# EUROPÄISCHE PATENTSCHRIFT

(11) **EP 0 984 962 B1**
(45) Veröffentlichungstag und Bekanntmachung des Hinweises auf die Patenterteilung: **09.04.2003**
(21) Anmeldenummer: 98922797.0
(22) Anmeldetag: 04.05.1998
(51) Int. Cl.: C07D 405/10, A01N 43/56, C07D 493/04, C07D 303/38, C07D 305/06

(54) **SUBSTITUIERTE 4-BENZOYL-PYRAZOLE**
SUBSTITUTED 4-BENZOYL-PYRAZOLES
4-BENZOYLE-PYRAZOLS SUBSTITUES

(30) Priorität: 07.05.1997 DE 19719387
(43) Veröffentlichungstag der Anmeldung: 15.03.2000
(73) Patentinhaber: BASF AKTIENGESELLSCHAFT, 67056 Ludwigshafen (DE)
(72) Erfinder: ENGEL, Stefan, D-55268 Nieder-Olm (DE); BAUMANN, Ernst, D-67373 Dudenhofen (DE); VON DEYN, Wolfgang, D-67435 Neustadt (DE); HILL, Regina, Luise, D-67376 Harthausen (DE); KARDORFF, Uwe, D-68159 Mannheim (DE); MAYER, Guido, D-67433 Neustadt (DE); OTTEN, Martina, D-67069 Ludwigshafen (DE); RHEINHEIMER, Joachim, D-67063 Ludwigshafen (DE); WAGNER, Oliver, D-67061 Ludwigshafen (DE); WITSCHEL, Matthias, D-67061 Ludwigshafen (DE); MISSLITZ, Ulf, D-67433 Neustadt (DE); WALTER, Helmut, D-67283 Obrigheim (DE); WESTPHALEN, Karl-Otto, D-67346 Speyer (DE)
(86) Internationale Anmeldenummer: EP9802622
(87) Internationale Veröffentlichungsnummer: WO98050379

(56) Entgegenhaltungen:
- EP-A- 0 352 543
- EP-A- 0 352 675
- WO-A-96/26206

## Beschreibung

Die vorliegende Erfindung betrifft substituierte 4-Benzoyl-pyrazole der Formel I 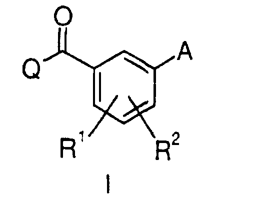 in der die Substituenten folgende Bedeutung haben:
- R¹, R²: Wasserstoff, Mercapto, Nitro, Halogen, Cyano, Rhodano, C₁-C₆-Alkyl, C₁-C₆-Halogenalkyl, C₁-C₆-Alkoxy, C₂-C₆-Alkenyl, C₂-C₆-Alkinyl, -OR¹⁰, -OCOR¹⁰, -OSO₂R¹⁰, -S(O)ₙR¹⁰, -SO₂OR¹⁰, -SO₂NR³R¹⁰, -NR¹⁰SO₂R¹⁰ oder -NR¹⁰COR;
- Q: ein in 4-Stellung verknüpftes Pyrazol der Formel II, 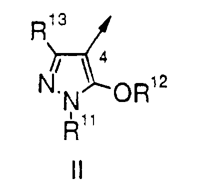
wobei
- R¹¹: für C₁-C₆-Alkyl, C₁-C₆-Halogenalkyl, Phenyl oder Phenyl das partiell oder vollständig halogeniert ist und/oder einen bis drei der folgenden Reste trägt:
Nitro, Cyano, C₁-C₄-Alkyl, C₁-C₄-Halogenalkyl, C₁-C₄-Alkoxy, C₁-C₄-Halogenalkoxy;
- R¹²: für Wasserstoff, C₁-C₆-Alkyl, C₁-C₆-Halogenalkyl, C₁-C₆-Alkylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, Phenylcarbonyl, Phenylcarbonylmethyl, Phenoxycarbonyl oder Phenyl-sulfonyl, wobei die vier letztgenannten Substituenten unsubstituiert sind oder der Phenylring jeweils partiell oder vollständig halogeniert ist und/oder einen bis drei der folgenden Reste trägt: Nitro, Cyano, C₁-C₄-Alkyl, C₁-C₄-Halogenalkyl, C₁-C₄-Alkoxy, C₁-C₄-Halogenalkoxy;
- R¹³: für Wasserstoff, C₁-C₆-Alkyl oder C₁-C₆-Halogen-alkyl;
stehen;
- A: eine Gruppe der Formel IIIa, IIIb oder IV 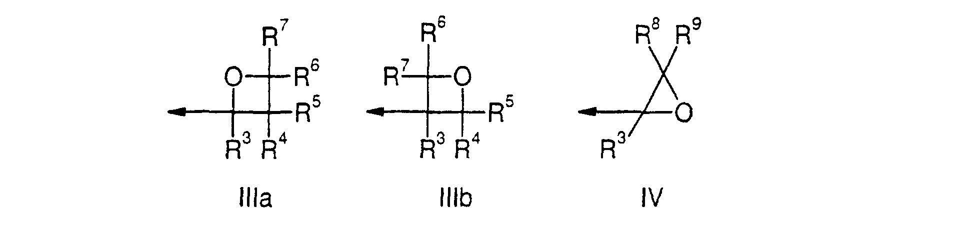 in der die Variablen folgende Bedeutung haben:
- R³: Wasserstoff, C₁-C₆-Alkyl oder Phenyl, wobei der genannte Alkyl und Phenylrest partiell oder vollständig halogeniert sein kann und/oder eine bis drei der folgenden Gruppen tragen kann:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
- R⁴ - R⁷: können gleich oder verschieden sein und stehen unabhängig voneinander für:
Wasserstoff, Hydroxy, Mercapto, Amino, Halogen, C₁-C₆-Alkyl, C₃-C₆-Cycloalkyl, C₄-C₆-Cycloalkenyl, Phenyl, -OR¹⁰, -S(O)ₙR¹⁰, -OS(O)ₙR¹⁰, -PO(OR¹⁰)₂, -NR³R¹⁰, -Si(R¹⁰)₃ oder -OCOR¹⁰, wobei die genannten Alkyl und Cycloalkylreste sowie R³ und R¹⁰ der Reste -OR¹⁰, -S(O)ₙR¹⁰, -OS(O)ₙR¹⁰, -PO(OR¹⁰)₂, -NR³R¹⁰, -Si(R¹⁰)₃, -OCOR¹⁰ partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
- R⁴, R⁵: können zusammen eine gegebenenfalls durch ein Stickstoff-oder ein Sauerstoffatom ein- oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰, NNR³R¹⁰ oder NOR¹⁰ stehen kann;
- R⁶, R⁷: können zusammen eine gegebenenfalls durch ein Stickstoff-oder ein Sauerstoffatom einfach oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰, NNR³R¹⁰ oder NOR¹⁰ stehen kann;
- n: null, eins, zwei;
- R⁵, R⁶: können darüber hinaus, wenn sie an benachbarte Kohlenstoffatome gebunden sind, zusammen eine gegebenenfalls durch ein Stickstoff- oder ein Sauerstoffatom unterbro-chene C₃-C₄-Alkylen- oder C₃-C₄-Alkenylenkette bilden, wenn R⁴ und R⁷ für Wasserstoff stehen;
- R⁸, R⁹: können gleich oder verschieden sein und stehen unabhängig voneinander für:
Wasserstoff, Nitro, Halogen, Cyano, C₁-C₆-Alkyl, C₃-C₆-Cycloalkyl, C₄-C₆-Cycloalkenyl, C₅-C₆-Heterocyclyl, -OR¹⁰, -SR¹⁰, -COR¹⁰, -COOR¹⁰, -CONR³R¹⁰, Phenyl, Phenyl-C₁-C₆-alkyl und fünf- oder sechsgliedriges Hetaryl, wobei die genannten Alkyl und Cycloalkylreste sowie R³ und R¹⁰ der Reste -OR¹⁰, -SR¹⁰, -COR¹⁰, -COOR¹⁰, -CONR³R¹⁰ partiell oder vollständig halogeniert sein können und/-oder eine bis drei der folgenden Gruppen tragen können:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
- R⁸, R⁹: können darüber hinaus zusammen eine gegebenenfalls durch ein Stickstoff- oder ein Sauerstoffatom ein- oder zweifach unterbrochene C₂-C₅-Alkylen- oder C₂-C₅-Alkenylenkette bilden;
- R¹⁰: Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl; wobei die genannten Alkylreste partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
sowie deren landwirtschaftlich brauchbaren Salze.

Außerdem betrifft die Erfindung Verfahren und Zwischenprodukte zur Herstellung von Verbindungen der Formel I, Mittel welche diese enthalten, sowie die Verwendung der Verbindungen der Formel I und diese enthaltende Mittel zur Schadpflanzenbekämpfung.

Aus der Literatur, beispielsweise aus EP-A 282 944 sind 4-Benzoyl-pyrazole bekannt.

In EP-O 352 543 werden unter anderem 4-Benzoylpyrazole offenbart, welche in 3-Position des Benzoylrests einen gegebenenfalls substituierten Alkoxyrest tragen.

Aus WO 96/26206 sind 4-Benzoylpyrazole mit 5- bzw. 6-gliedrigen heterocyclischen Substituenten bekannt.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die 4-Benzoyl-pyrazole der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Gegenstand der vorliegenden Erfindung sind auch Stereoisomere der Verbindungen der Formel I. Es werden sowohl reine Stereoisomere als auch Gemische hiervon erfaßt.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl oder Hydroxy-C₁-C₄-alkyl und/oder ein Phenyl oder Benzyl ersetzt sein können, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)-sulfonium und Sulfoxoniumionen, vorzugsweise Tri(C₁-C₄-alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

### Verfahren A:

Umsetzungen von Pyrazolen der Formel II (mit R¹² = H) mit einer aktivierten Carbonsäure Va oder einer Carbonsäure Vb, die vorzugsweise in situ aktiviert wird, zu dem Acylierungsprodukt VII und anschließende Umlagerung zu den erfindungsgemäßen Verbindungen der Formel I. 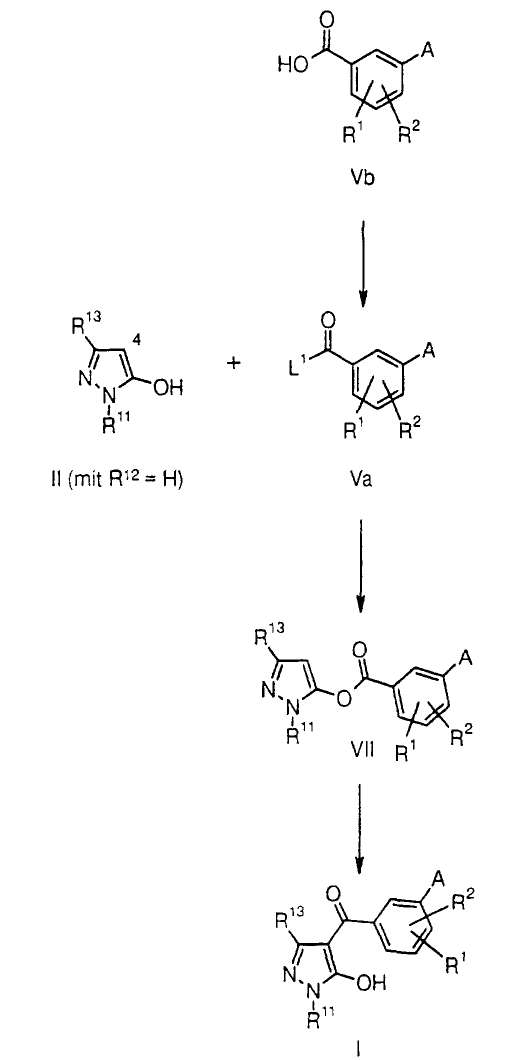 L¹ steht für eine nucleophil austauschbare Abgangsgruppe, wie Halogen z.B. Brom, Chlor, Hetaryl, z.B. Imidazolyl, Pyridyl, Carboxylat, z.B. Acetat, Trifluoracetat etc.

Die aktivierte Carbonsäure kann direkt eingesetzt werden, wie im Fall der Carbonsäurehalogenide oder in situ erzeugt werden, z.B. mit Dicyclohexylcarbodiimid, Triphenylphosphin/Azodicarbonsäureester, 2-Pyridindisulfit/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Carbonsäurehalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels wird der rohe Enolester der Formel VII vorzugsweise durch Chromatographie gereinigt. Es ist aber auch möglich, den rohen Enolester der Formel VII ohne weitere Reinigung zur Umlagerung einzusetzen.

Die Umlagerung der Enolester der Formel VII zu den Verbindungen der Formel I erfolgt zweckmäßigerweise bei Temperaturen von 20 bis 40°C in einem Lösungsmittel und in Gegenwart einer Base sowie gegebenenfalls in Gegenwart einer Cyanoverbindung.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonate verwendet.

Als Cyanoverbindungen kommen anorganische Cyanide, wie Natriumcyanid, Kaliumcyanid und organische Cyanoverbindungen, wie Acetoncyanhydrin, Trimethylsilycyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Besonders bevorzugt werden Alkalicarbonate, wie Kaliumcarbonat, in Acetonitril oder Dioxan eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid, Essigsäureethylester extrahiert. Der organische Extrakt kann mit 5-10%iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt. (Beispiele für die Darstellung von Estern von Hydroxypyrazolen und für die Umlagerung der Ester sind z.B. in EP-A 282 944 oder US 4 643 757 genannt).

### Verfahren B:

Umsetzungen von 4-Benzoyl-pyrazolen der Formel I (mit R¹² = H) mit einer Verbindung der Formel VI (mit R¹² ≠ H): 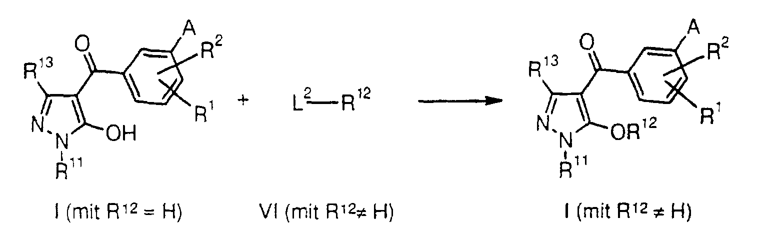 L² steht für eine nucleophil austauschbare Abgangsgruppe, wie Halogen, z.B. Brom, Chlor, Hetaryl, z.B. Imidazolyl, Pyridyl, Carboxylat, z.B. Acetat, Trifluoracetat, Sulfonat, z.B. Mesylat, Triflat etc.

Die Verbindungen der Formel V können direkt eingesetzt werden, wie z.B. im Fall der Alkylhalogenide, Carbonsäurehalogenide, Sulfonsäurehalogenide. Carbonsäureanhydride und Sulfonsäureanhydride oder in situ erzeugt werden, z.B. aktivierte Carbonsäuren (mittels Carbonsäure und Dicyclohexylcarbodiimid, Carbonyldiimidazol etc.).

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuß der Hilfsbase z.B. 1,5 bis 3 Moläquivalente, bezogen auf II, kann unter Umständen vorteilhaft sein.

Als Hilfsbasen eignen sich tertiäre Alkylamine, wie Triethylamin, Pyridin, Alkalimetallcarbonate, z.B. Natriumcarbonat, Kaliumcarbonat und Alkalimetallhydride, z.B. Natriumhydrid. Bevorzugt verwendet werden Triethylamin, Pyridin und Kaliumcarbonat.

Als Lösungsmittel kommen z.B. chlorierte Kohlenwasserstoffe, wie Methylenchlorid, 1,2-Dichlorethan, aromatische Kohlenwasserstoffe, z.B. Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

Die als Ausgangsmaterialien verwendeten Pyrazole der Formel II (mit R¹² = H) sind bekannt oder können nach an sich bekannten Verfahren hergestellt werden (z.B. EP-A 240 001, J. Prakt. Chem. 315, 383 (1973)).

Die Benzoesäuren der Formel V sind neu, 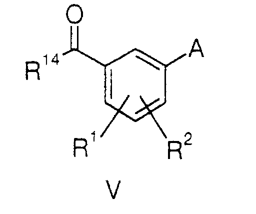 wobei die Variablen folgende Bedeutung haben:
- R¹, R²: Wasserstoff, Mercapto, Nitro, Halogen, Cyano, Rhodano, C₁-C₆-Alkyl, C₁-C₆-Halogenalkyl, C₁-C₆-Alkoxy, C₂-C₆-Alkenyl, C₂-C₆-Alkinyl, -OR¹⁰, -OCOR¹⁰, -OSO₂R¹⁰, -S(O)ₙR¹⁰, -SO₂OR¹⁰, -SO₂NR³R¹⁰, -NR¹⁰SO₂R¹⁰ oder -NR¹⁰COR;
- A: eine Gruppe der Formel IIIa, IIIb oder IV 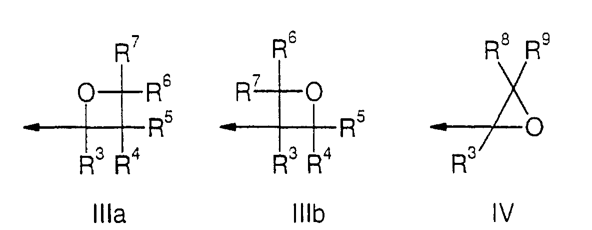 in der die Variablen folgende Bedeutung haben:
- R³: Wasserstoff, C₁-C₆-Alkyl oder Phenyl,
wobei der genannte Alkyl und Phenylrest partiell oder vollständig halogeniert sein kann und/oder eine bis drei der folgenden Gruppen tragen kann:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein
- R⁴ - R⁷: können gleich oder verschieden sein und stehen unabhängig voneinander für:
Wasserstoff, Hydroxy, Mercapto, Amino, Halogen, C₁-C₆-Alkyl, C₃-C₆-Cycloalkyl, C₄-C₆-Cycloalkenyl, Phenyl, -OR¹⁰, -S(O)ₙR¹⁰, -OS(O)ₙR¹⁰, -PO(OR¹⁰)₂, -NR³R¹⁰, -Si(R¹⁰)₃ oder -OCOR¹⁰,
wobei die genannten Alkyl und Cycloalkylreste sowie R³ und R¹⁰ der Reste -OR¹⁰, -S(O)ₙR¹⁰, -OS(O)ₙR¹⁰, -PO(OR¹⁰)₂, -NR³R¹⁰, -Si(R¹⁰)₃, -OCOR¹⁰ partiell oder vollständig halogeniert sein können und/ oder eine bis drei der folgenden Gruppen tragen können:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
- R⁴, R⁵: können zusammen eine gegebenenfalls durch ein Stickstoffoder ein Sauerstoffatom ein- oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰, NNR³R¹⁰ oder NOR¹⁰ stehen kann;
- R⁶, R⁷: können zusammen eine gegebenenfalls durch ein Stickstoff-oder ein Sauerstoffatom einfach oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰ oder NOR¹⁰, NNR³R¹⁰ stehen kann;
- n: null, eins, zwei;
- R⁵, R⁶: können darüber hinaus, wenn sie an benachbarte Kohlen-stoffatome gebunden sind zusammen eine gegebenenfalls durch ein Stickstoff- oder ein Sauerstoffatom unterbrochene C₃-C₄-Alkylen- oder C₃-C₄-Alkenylenkette bilden, wenn R⁴ und R⁷ für Wasserstoff stehen;
- R⁸, R⁹: können gleich oder verschieden sein und stehen unabhängig voneinander für:
Wasserstoff, Nitro, Halogen, Cyano, C₁-C₆-Alkyl, C₃-C₆-Cycloalkyl, C₄-C₆-Cycloalkenyl, C₅-C₆-Heterocyclyl, -OR¹⁰, -SR¹⁰, -COR¹⁰, -COOR¹⁰, -CONR³R¹⁰, Phenyl, Phenyl-C₁-C₆-alkyl und fünf- oder sechsgliedriges Hetaryl,
wobei die genannten Alkyl und Cycloalkylreste sowie R³ und R¹⁰ der Reste -OR¹⁰, -SR¹⁰, -COR¹⁰, -COOR¹⁰, -CONR³R¹⁰ partiell oder vollständig halogeniert sein können und/-oder eine bis drei der folgenden Gruppen tragen können:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
- R⁸, R⁹: können darüber hinaus zusammen eine gegebenenfalls durch ein Stickstoff- oder ein Sauerstoffatom ein- oder zweifach unterbrochene C₂-C₅-Alkylen- oder C₂-C₅-Alkenylenkette bilden;
- R¹⁰: Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl;
- R¹⁴: Hydroxy oder ein hydrolysierbarer Rest.

Beispiele für hydrolysierbare Reste sind Alkoxy-, Phenoxy-, Alkylthio-, Phenylthioreste, die substituiert sein können, Halogenide, Hetarylreste, die über Stickstoff gebunden sind, Amino-, Iminoreste, die substituiert sein können, etc.

Bevorzugt sind Benzoesäurehalogenide Va mit L¹ = Halogen (=̂ V mit R¹⁴ = Halogen), 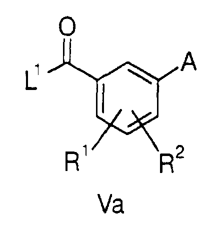 wobei die Variablen R¹, R², und A die unter Formel V genannte Bedeutung haben und
- L: Halogen, insbesondere Chlor oder Brom, bedeuten.

Ebenso bevorzugt sind Benzoesäuren der Formel Vb (=̂ V mit R¹⁴ = Hydroxy), 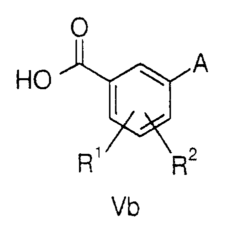 wobei die Variablen R¹, R², und A die unter Formel V genannte Bedeutung haben.

Ebenso bevorzugt sind Benzoesäureester der Formel Vc (=̂ V mit R¹⁴ = C₁-C₆-Alkoxy), 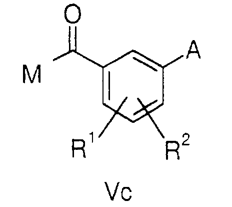 wobei die Variablen R¹, R², und A die unter Formel V gennante Bedeutung haben und
- M: C₁-C₆-Alkoxy
bedeutet.

Die Verbindungen der Formel Va (mit L¹ = Halogen) können in Analogie zu literaturbekannten Methoden (vgl. L.G. Fieser, M. Fieser "Reagents for Organic Synthesis", Bd. I, S. 767-769 (1967)) durch Umsetzung von Benzoesäuren der Formel Vb mit Halogenierungsreagentien wie Thionylchlorid, Thionylbromid, Phosgen, Diphosgen, Triphosgen, Oxalylchlorid, Oxalylbromid dargestellt werden.

Die Benzoesäuren der Formel Vb können u.a. durch Verseifung der Benzoesäureester der Formel Vc (mit M = C₁-C₆-Alkoxy) erhalten werden.

Die erfindungsgemäßen Benzoesäureester der Formel Vc sind nach verschiedenen literaturbekannten Methoden (z.B. a. G. Dittus in Houben-Weyl, Methoden der Organischen Chemie, Band VI/3, Sauerstoff-Verbindungen I, 4. Aufl., S. 493 ff., Georg Thieme Verlag, 1965; b. T. L. Gilchrist, Heterocyclenchemie, 2. Aufl., Verlag Chemie, 1995) darstellbar, wie in den nachfolgenden Beispielen illustriert.

### Verfahren A:

Cyclisierung von 1,3-Halogenhydrinen der Formel VIIIa oder VIIIb unter alkalischen Reaktionsbedingungen zu den erfindungsgemäßen Benzoesäureestern der Formel Vc, in der die Variablen R¹, R² und M die unter Formel V genannte Bedeutung haben, A für eine Gruppe der Formel IIIa oder IIIb und L² für eine nucleophil austauschbare Abgangsgruppe, vorzugsweise Iod, Brom oder Chlor steht.

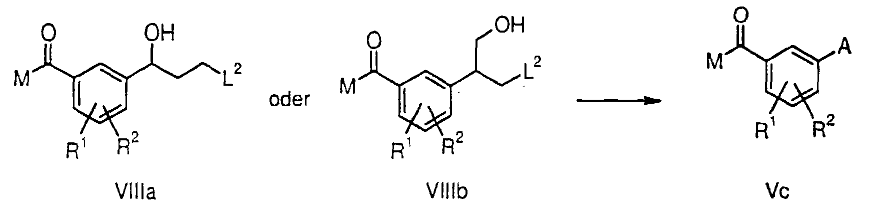

Als Abspaltungsmittel werden vor allem Alkali- und Erdalkalihydroxide, z.B. Kaliumhydroxid oder Natriumhydroxid oder organische Basen, z.B. Alkoholate, wie z.B. Natriummethanolat oder sekundäre Amine, wie z.B. Diethylamin verwendet.

Die Abspaltung von Halogenwasserstoff kann bereits durch alkalisch reagierende Salze, z.B. Kaliumfluorid erfolgen (E. Gryszkiewics-Trochimowski, O. Gryszkiewics-Trochimowski, Bulletin de la Société Chimique de France, Mémoires 123 (1953)).

Die Abspaltungsmittel können sowohl in Lösung als auch in Substanz, vorzugsweise in Lösung, z.B. methanolische Natriummethanolat-Lösung eingesetzt werden.

Die Durchführung der Cyclisierung erfolgt in indifferenten Lösungsmitteln, z.B. in Alkoholen, wie z.B. Methanol oder Ethanol.

### Verfahren B:

Photochemische Cycloadditionen von Aldehyden der Formel IX oder Ketonen der Formel X mit Olefinen XI, vorzugsweise mit Enolethern der Formel XI (mit R⁷ = OR¹⁰) zu den erfindungsgemäßen Benzoesäureestern der Formel Vc, in der die Variablen R¹, R², R³, R¹⁰ und M die unter Formel V genannte Bedeutung haben und A für eine Gruppe der Formel IIIa steht.

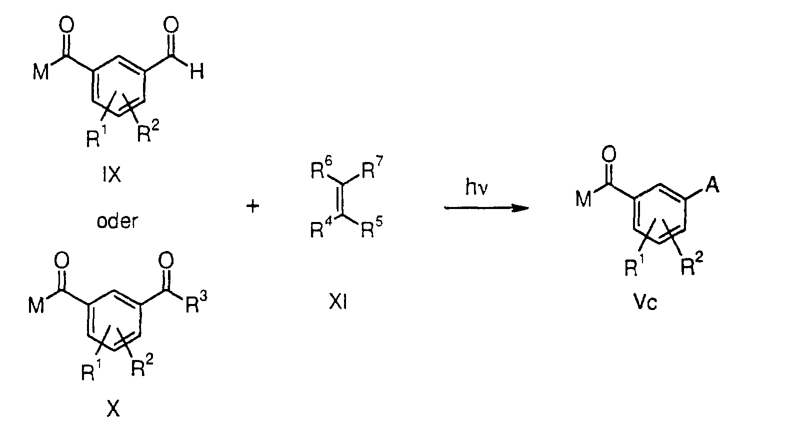

Die Durchführung der photochemischen Cycloaddition erfolgt in indifferenten Lösungsmitteln, die im eingesetzten Spektralbereich keine signifikante UV-Absorptionen aufweisen. So können z.B. Acetonitril oder aliphatische oder aromatische Kohlenwasserstoffe, wie z.B. n-Hexan oder Toluol, eingesetzt werden.

Als Strahlungsquellen werden geeignete UV-Strahler, bevorzugt Niederdruck- oder Mittel- bzw. Hochdruck-Quecksilber-Lampen eingesetzt (A.M. Braun, M.T. Maurette, E. Oliveros, Photochemical Technology, John Wiley & Sons Ltd 1991). Niederdruck-Quecksilber-Lampen emittieren bei 189 und 253 nm, wobei die Emission bei 189 nm fast vollständig durch Sauerstoff, Wasser oder Lösungsmittel absorbiert wird, so daß Niederdruck-Quecksilber-Lampen praktisch eine monochromatische Strahlung bei 253 nm emittieren.

Mittel- bzw. Hochdruck-Quecksilber-Lampen werden bei Drücken zwischen 1 und 100 atm betrieben und emittieren je nach Druck und Temperatur in einem Wellenlängenbereich zwischen 200 und 600 nm, wobei Mitteldruck-Quecksilber-Lampen eine dominante Emissionslinie bei 366 nm und Hochdruck-Quecksilber-Lampen zwei dominante Emissionen bei 436 und 546 nm aufweisen.

Darüber hinaus können mit Metallsalzen, wie z.B. Thallium-, Indium-, Natrium- oder Galliumhalogenide dotierte Mittel- bzw. Hochdruck-Quecksilber-Lampen eingesetzt werden. Die Dotierung bewirkt eine Modifikation des Emissionsspektrums der Mittel- bzw. Hochdruck-Quecksilber-Lampen und führt zur Emission zusätzlicher für die jeweilige Dotierung charakteristischen Emissionslinien.

Zusätzlich können noch geeignete Filter eingesetzt werden, die nicht gewünschte Wellenlängenbereiche unterdrücken.

Geeignete Photoreaktoren sollten nicht im eingesetzten UV-Bereich absorbieren, sondern für die durch einen geeigneten UV-Strahler emittierten Wellenlängen transparent sein.

Dafür eignen sich insbesondere Borosilikat-Glas, z.B. Borosilikat BK7 von Schott oder Corning 7740 von Pyrex oder Quartz-Glas, das im UV-Bereich eine noch bessere Durchlässigkeit als Borosilikat-Glas aufweist.

Bevorzugte Photoreaktoren sind Falling Film-Reaktoren.

### Verfahren C:

Intramolekulare Cyclisierung von β-Halogenfettsäuren bzw. deren Alkalisalzen der Formel XIIa oder XIIb zu den erfindungsgemäßen Benzoesäureestern der Formel Vc, in der die Variablen R¹, R² und M die unter Formel V genannte Bedeutung haben, A für eine Gruppe der Formel IIIa oder IIIb und L² für eine nucleophil verdrängbare Abgangsgruppe, vorzugsweise Iod, Brom oder Chlor steht.

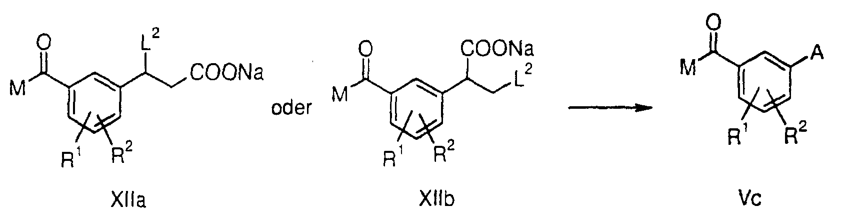

Die Abspaltung von Halogenwasserstoff aus β-Halogenfettsäuren oder deren Salzen, wie z.B. den Natriumsalzen durch wäßrige Lösungen von Alkalimetallsalzen, z.B. Natriumcarbonatlösung führt zur Bildung von β-Lactonen (A. Einhorn, Chem. Ber. **16**, 2208 (1983)). Statt mit Natriumcarbonat gelingt die Abspaltung von Halogenwasserstoff häufig mit Silberoxid bzw. mit Silbersalzen.

Aufgrund der Labilität der β-Lactone gegenüber wäßrigen Alkalihalogeniden ist es notwendig, bei der Darstellung wasserlöslicher β-Lactone in Gegenwart von Lösungsmitteln zu arbeiten, damit das entstandene β-Lacton möglichst rasch aus der wäßrigen Phase entfernt wird (Org. Reactions **8**, 309 (1954)). Geeignete Lösungsmittel sind z.B. Diethylether oder Chloroform.

### Verfahren D:

Cycloaddition von Aldehyden der Formel IX oder Ketonen der Formel X mit Ketenen XIII zu den erfindungsgemäßen Benzoesäureestern der Formel Vc, in der die Variablen R¹, R², R³, R⁴, R⁵ und M die unter Formel V genannte Bedeutung haben und A für eine Gruppe der Formel IIIa oder IIIb steht.

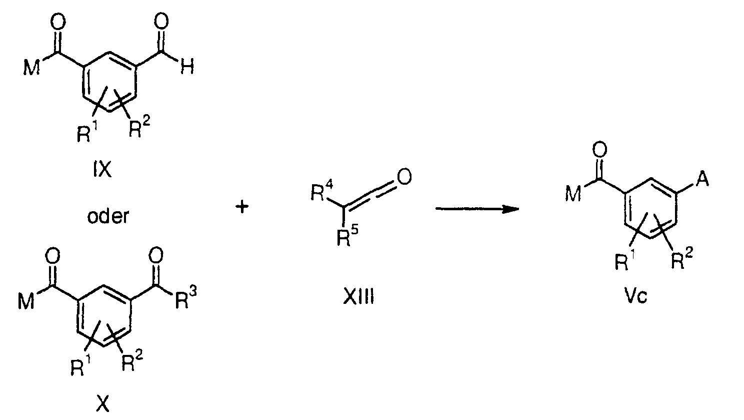

Keten reagieren mit Carbonylverbindungen je nach Art des Katalysators und der Reaktionsbedingungen bei Temperaturen von 20 bis 100°C, bevorzugt bei Temperaturen von 40 bis 80°C, unter Bildung von Enolacetaten oder von β-Lactonen (z.B. H. Kröper in Houben-Weyl, Methoden der Organischen Chemie, Band VI/2, Sauerstoffverbindungen I, Teil 2. 4. Aufl., S. 511ff., Georg Thieme Verlag, 1965). Werden basische Katalysatoren, wie z.B. tertiäre Amine oder Alkali- oder Erdalkalimetalle eingesetzt, erfolgt die Bildung von β-Lactonen nach C-Acetylierung (J.A. Spence, E.F. Degering, Chem. Abst. **43,** 6654 (1949)). In Gegenwart spezieller Katalysatoren kondensieren sich Aldehyde oder Ketone schon bei niedrigeren Temperaturen von 0 bis 10°C mit dem einfachsten Keten unter Bildung von β-Lactonen.

Die Wahl des Katalysators ist von den einzelnen Carbonylverbindungen abhängig. Als Katalysatoren für aromtische Aldehyde können z.B. Borsäure, Triacetylborat, Zinkrhodanid und Zinkchlorid, Aluminiumchlorid, Quecksilber-(II)-Chlorid, sowie aktivierte Tonerde und Siliciumdioxid verwendet werden.

Die Reaktion muß zur Erzielung hoher Ausbeuten in wasserfreiem Medium durchgeführt werden.

Als Lösungsmittel eignen sich Ether, z.B. Diethylether, Halogenalkane, z.B. Methylenchlorid oder Chloroform, und wegen der Polymerisationstendenz der β-Lactone bevorzugt Ketone.

### Verfahren E:

Addition von Thiolesterenolaten XIV an Aldehyde der Formel IX oder Ketone der Formel X in Analogie zu literaturbekannten Verfahren (R. L. Danheiser, J. S. Nowick, Journal of Organic Chemistry 56, 1176 (1991)) zu den erfindungsgemäßen Benzoesäureestern der Formel Vc, in der die Variablen R¹, R², R³, R⁴, R⁵ und M die unter Formel V genannte Bedeutung haben und A für eine Gruppe der Formel IIIa oder IIIb steht. 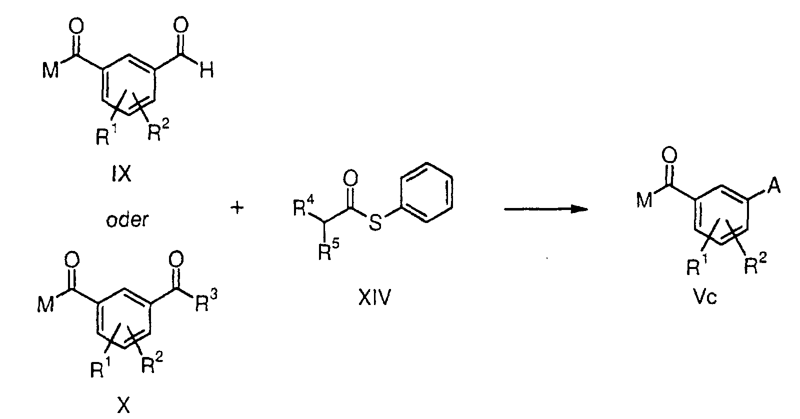 Die als Edukte eingesetzten Thioesterenolate XIV können in einer einfachen Einstufenreaktion aus Carbonsäurederivaten bereitgestellt werden (z.B. T. Mukaiyama, T. Takeda, K. Atsumi, Chemistry Letters **1974**, 187).

In Gegenwart von einem Equivalent einer Base, bevorzugt einer Lithiumbase, wie z.B. Lithiumdiisopropylamid, wird das entsprechende Enolat gebildet, das mit Aldehyden oder Ketonen die gewünschten Verbindungen der Formel Vc, insbesondere β-Lactone bildet.

### Verfahren F:

Cyclisierung von 1,2-Halogenhydrinen der Formel XVa oder XVb unter alkalischen Reaktionsbedingungen zu den erfindungsgemäßen Benzoesäureestern der Formel Vc, in der die Variablen R¹, R² und M die unter Formel V genannte Bedeutung haben, A für eine Gruppe der Formel IV und L² für eine nucleophil verdrängbare Abgangsgruppe, vorzugsweise Iod, Brom oder Chlor steht. 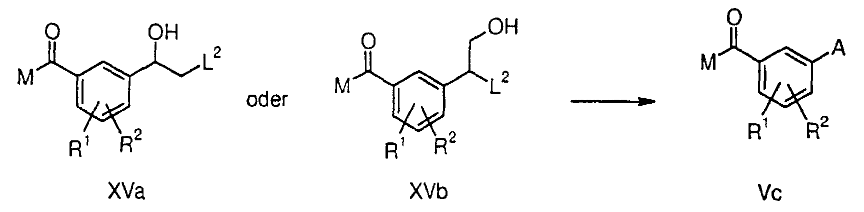 Anstelle der 1,2-Halogenhydrine können in Analogie auch deren Acetate als Ausgangsmaterial eingesetzt werden.

Den beschriebenen Methoden verwandt ist die Darstellung der erfindungsgemäßen Benzoesäureestern der Formel Vc durch Abspaltung von *p*-Toluolsulfonsäure aus 2-Hydroxy-toluolsulfonaten (z.B. H. Ohle, L. v. Vargha, Chemische Berichte 62, 2440 (1929).

Als Abspaltungsmittel werden vor allem Alkali- und Erdalkalihydroxide verwendet, seltener organische Basen, z.B. Alkoholate, sekundäre Amine oder Pyridin bzw. seine Homologen, wie z.B. Kollidin. Bevorzugte Alkali- und Erdalkalihydroxide sind z.B. Kaliumhydroxid, Natriumhydroxid oder Calciumhydroxid.

Die Abspaltung von Halogenwasserstoff erfolgt bisweilen schon durch alkalisch reagierende Salze, z.B. Kaliumcarbonat, Bariumcarbonat oder Kaliumfluorid. Ferner ist die Verwendung von Aluminaten, Silikaten und Zinkaten (z.B. J. D. Zech, Chemical Abstracts, 46, 8672 (1952)), Blei-(II)-oxid, Aluminiumoxid, Silbernitrat oder alkalischer Ionenaustauscher beschrieben.

Die Abspaltungsmittel können sowohl in Lösung als auch in Substanz, teilweise in gepulverter Form, z.B. gepulvertes Kaliumhydroxid verwendet werden.

Die Durchführung der Cyclisierung erfolgt in indifferenten Lösungsmitteln. Geeignet sind offenkettige oder cyclische Ether, z.B. Diethylether oder Dioxan oder aromatische Kohlenwasserstoffe, z.B. Benzol oder Toluol.

### Verfahren G:

Epoxidierung von Olefinen der Formel XVI zu den erfindungsgemäßen Benzoesäureestern der Formel Vc, in der die Variablen R¹, R² und M die unter Formel V genannte Bedeutung haben, A für eine Gruppe der Formel IV steht.

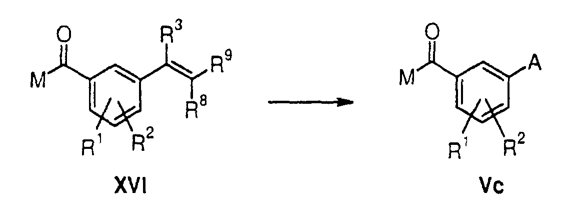

Die Epoxidierung wird in Analogie zu literaturbekannten Verfahren häufig mit Persäuren, z.B. Perbenzoesäure, Monoperphthalsäure, Perameisensäure, Peressigsäure, Trifluorperessigsäure oder Propionpersäure (z.B. R. Criegee, Houben-Weyl, "Methoden der Organischen Chemie", Band VIII, Sauerstoff-Verbindungen III, 4. Aufl., S. 40 ff., Georg Thieme Verlag, 1965), Wasserstoffperoxid oder *tert.*-Butylhydroperoxid in alkalischer Lösung, vorzugsweise in wäßriger Natriumhydroxid-Lösung oder mit Dioxiranen, z.B. Dimethyldioxiran bzw. Derivaten, wie z.B. (Trifluormethyl)-methyldioxiran (W. Adam, A. K. Smerz, Bulletin des Sociétés Chimiques Belges 105, 581 (1996)) durchgeführt.

Die Durchführung der Epoxidierung mit Persäuren erfolgt in indifferenten Lösungsmitteln, z.B. Diethylether, Chloroform, Tetrachlormethan, Ethylchlorid oder gelegentlich auch in Eisessig, während Dioxirane vorzugsweise in Aceton bzw. Derivaten, wie z.B. (Trifluormethyl)-methylketon eingesetzt werden.

Die Persäure, Wasserstoffperoxid, tert.-Butylhydroperoxid oder das Dioxiran wird oft in geringem Überschuß zur Reaktion gebracht, doch kann auch, wenn das Oxidationsmittel restlos ausgenutzt werden soll, ein Überschuß des Olefins zweckmäßig sein.

### Verfahren H:

Kondensation von Aldehyden der Formel IX mit α-Halogen-fettsäureestern XVII oder verwandten α-Halogenfettsäure-Derivaten, z.B. α-Halogen-fettsäureamide, -nitrile oder -ketone in Gegenwart alkalischer Kondensationsmittel entsprechend literaturbekannter Verfahren (z.B. M. Ballester, Chemical Reviews 55, 283 (1955)) zu den erfindungsgemäßen Benzoesäureestern der Formel Vc, in der die

Variablen R¹, R², R¹⁰ und M die unter Formel V genannte Bedeutung haben und A für eine Gruppe der Formel IV steht.

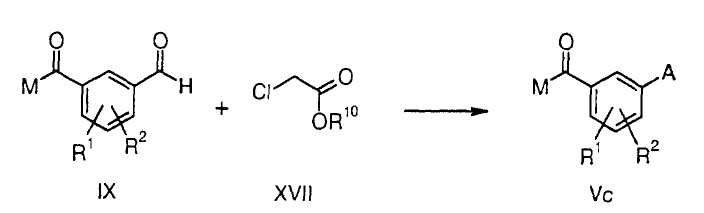

Als Kondensationsmittel eignen sich anorganische oder organische Katalysatoren, z.B. Natriumhydrid in Mineralöl, Lithiumhydrid, Tetraethylammoniumethylat, Natriumethanolat, Kalium-*tert.*-butylat oder Diisopropylmagnesiumbromid.

Die Durchführung erfolgt in indifferenten Lösungsmitteln, z.B. in Xylol, Diethylether, Methanol, Ethanol oder tert.-Butanol.

Hervorzuheben sind die erfindungsgemäßen Verbindungen der Formel I, wobei A für eine Gruppe der Formel IIIa oder IIIb steht und
- R⁴- R⁷: Wasserstoff, Hydroxy, Mercapto, Amino, Halogen, C₁-C₆-Alkyl, C₃-C₆-Cycloalkyl, Phenyl, -OR¹⁰, -S(O)ₙR¹⁰, -OS(O)ₙR¹⁰, -PO(OR¹⁰)₂, -NR³R¹⁰, -Si(R¹⁰)₃ oder -OCOR¹⁰, wobei die genannten Alkyl und Cycloalkylreste sowie R³ und R¹⁰ der Reste -OR¹⁰, -S(O)ₙR¹⁰, -OS(O)ₙR¹⁰, -PO(OR¹⁰)₂, -NR³R¹⁰, -Si(R¹⁰)₃, -OCOR¹⁰ partiell oder vollständig halogeniert sein können und/ oder eine bis drei der folgenden Gruppen tragen können:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
bedeuten.

Darüber hinaus sind die erfindungsgemäßen Verbindungen der Formel I hervorzuheben, wobei A für eine Gruppe der Formel IIIa oder IIIb steht und
- R⁴, R⁵: zusammen eine gegebenenfalls durch ein Stickstoff- oder ein Sauerstoffatom ein- oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰ oder NOR¹⁰ steht;
und/oder
- R⁶, R⁷: zusammen eine gegebenenfalls durch ein Stickstoff- oder ein Sauerstoffatom einfach oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰ oder NOR¹⁰ steht.

Weiterhin sind die erfindungsgemäßen Verbindungen der Formel I hervorzuheben, wobei A für eine Gruppe der Formel IIIa oder IIIb steht und
- R⁵, R⁶: wenn sie an benachbarte Kohlenstoffatome gebunden sind zusammen eine gegebenenfalls durch ein Stickstoff- oder ein Sauerstoffatom unterbrochene C₃-C₄-Alkylen- oder C₃-C₄-Alkenylenkette bilden, wenn R⁴ und R⁷ für Wasserstoff stehen.

Hervorzuheben sind desweiteren die erfindungsgemäßen Verbindungen der Formel I, wobei A für eine Gruppe der Formel IVb steht und
- R⁸, R⁹: Wasserstoff, Nitro, Halogen, Cyano, C₁-C₆-Alkyl, C₃-C₆-Cycloalkyl, C₅-C₆-Heterocyclyl, -OR¹⁰, -SR¹⁰, -COR¹⁰, -COOR¹⁰, -CONR³R¹⁰, Phenyl, Phenyl-C₁-C₆-alkyl und fünfoder sechsgliedriges Hetaryl,
wobei die genannten Alkyl und Cycloalkylreste sowie R³ und R¹⁰ der Reste -OR¹⁰, -SR¹⁰, -COR¹⁰, -COOR¹⁰, -CONR³R¹⁰ partiell oder vollständig halogeniert sein können und/-oder eine bis drei der folgenden Gruppen tragen können: Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxyamino, C₁-C₄-Alkylcarbonyl, C₁-C₄-Alkoxy-C₂-C₆- alkoxycarbonyl, C₁-C₄-Alkylsulfonyl, Heterocyclyl, Heterocyclyloxy, Phenyl, Benzyl, Hetaryl, Phenoxy, Benzyloxy und Hetaryl-oxy, wobei die acht letztgenannten Reste ihrerseits substituiert sein können;
bedeuten.

Darüber hinaus sind die erfindungsgemäßen Verbindungen der Formel I hervorzuheben, wobei A für eine Gruppe der Formel IVb steht und
- R⁸, R⁹: zusammen eine gegebenenfalls durch ein Stickstoff- oder ein Sauerstoffatom ein- oder zweifach unterbrochene C₂-C₅-Alkylen- oder C₂-C₅-Alkenylenkette
bilden.

Die für die Substituenten R¹-R¹³ oder als Reste an Phenyl-, Hetaryl- und Heterocyclylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cycloalkyl-, Alkoxyalkyl-, Alkoxy-, Halogenalkoxy-, Alkyliminooxy-, Alkoxyamino-, Alkylsulfonyl-, Halogenalkylsulfonyl, Alkylcarbonyl-, Halogenalkylcarbonyl, Alkoxycarbonyl-, Alkoxyalkoxycarbonyl-, Alkenyl-, Cycloalkenyl-, Alkinyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl, sowie die Alkylteile von C₁-C₄-Alkylcarbonyl: Methyl, Ethyl, n-Propyl, 1-Methylethyl, Butyl, 1-Methylpropyl, 2-Methylpropyl und 1,1-Dimethylethyl;
- C₁-C₆-Alkyl, sowie die Alkylteile von C₁-C₆-Alkoxy-C₁-C₆-alkyl und C₁-C₆-Alkylcarbonyl: C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Halogenalkyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl, sowie die Halogenalkylteile von C₁-C₆-Halogenalkylcarbonyl: C₁-C₄-Halogenalkyl wie voranstehend genannt, sowie 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
- C₁-C₄-Alkoxy, sowie die Alkoxyteile von C₁-C₄-Alkoxyamino, C₁-C₄-Alkoxy-C₂-C₆-alkoxycarbonyl und C₁-C₄-Alkoxycarbonyl: Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy, sowie die Alkoxyteile von C₁-C₆-Alkoxy-C₁-C₆alkyl, C₁-C₆-Alkoxy-C₂-C₆-alkyl, C₁-C₄-Alkoxy-C₂-C₆-alkoxycarbonyl und C₁-C₆-Alkoxycarbonyl: C₁-C₄-Alkoxy wie voranstehend genannt, sowie Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₄-Alkylsulfonyl (C₁-C₄-Alkyl-S(=O)₂-): Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
- C₂-C₆-Alkylsulfonyl: C₁-C₄-Alkylsulfonyl wie voranstehend genannt, sowie Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, 1,1-Dimethylpropylsulfonyl, 1,2-Dimethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, l-Ethyl-l-methylpropylsulfonyl und 1-Ethyl-2-methylpropylsulfonyl;
- C₁-C₆-Halogenalkylsulfonyl: einen C₁-C₆-Alkylsulfonylrest wie voranstehend genannt, der partiell oder vollständig durch Fluor, Chlor, Brom und/oder Iod substituiert ist, also Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2,2,2-Trichlorethylsulfonyl, 2-Chlor-2-fluorethylsulfonyl, 2-Chlor-2,2-difluorethylsulfonyl, 2,2-Dichlor-2-fluorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl und Dodecafluorhexylsulfonyl;
- C₁-C₄-Alkyliminooxy: Methyliminooxy, Ethyliminooxy, 1-Propyliminooxy, 2-Propyliminooxy, 1-Butyliminooxy und 2-Butyliminooxy;
- C₃-C₆-Alkenyl: Prop-1-en-1-yl, Prop-2-en-1-yl, 1-Methylethenyl, Buten-1-yl, Buten-2-yl, Buten-3-yl, 1-Methylprop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-1-yl, 2-Methyl-but-l-en-l-yl, 3-Methyl-but-1-en-1-yl, l-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-l-yl, 1,2-Dimethyl-prop-1-en-1-yl, 1,2-Dimethyl-prop-2-en-1-yl, 1-Ethyl-prop-1-en-2-yl, l-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-l-yl, 2-Methyl-pent-l-en-1-yl, 3-Methyl-pent-1-en-1-yl, 4-Methyl-pent-1-en-l-yl, l-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, l-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-l-en-l-yl, 1,2-Dimethyl-but-2-en-1-yl, 1,2-Dimethyl-but-3-en-1-yl, 1,3-Dimethyl-but-l-en-l-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-l-en-1-yl, 3,3-Dimethyl-but-2-en-1-yl, 1-Ethyl-but-1-en-1-yl, 1-Ethyl-but-2-en-1-yl, 1-Ethylbut-3-en-1-yl, 2-Ethyl-but-l-en-l-yl, 2-Ethyl-but-2-en-1-yl, 2-Ethyl-but-3-en-1-yl, 1,1,2-Trimethyl-prop-2-en-1-yl, 1-Ethyl-l-methyl-prop-2-en-1-yl, 1-Ethyl-2-methyl prop-1-en-1-yl und l-Ethyl-2-methyl-prop-2-en-1-yl;
- C₂-C₆-Alkenyl: C₃-C₆-Alkenyl wie voranstehend genannt, sowie Ethenyl;
- C₃-C₆-Alkinyl: Prop-1-in-1-yl, Prop-2-in-1-yl, But-1-in-1-yl, But-1-in-3-yl, But-1-in-4-yl, But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-1-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-l-in-1-yl, 3-Methyl-pent-1-in-3-yl, 3-Methyl-pent-l-in-4-yl, 3-Methyl-pent-1-in-5-yl, 4-Methyl-pent-1-in-l-yl, 4-Methyl-pent-2-in-4-yl und 4-Methyl-pent-2-in-5-yl;
- C₂-C₆-Alkinyl: C₃-C₆-Alkinyl, wie voranstehend genannt, sowie Ethinyl:
- C₃-C₆-Cycloalkyl: Cyclopropyl, Cyclobutyl, Cyclopentyl und Cyclohexyl;
- C₄-C₆-Cycloalkenyl: Cyclobuten-1-yl, Cyclobuten-3-yl, Cyclopenten-1-yl, Cyclopenten-3-yl, Cyclopenten-4-yl, Cyclohexen-1-yl, Cyclohexen-3-yl und Cyclohexen-4-yl;
- Heterocyclyl, sowie die Heteroxyclylreste in Heterocyclyloxy: drei- bis siebengliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, wie Oxiranyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofuran-2-yl, 2,3-Dihydrofuran-3-yl, 2,3-Dihydrofuran-4-yl, 2,3-Dihydrofuran-5-yl, 2,5-Dihydrofuran-2-yl, 2,5-Dihydrofuran-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroxazol-5-yl, 2,3-Dihydroisothiazol-3-yl, 2,3-Dihydroisothiazol-4-yl, 2,3-Dihydroisothiazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydropyrazol-3-yl, 2,3-Dihydropyrazol-4-yl, 2,3-Dihydropyrazol-5-yl, 4,5-Dihydropyrazol-3-yl, 4,5-Dihydropyrazol-4-yl, 4,5-Dihydropyrazol-5-yl, 2,5-Dihydropyrazol-3-yl, 2,5-Dihydropyrazol-4-yl, 2,5-Dihydropyrazol-5-yl, 2,3-Dihydrooxazol-2-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 3-Tetrahydropyranyl, 4-Tetrahydropyranyl, 2-Tetrahydrothiopyranyl, 3-Tetrahydrothiopyranyl, 4-Tetrahydrothiopyranyl, 1,3-Dioxolan-2-yl, 3,4,5,6-Tetrahydropyridin-2-yl, 4H-1,3-Thiazin-2-yl, 4H-3,l-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl,
- Hetaryl, sowie die Hetarylreste in Hetaryloxy:
   aromatische mono- oder polycyclische Reste, welche neben Kohlenstoffringgliedern zusätzlich ein bis vier Stickstoffatome oder ein bis drei Stickstoffatome und ein Sauerstoffoder ein Schwefelatom oder ein Sauerstoff- oder ein Schwefelatom enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4,5-Tetrazin-3-yl, sowie die entsprechenden benzokondensierten Derivate.

Alle Phenyl-, Hetaryl- und Heterocyclylringe sind vorzugsweise unsubstituiert oder tragen ein bis drei Halogenatome und/oder einen oder zwei Reste aus folgender Gruppe: Nitro, Cyano, Methyl, Trifluormethyl, Methoxy, Trifluormethoxy oder Methoxycarbonyl.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹: Nitro, Halogen, Cyano, Rhodano, C₁-C₆-Alkyl, C₁-C₆-Halogen-alkyl, C₁-C₆-Alkoxy-C₁-C₆-alkyl, C₂-C₆-Alkenyl, C₂-C₆-Alkinyl, -OR⁵ oder -S(O)ₙR⁷;
besonders bevorzugt Nitro, Halogen wie z.B. Fluor, Chlor oder Brom, C₁-C₆-Halogenalkyl, -OR⁵ oder -SO₂R⁷;
- R²: Wasserstoff, Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵ oder -S(O)ₙR⁷;
besonders bevorzugt Wasserstoff, Nitro, Halogen wie z.B. Fluor, Chlor oder Brom, C₁-C₆-Alkyl, C₁-C₆-Halogenalkyl, -OR⁵ oder -SO₂R⁷;
- R⁴- R⁷: Wasserstoff, Hydroxy, Mercapto, Amino, Halogen, C₁-C₄-Alkyl, C₅-C₆-Cycloalkyl, C₅-C₆-Cycloalkenyl, Phenyl, -OR¹⁰, -S(O)ₙR¹⁰, -OS(O)ₙR¹⁰, -PO(OR¹⁰)₂, -NR³R¹⁰, -Si(R¹⁰)₃ oder -OCOR¹⁰,
wobei die genannten Alkyl und Cycloalkylreste sowie R³ und R¹⁰ der Reste -OR¹⁰, -S(O)ₙR¹⁰, -OS(O)ₙR¹⁰, -PO(OR¹⁰)₂, -NR³R¹⁰, -Si(R¹⁰)₃, -OCOR¹⁰ partiell oder vollständig halogeniert sein können und/oder eine bis drei der folgenden Gruppen tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl; besonders bevorzugt Wasserstoff, Hydroxy, Mercapto, Halogen, wie z. B. Fluor, Chlor oder Brom, C₁-C₄-Alkyl, -OSO₂R¹⁰, -OPO₃R¹⁰, -Si(CH₃)₃;
- R⁴, R⁵: bilden bevorzugt zusammen eine gegebenenfalls durch ein Sauerstoffatom ein- oder zweifach unterbrochene C₂-C₅-Alkylen- oder C₂-C₅-Alkenylenkette oder eine Gruppe =X, wobei X vorzugsweise für ein Sauerstoffatom oder NR¹⁰ steht; besonders bevorzugt bilden R⁴ und R⁵ eine Gruppe =X, wobei =X vorzugsweise für ein Sauerstoffatom steht;
- R⁶, R⁷: bilden bevorzugt zusammen eine gegebenenfalls durch ein Sauerstoffatom ein- oder zweifach unterbrochene C₂-C₅-Alkylen- oder C₂-C₅-Alkenylenkette oder eine Gruppe =X, wobei X vorzugsweise für ein Sauerstoffatom oder NR¹⁰ steht;
besonders bevorzugt bilden R⁶ und R⁷ eine Gruppe =X, wobei =X vorzugsweise für ein Sauerstoffatom steht;
- n: zwei;
- R⁵, R⁶: bilden bevorzugt zusammen, wenn sie an benachbarte Kohlenstoffatome gebunden sind, eine gegebenenfalls durch ein Stickstoff- oder ein Sauerstoffatom unterbrochene C₃-C₄-Alkylen- oder C₃-C₄-Alkenylenkette, wenn R⁴ und R⁷ für Wasserstoff stehen;
- R⁸, R⁹: Wasserstoff, Nitro, Halogen, Cyano, C₁-C₄-Alkyl, C₅-C₆-Cycloalkyl, C₅-C₆-Cycloalkenyl, C₅-C₆-Heterocyclyl, -OR¹⁰, -SR¹⁰, -COR¹⁰, -COOR¹⁰, -CONR³R¹⁰, Phenyl, Phenyl-C₁-C₄-alkyl und fünf- oder sechsgliedriges Hetaryl, wobei die genannten Alkyl und Cycloalkylreste sowie R³ und R¹⁰ der Reste -OR¹⁰, -SR¹⁰, -COR¹⁰, -COOR¹⁰, -CONR³R¹⁰ partiell oder vollständig halogeniert sein können und/-oder eine bis drei der folgenden Gruppen tragen können: Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
- R¹⁰: Wasserstoff, C₁-C₄-Alkyl, C₁-C₄-Halogenalkyl, C₂-C₄-Alkenyl, Phenyl oder Phenyl-C₁-C₄-alkyl, wobei der genannte Phenylrest partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl.
- R¹¹: C₁-C₆-Alkyl oder C₁-C₆-Halogenalkyl; besonders bevorzugt Methyl, Ethyl, Propyl, Isopropyl, Butyl oder Isobutyl;
- R¹²: Wasserstoff, C₁-C₆-Alkyl, C₁-C₆-Alkylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, Phenyl-carbonylmethyl, oder Phenylsulfonyl, wobei der Phenylring der zwei letztgenannten Substituenten partiell oder vollständig halogeniert sein kann und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy;
- R¹³: Wasserstoff, C₁-C₆-Alkyl oder C₁-C₆-Halogenalkyl; besonders bevorzugt Wasserstoff, Methyl, Ethyl oder Trifluor-methyl.

Insbesondere bevorzugt sind die Verbindungen der Formel Ia, wobei R¹ in Position 2 und R² in Position 4 des Phenylringes gebunden sind.

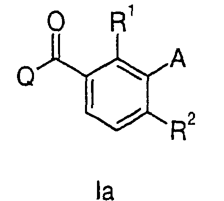

Außerordentlich bevorzugt sind die Verbindungen der Formel Ia, in der die Substituenten R¹, R² und Q die oben genannte Bedeutung haben, A für eine Gruppe der Formel IIIa oder IIIb steht und
- R⁴ - R⁷: Wasserstoff, Hydroxy, Amino, Halogen, C₁-C₄-Alkyl, C₅-C₆-Cycloalkyl, Phenyl, -OR¹⁰, -SO₂R¹⁰, -OSO₂R¹⁰, -PO(OR¹⁰)₂, -NR³R¹⁰ oder -OCOR¹⁰,
wobei die genannten Alkyl und Cycloalkylreste sowie R³ und R¹⁰ der Reste -OR¹⁰, -SO₂R¹⁰, -OSO₂R¹⁰, -PO(OR¹⁰)₂, -NR³R¹⁰, -OCOR¹⁰ partiell oder vollständig halogeniert sein können und/ oder eine bis drei der folgenden Gruppen tragen können:
Hydroxy, Amino, Cyano, R¹⁰, -OR¹⁰, -NR³R¹⁰, -OCOR¹⁰, -CO₂R¹⁰, C₁-C₄-Alkylcarbonyl, C₁-C₄-Alkylsulfonyl, Phenyl, Benzyl, Phenoxy und Benzyloxy, wobei die vier letztgenannten Reste ihrerseits substituiert sein können;
bedeuten.

Darüber hinaus sind die erfindungsgemäßen Verbindungen der Formel Ia außerordentlich bevorzugt, in der die Substituenten R¹, R² und Q die oben genannte Bedeutung haben, A für eine Gruppe der Formel IIIa oder IIIb steht und
- R⁴, R⁵: zusammen eine gegebenenfalls durch ein Sauerstoffatom ein- oder zweifach unterbrochene C₂-C₅-Alkylen- oder C₂-C₅-Alkenylenkette oder eine Gruppe =X bilden, wobei X vorzugsweise für ein Sauerstoffatom steht;
und/oder
- R⁶, R⁷: zusammen eine gegebenenfalls durch ein Sauerstoffatom ein- oder zweifach unterbrochene C₂-C₅-Alkylen- oder C₂-C₅-Alkenylenkette oder eine Gruppe =X bilden, wobei X vorzugsweise für ein Sauerstoffatom steht.

Weiterhin sind die erfindungsgemäßen Verbindungen der Formel Ia außerordentlich bevorzugt, in der die Variablen R¹, R² und Q die oben genannte Bedeutung haben, A für eine Gruppe der Formel IIIa oder IIIb steht und
- R⁵, R⁶: wenn sie an benachbarte Kohlenstoffatome gebunden sind zusammen eine gegebenenfalls durch ein Stickstoff- oder ein Sauerstoffatom unterbrochene C₃-C₄-Alkylen- oder C₃-C₄-Alkenylenkette bilden, wenn R⁴ und R⁷ für Wasserstoff stehen.

Außerordentlich bevorzugt sind die Verbindungen der Formel Ia, in der die Variablen R¹, R² und Q die oben genannte Bedeutung haben, A für eine Gruppe der Formel IVb steht und
- R⁸, R⁹: Wasserstoff, Nitro, Halogen, Cyano, C₁-C₄-Alkyl, C₅-C₆-Cycloalkyl, C₅-C₆-Cycloalkenyl, C₅-C₆-Heterocyclyl, -OR¹⁰, -COR¹⁰, -COOR¹⁰, -CONR³R¹⁰, Phenyl, Phenyl-C₁-C₄-alkyl und fünf- oder sechsgliedriges Hetaryl, wobei die genannten Alkyl und Cycloalkylreste sowie R³ und R¹⁰ der Reste -OR¹⁰, -COR¹⁰, -COOR¹⁰, -CONR³R¹⁰ partiell oder vollständig halogeniert sein können und/oder eine bis drei der folgenden Gruppen tragen können:
Hydroxy, Amino, Cyano, R¹⁰, -OR¹⁰, -NR³R¹⁰, -OCOR¹⁰, -CO₂R¹⁰, C₁-C₄-Alkylcarbonyl, C₁-C₄-Alkylsulfonyl, Phenyl, Benzyl, Phenoxy und Benzyloxy, wobei die vier letztgenannten Reste ihrerseits substituiert sein können;
bedeuten.

Darüber hinaus sind die erfindungsgemäßen Verbindungen der Formel Ia außerordentlich bevorzugt, in der die Variablen R¹, R² und Q die oben genannte Bedeucung haben, A für eine Gruppe der Formel IVb steht und
- R⁸, R⁹: zusammen eine gegebenenfalls durch ein Sauerstoffatom ein- oder zweifach unterbrochene C₂-C₅-Alkylen- oder C₂-C₅-Alkenylenkette bilden.

Insbesondere bevorzugt sind die Verbindungen Ib der Tabellen 1 bis 144.

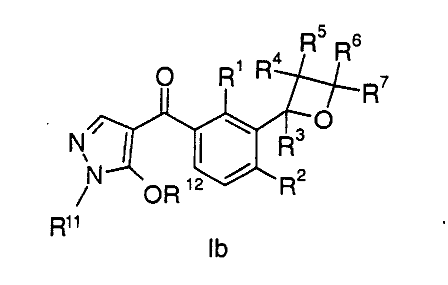

Die Substituenten R³ bis R⁷ für jede einzelne Verbindung Ib entsprechen jeweils einer Zeile der Tabelle A.

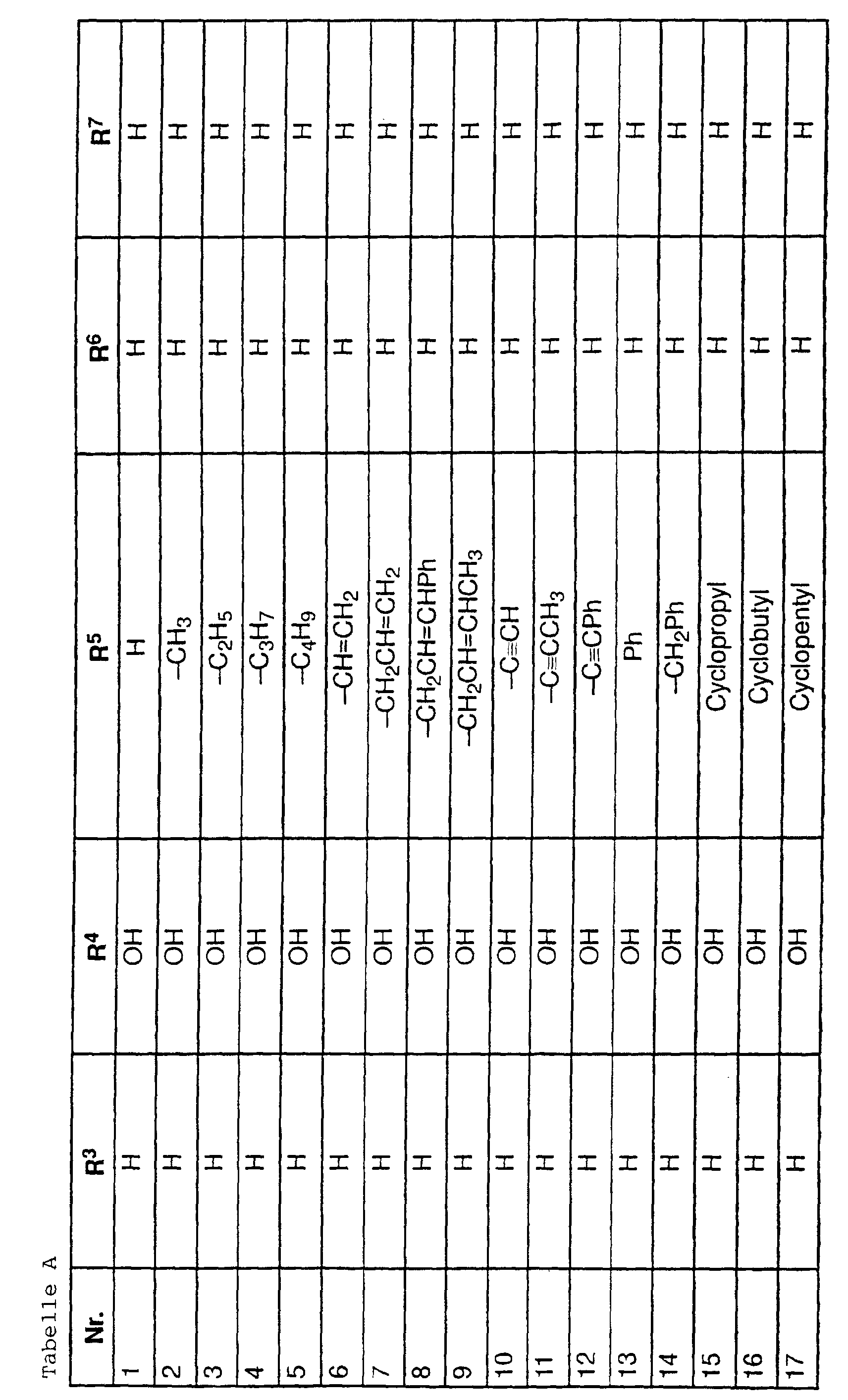

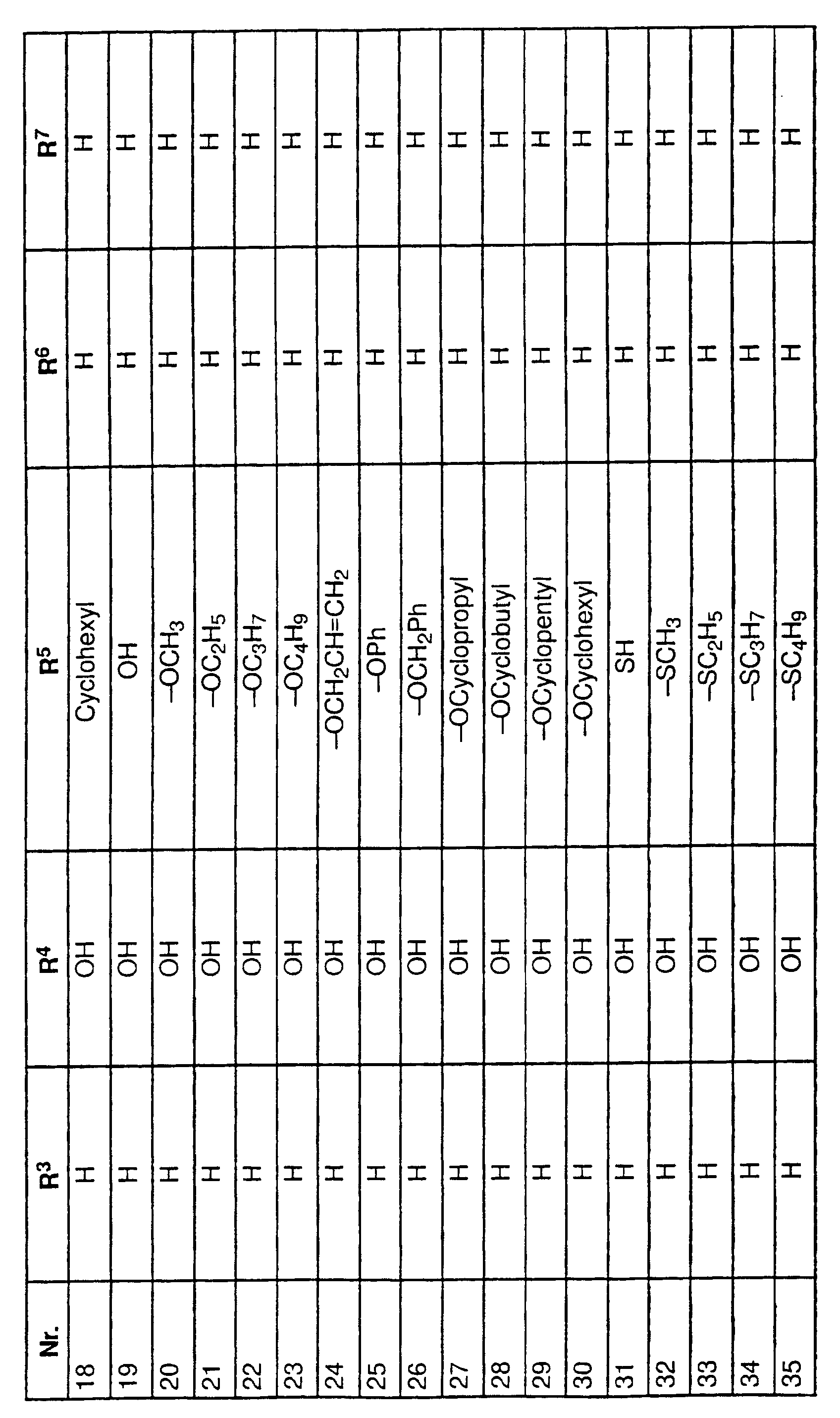

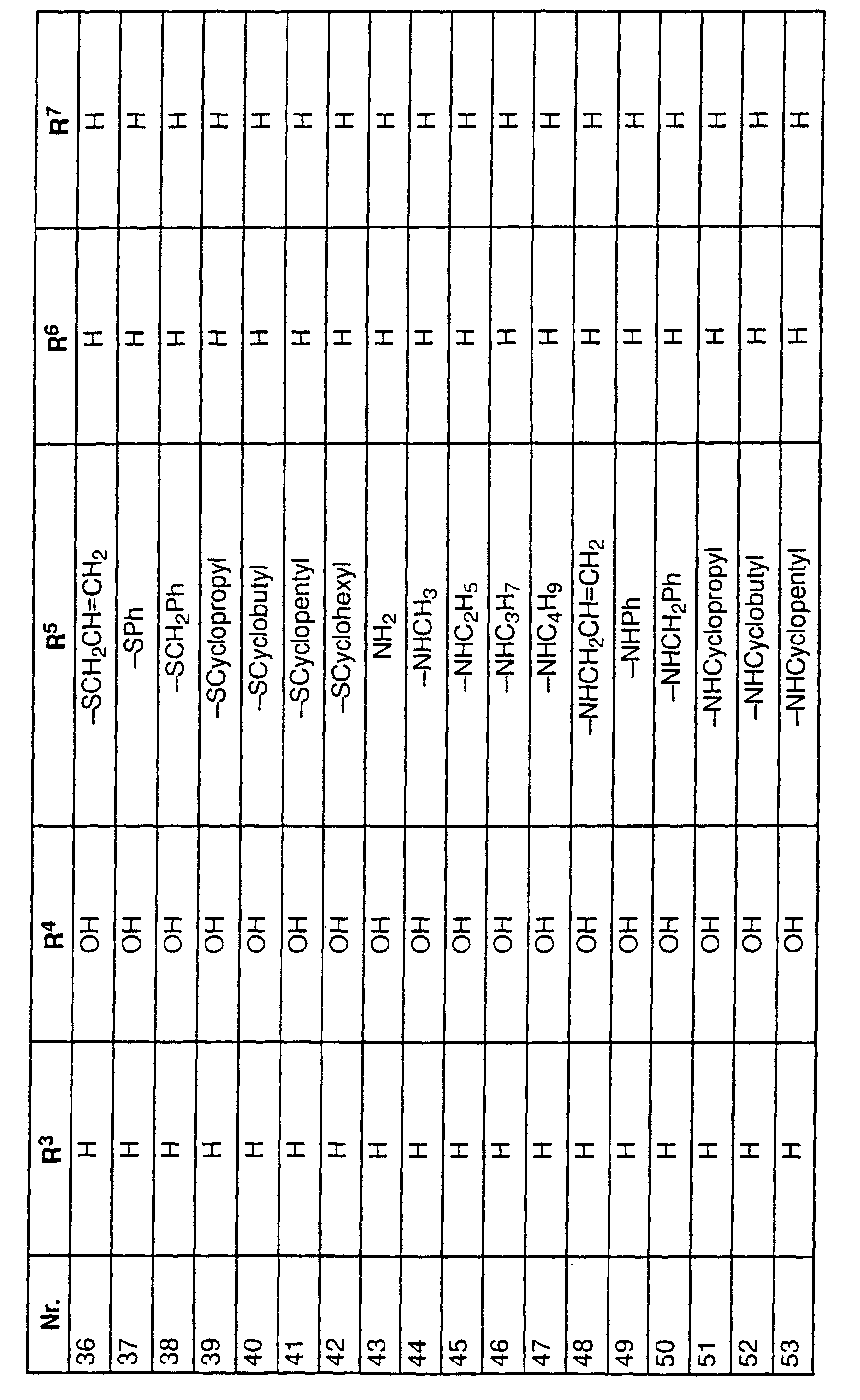

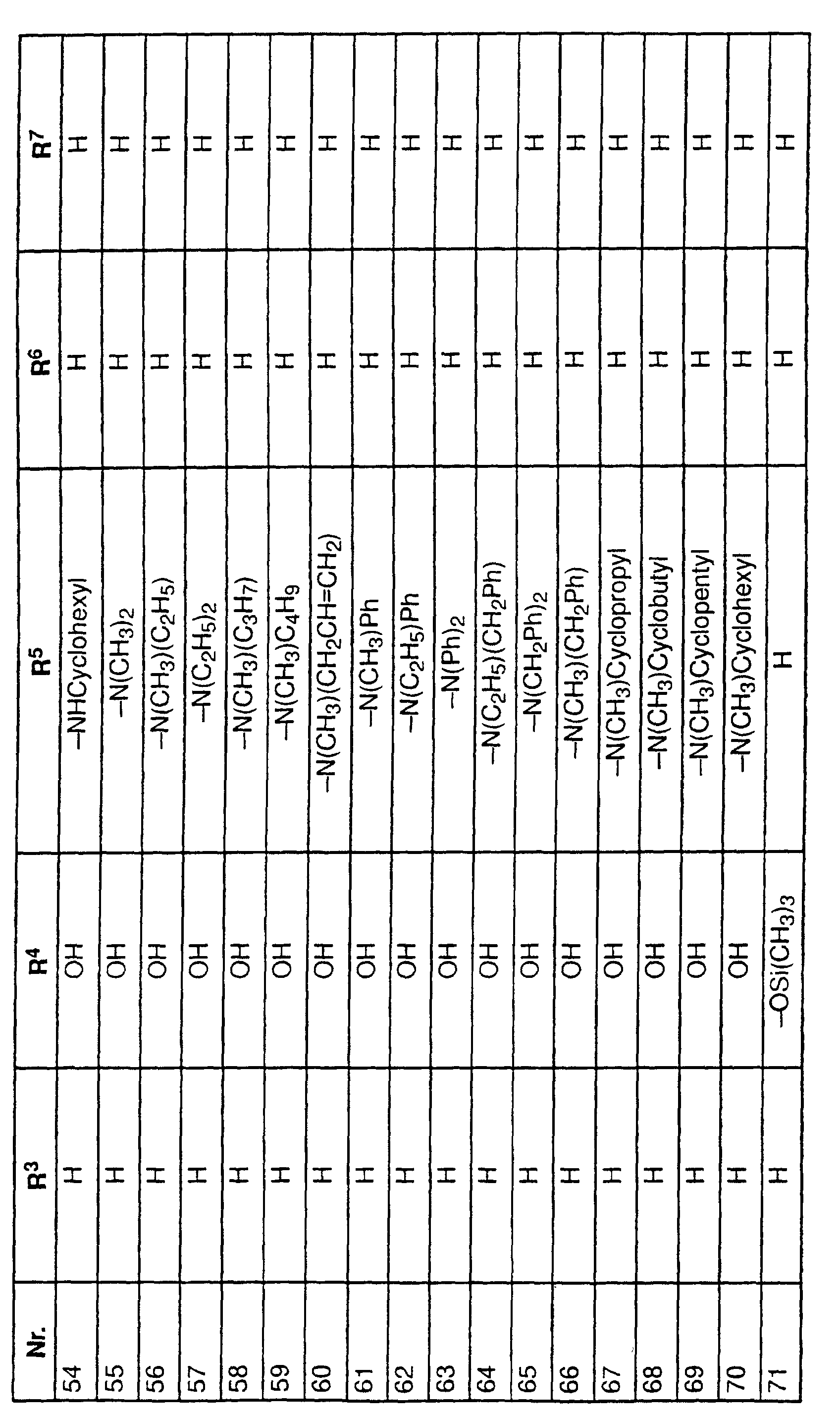

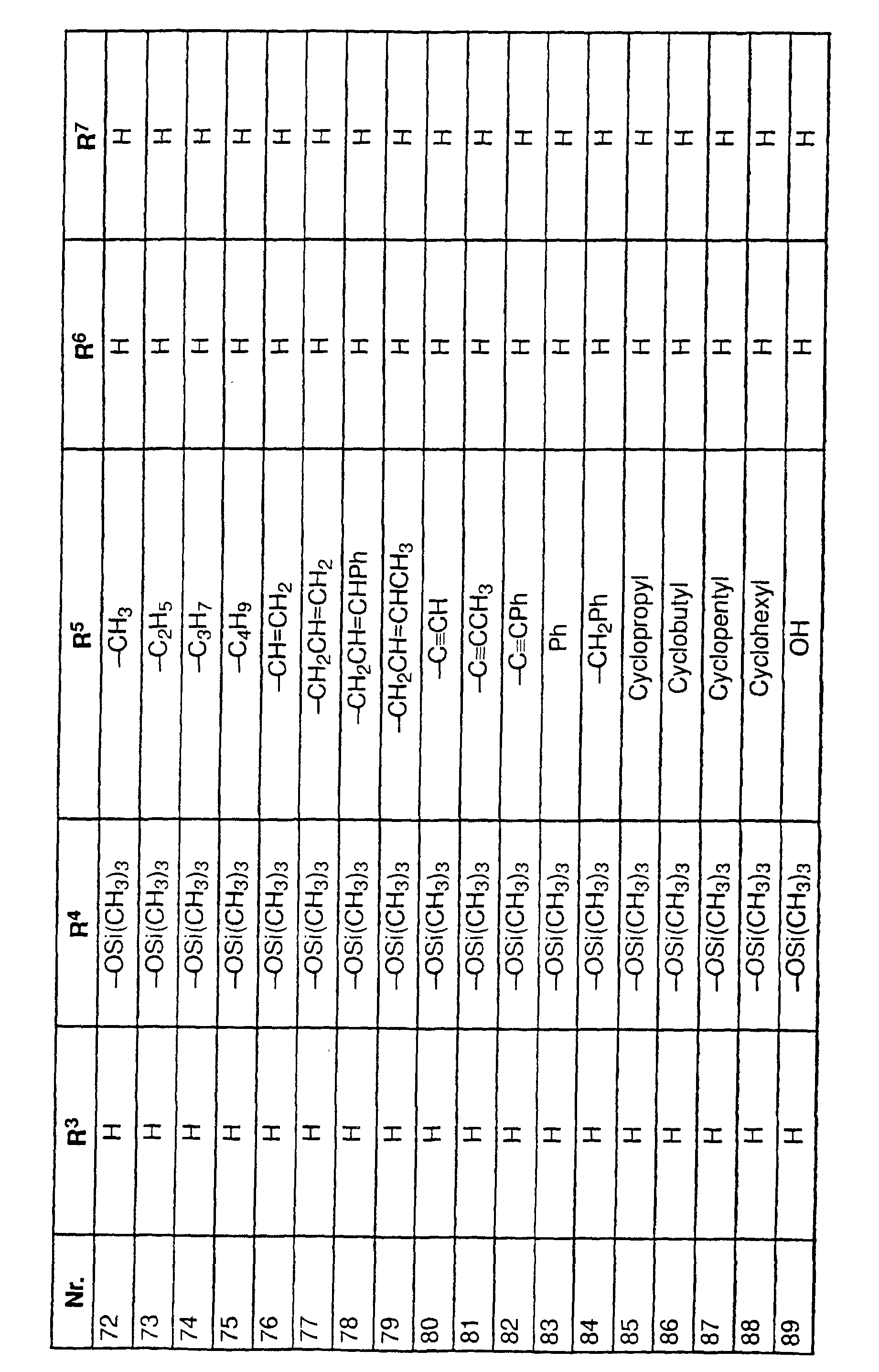

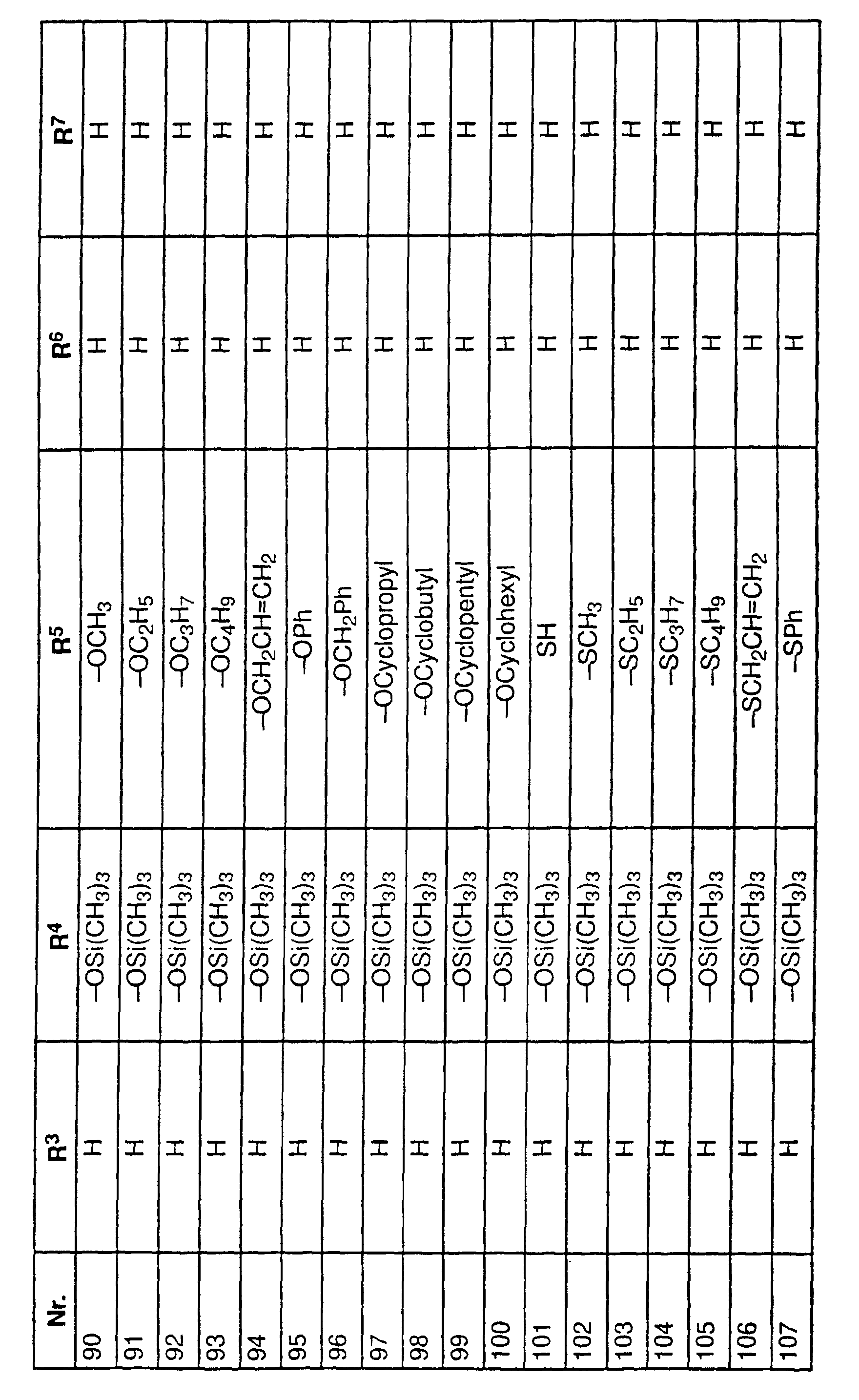

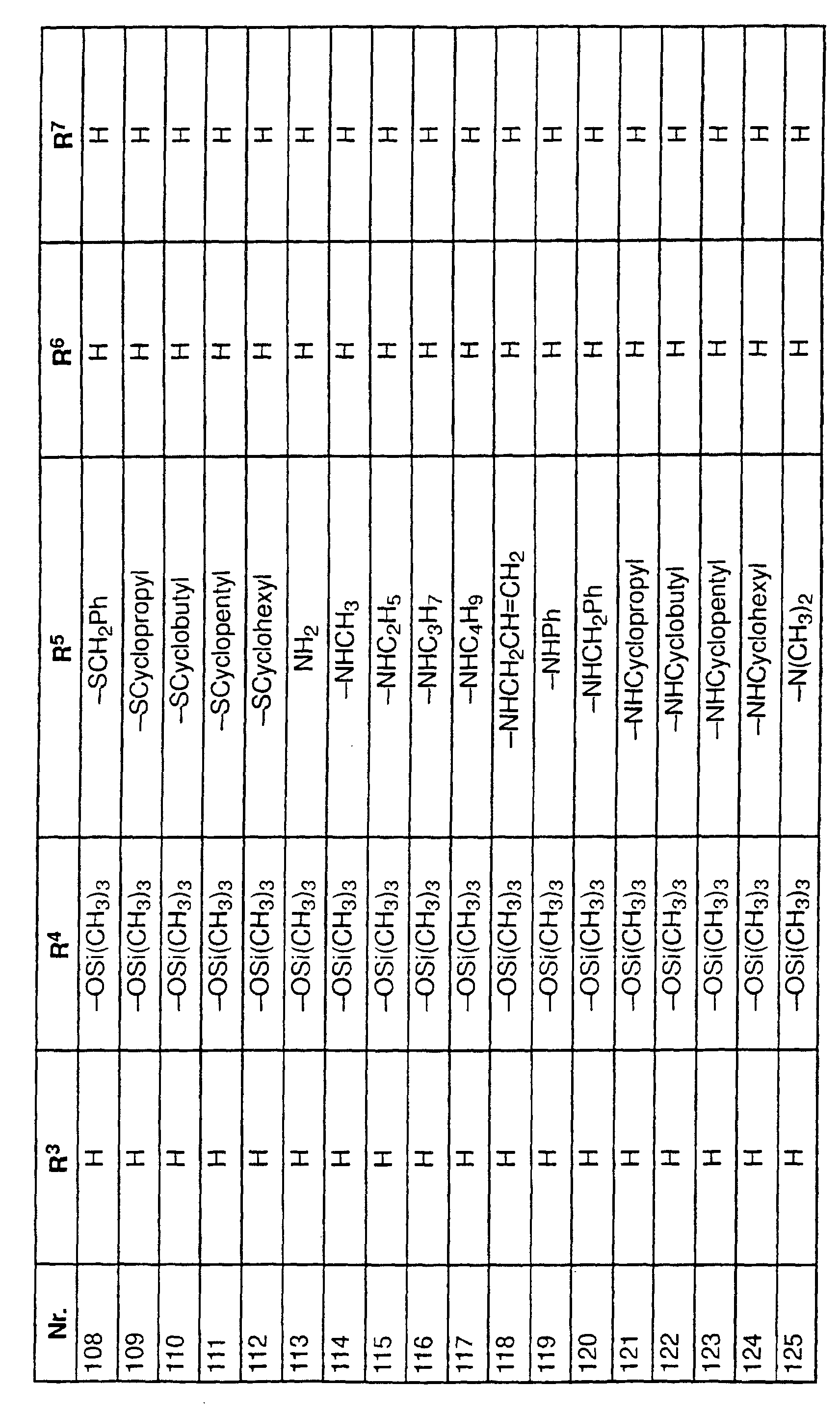

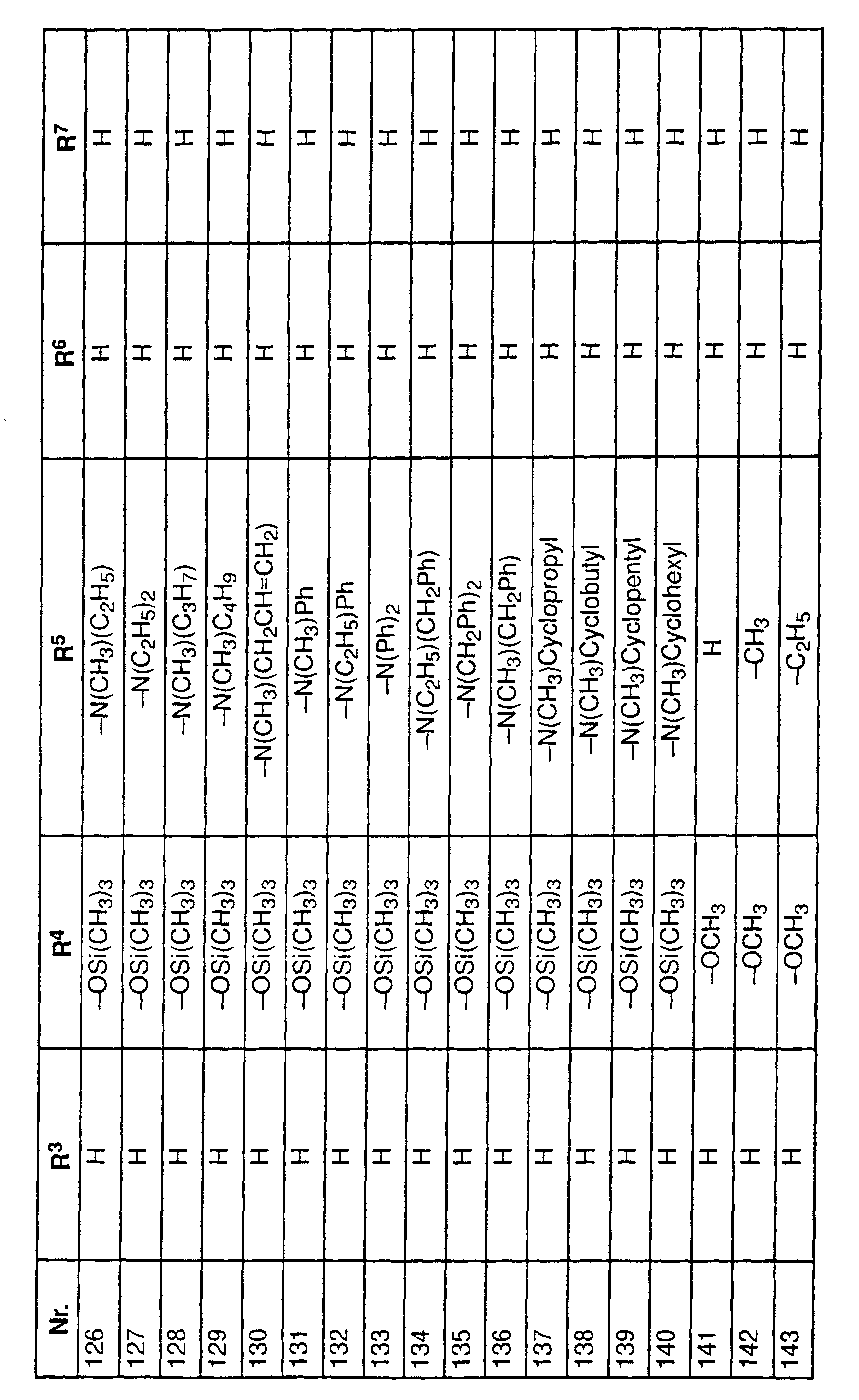

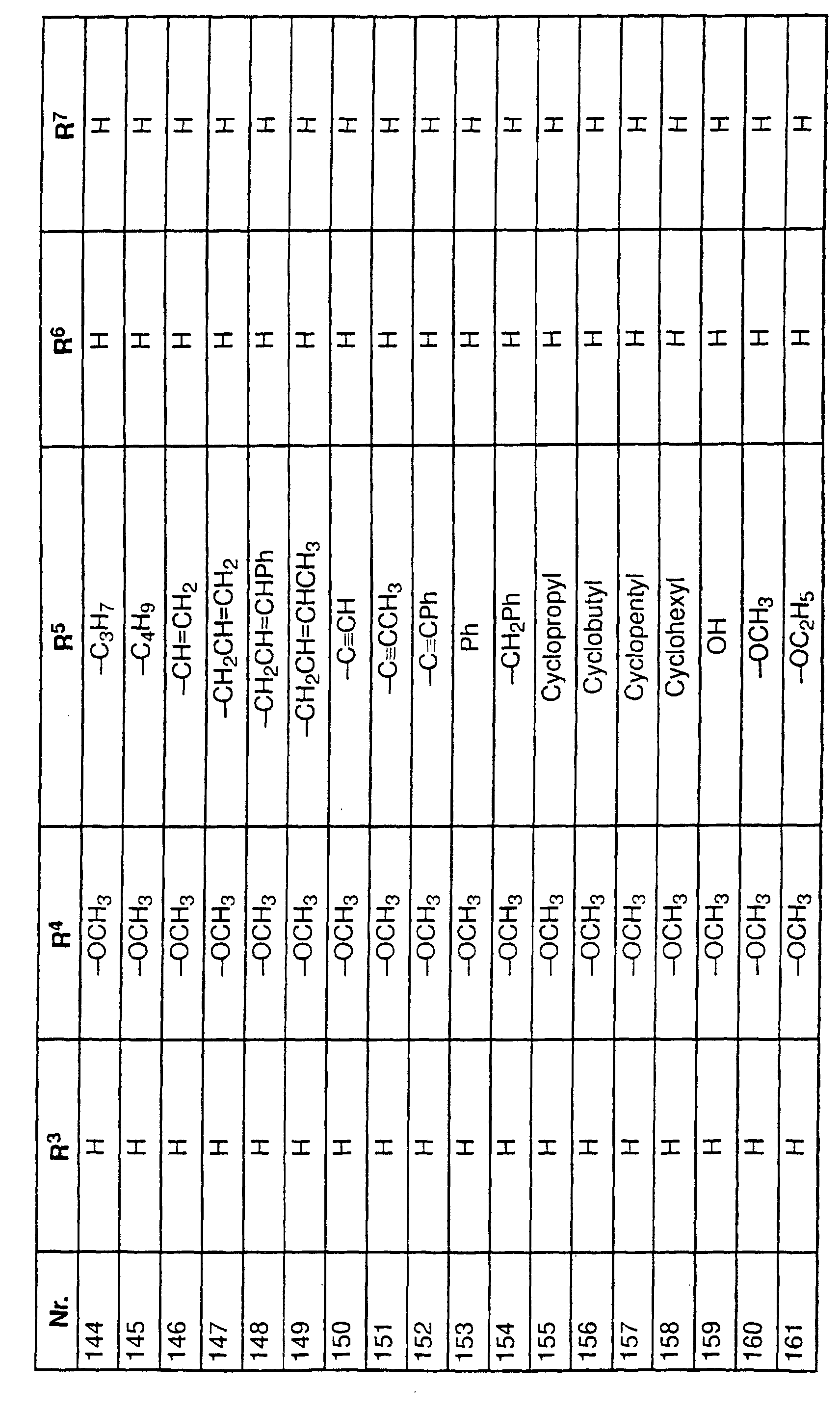

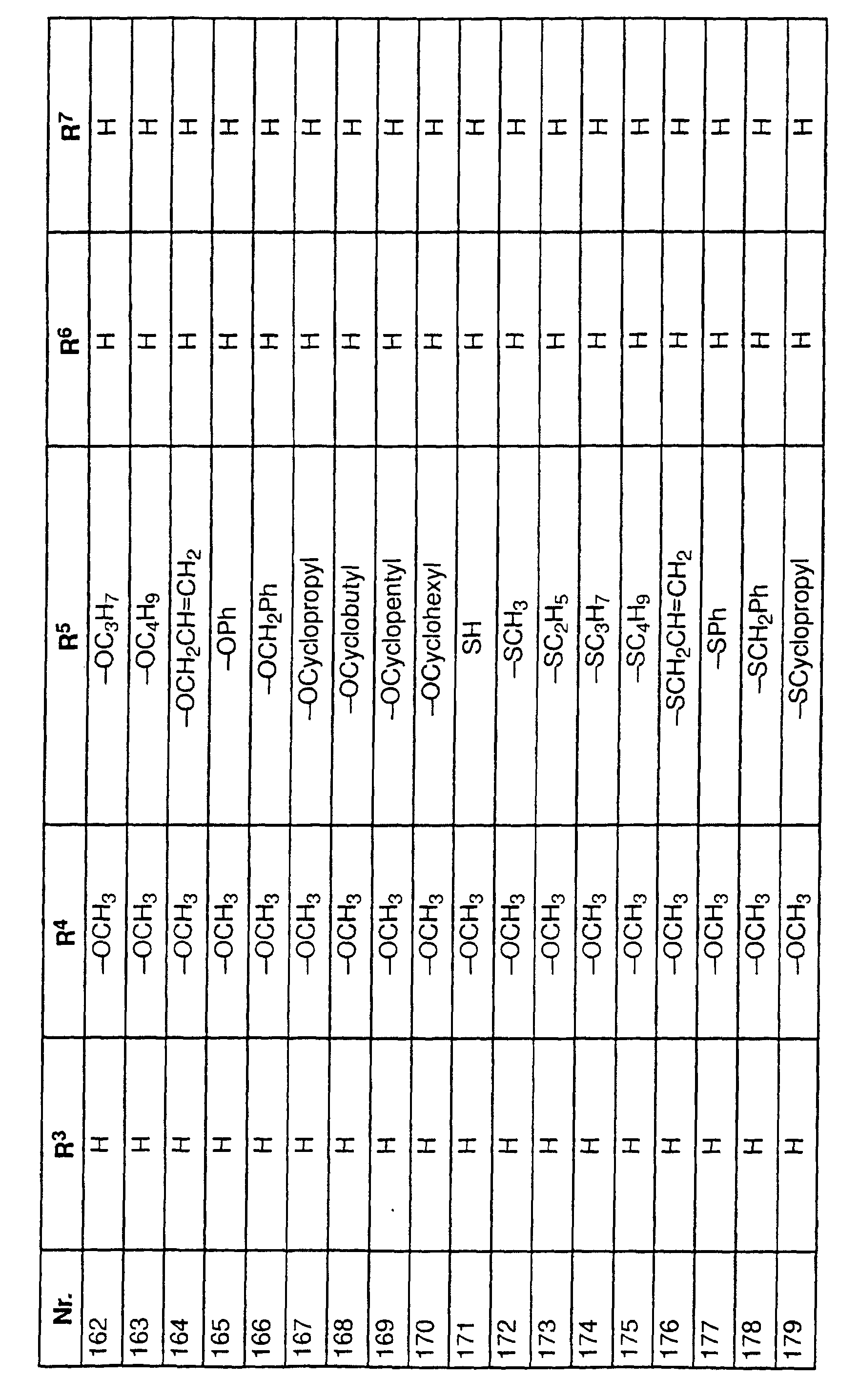

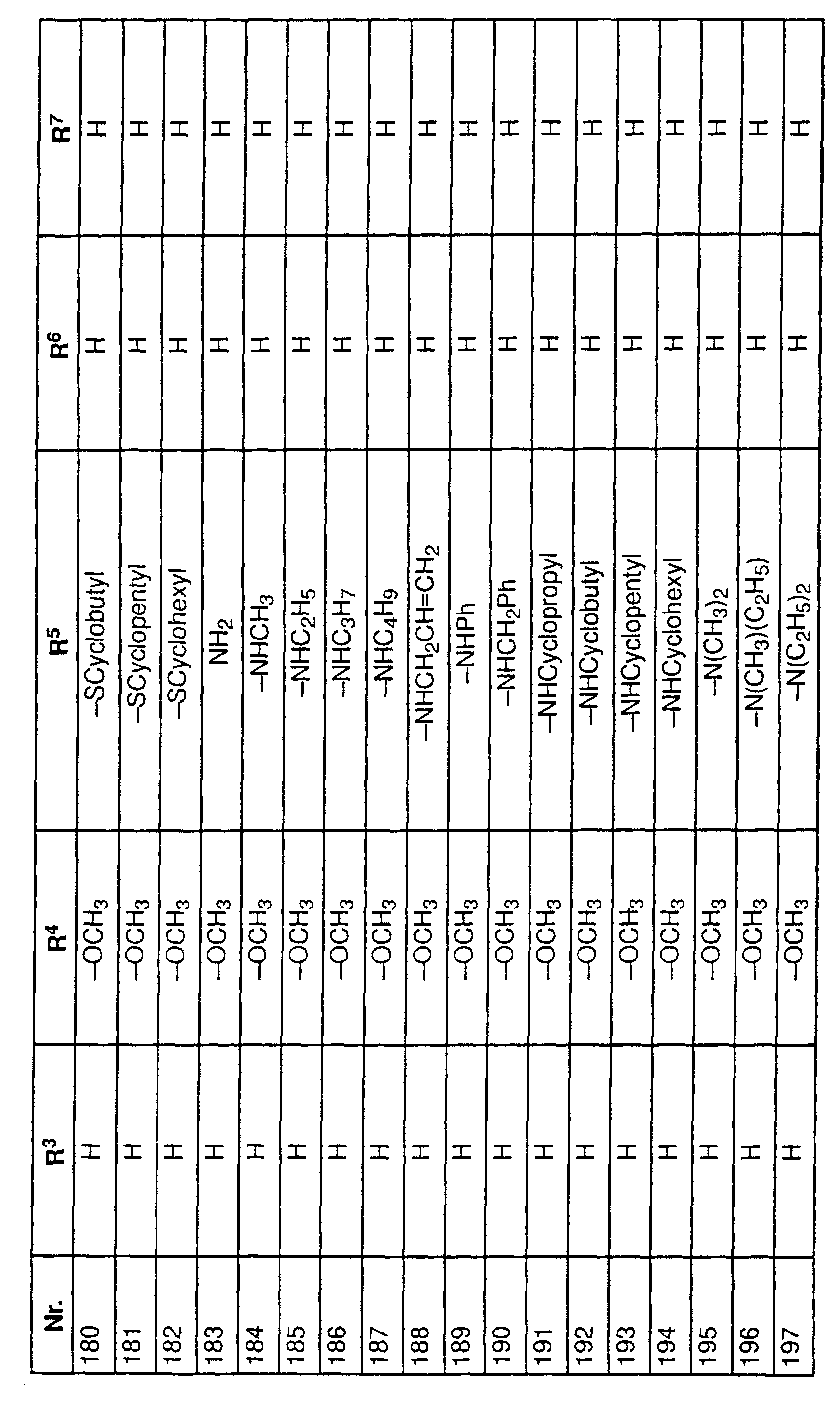

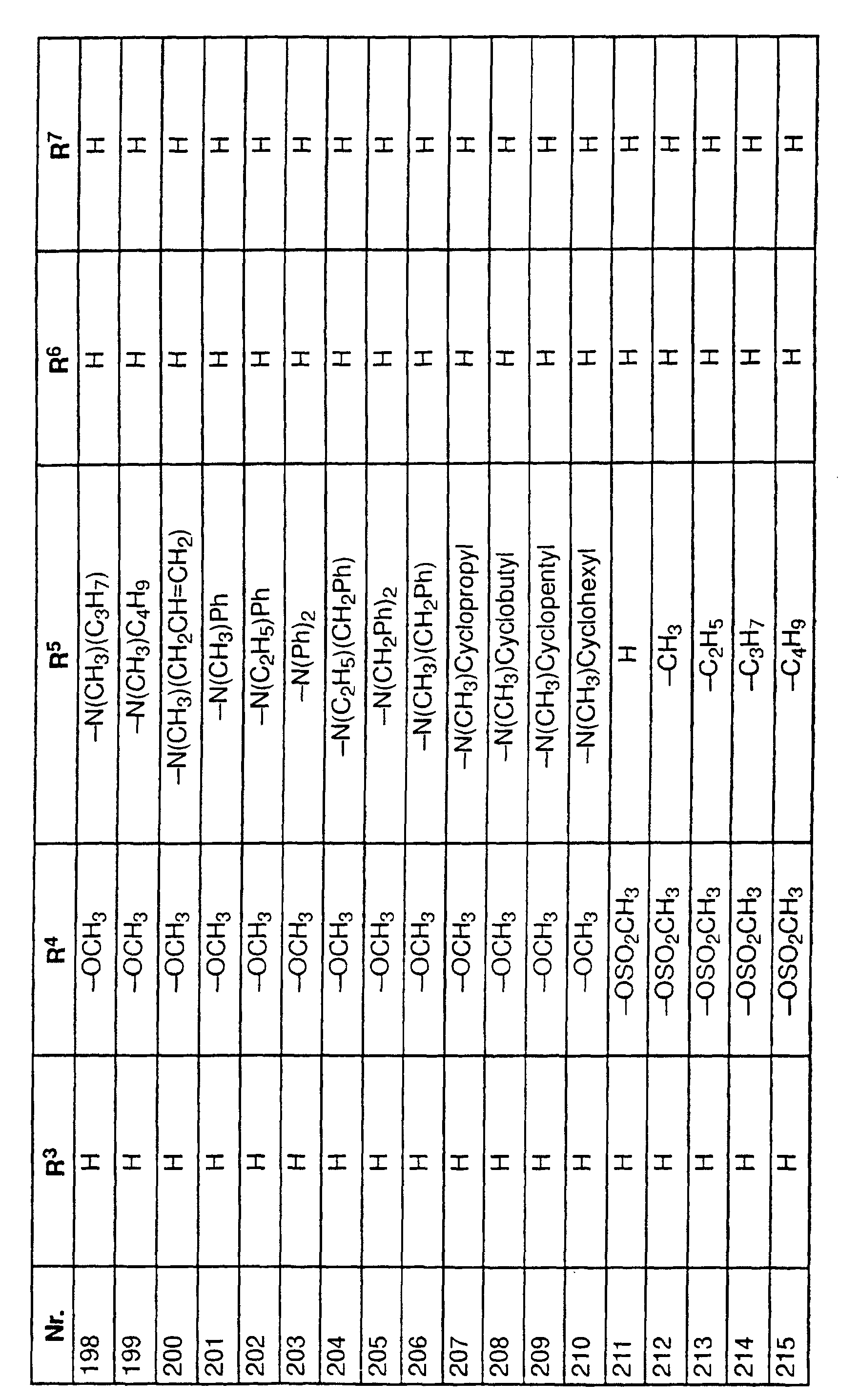

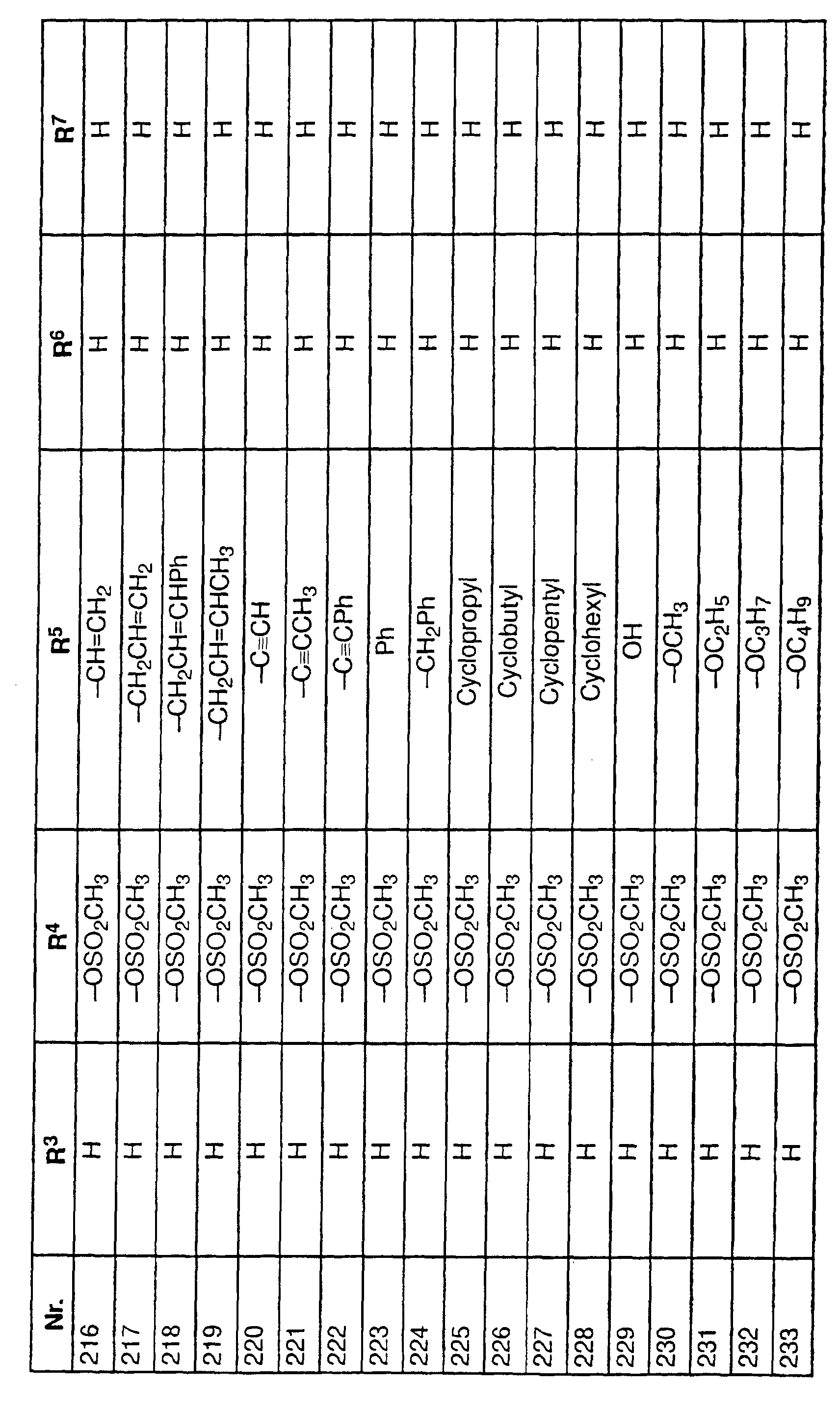

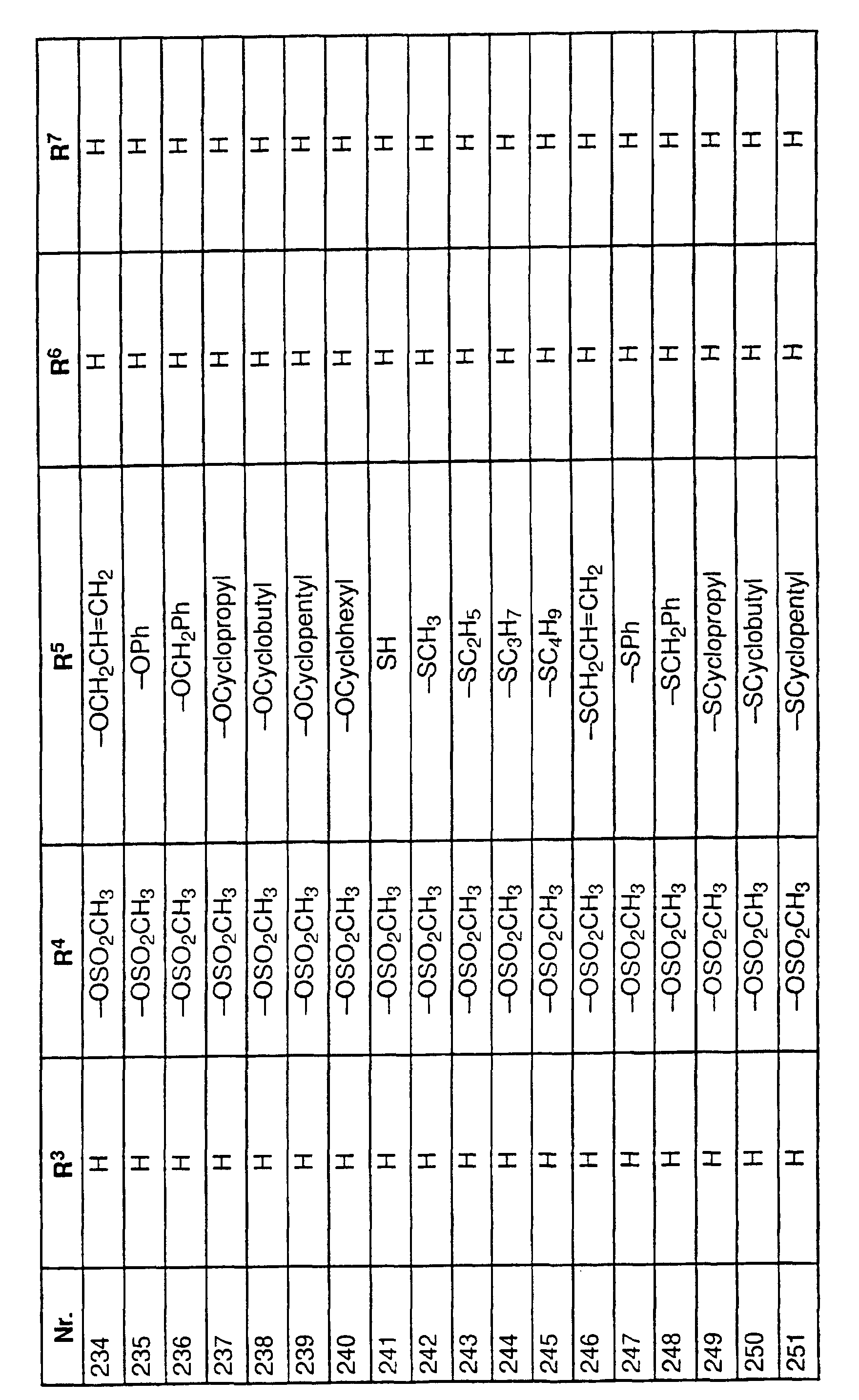

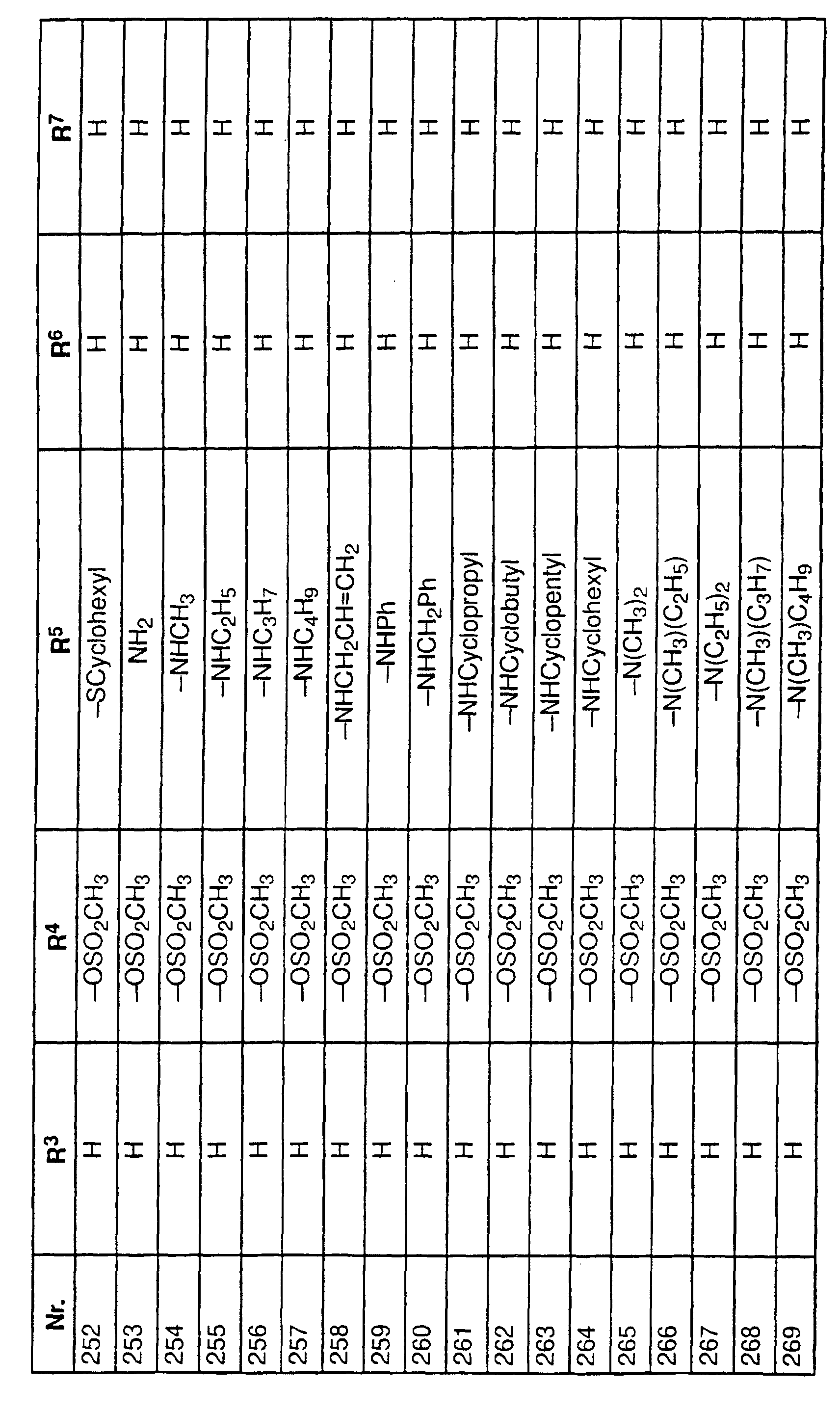

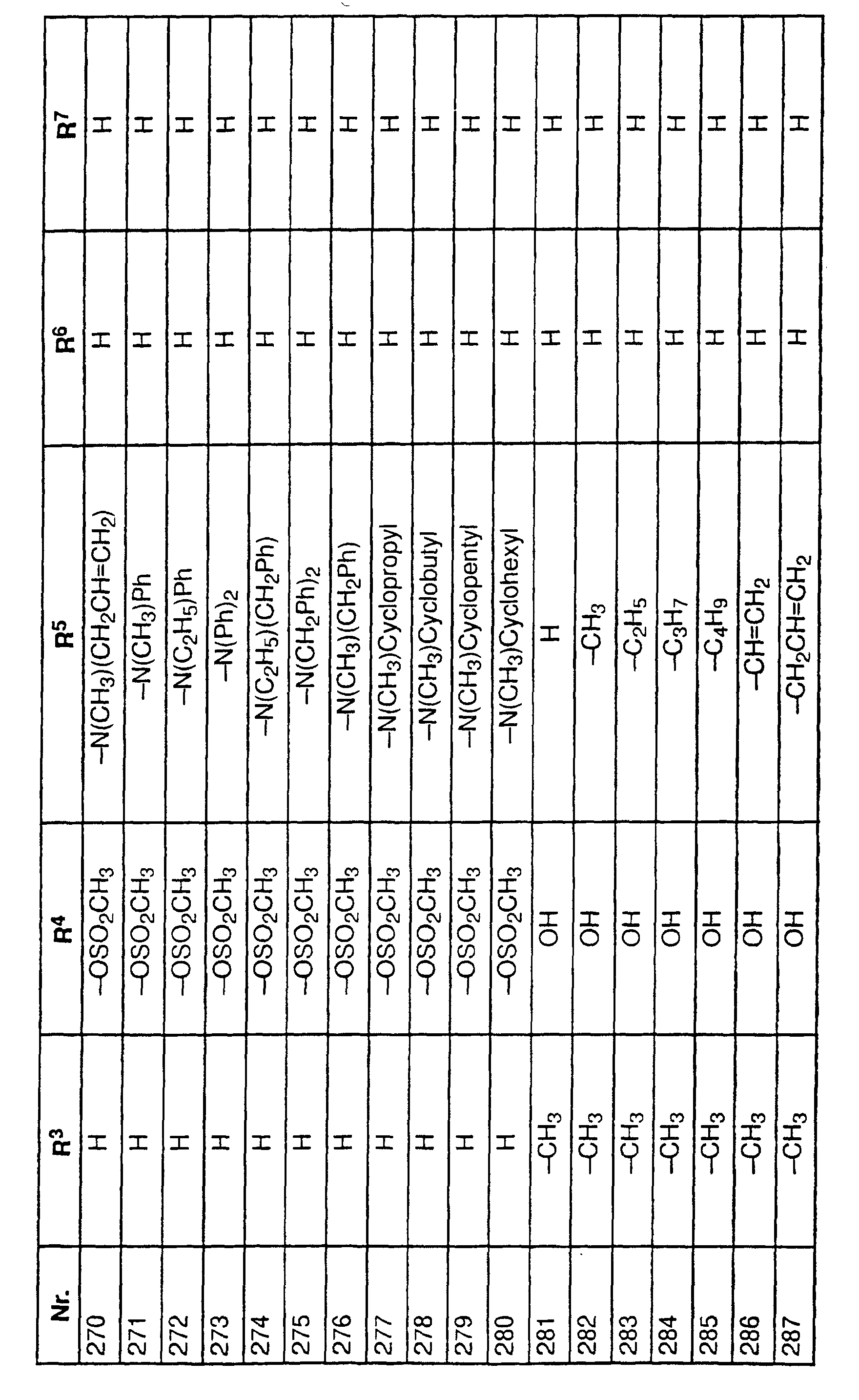

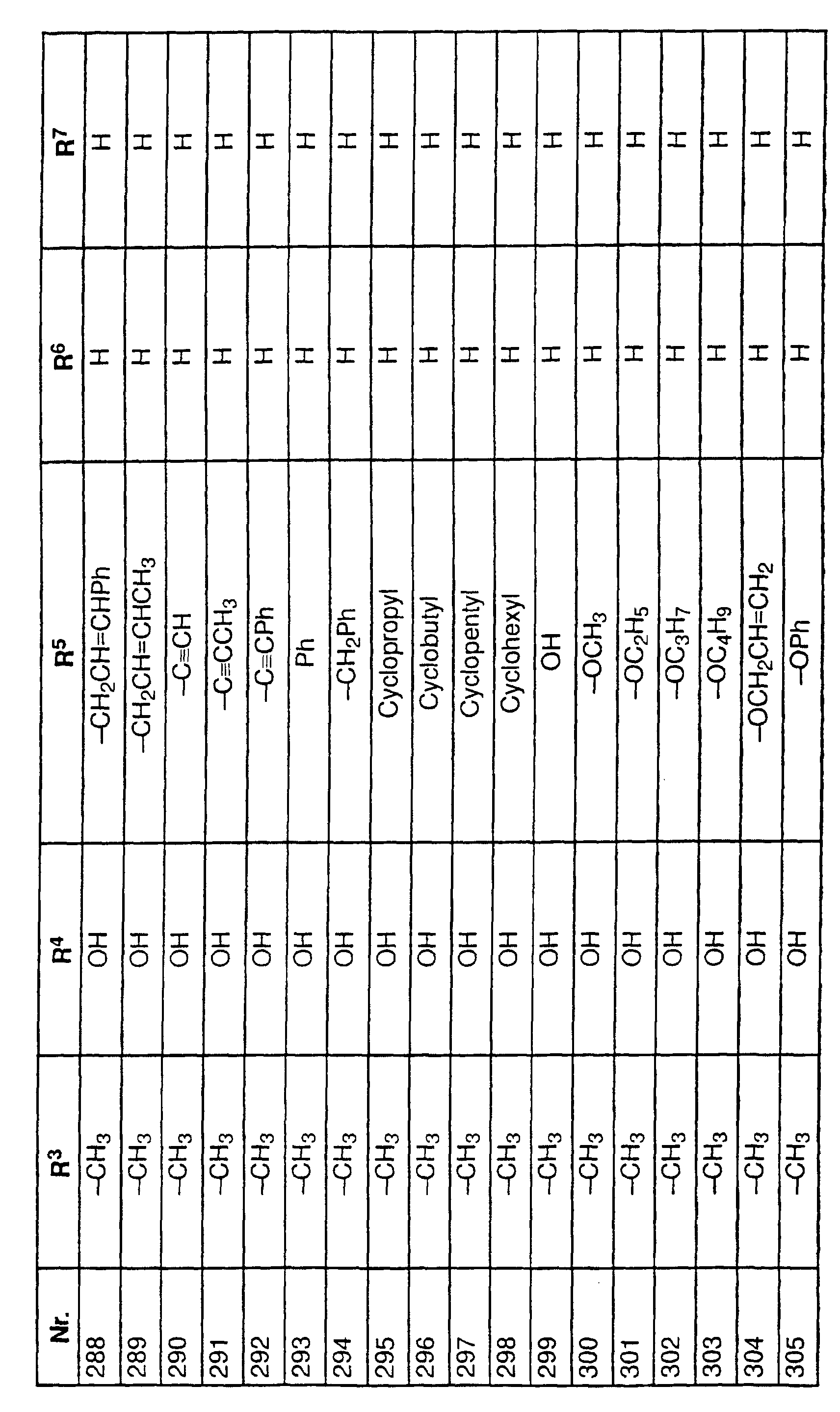

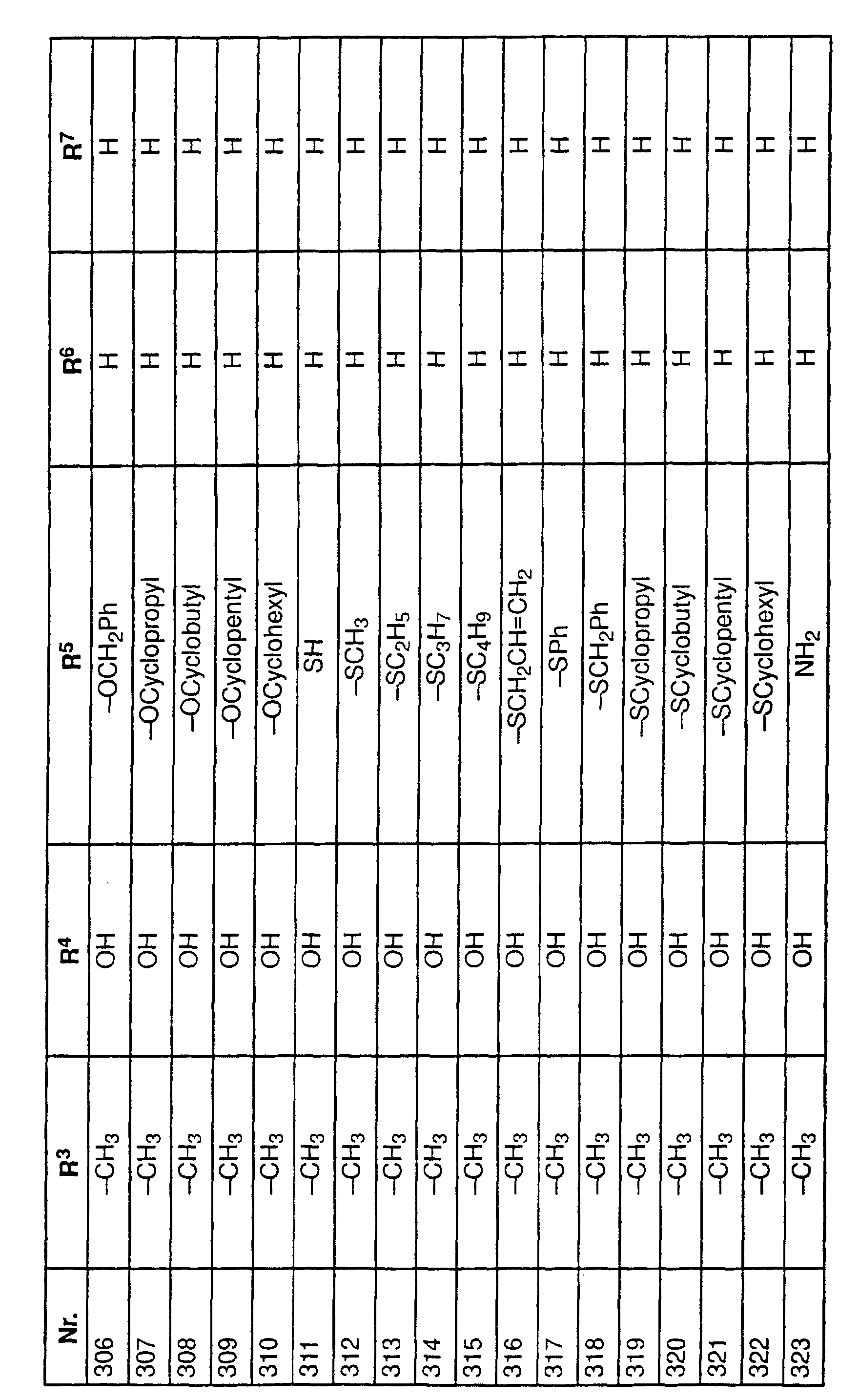

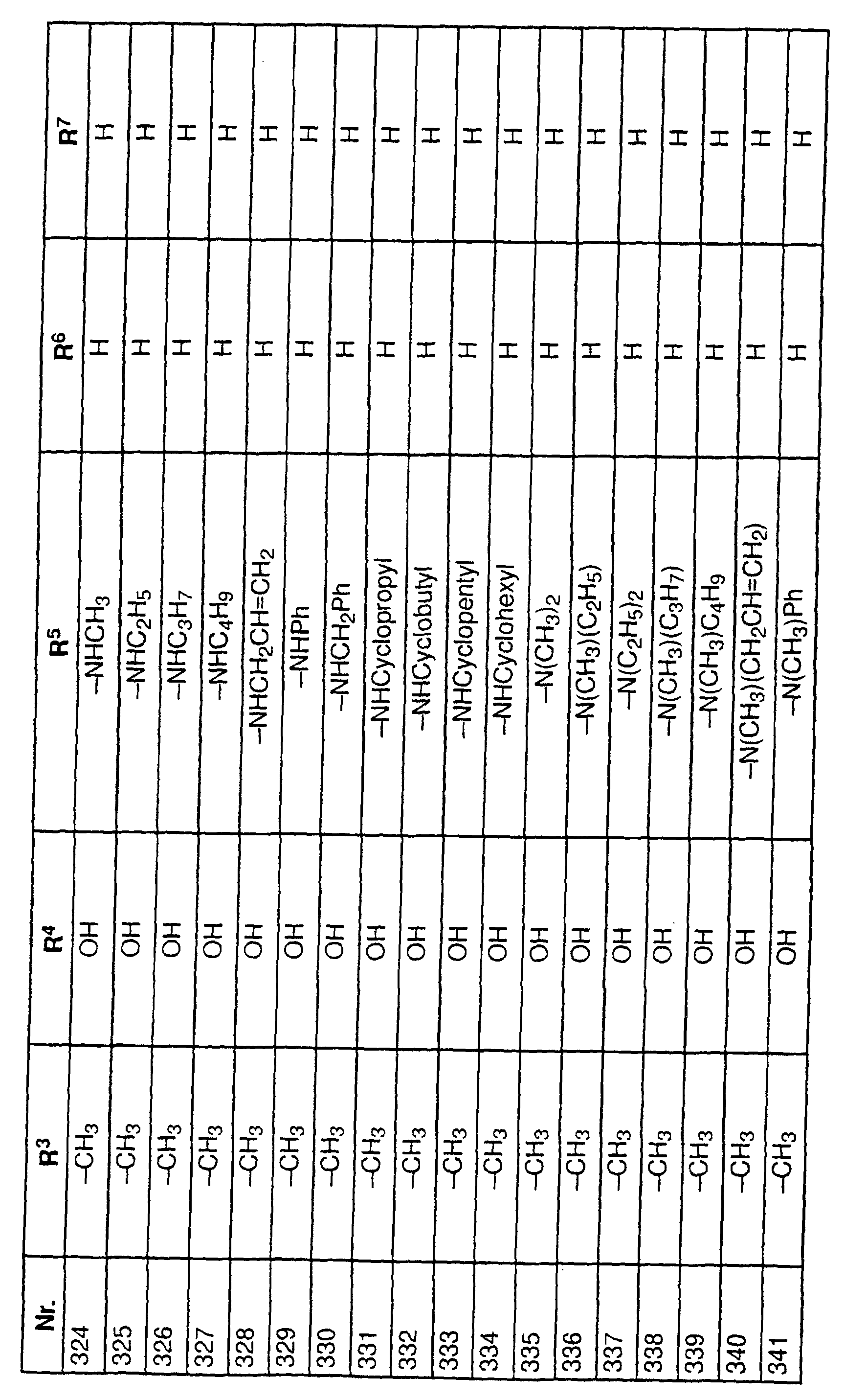

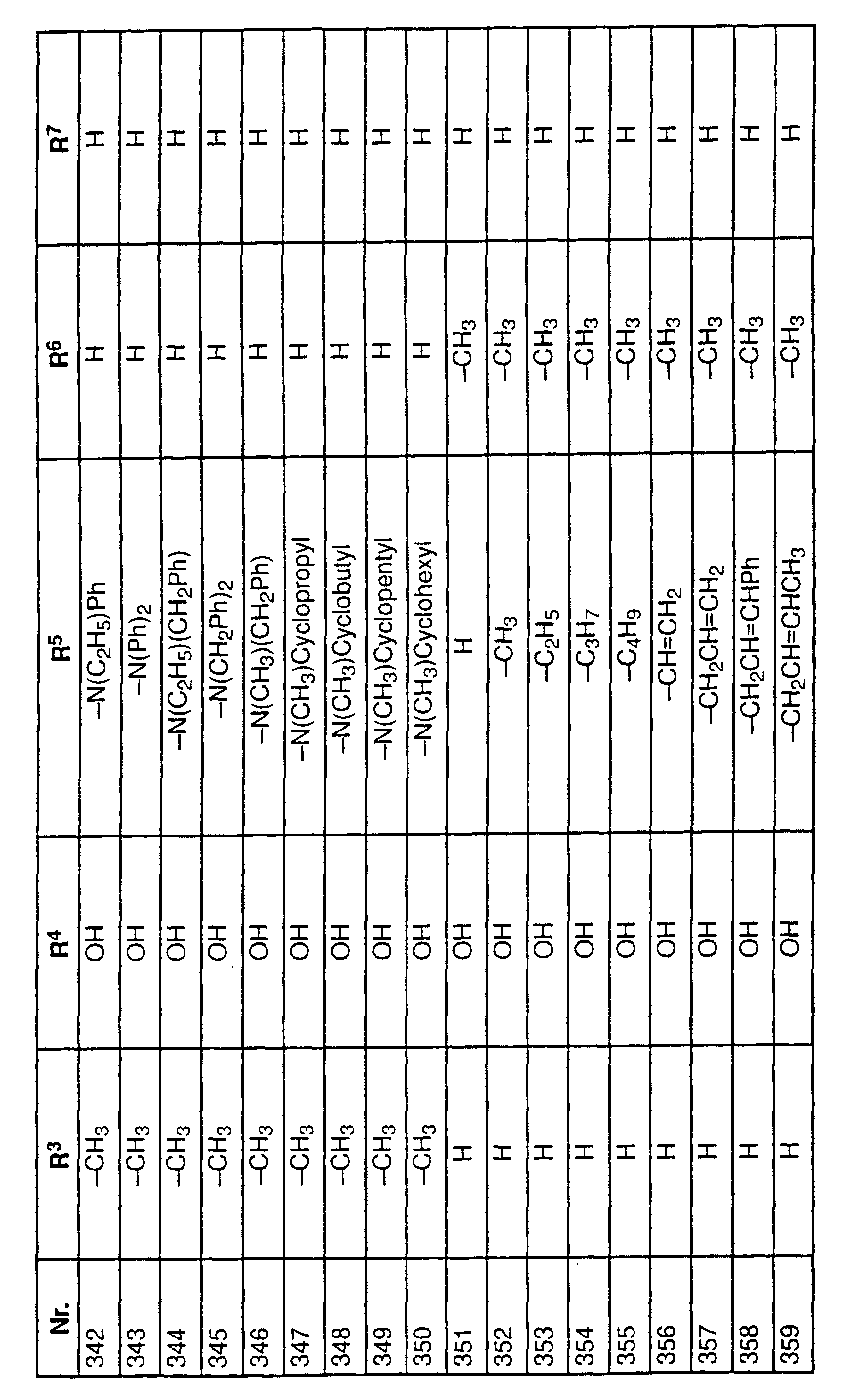

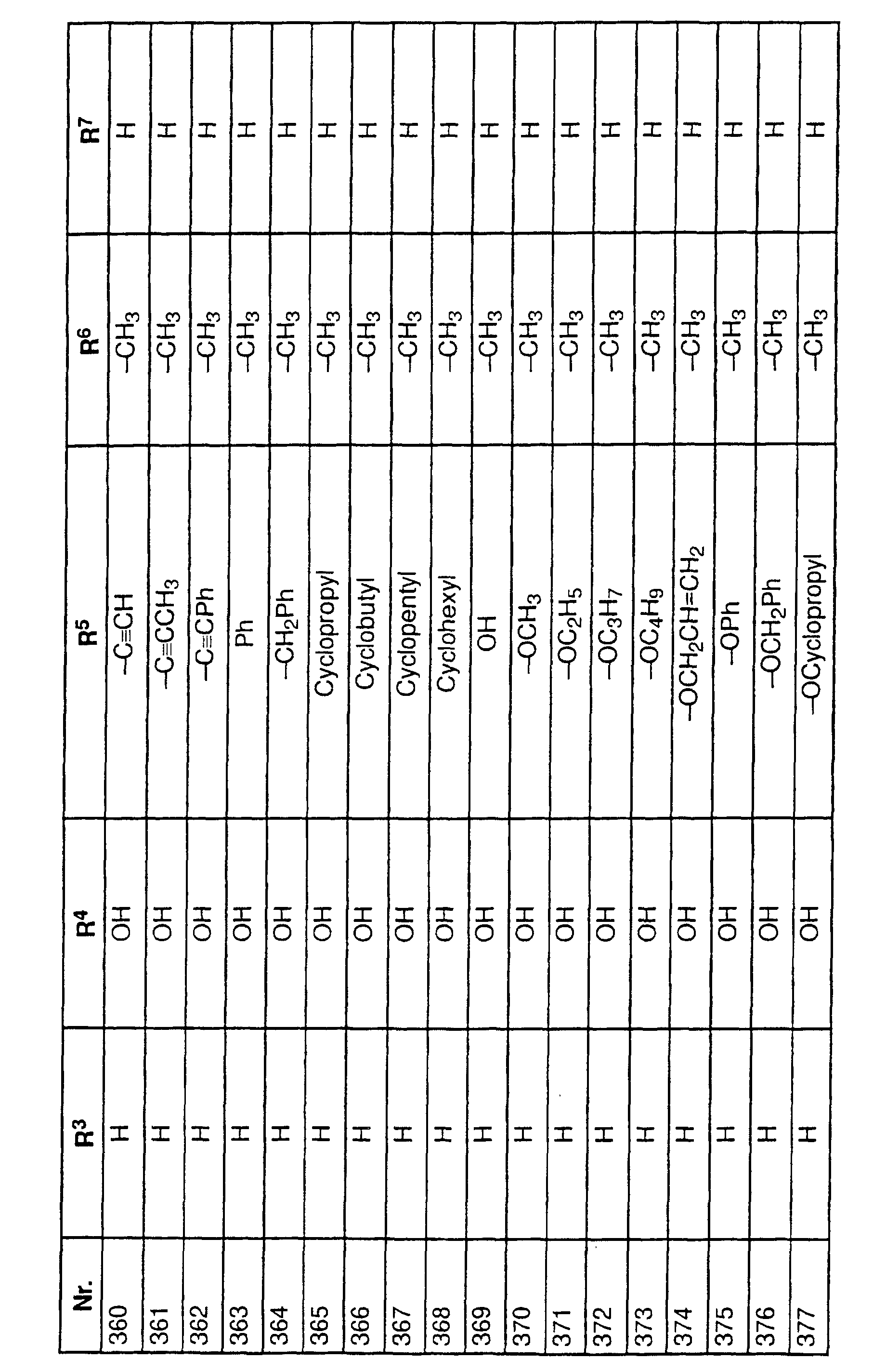

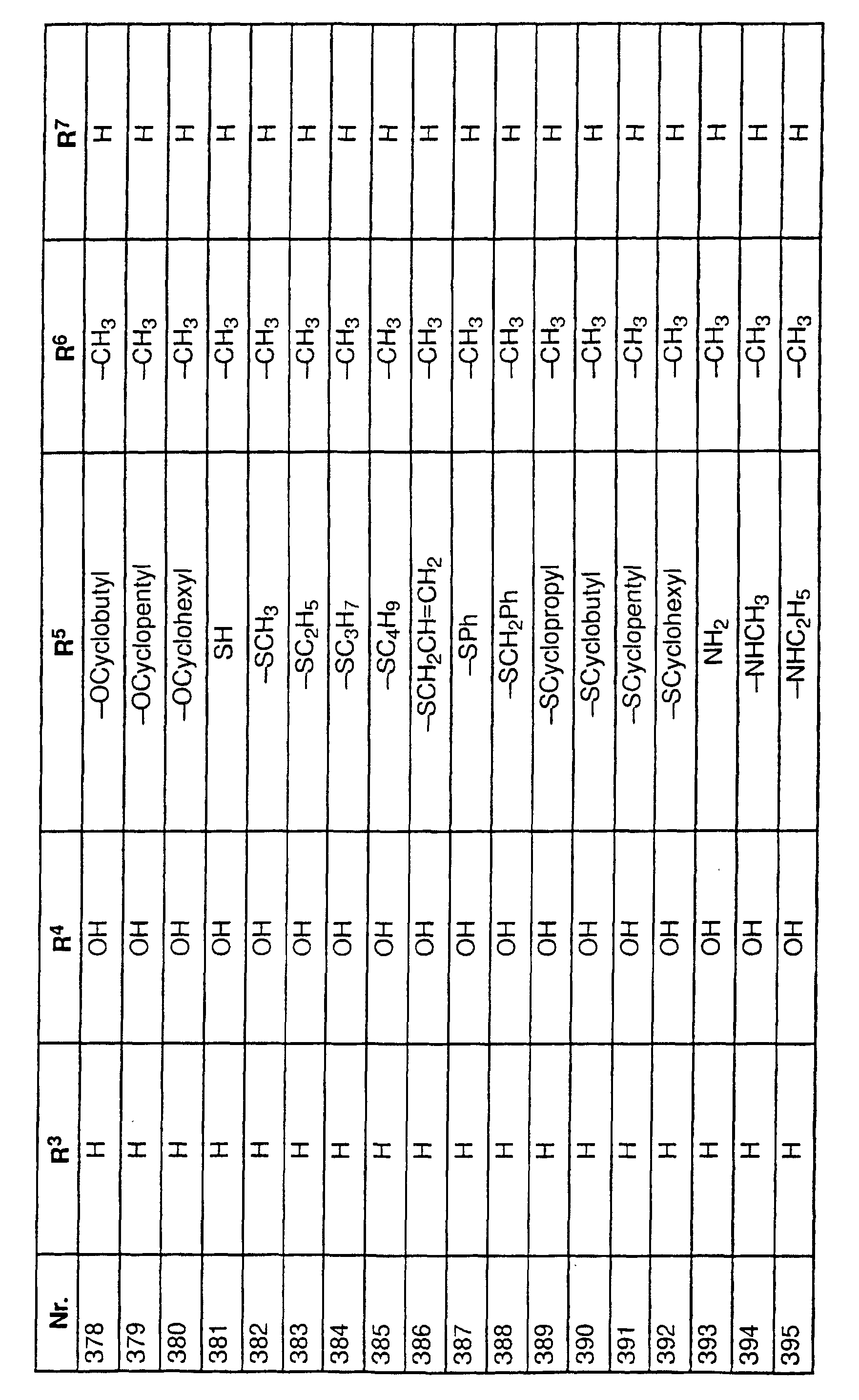

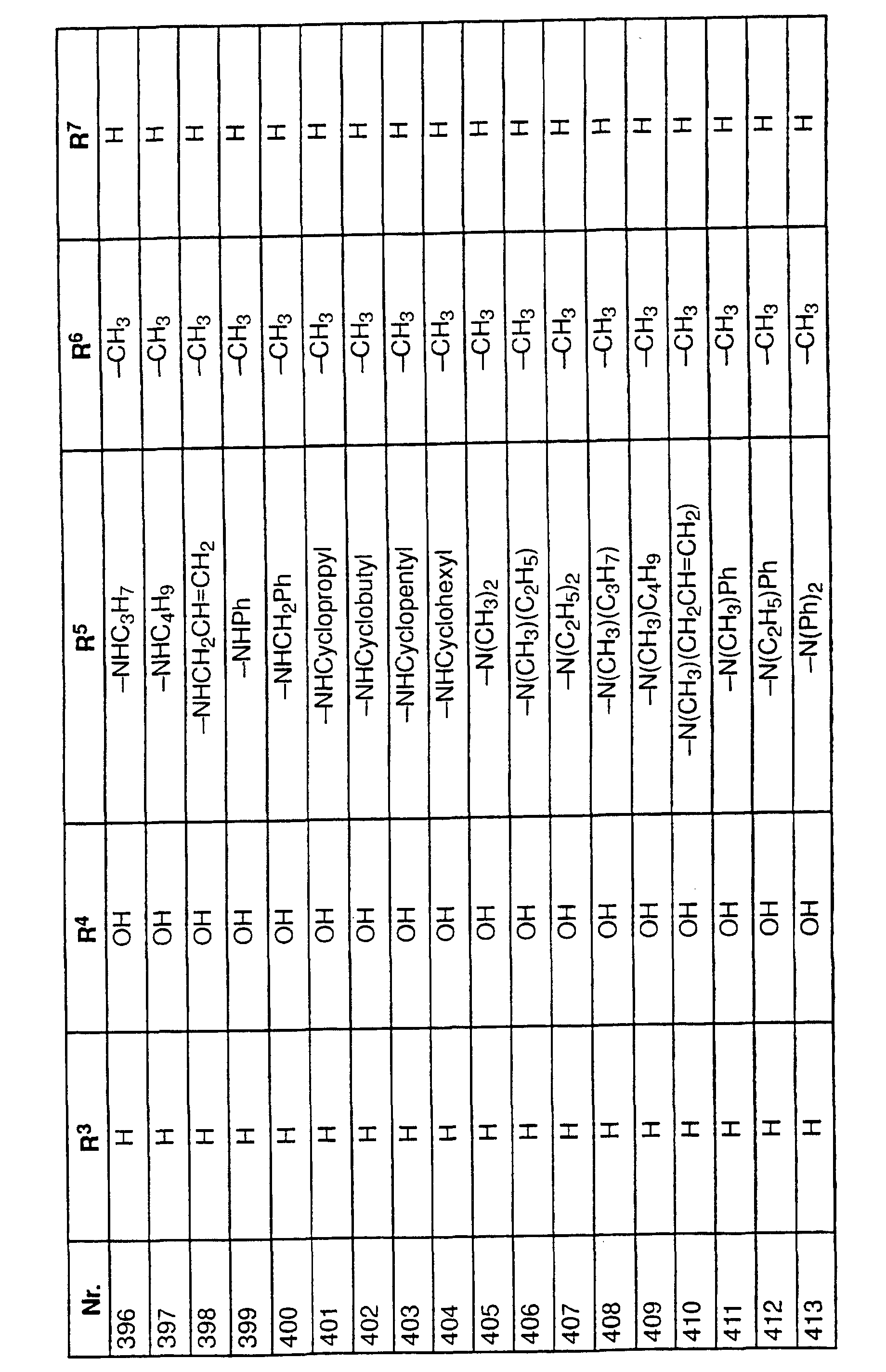

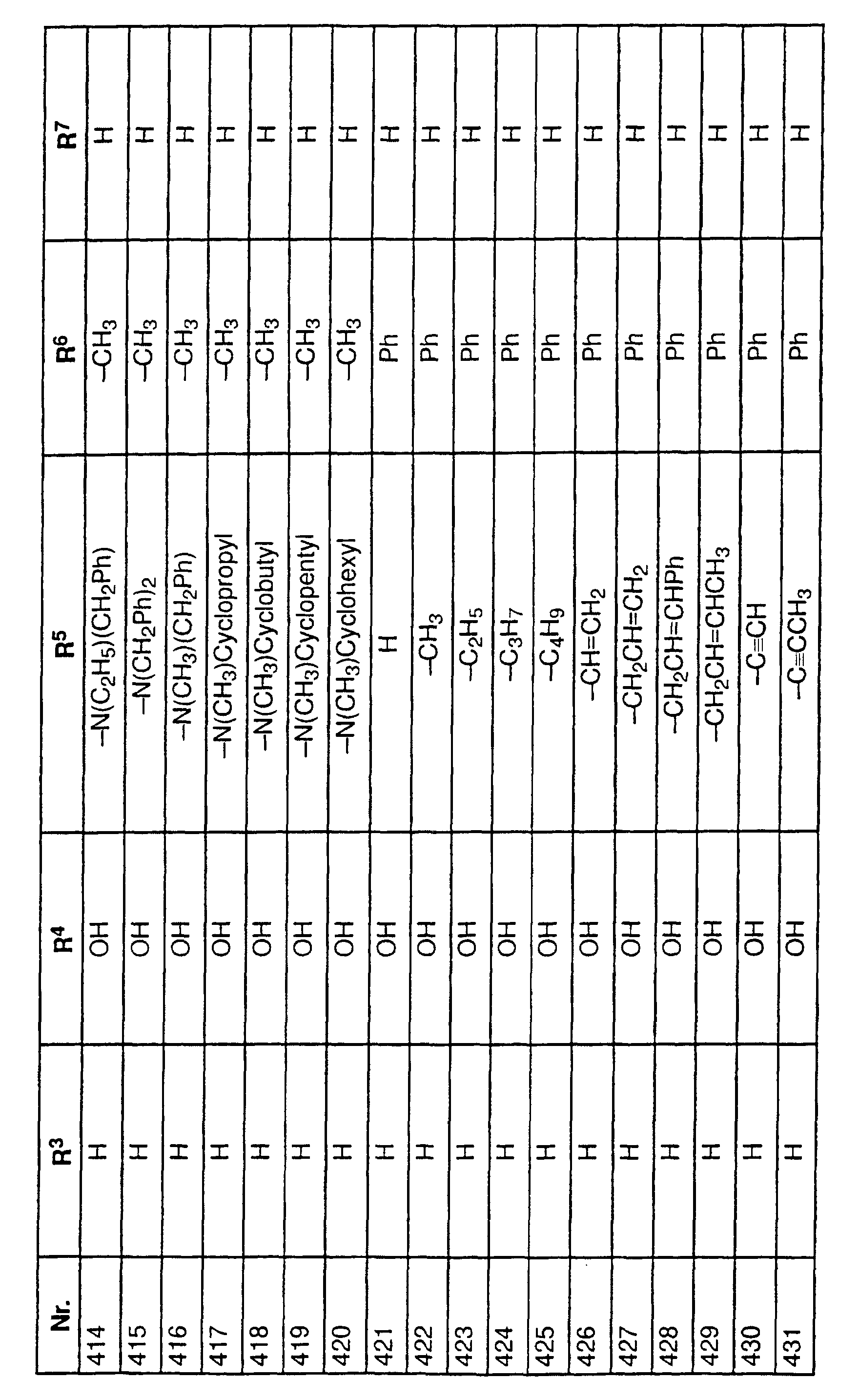

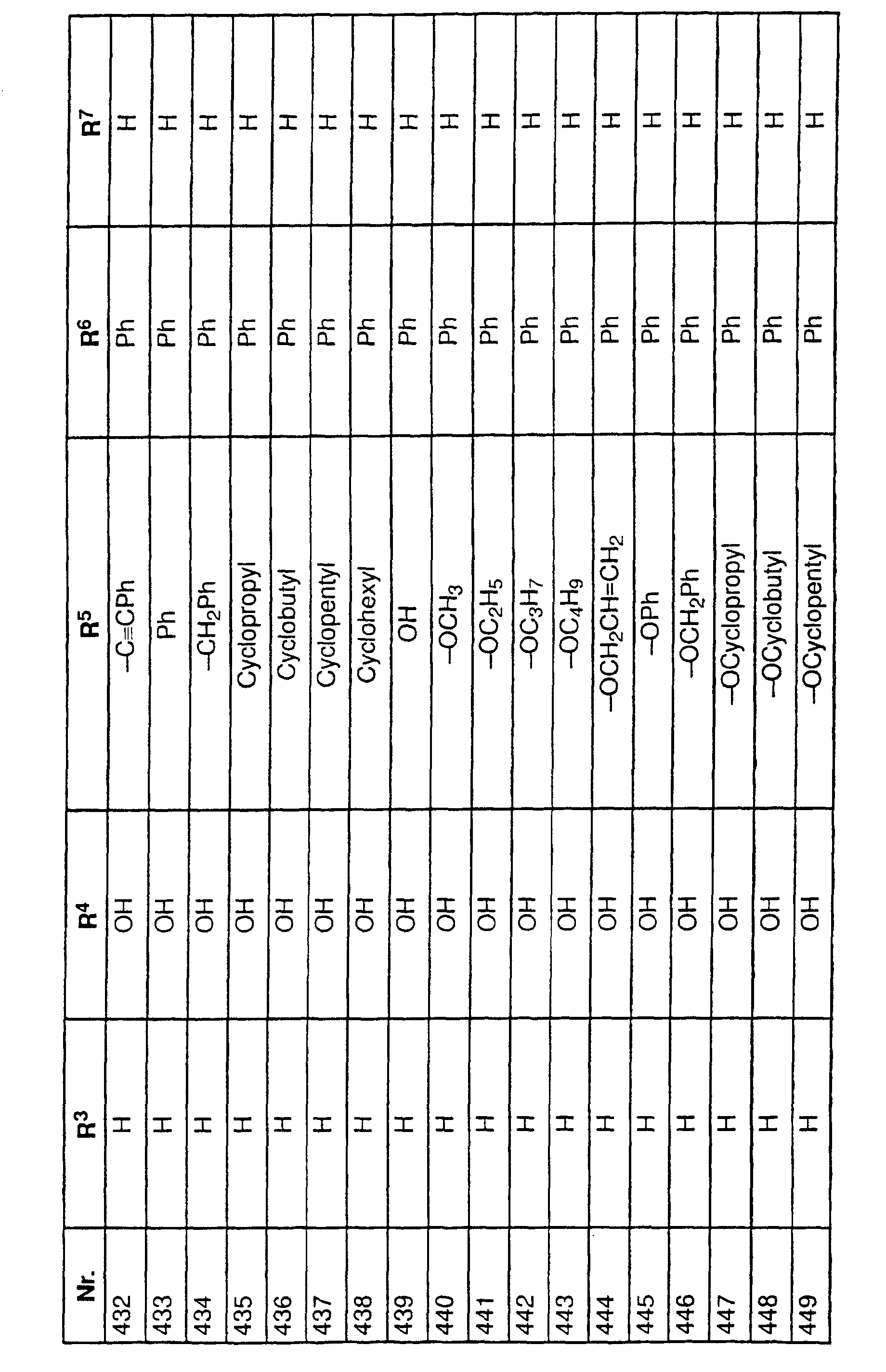

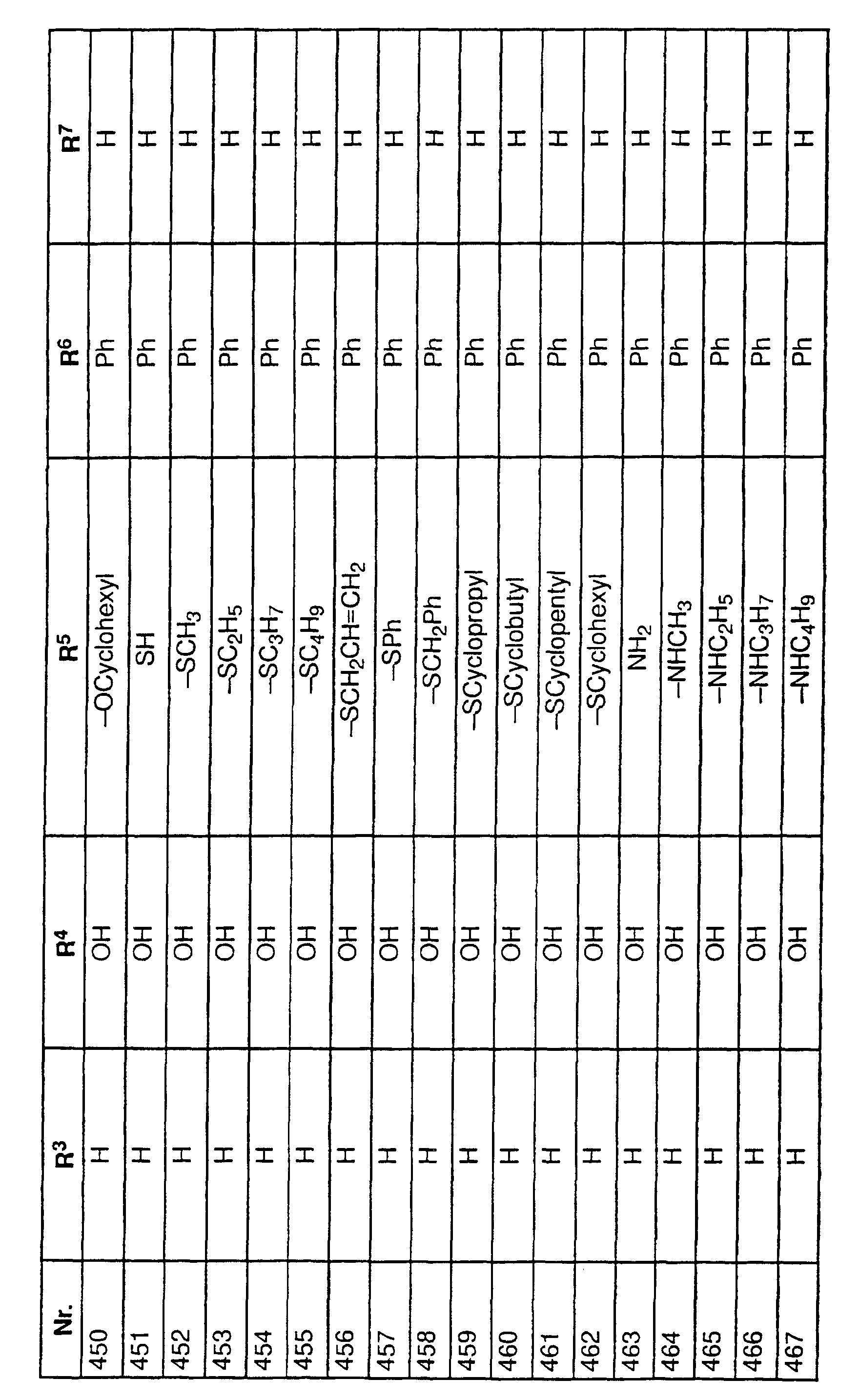

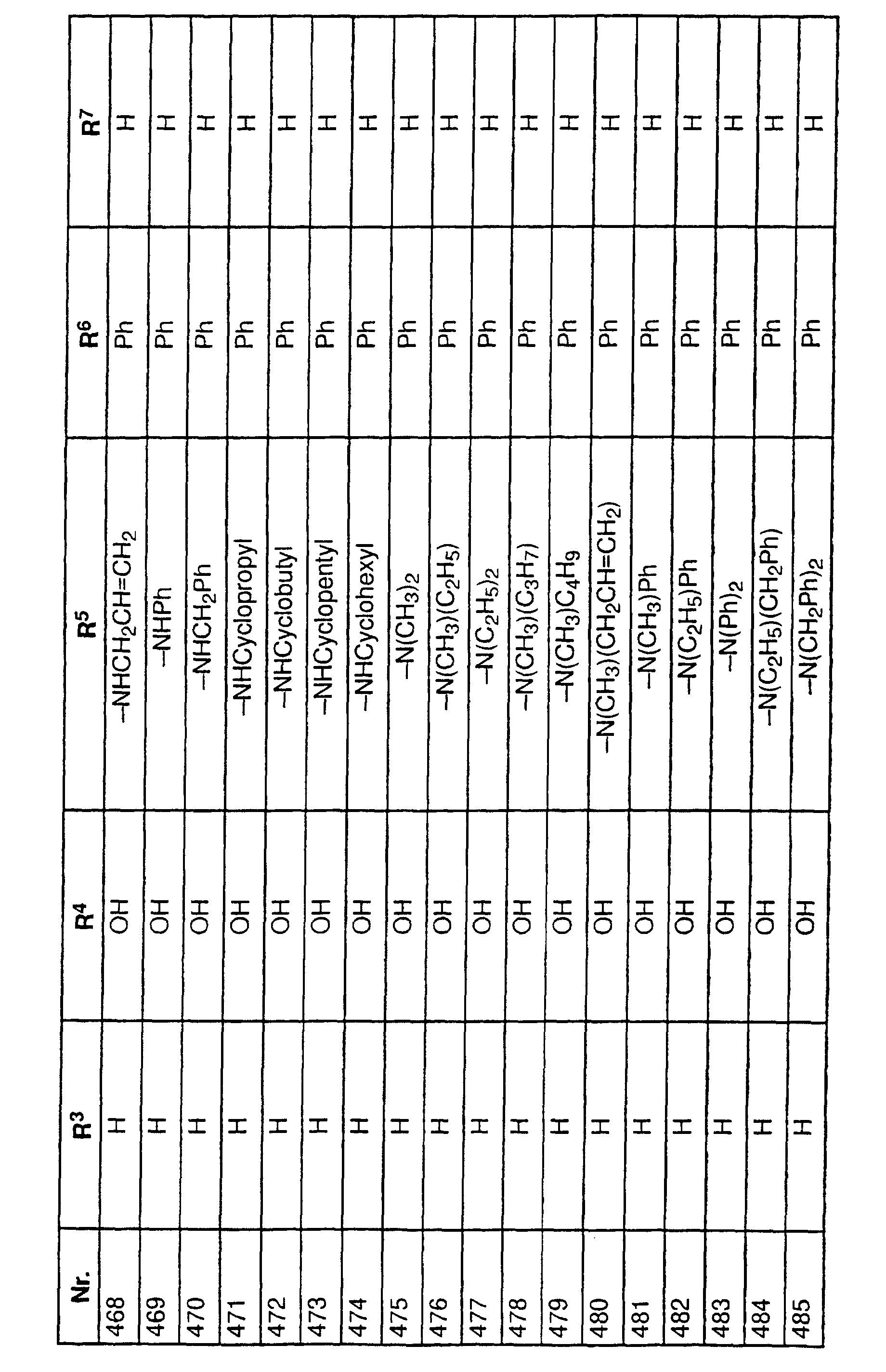

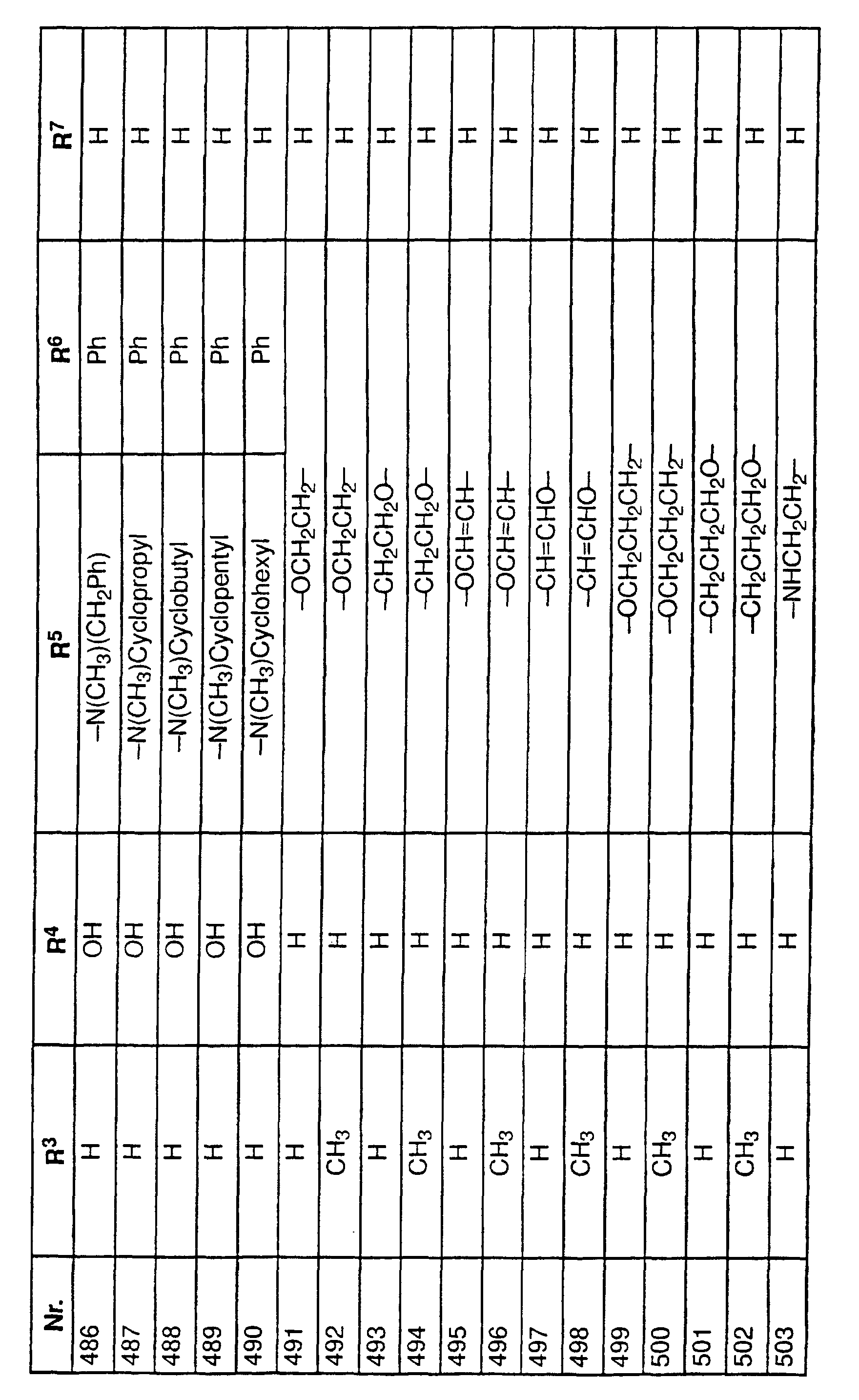

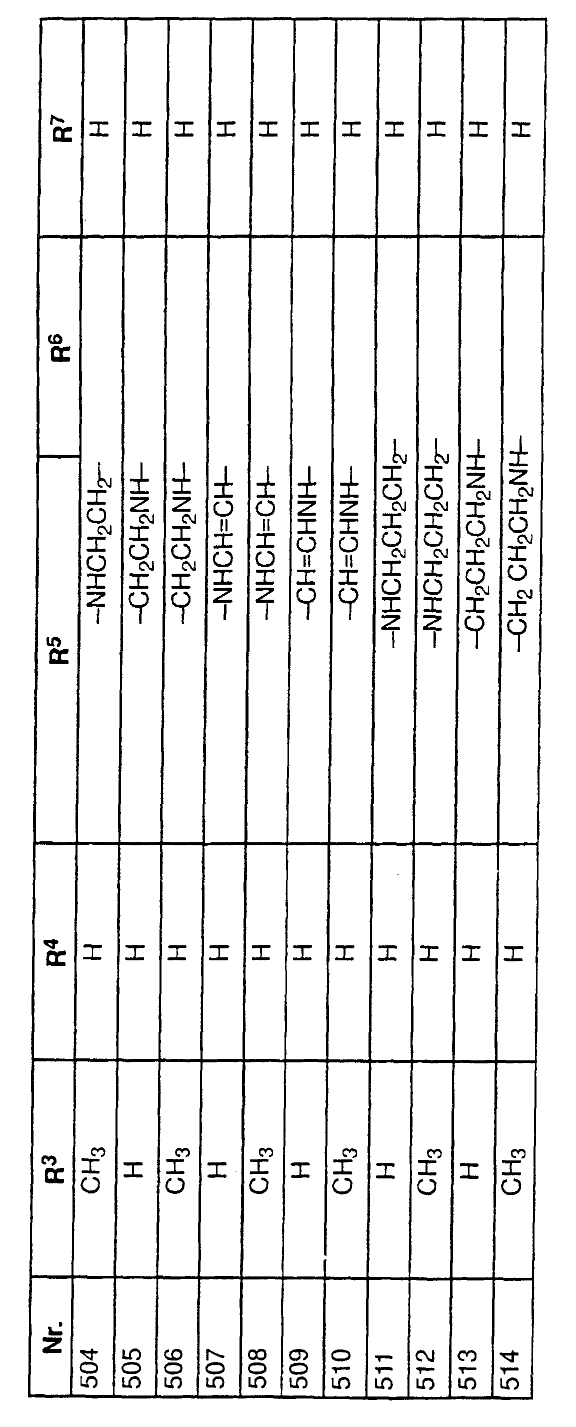

Die folgenden Tabellen 1-144 basieren auf den 4-Benzoyl-pyrazolen der Formel Ib.

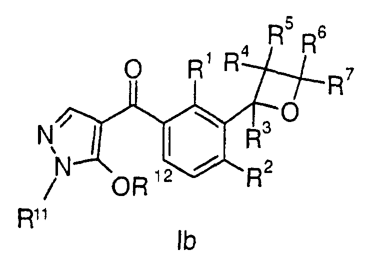

### Tabelle 1: Verbindungen 1.1-1.514

Verbindungen der allgemeinen Formel Ib, in der R¹ Cl, R² Methylsulfonyl, R¹¹ Methyl und R¹² Wasserstoff bedeutet und die Substituenten R³-R⁷ für jede einzelne Verbindung jeweils einer Zeile der Tabelle A entsprechen.

### Tabelle 2: Verbindungen 2.1-2.514

Verbindungen der allgemeinen Formel Ib, in der R¹ Cl, R² Methylsulfonyl, R¹¹ Ethyl, R¹² Wasserstoff bedeutet und die Substituenten R³-R⁷ für jede einzelne Verbindung jeweils einer Zeile der Tabelle A entsprechen.

### Tabelle 3: Verbindungen 3.1-3.514

Verbindungen der allgemeinen Formel Ib, in der R¹ Cl, R² Methylsulfonyl, R¹¹ n-Propyl, R¹² Wasserstoff bedeutet und die Substituenten R³-R⁷ für jede einzelne Verbindung jeweils einer Zeile der Tabelle A entsprechen.

### Tabelle 4: Verbindungen 4.1-4.514

Verbindungen der allgemeinen Formel Ib, in der R¹ Cl, R² Methylsulfonyl, R¹¹ Methyl, R¹² Methyl bedeutet und die Substituenten R³-R⁷ für jede einzelne Verbindung jeweils einer Zeile der Tabelle A entsprechen.

### Tabelle 5: Verbindungen 5.1-5.514

Verbindungen der allgemeinen Formel Ib, in der R¹ Cl, R² Methylsulfonyl, R¹¹ Ethyl, R¹² Methyl bedeutet und die Substituenten R³-R⁷ für jede einzelne Verbindung jeweils einer Zeile der Tabelle A entsprechen.

### Tabelle 6: Verbindungen 6.1-6.514

Verbindungen der allgemeinen Formel Ib, in der R¹ Cl, R² Methylsulfonyl, R¹¹ n-Propyl, R¹² Methyl bedeutet und die Substituenten R³-R⁷ für jede einzelne Verbindung jeweils einer Zeile der Tabelle A entsprechen.

### Tabelle 7: Verbindungen 7.1-7.514

Verbindungen der allgemeinen Formel Ib, in der R¹ Cl, R² Methylsulfonyl, R¹¹ Methyl, R¹² Ethyl bedeutet und die Substituenten R³-R⁷ für jede einzelne Verbindung jeweils einer Zeile der Tabelle A entsprechen.

### Tabelle 8: Verbindungen 8.1-8.514

Verbindungen der allgemeinen Formel Ib, in der R¹ Cl, R² Methylsulfonyl, R¹¹ Ethyl, R¹² Ethyl bedeutet und die Substituenten R³-R⁷ für jede einzelne Verbindung jeweils einer Zeile der Tabelle A entsprechen.

### Tabelle 9: Verbindungen 9.1-9.514

Verbindungen der allgemeinen Formel Ib, in der R¹ Cl, R² Methylsulfonyl, R¹¹ n-Propyl, R¹² Ethyl bedeutet und die Substituenten R³-R⁷ für jede einzelne Verbindung jeweils einer Zeile der Tabelle A entsprechen.

### Tabelle 10: Verbindungen 10.1-10.514

Verbindungen der allgemeinen Formel Ib, in der R¹ Cl, R² Methylsulfonyl, R¹¹ Methyl, R¹² Methylcarbonyl bedeutet und die Substituenten R³-R⁷ für jede einzelne Verbindung jeweils einer Zeile der Tabelle A entsprechen.

### Tabelle 11: Verbindungen 11.1-11.514

Verbindungen der allgemeinen Formel Ib, in der R² Cl, R² Methylsulfonyl, R¹¹ Ethyl, R¹² Methylcarbonyl bedeutet und die Substituenten R³-R⁷ für jede einzelne Verbindung jeweils einer Zeile der Tabelle A entsprechen.

### Tabelle 12: Verbindungen 12.1-12.514

Verbindungen der allgemeinen Formel Ib, in der R¹ Cl, R² Methylsulfonyl, R¹¹ n-Propyl, R¹² Methylcarbonyl bedeutet und die Substituenten R³-R⁷ für jede einzelne Verbindung jeweils einer Zeile der Tabelle A entsprechen.

### Tabelle 13: Verbindungen 13.1-13.514

Verbindungen der allgemeinen Formel Ib, in der R¹ Cl, R² Methylsulfonyl, R¹¹ Methyl, R¹² Ethylcarbonyl bedeutet und die Substituenten R³-R⁷ für jede einzelne Verbindung jeweils einer Zeile der Tabelle A entsprechen.

### Tabelle 14: Verbindungen 14.1-14.514

Verbindungen der allgemeinen Formel Ib, in der R¹ Cl, R² Methylsulfonyl, R¹¹ Ethyl, R¹² Ethylcarbonyl bedeutet und die Substituenten R³-R⁷ für jede einzelne Verbindung jeweils einer Zeile der Tabelle A entsprechen.

### Tabelle 15: Verbindungen 15.1-15.514

Verbindungen der allgemeinen Formel Ib, in der R¹ Cl, R² Methylsulfonyl, R¹¹ n-Propyl, R¹² Ethylcarbonyl bedeutet und die Substituenten R³-R⁷ für jede einzelne Verbindung jeweils einer Zeile der Tabelle A entsprechen.

### Tabelle 16: Verbindungen 16.1-16.514

Verbindungen der allgemeinen Formel Ib, in der R¹ Cl, R² Methylsulfonyl, R¹¹ Methyl, R¹² Methylsulfonyl bedeutet und die Substituenten R³-R⁷ für jede einzelne Verbindung jeweils einer Zeile der Tabelle A entsprechen.

### Tabelle 17: Verbindungen 17.1-17.514

Verbindungen der allgemeinen Formel Ib, in der R¹ Cl, R² Methylsulfonyl, R¹¹ Ethyl, R¹² Methylsulfonyl bedeutet und die Substituenten R³-R⁷ für jede einzelne Verbindung jeweils einer Zeile der Tabelle A entsprechen.

### Tabelle 18: Verbindungen 18.1-18.514

Verbindungen der allgemeinen Formel Ib, in der R¹ Cl, R² Methylsulfonyl, R¹¹ n-Propyl, R¹² Methylsulfonyl bedeutet und die Substituenten R³-R⁷ für jede einzelne Verbindung jeweils einer Zeile der Tabelle A entsprechen.

### Tabelle 19: Verbindungen 19.1-19.514

Verbindungen der allgemeinen Formel Ib, in der R¹ Cl, R² Methylsulfonyl, R¹¹ Methyl, R¹² Ethylsulfonyl bedeutet und die Substituenten R³-R⁷ für jede einzelne Verbindung jeweils einer Zeile der Tabelle A entsprechen.

### Tabelle 20: Verbindungen 20.1-20.514

Verbindungen der allgemeinen Formel Ib, in der R¹ Cl, R² Methylsulfonyl, R¹¹ Ethyl, R¹² Ethylsulfonyl bedeutet und die Substituenten R³-R⁷ für jede einzelne Verbindung jeweils einer Zeile der Tabelle A entsprechen.

### Tabelle 21: Verbindungen 21.1-21.514

Verbindungen der allgemeinen Formel Ib, in der R¹ Cl, R² Methylsulfonyl, R¹¹ n-Propyl, R¹² Ethylsulfonyl bedeutet und die Substituenten R³-R⁷ für jede einzelne Verbindung jeweils einer Zeile der Tabelle A entsprechen.

### Tabelle 22: Verbindungen 22.1-22.514

Verbindungen der allgemeinen Formel Ib, in der R¹ Cl, R² Methylsulfonyl, R¹¹ Methyl, R¹² 4-Methylphenylsulfonyl bedeutet und die Substituenten R³-R⁷ für jede einzelne Verbindung jeweils einer Zeile der Tabelle A entsprechen.

### Tabelle 23: Verbindungen 23.1-23.514

Verbindungen der allgemeinen Formel Ib, in der R¹ Cl, R² Methylsulfonyl, R¹¹ Ethyl, R¹² 4-Methylphenylsulfonyl bedeutet und die Substituenten R³-R⁷ für jede einzelne Verbindung jeweils einer Zeile der Tabelle A entsprechen.

### Tabelle 24: Verbindungen 24.1-24.514

Verbindungen der allgemeinen Formel Ib, in der R¹ Cl, R² Methylsulfonyl, R¹¹ n-Propyl, R¹² 4-Methylphenylsulfonyl bedeutet und die Substituenten R³-R⁷ für jede einzelne Verbindung jeweils einer Zeile der Tabelle A entsprechen.

### Tabelle 25: Verbindungen 25.1-25.514

Verbindungen der allgemeinen Formel Ib, in der R¹ Cl, R² Cl, R¹¹ Methyl und R¹² Wasserstoff bedeutet und die Substituenten R³-R⁷ für jede einzelne Verbindung jeweils einer Zeile der Tabelle A entsprechen.

### Tabelle 26: Verbindungen 26.1-26.514

Verbindungen der allgemeinen Formel Ib, in der R¹ Cl, R² Cl, R¹¹ Ethyl, R¹² Wasserstoff bedeutet und die Substituenten R³-R⁷ für jede einzelne Verbindung jeweils einer Zeile der Tabelle A entsprechen.

### Tabelle 27: Verbindungen 27.1-27.514

Verbindungen der allgemeinen Formel Ib, in der R¹ Cl, R² Cl, R¹¹ n-Propyl, R¹² Wasserstoff bedeutet und die Substituenten R³-R⁷ für jede einzelne Verbindung jeweils einer Zeile der Tabelle A entsprechen.

### Tabelle 28: Verbindungen 28.1-28.514

Verbindungen der allgemeinen Formel Ib, in der R¹ Cl, R² Cl, R¹¹ Methyl, R¹² Methyl bedeutet und die Substituenten R³-R⁷ für jede einzelne Verbindung jeweils einer Zeile der Tabelle A entsprechen.

### Tabelle 29: Verbindungen 29.1-29.514

Verbindungen der allgemeinen Formel Ib, in der R¹ Cl, R² Cl, R¹¹ Ethyl, R¹² Methyl bedeutet und die Substituenten R³-R⁷ für jede einzelne Verbindung jeweils einer Zeile der Tabelle A entsprechen.

### Tabelle 30: Verbindungen 30.1-30.514

Verbindungen der allgemeinen Formel Ib, in der R¹ Cl, R² Cl, R¹¹ n-Propyl, R¹² Methyl bedeutet und die Substituenten R³-R⁷ für jede einzelne Verbindung jeweils einer Zeile der Tabelle A entsprechen.

### Tabelle 31: Verbindungen 31.1-31.514

Verbindungen der allgemeinen Formel Ib, in der R¹ Cl, R² Cl, R¹¹ Methyl, R¹² Ethyl bedeutet und die Substituenten R³-R⁷ für jede einzelne Verbindung jeweils einer Zeile der Tabelle A entsprechen.

### Tabelle 32: Verbindungen 32.1-32.514

Verbindungen der allgemeinen Formel Ib, in der R¹ Cl, R² Cl, R¹¹ Ethyl, R¹² Ethyl bedeutet und die Substituenten R³-R⁷ für jede einzelne Verbindung jeweils einer Zeile der Tabelle A entsprechen.

### Tabelle 33: Verbindungen 33.1-33.514

Verbindungen der allgemeinen Formel Ib, in der R¹ Cl, R² Cl, R¹¹ n-Propyl, R¹² Ethyl bedeutet und die Substituenten R³-R⁷ für jede einzelne Verbindung jeweils einer Zeile der Tabelle A entsprechen.

### Tabelle 34: Verbindungen 34.1-34.514

Verbindungen der allgemeinen Formel Ib, in der R¹ Cl, R² Cl, R¹¹ Methyl, R¹² Methylcarbonyl bedeutet und die Substituenten R³-R⁷ für jede einzelne Verbindung jeweils einer Zeile der Tabelle A entsprechen.

### Tabelle 35: Verbindungen 35.1-35.514

Verbindungen der allgemeinen Formel Ib, in der R¹ Cl, R² Cl, R¹¹ Ethyl, R¹² Methylcarbonyl bedeutet und die Substituenten R³-R⁷ für jede einzelne Verbindung jeweils einer Zeile der Tabelle A entsprechen.

### Tabelle 36: Verbindungen 36.1-36.514

Verbindungen der allgemeinen Formel Ib, in der R¹ Cl, R² Cl, R¹¹ n-Propyl, R¹² Methylcarbonyl bedeutet und die Substituenten R³-R⁷ für jede einzelne Verbindung jeweils einer Zeile der Tabelle A entsprechen.

### Tabelle 37: Verbindungen 37.1-37.514

Verbindungen der allgemeinen Formel Ib, in der R¹ Cl, R² Cl, R¹¹ Methyl, R¹² Ethylcarbonyl bedeutet und die Substituenten R³-R⁷ für jede einzelne Verbindung jeweils einer Zeile der Tabelle A entsprechen.

### Tabelle 38: Verbindungen 38.1-38.514

Verbindungen der allgemeinen Formel Ib, in der R¹ Cl, R² Cl, R¹¹ Ethyl, R¹² Ethylcarbonyl bedeutet und die Substituenten R³-R⁷ für jede einzelne Verbindung jeweils einer Zeile der Tabelle A entsprechen.

### Tabelle 39: Verbindungen 39.1-39.514

Verbindungen der allgemeinen Formel Ib, in der R¹ Cl, R² Cl, R¹¹ n-Propyl, R¹² Ethylcarbonyl bedeutet und die Substituenten R³-R⁷ für jede einzelne Verbindung jeweils einer Zeile der Tabelle A entsprechen.

### Tabelle 40: Verbindungen 40.1-40.514

Verbindungen der allgemeinen Formel Ib, in der R¹ Cl, R² Cl, R¹¹ Methyl, R¹² Methylsulfonyl bedeutet und die Substituenten R³-R⁷ für jede einzelne Verbindung jeweils einer Zeile der Tabelle A entsprechen.

### Tabelle 41: Verbindungen 41.1-41.514

Verbindungen der allgemeinen Formel Ib, in der R¹ Cl, R² Cl, R¹¹ Ethyl, R¹² Methylsulfonyl bedeutet und die Substituenten R³-R⁷ für jede einzelne Verbindung jeweils einer Zeile der Tabelle A entsprechen.

### Tabelle 42: Verbindungen 42.1-42.514

Verbindungen der allgemeinen Formel Ib, in der R¹ Cl, R² Cl, R¹¹ n-Propyl, R¹² Methylsulfonyl bedeutet und die Substituenten R³-R⁷ für jede einzelne Verbindung jeweils einer Zeile der Tabelle A entsprechen.

### Tabelle 43: Verbindungen 43.1-43.514

Verbindungen der allgemeinen Formel Ib, in der R¹ Cl, R² Cl, R¹¹ Methyl, R¹² Ethylsulfonyl bedeutet und die Substituenten R³-R⁷ für jede einzelne Verbindung jeweils einer Zeile der Tabelle A entsprechen.

### Tabelle 44: Verbindungen 44.1-44.514

Verbindungen der allgemeinen Formel Ib, in der R¹ Cl, R² Cl, R¹¹ Ethyl, R¹² Ethylsulfonyl bedeutet und die Substituenten R³-R⁷ für jede einzelne Verbindung jeweils einer Zeile der Tabelle A entsprechen.

### Tabelle 45: Verbindungen 45.1-45.514

Verbindungen der allgemeinen Formel Ib, in der R¹ Cl, R² Cl, R¹¹ n-Propyl, R¹² Ethylsulfonyl bedeutet und die Substituenten R³-R⁷ für jede einzelne Verbindung jeweils einer Zeile der Tabelle A entsprechen.

### Tabelle 46: Verbindungen 46.1-46.514

Verbindungen der allgemeinen Formel Ib, in der R¹ Cl, R² Cl, R¹¹ Methyl, R¹² 4-Methylphenylsulfonyl bedeutet und die Substituenten R³-R⁷ für jede einzelne Verbindung jeweils einer Zeile der Tabelle A entsprechen.

### Tabelle 47: Verbindungen 47.1-47.514

Verbindungen der allgemeinen Formel Ib, in der R¹ Cl, R² Cl, R¹¹ Ethyl, R¹² 4-Methylphenylsulfonyl bedeutet und die Substituenten R³-R⁷ für jede einzelne Verbindung jeweils einer Zeile der Tabelle A entsprechen.

### Tabelle 48: Verbindungen 48.1-48.514

Verbindungen der allgemeinen Formel Ib, in der R¹ Cl, R² Cl, R¹¹ n-Propyl, R¹² 4-Methylphenylsulfonyl bedeutet und die Substituenten R³-R⁷ für jede einzelne Verbindung jeweils einer Zeile der Tabelle A entsprechen.

### Tabelle 49: Verbindungen 49.1-49.514

Verbindungen der allgemeinen Formel Ib, in der R¹ Cl, R² Trifluormethyl, R¹¹ Methyl und R¹² Wasserstoff bedeutet und die Substituenten R³-R⁷ für jede einzelne Verbindung jeweils einer Zeile der Tabelle A entsprechen.

### Tabelle 50: Verbindungen 50.1-50.514

Verbindungen der allgemeinen Formel Ib, in der R¹ Cl, R² Trifluormethyl, R¹¹ Ethyl, R¹² Wasserstoff bedeutet und die Substituenten R³-R⁷ für jede einzelne Verbindung jeweils einer Zeile der Tabelle A entsprechen.

### Tabelle 51: Verbindungen 51.1-51.514

Verbindungen der allgemeinen Formel Ib, in der R¹ Cl, R² Trifluormethyl, R¹¹ n-Propyl, R¹² Wasserstoff bedeutet und die Substituenten R³-R⁷ für jede einzelne Verbindung jeweils einer Zeile der Tabelle A entsprechen.

### Tabelle 52: Verbindungen 52.1-52.514

Verbindungen der allgemeinen Formel Ib, in der R¹ Cl, R² Trifluormethyl, R¹¹ Methyl, R¹² Methyl bedeutet und die Substituenten R³-R⁷ für jede einzelne Verbindung jeweils einer Zeile der Tabelle A entsprechen.

### Tabelle 53: Verbindungen 53.1-53.514

Verbindungen der allgemeinen Formel Ib, in der R¹ Cl, R² Trifluormethyl, R¹¹ Ethyl, R¹² Methyl bedeutet und die Substituenten R³-R⁷ für jede einzelne Verbindung jeweils einer Zeile der Tabelle A entsprechen.

### Tabelle 54: Verbindungen 54.1-54.514

Verbindungen der allgemeinen Formel Ib, in der R¹ Cl, R² Trifluormethyl, R¹¹ n-Propyl, R¹² Methyl bedeutet und die Substituenten R³-R⁷ für jede einzelne Verbindung jeweils einer Zeile der Tabelle A entsprechen.

### Tabelle 55: Verbindungen 55.1-55.514

Verbindungen der allgemeinen Formel Ib, in der R¹ Cl, R² Trifluormethyl, R¹¹ Methyl, R¹² Ethyl bedeutet und die Substituenten R³-R⁷ für jede einzelne Verbindung jeweils einer Zeile der Tabelle A entsprechen.

### Tabelle 56: Verbindungen 56.1-56.514

Verbindungen der allgemeinen Formel Ib, in der R¹ Cl, R² Trifluormethyl, R¹¹ Ethyl, R¹² Ethyl bedeutet und die Substituenten R³-R⁷ für jede einzelne Verbindung jeweils einer Zeile der Tabelle A entsprechen.

### Tabelle 57: Verbindungen 57.1-57.514

Verbindungen der allgemeinen Formel Ib, in der R¹ Cl, R² Trifluormethyl, R¹¹ n-Propyl, R¹² Ethyl bedeutet und die Substituenten R³-R⁷ für jede einzelne Verbindung jeweils einer Zeile der Tabelle A entsprechen.

### Tabelle 58: Verbindungen 58.1-58.514

Verbindungen der allgemeinen Formel Ib, in der R¹ Cl, R² Trifluormethyl, R¹¹ Methyl, R¹² Methylcarbonyl bedeutet und die Substituenten R³-R⁷ für jede einzelne Verbindung jeweils einer Zeile der Tabelle A entsprechen.

### Tabelle 59: Verbindungen 59.1-59.514

Verbindungen der allgemeinen Formel Ib, in der R¹ Cl, R² Trifluormethyl, R¹¹ Ethyl, R¹² Methylcarbonyl bedeutet und die Substituenten R³-R⁷ für jede einzelne Verbindung jeweils einer Zeile der Tabelle A entsprechen.

### Tabelle 60: Verbindungen 60.1-60.514

Verbindungen der allgemeinen Formel Ib, in der R¹ Cl, R² Trifluormethyl, R¹¹ n-Propyl, R¹² Methylcarbonyl bedeutet und die Substituenten R³-R⁷ für jede einzelne Verbindung jeweils einer Zeile der Tabelle A entsprechen.

### Tabelle 61: Verbindungen 61.1-61.514

Verbindungen der allgemeinen Formel Ib, in der R¹ Cl, R² Trifluormethyl, R¹¹ Methyl, R¹² Ethylcarbonyl bedeutet und die Substituenten R³-R⁷ für jede einzelne Verbindung jeweils einer Zeile der Tabelle A entsprechen.

### Tabelle 62: Verbindungen 62.1-62.514

Verbindungen der allgemeinen Formel Ib, in der R¹ Cl, R² Trifluormethyl, R¹¹ Ethyl, R¹² Ethylcarbonyl bedeutet und die Substituenten R³-R⁷ für jede einzelne Verbindung jeweils einer Zeile der Tabelle A entsprechen.

### Tabelle 63: Verbindungen 63.1-63.514

Verbindungen der allgemeinen Formel Ib, in der R¹ Cl, R² Trifluormethyl, R¹¹ n-Propyl, R¹² Ethylcarbonyl bedeutet und die Substituenten R³-R⁷ für jede einzelne Verbindung jeweils einer Zeile der Tabelle A entsprechen.

### Tabelle 64: Verbindungen 64.1-64.514

Verbindungen der allgemeinen Formel Ib, in der R¹ Cl, R² Trifluormethyl, R¹¹ Methyl, R¹² Methylsulfonyl bedeutet und die Substituenten R³-R⁷ für jede einzelne Verbindung jeweils einer Zeile der Tabelle A entsprechen.

### Tabelle 65: Verbindungen 65.1-65.514

Verbindungen der allgemeinen Formel Ib, in der R¹ Cl, R² Trifluormethyl, R¹¹ Ethyl, R¹² Methylsulfonyl bedeutet und die Substituenten R³-R⁷ für jede einzelne Verbindung jeweils einer Zeile der Tabelle A entsprechen.

### Tabelle 66: Verbindungen 66.1-66.514

Verbindungen der allgemeinen Formel Ib, in der R¹ Cl, R² Trifluormethyl, R¹¹ n-Propyl, R¹² Methylsulfonyl bedeutet und die Substituenten R³-R⁷ für jede einzelne Verbindung jeweils einer Zeile der Tabelle A entsprechen.

### Tabelle 67: Verbindungen 67.1-67.514

Verbindungen der allgemeinen Formel Ib, in der R¹ Cl, R² Trifluormethyl, R¹¹ Methyl, R¹² Ethylsulfonyl bedeutet und die Substituenten R³-R⁷ für jede einzelne Verbindung jeweils einer Zeile der Tabelle A entsprechen.

### Tabelle 68: Verbindungen 68.1-68.514

Verbindungen der allgemeinen Formel Ib, in der R¹ Cl, R² Trifluormethyl, R¹¹ Ethyl, R¹² Ethylsulfonyl bedeutet und die Substituenten R³-R⁷ für jede einzelne Verbindung jeweils einer Zeile der Tabelle A entsprechen.

### Tabelle 69: Verbindungen 69.1-69.514

Verbindungen der allgemeinen Formel Ib, in der R¹ Cl, R² Trifluormethyl, R¹¹ n-Propyl, R¹² Ethylsulfonyl bedeutet und die Substituenten R³-R⁷ für jede einzelne Verbindung jeweils einer Zeile der Tabelle A entsprechen.

### Tabelle 70: Verbindungen 70.1-70.514

Verbindungen der allgemeinen Formel Ib, in der R¹ Cl, R² Trifluormethyl, R¹¹ Methyl, R¹² 4-Methylphenylsulfonyl bedeutet und die Substituenten R³-R⁷ für jede einzelne Verbindung jeweils einer Zeile der Tabelle A entsprechen.

### Tabelle 71: Verbindungen 71.1-71.514

Verbindungen der allgemeinen Formel Ib, in der R¹ Cl, R² Trifluormethyl, R¹¹ Ethyl, R¹² 4-Methylphenylsulfonyl bedeutet und die Substituenten R³-R⁷ für jede einzelne Verbindung jeweils einer Zeile der Tabelle A entsprechen.

### Tabelle 72: Verbindungen 72.1-72.514

Verbindungen der allgemeinen Formel Ib, in der R¹ Cl, R² Trifluormethyl, R¹¹ n-Propyl, R¹² 4-Methylphenylsulfonyl bedeutet und die Substituenten R³-R⁷ für jede einzelne Verbindung jeweils einer Zeile der Tabelle A entsprechen.

### Tabelle 73: Verbindungen 73.1-73.514

Verbindungen der allgemeinen Formel Ib, in der R¹ Methyl, R² Cl, R¹¹ Methyl und R¹² Wasserstoff bedeutet und die Substituenten R³-R⁷ für jede einzelne Verbindung jeweils einer Zeile der Tabelle A entsprechen.

### Tabelle 74: Verbindungen 74.1-74.514

Verbindungen der allgemeinen Formel Ib, in der R¹ Methyl, R² Cl, R¹¹ Ethyl, R¹² Wasserstoff bedeutet und die Substituenten R³-R⁷ für jede einzelne Verbindung jeweils einer Zeile der Tabelle A entsprechen.

### Tabelle 75: Verbindungen 75.1-75.514

Verbindungen der allgemeinen Formel Ib, in der R¹ Methyl, R² Cl, R¹¹ n-Propyl, R¹² Wasserstoff bedeutet und die Substituenten R³-R⁷ für jede einzelne Verbindung jeweils einer Zeile der Tabelle A entsprechen.

### Tabelle 76: Verbindungen 76.1-76.514

Verbindungen der allgemeinen Formel Ib, in der R¹ Methyl, R² Cl, R¹¹ Methyl, R¹² Methyl bedeutet und die Substituenten R³-R⁷ für jede einzelne Verbindung jeweils einer Zeile der Tabelle A entsprechen.

### Tabelle 77: Verbindungen 77.1-77.514

Verbindungen der allgemeinen Formel Ib, in der R¹ Methyl, R² Cl, R¹¹ Ethyl, R¹² Methyl bedeutet und die Substituenten R³-R⁷ für jede einzelne Verbindung jeweils einer Zeile der Tabelle A entsprechen.

### Tabelle 78: Verbindungen 78.1-78.514

Verbindungen der allgemeinen Formel Ib, in der R¹ Methyl, R² Cl, R¹¹ n-Propyl, R¹² Methyl bedeutet und die Substituenten R³-R⁷ für jede einzelne Verbindung jeweils einer Zeile der Tabelle A entsprechen.

### Tabelle 79: Verbindungen 79.1-79.514

Verbindungen der allgemeinen Formel Ib, in der R¹ Methyl, R² Cl, R¹¹ Methyl, R¹² Ethyl bedeutet und die Substituenten R³-R⁷ für jede einzelne Verbindung jeweils einer Zeile der Tabelle A entsprechen.

### Tabelle 80: Verbindungen 80.1-80.514

Verbindungen der allgemeinen Formel Ib, in der R¹ Methyl, R² Cl, R¹¹ Ethyl, R¹² Ethyl bedeutet und die Substituenten R³-R⁷ für jede einzelne Verbindung jeweils einer Zeile der Tabelle A entsprechen.

### Tabelle 81: Verbindungen 81.1-81.514

Verbindungen der allgemeinen Formel Ib, in der R¹ Methyl, R² Cl, R¹¹ n-Propyl, R¹² Ethyl bedeutet und die Substituenten R³-R⁷ für jede einzelne Verbindung jeweils einer Zeile der Tabelle A entsprechen.

### Tabelle 82: Verbindungen 82.1-82.514

Verbindungen der allgemeinen Formel Ib, in der R¹ Methyl, R² Cl, R¹¹ Methyl, R¹² Methylcarbonyl bedeutet und die Substituenten R³-R⁷ für jede einzelne Verbindung jeweils einer Zeile der Tabelle A entsprechen.

### Tabelle 83: Verbindungen 83.1-83.514

Verbindungen der allgemeinen Formel Ib, in der R¹ Methyl, R² Cl, R¹¹ Ethyl, R¹² Methylcarbonyl bedeutet und die Substituenten R³-R⁷ für jede einzelne Verbindung jeweils einer Zeile der Tabelle A entsprechen.

### Tabelle 84: Verbindungen 84.1-84.514

Verbindungen der allgemeinen Formel Ib, in der R¹ Methyl, R² Cl, R¹¹ n-Propyl, R¹² Methylcarbonyl bedeutet und die Substituenten R³-R⁷ für jede einzelne Verbindung jeweils einer Zeile der Tabelle A entsprechen.

### Tabelle 85: Verbindungen 85.1-85.514

Verbindungen der allgemeinen Formel Ib, in der R¹ Methyl, R² Cl, R¹¹ Methyl, R¹² Ethylcarbonyl bedeutet und die Substituenten R³-R⁷ für jede einzelne Verbindung jeweils einer Zeile der Tabelle A entsprechen.

### Tabelle 86: Verbindungen 86.1-86.514

Verbindungen der allgemeinen Formel Ib, in der R¹ Methyl, R² Cl, R¹¹ Ethyl, R¹² Ethylcarbonyl bedeutet und die Substituenten R³-R⁷ für jede einzelne Verbindung jeweils einer Zeile der Tabelle A entsprechen.

### Tabelle 87: Verbindungen 87.1-87.514

Verbindungen der allgemeinen Formel Ib, in der R¹ Methyl, R² Cl, R¹¹ n-Propyl, R¹² Ethylcarbonyl bedeutet und die Substituenten R³-R⁷ für jede einzelne Verbindung jeweils einer Zeile der Tabelle A entsprechen.

### Tabelle 88: Verbindungen 88.1-88.514

Verbindungen der allgemeinen Formel Ib, in der R¹ Methyl, R² Cl, R¹¹ Methyl, R¹² Methylsulfonyl bedeutet und die Substituenten R³-R⁷ für jede einzelne Verbindung jeweils einer Zeile der Tabelle A entsprechen.

### Tabelle 89: Verbindungen 89.1-89.514

Verbindungen der allgemeinen Formel Ib, in der R¹ Methyl, R² Cl, R¹¹ Ethyl, R¹² Methylsulfonyl bedeutet und die Substituenten R³-R⁷ für jede einzelne Verbindung jeweils einer Zeile der Tabelle A entsprechen.

### Tabelle 90: Verbindungen 90.1-90.514

Verbindungen der allgemeinen Formel Ib, in der R¹ Methyl, R² Cl, R¹¹ n-Propyl, R¹² Methylsulfonyl bedeutet und die Substituenten R³-R⁷ für jede einzelne Verbindung jeweils einer Zeile der Tabelle A entsprechen.

### Tabelle 91: Verbindungen 91.1-91.514

Verbindungen der allgemeinen Formel Ib, in der R¹ Methyl, R² Cl, R¹¹ Methyl, R¹² Ethylsulfonyl bedeutet und die Substituenten R³-R⁷ für jede einzelne Verbindung jeweils einer Zeile der Tabelle A entsprechen.

### Tabelle 92: Verbindungen 92.1-92.514

Verbindungen der allgemeinen Formel Ib, in der R¹ Methyl, R² Cl, R¹¹ Ethyl, R¹² Ethylsulfonyl bedeutet und die Substituenten R³-R⁷ für jede einzelne Verbindung jeweils einer Zeile der Tabelle A entsprechen.

### Tabelle 93: Verbindungen 93.1-93.514

Verbindungen der allgemeinen Formel Ib, in der R¹ Methyl, R² Cl, R¹¹ n-Propyl, R¹² Ethylsulfonyl bedeutet und die Substituenten R³-R⁷ für jede einzelne Verbindung jeweils einer Zeile der Tabelle A entsprechen.

### Tabelle 94: Verbindungen 94.1-94.514

Verbindungen der allgemeinen Formel Ib, in der R¹ Methyl, R² Cl, R¹¹ Methyl, R¹² 4-Methylphenylsulfonyl bedeutet und die Substituenten R³-R⁷ für jede einzelne Verbindung jeweils einer Zeile der Tabelle A entsprechen.

### Tabelle 95: Verbindungen 95.1-95.514

Verbindungen der allgemeinen Formel Ib, in der R¹ Methyl, R² Cl, R¹¹ Ethyl, R¹² 4-Methylphenylsulfonyl bedeutet und die Substituenten R³-R⁷ für jede einzelne Verbindung jeweils einer Zeile der Tabelle A entsprechen.

### Tabelle 96: Verbindungen 96.1-96.514

Verbindungen der allgemeinen Formel Ib, in der R¹ Methyl, R² Cl, R¹¹ n-Propyl, R¹² 4-Methylphenylsulfonyl bedeutet und die Substituenten R³-R⁷ für jede einzelne Verbindung jeweils einer Zeile der Tabelle A entsprechen.

### Tabelle 97: Verbindungen 97.1-97.514

Verbindungen der allgemeinen Formel Ib, in der R¹ Methyl, R² Methylsulfonyl, R¹¹ Methyl und R¹² Wasserstoff bedeutet und die Substituenten R³-R⁷ für jede einzelne Verbindung jeweils einer Zeile der Tabelle A entsprechen.

### Tabelle 98: Verbindungen 98.1-98.514

Verbindungen der allgemeinen Formel Ib, in der R¹ Methyl, R² Methylsulfonyl, R¹¹ Ethyl, R¹² Wasserstoff bedeutet und die Substituenten R³-R⁷ für jede einzelne Verbindung jeweils einer Zeile der Tabelle A entsprechen.

### Tabelle 99: Verbindungen 99.1-99.514

Verbindungen der allgemeinen Formel Ib, in der R¹ Methyl, R² Methylsulfonyl, R¹¹ n-Propyl, R¹² Wasserstoff bedeutet und die Substituenten R³-R⁷ für jede einzelne Verbindung jeweils einer Zeile der Tabelle A entsprechen.

### Tabelle 100: Verbindungen 100.1-100.514

Verbindungen der allgemeinen Formel Ib, in der R¹ Methyl, R² Methylsulfonyl, R¹¹ Methyl, R¹² Methyl bedeutet und die Substituenten R³-R⁷ für jede einzelne Verbindung jeweils einer Zeile der Tabelle A entsprechen.

### Tabelle 101: Verbindungen 101.1-101.514

Verbindungen der allgemeinen Formel Ib, in der R¹ Methyl, R² Methylsulfonyl, R¹¹ Ethyl, R¹² Methyl bedeutet und die Substituenten R³-R⁷ für jede einzelne Verbindung jeweils einer Zeile der Tabelle A entsprechen.

### Tabelle 102: Verbindungen 102.1-102.514

Verbindungen der allgemeinen Formel Ib, in der R¹ Methyl, R² Methylsulfonyl, R¹¹ n-Propyl, R¹² Methyl bedeutet und die Substituenten R³-R⁷ für jede einzelne Verbindung jeweils einer Zeile der Tabelle A entsprechen.

### Tabelle 103: Verbindungen 103.1-103.514

Verbindungen der allgemeinen Formel Ib, in der R¹ Methyl, R² Methylsulfonyl, R¹¹ Methyl, R¹² Ethyl bedeutet und die Substituenten R³-R⁷ für jede einzelne Verbindung jeweils einer Zeile der Tabelle A entsprechen.

### Tabelle 104: Verbindungen 104.1-104.514

Verbindungen der allgemeinen Formel Ib, in der R¹ Methyl, R² Methylsulfonyl, R¹¹ Ethyl, R¹² Ethyl bedeutet und die Substituenten R³-R⁷ für jede einzelne Verbindung jeweils einer Zeile der Tabelle A entsprechen.

### Tabelle 105: Verbindungen 105.1-105.514

Verbindungen der allgemeinen Formel Ib, in der R¹ Methyl, R² Methylsulfonyl, R¹¹ n-Propyl, R¹² Ethyl bedeutet und die Substituenten R³-R⁷ für jede einzelne Verbindung jeweils einer Zeile der Tabelle A entsprechen.

### Tabelle 106: Verbindungen 106.1-106.514

Verbindungen der allgemeinen Formel Ib, in der R¹ Methyl, R² Methylsulfonyl, R¹¹ Methyl, R¹² Methylcarbonyl bedeutet und die Substituenten R³-R⁷ für jede einzelne Verbindung jeweils einer Zeile der Tabelle A entsprechen.

### Tabelle 107: Verbindungen 107.1-107.514

Verbindungen der allgemeinen Formel Ib, in der R¹ Methyl, R² Methylsulfonyl, R¹¹ Ethyl, R¹² Methylcarbonyl bedeutet und die Substituenten R³-R⁷ für jede einzelne Verbindung jeweils einer Zeile der Tabelle A entsprechen.

### Tabelle 108: Verbindungen 108.1-108.514

Verbindungen der allgemeinen Formel Ib, in der R¹ Methyl, R² Methylsulfonyl, R¹¹ n-Propyl, R¹² Methylcarbonyl bedeutet und die Substituenten R³-R⁷ für jede einzelne Verbindung jeweils einer Zeile der Tabelle A entsprechen.

### Tabelle 109: Verbindungen 109.1-109.514

Verbindungen der allgemeinen Formel Ib, in der R¹ Methyl, R² Methylsulfonyl, R¹¹ Methyl, R¹² Ethylcarbonyl bedeutet und die Substituenten R³-R⁷ für jede einzelne Verbindung jeweils einer Zeile der Tabelle A entsprechen.

### Tabelle 110: Verbindungen 110.1-110.514

Verbindungen der allgemeinen Formel Ib, in der R¹ Methyl, R² Methylsulfonyl, R¹¹ Ethyl, R¹² Ethylcarbonyl bedeutet und die Substituenten R³-R⁷ für jede einzelne Verbindung jeweils einer Zeile der Tabelle A entsprechen.

### Tabelle 111: Verbindungen 111.1-111.514

Verbindungen der allgemeinen Formel Ib, in der R¹ Methyl, R² Methylsulfonyl, R¹¹ n-Propyl, R¹² Ethylcarbonyl bedeutet und die Substituenten R³-R⁷ für jede einzelne Verbindung jeweils einer Zeile der Tabelle A entsprechen.

### Tabelle 112: Verbindungen 112.1-112.514

Verbindungen der allgemeinen Formel Ib, in der R¹ Methyl, R² Methylsulfonyl, R¹¹ Methyl, R¹² Methylsulfonyl bedeutet und die Substituenten R³-R⁷ für jede einzelne Verbindung jeweils einer Zeile der Tabelle A entsprechen.

### Tabelle 113: Verbindungen 113.1-113.514

Verbindungen der allgemeinen Formel Ib, in der R¹ Methyl, R² Methylsulfonyl, R¹¹ Ethyl, R¹² Methylsulfonyl bedeutet und die Substituenten R³-R⁷ für jede einzelne Verbindung jeweils einer Zeile der Tabelle A entsprechen.

### Tabelle 114: Verbindungen 114.1-114.514

Verbindungen der allgemeinen Formel Ib, in der R¹ Methyl, R² Methylsulfonyl, R¹¹ n-Propyl, R¹² Methylsulfonyl bedeutet und die Substituenten R³-R⁷ für jede einzelne Verbindung jeweils einer Zeile der Tabelle A entsprechen.

### Tabelle 115: Verbindungen 115.1-115.514

Verbindungen der allgemeinen Formel Ib, in der R¹ Methyl, R² Methylsulfonyl, R¹¹ Methyl, R¹² Ethylsulfonyl bedeutet und die Substituenten R³-R⁷ für jede einzelne Verbindung jeweils einer Zeile der Tabelle A entsprechen.

### Tabelle 116: Verbindungen 116.1-116.514

Verbindungen der allgemeinen Formel Ib, in der R¹ Methyl, R² Methylsulfonyl, R¹¹ Ethyl, R¹² Ethylsulfonyl bedeutet und die Substituenten R³-R⁷ für jede einzelne Verbindung jeweils einer Zeile der Tabelle A entsprechen.

### Tabelle 117: Verbindungen 117.1-117.514

Verbindungen der allgemeinen Formel Ib, in der R¹ Methyl, R² Methylsulfonyl, R¹¹ n-Propyl, R¹² Ethylsulfonyl bedeutet und die Substituenten R³-R⁷ für jede einzelne Verbindung jeweils einer Zeile der Tabelle A entsprechen.

### Tabelle 118: Verbindungen 118.1-118.514

Verbindungen der allgemeinen Formel Ib, in der R¹ Methyl, R² Methylsulfonyl, R¹¹ Methyl, R¹² 4-Methylphenylsulfonyl bedeutet und die Substituenten R³-R⁷ für jede einzelne Verbindung jeweils einer Zeile der Tabelle A entsprechen.

### Tabelle 119: Verbindungen 119.1-119.514

Verbindungen der allgemeinen Formel Ib, in der R¹ Methyl, R² Methylsulfonyl, R¹¹ Ethyl, R¹² 4-Methylphenylsulfonyl bedeutet und die Substituenten R³-R⁷ für jede einzelne Verbindung jeweils einer Zeile der Tabelle A entsprechen.

### Tabelle 120: Verbindungen 120.1-120.514

Verbindungen der allgemeinen Formel Ib, in der R¹ Methyl, R² Methylsulfonyl, R¹¹ n-Propyl, R¹² 4-Methylphenylsulfonyl bedeutet und die Substituenten R³-R⁷ für jede einzelne Verbindung jeweils einer Zeile der Tabelle A entsprechen.

### Tabelle 121: Verbindungen 121.1-121.514

Verbindungen der allgemeinen Formel Ib, in der R¹ Methyl, R² Trifluormethyl, R¹¹ Methyl und R¹² Wasserstoff bedeutet und die Substituenten R³-R⁷ für jede einzelne Verbindung jeweils einer Zeile der Tabelle A entsprechen.

### Tabelle 122: Verbindungen 122.1-122.514

Verbindungen der allgemeinen Formel Ib, in der R¹ Methyl, R² Trifluormethyl, R¹¹ Ethyl, R¹² Wasserstoff bedeutet und die Substituenten R³-R⁷ für jede einzelne Verbindung jeweils einer Zeile der Tabelle A entsprechen.

### Tabelle 123: Verbindungen 123.1-123.514

Verbindungen der allgemeinen Formel Ib, in der R¹ Methyl, R² Trifluormethyl, R¹¹ n-Propyl, R¹² Wasserstoff bedeutet und die Substituenten R³-R⁷ für jede einzelne Verbindung jeweils einer Zeile der Tabelle A entsprechen.

### Tabelle 124: Verbindungen 124.1-124.514

Verbindungen der allgemeinen Formel Ib, in der R¹ Methyl, R² Trifluormethyl, R¹¹ Methyl, R¹² Methyl bedeutet und die Substituenten R³-R⁷ für jede einzelne Verbindung jeweils einer Zeile der Tabelle A entsprechen.

### Tabelle 125: Verbindungen 125.1-125.514

Verbindungen der allgemeinen Formel Ib, in der R¹ Methyl, R² Trifluormethyl, R¹¹ Ethyl, R¹² Methyl bedeutet und die Substituenten R³-R⁷ für jede einzelne Verbindung jeweils einer Zeile der Tabelle A entsprechen.

### Tabelle 126: Verbindungen 126.1-126.514

Verbindungen der allgemeinen Formel Ib, in der R¹ Methyl, R² Trifluormethyl, R¹¹ n-Propyl, R¹² Methyl bedeutet und die Substituenten R³-R⁷ für jede einzelne Verbindung jeweils einer Zeile der Tabelle A entsprechen.

### Tabelle 127: Verbindungen 127.1-127.514

Verbindungen der allgemeinen Formel Ib, in der R¹ Methyl, R² Trifluormethyl, R¹¹ Methyl, R¹² Ethyl bedeutet und die Substituenten R³-R⁷ für jede einzelne Verbindung jeweils einer Zeile der Tabelle A entsprechen.

### Tabelle 128: Verbindungen 128.1-128.514

Verbindungen der allgemeinen Formel Ib, in der R¹ Methyl, R² Trifluormethyl, R¹¹ Ethyl, R¹² Ethyl bedeutet und die Substituenten R³-R⁷ für jede einzelne Verbindung jeweils einer Zeile der Tabelle A entsprechen.

### Tabelle 129: Verbindungen 129.1-129.514

Verbindungen der allgemeinen Formel Ib, in der R¹ Methyl, R² Trifluormethyl, R¹¹ n-Propyl, R¹² Ethyl bedeutet und die Substituenten R²-R⁷ für jede einzelne Verbindung jeweils einer Zeile der Tabelle A entsprechen.

### Tabelle 130: Verbindungen 130.1-130.514

Verbindungen der allgemeinen Formel Ib, in der R¹ Methyl, R² Trifluormethyl, R¹¹ Methyl, R¹² Methylcarbonyl bedeutet und die Substituenten R³-R⁷ für jede einzelne Verbindung jeweils einer Zeile der Tabelle A entsprechen.

### Tabelle 131: Verbindungen 131.1-131.514

Verbindungen der allgemeinen Formel Ib, in der R¹ Methyl, R² Trifluormethyl, R¹¹ Ethyl, R¹² Methylcarbonyl bedeutet und die Substituenten R³-R⁷ für jede einzelne Verbindung jeweils einer Zeile der Tabelle A entsprechen.

### Tabelle 132: Verbindungen 132.1-132.514

Verbindungen der allgemeinen Formel Ib, in der R¹ Methyl, R² Trifluormethyl, R¹¹ n-Propyl, R¹² Methylcarbonyl bedeutet und die Substituenten R³-R⁷ für jede einzelne Verbindung jeweils einer Zeile der Tabelle A entsprechen.

### Tabelle 133: Verbindungen 133.1-133.514

Verbindungen der allgemeinen Formel Ib, in der R¹ Methyl, R² Trifluormethyl, R¹¹ Methyl, R¹² Ethylcarbonyl bedeutet und die Substituenten R³-R⁷ für jede einzelne Verbindung jeweils einer Zeile der Tabelle A entsprechen.

### Tabelle 134: Verbindungen 134.1-134.514

Verbindungen der allgemeinen Formel Ib, in der R¹ Methyl, R² Trifluormethyl, R¹¹ Ethyl, R¹² Ethylcarbonyl bedeutet und die Substituenten R³-R⁷ für jede einzelne Verbindung jeweils einer Zeile der Tabelle A entsprechen.

### Tabelle 135: Verbindungen 135.1-135.514

Verbindungen der allgemeinen Formel Ib, in der R¹ Methyl, R² Trifluormethyl, R¹¹ n-Propyl, R¹² Ethylcarbonyl bedeutet und die Substituenten R³-R⁷ für jede einzelne Verbindung jeweils einer Zeile der Tabelle A entsprechen.

### Tabelle 136: Verbindungen 136.1-136.514

Verbindungen der allgemeinen Formel Ib, in der R¹ Methyl, R² Trifluormethyl, R¹¹ Methyl, R¹² Methylsulfonyl bedeutet und die Substituenten R³-R⁷ für jede einzelne Verbindung jeweils einer Zeile der Tabelle A entsprechen.

### Tabelle 137: Verbindungen 137.1-137.514

Verbindungen der allgemeinen Formel Ib, in der R¹ Methyl, R² Trifluormethyl, R¹¹ Ethyl, R¹² Methylsulfonyl bedeutet und die Substituenten R³-R⁷ für jede einzelne Verbindung jeweils einer Zeile der Tabelle A entsprechen.

### Tabelle 138: Verbindungen 138.1-138.514

Verbindungen der allgemeinen Formel Ib, in der R¹ Methyl, R² Trifluormethyl, R¹¹ n-Propyl, R¹² Methylsulfonyl bedeutet und die Substituenten R³-R⁷ für jede einzelne Verbindung jeweils einer Zeile der Tabelle A entsprechen.

### Tabelle 139: Verbindungen 139.1-139.514

Verbindungen der allgemeinen Formel Ib, in der R¹ Methyl, R² Trifluormethyl, R¹¹ Methyl, R¹² Ethylsulfonyl bedeutet und die Substituenten R³-R⁷ für jede einzelne Verbindung jeweils einer Zeile der Tabelle A entsprechen.

### Tabelle 140: Verbindungen 140.1-140.514

Verbindungen der allgemeinen Formel Ib, in der R¹ Methyl, R² Trifluormethyl, R¹¹ Ethyl, R¹² Ethylsulfonyl bedeutet und die Substituenten R³-R⁷ für jede einzelne Verbindung jeweils einer Zeile der Tabelle A entsprechen.

### Tabelle 141: Verbindungen 141.1-141.514

Verbindungen der allgemeinen Formel Ib, in der R¹ Methyl, R² Trifluormethyl, R¹¹ n-Propyl, R¹² Ethylsulfonyl bedeutet und die Substituenten R³-R⁷ für jede einzelne Verbindung jeweils einer Zeile der Tabelle A entsprechen.

### Tabelle 142: Verbindungen 142.1-142.514

Verbindungen der allgemeinen Formel Ib, in der R¹ Methyl, R² Trifluonnethyl, R¹¹ Methyl, R¹² 4-Methylphenylsulfonyl bedeutet und die Substituenten R³-R⁷ für jede einzelne Verbindung jeweils einer Zeile der Tabelle A entsprechen.

### Tabelle 143: Verbindungen 143.1-143.514

Verbindungen der allgemeinen Formel Ib, in der R¹ Methyl, R² Trifluormethyl, R¹¹ Ethyl, R¹² 4-Methylphenylsulfonyl bedeutet und die Substituenten R³-R⁷ für jede einzelne Verbindung jeweils einer Zeile der Tabelle A entsprechen.

### Tabelle 144: Verbindungen 144.1-144.514

Verbindungen der allgemeinen Formel Ib, in der R¹ Methyl, R² Trifluormethyl, R¹¹ n-Propyl, R¹² 4-Methylphenylsulfonyl bedeutet und die Substituenten R³-R⁷ für jede einzelne Verbindung jeweils einer Zeile der Tabelle A entsprechen.

Beispielsweise seien die Verbindungen Ic der Tabellen 145 bis 288 genannt, wobei die Substituenten R³ bis R⁵ für jede einzelne Verbindung Ic jeweils einer Zeile der Tabelle B entsprechen.

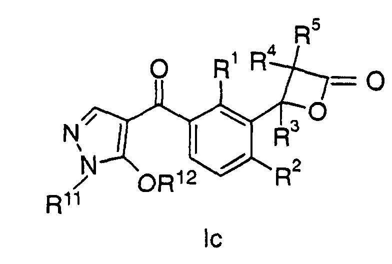

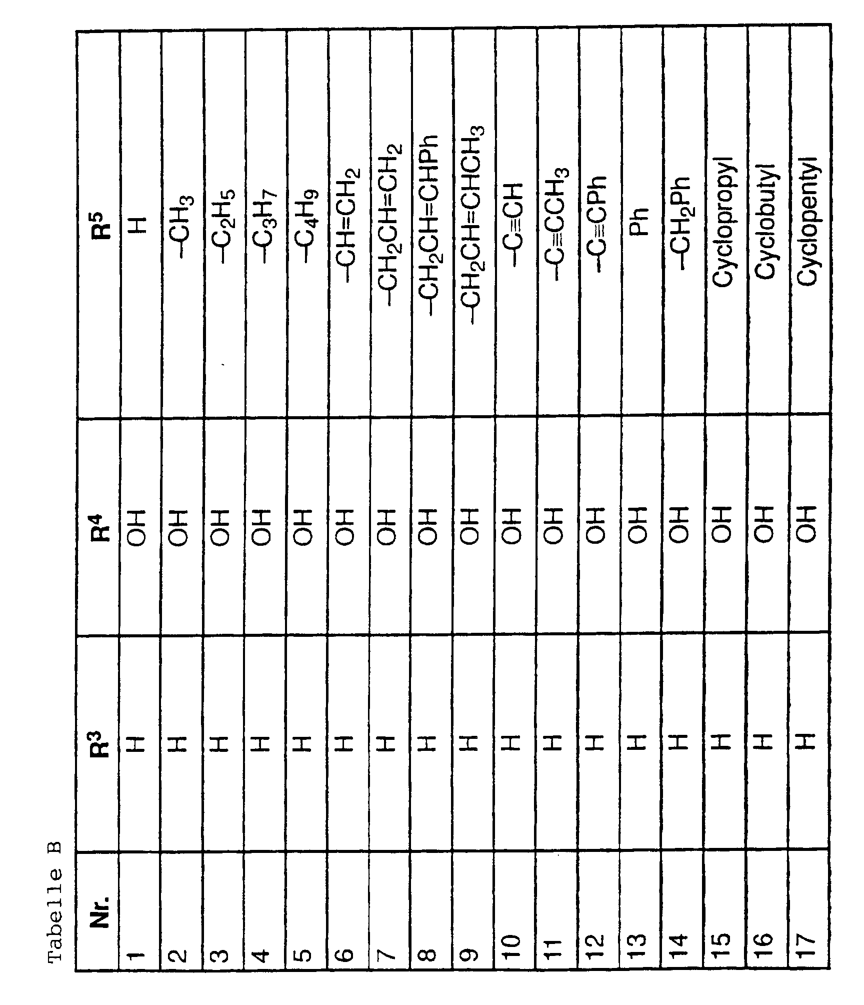

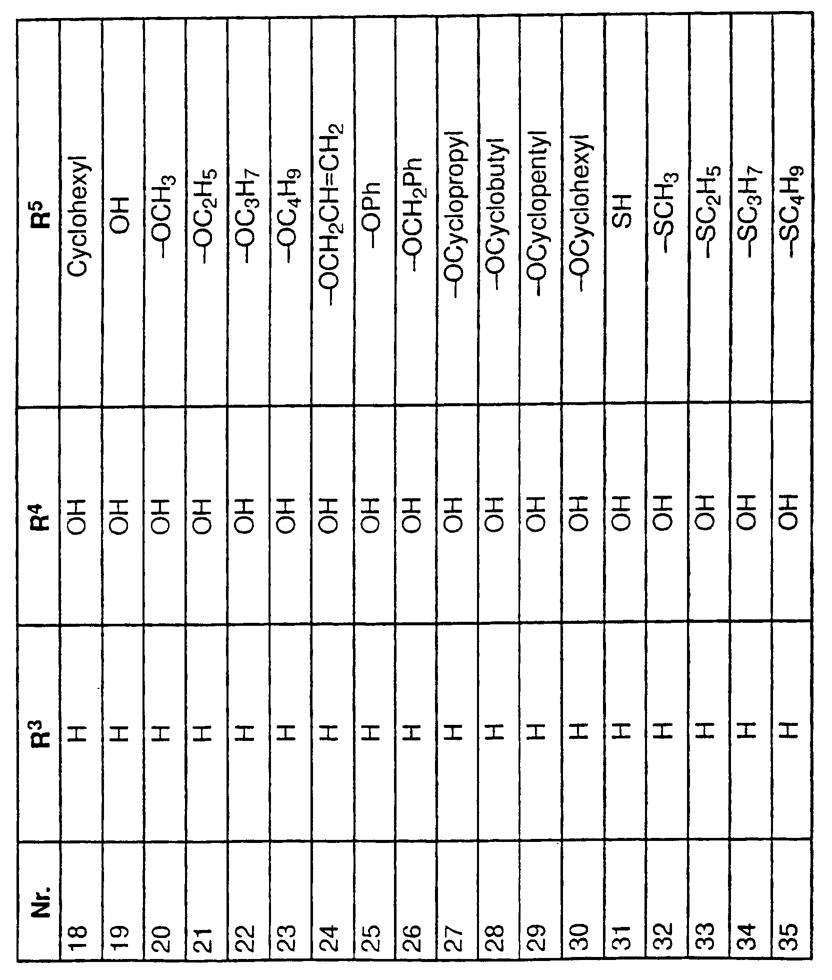

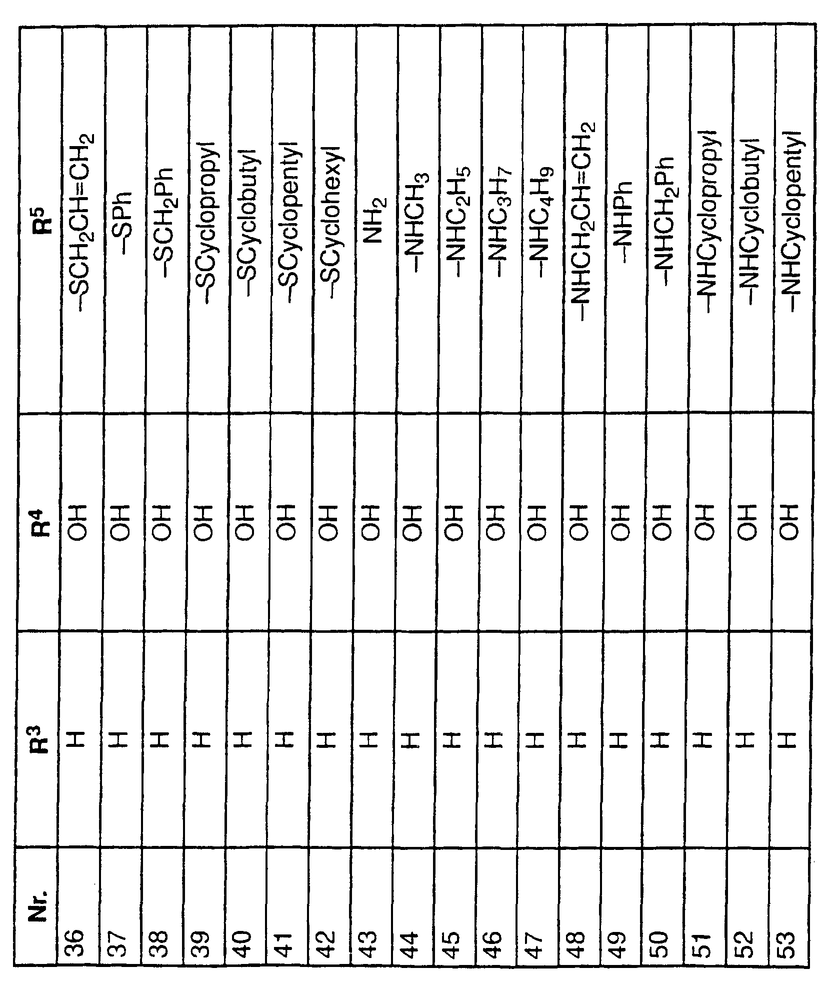

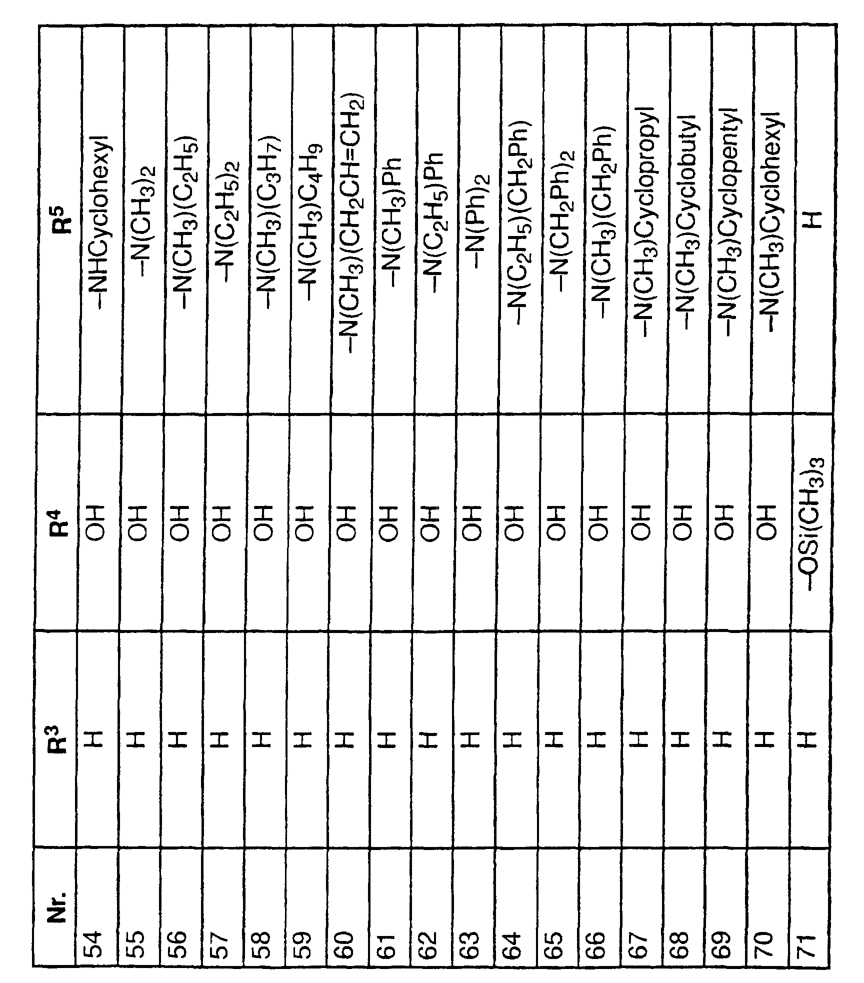

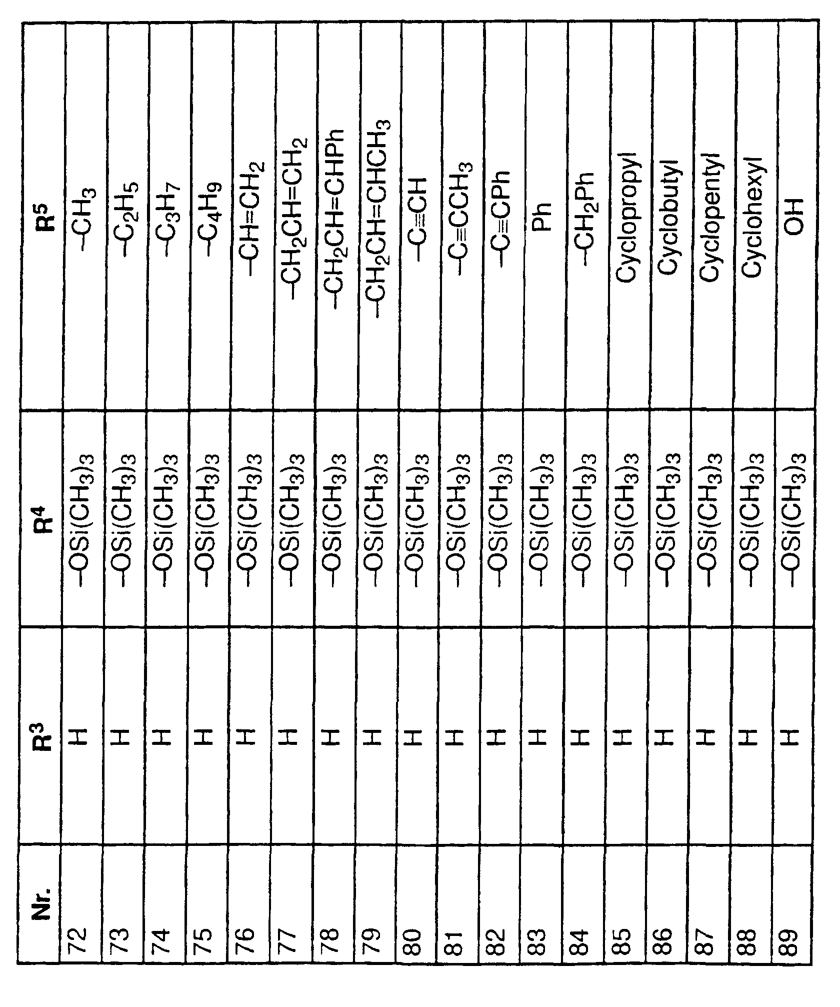

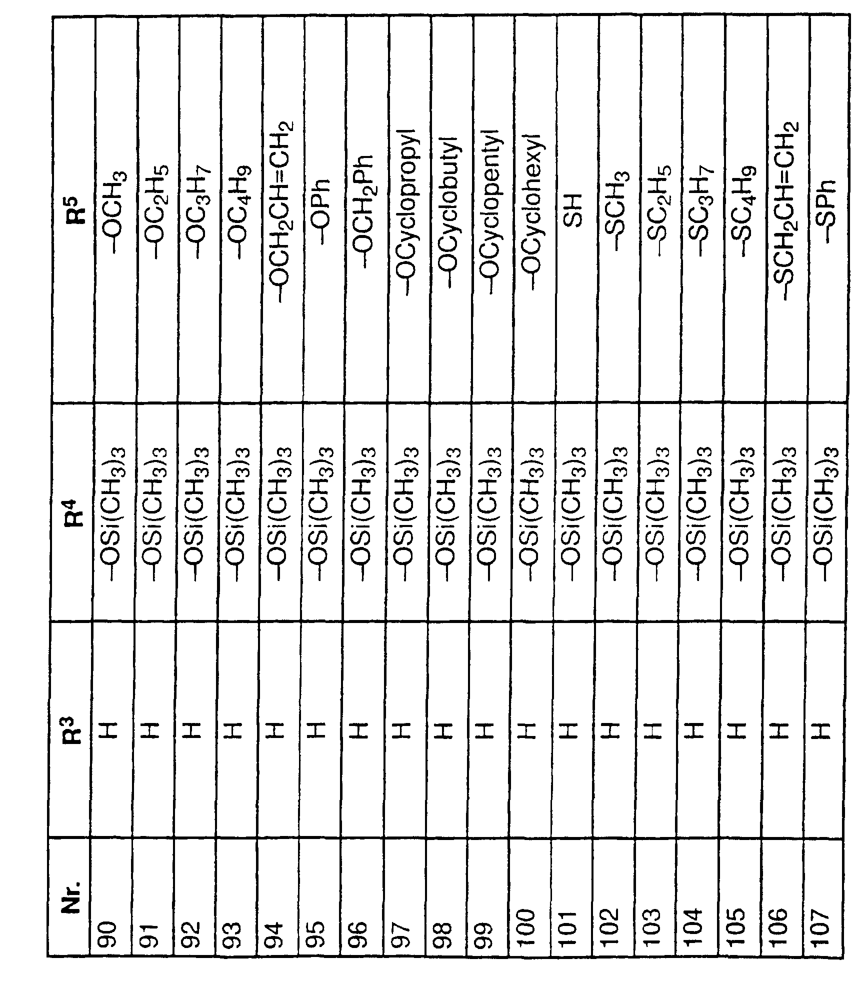

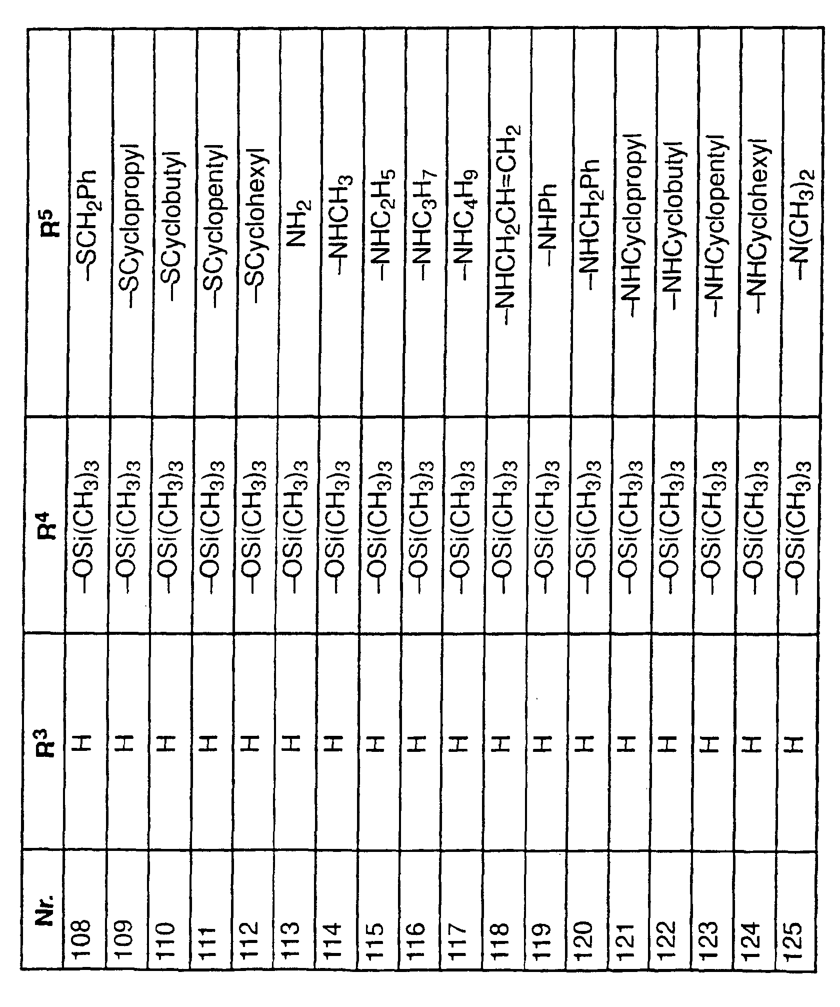

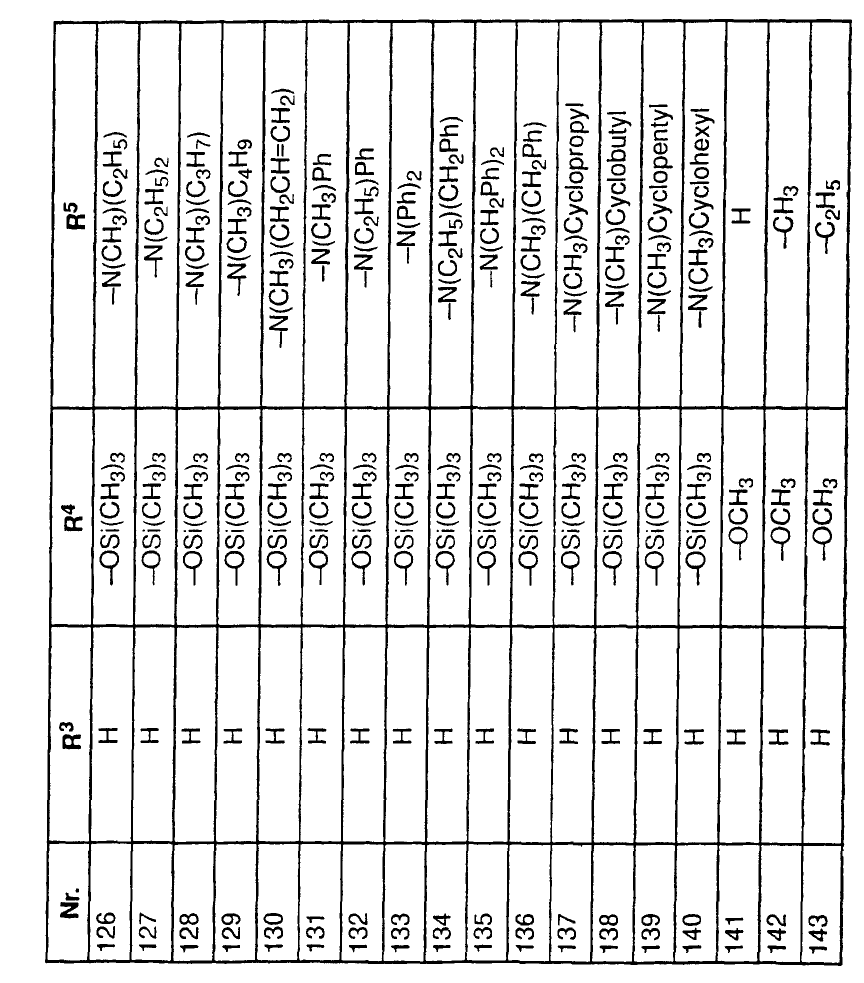

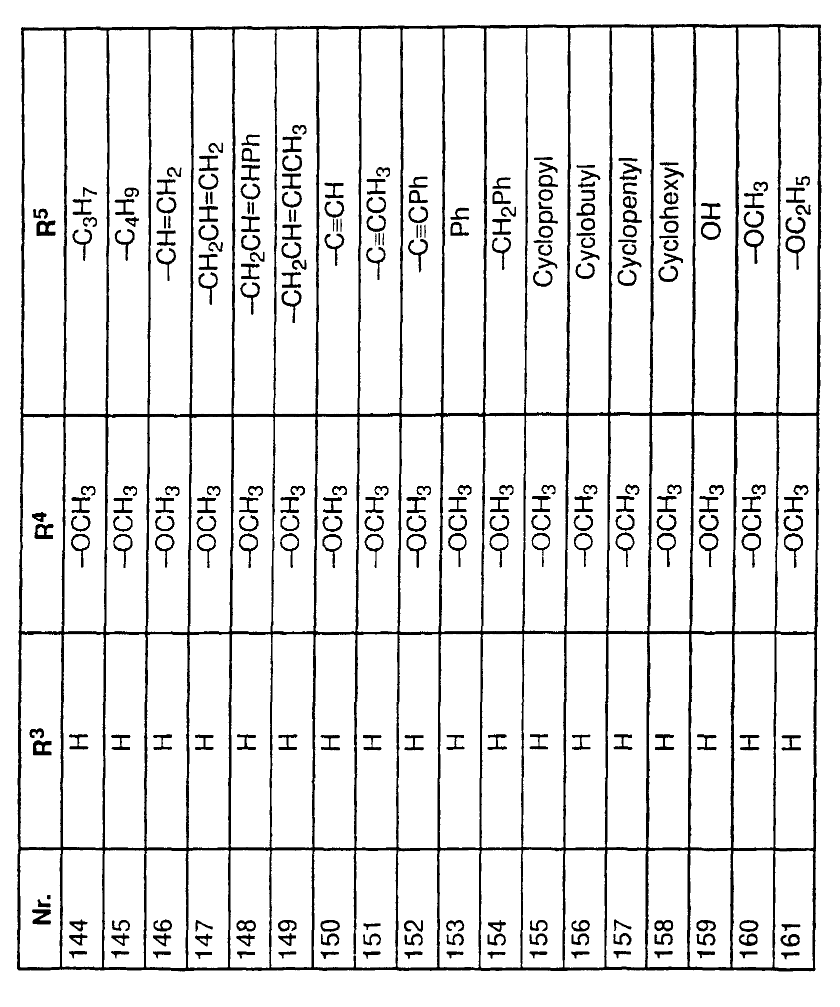

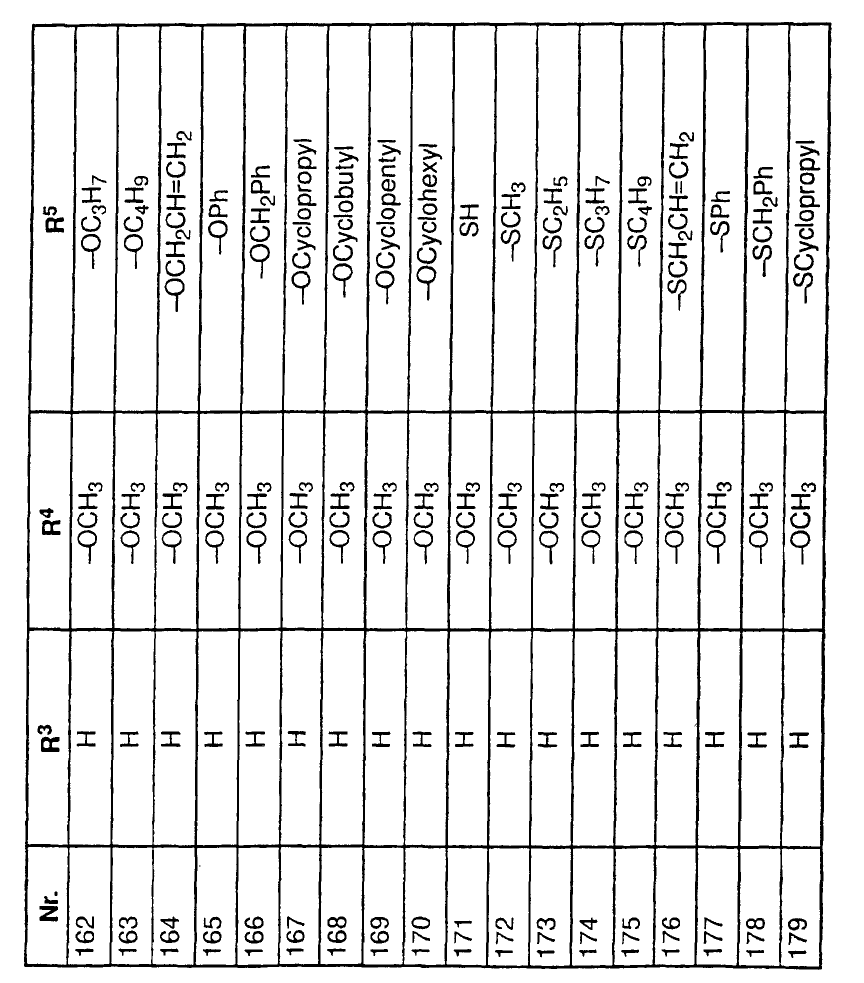

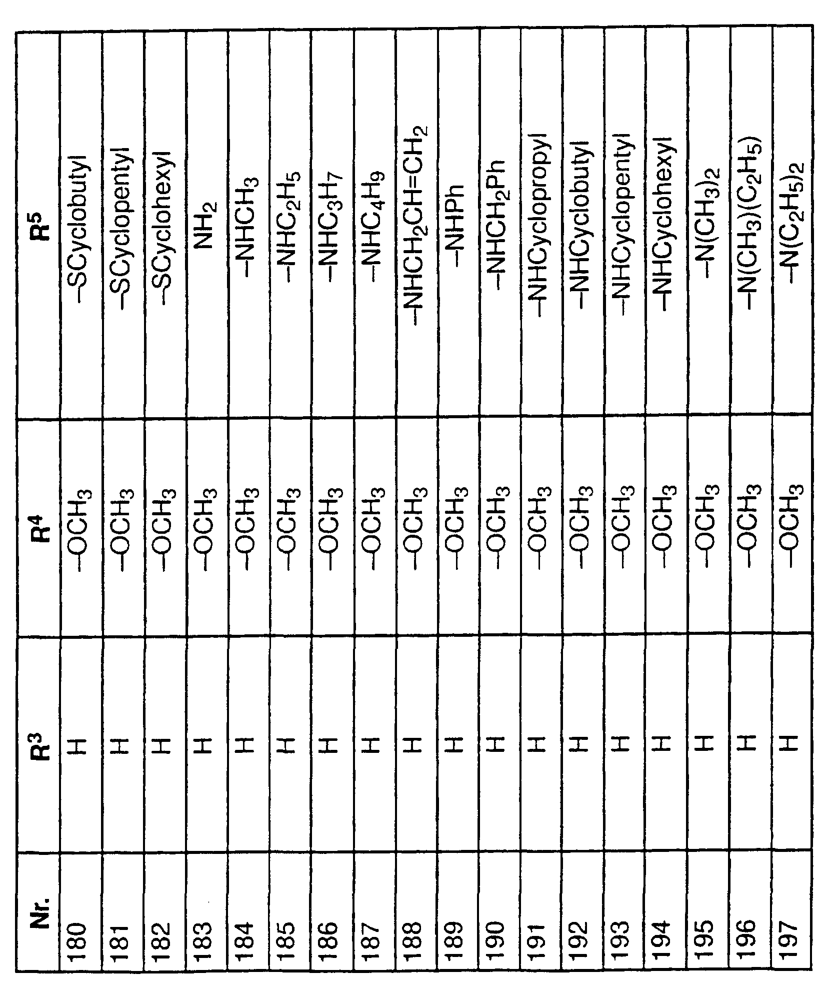

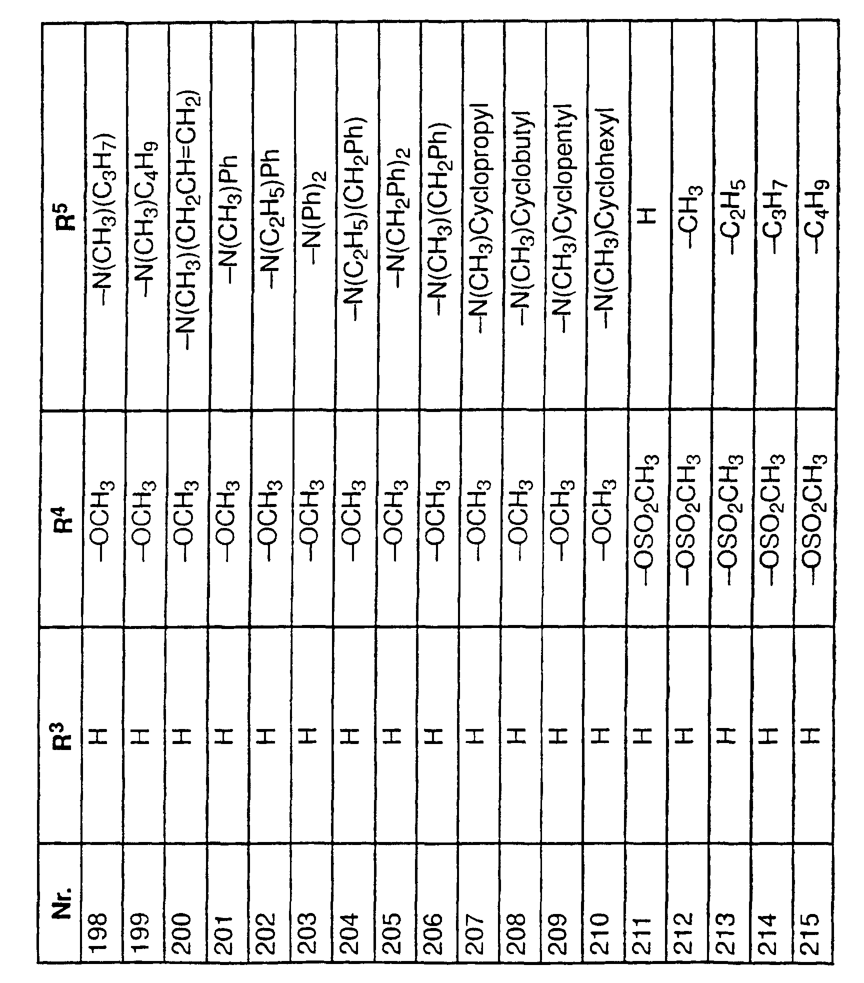

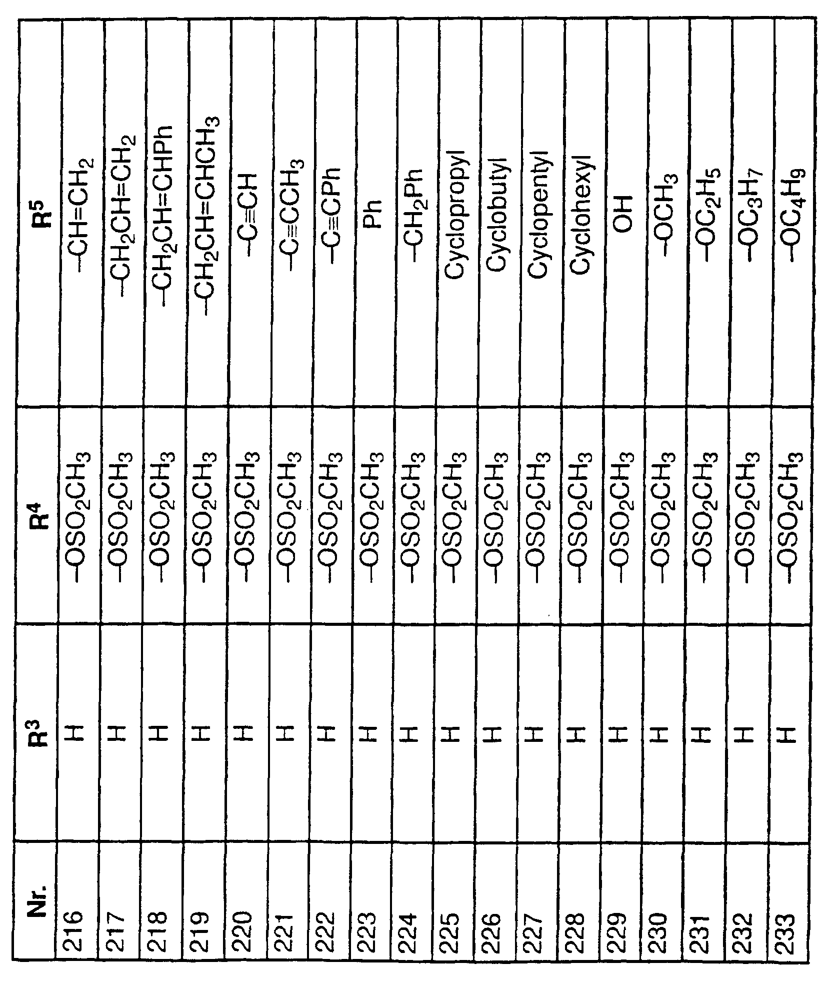

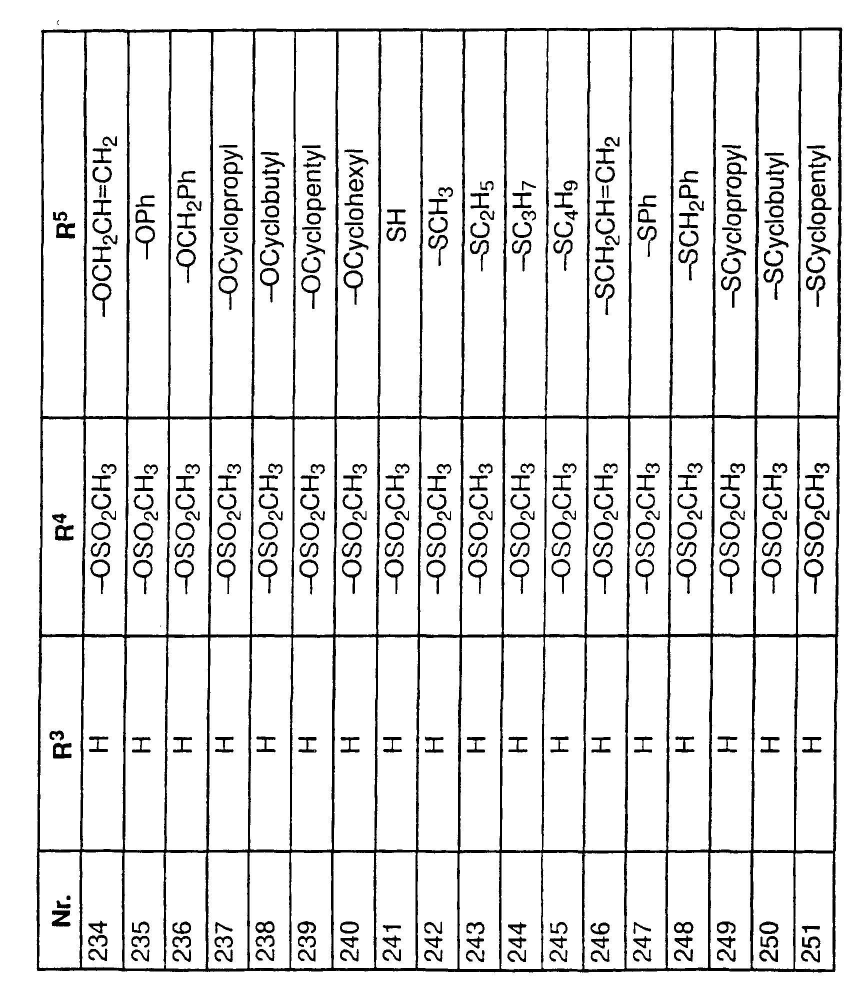

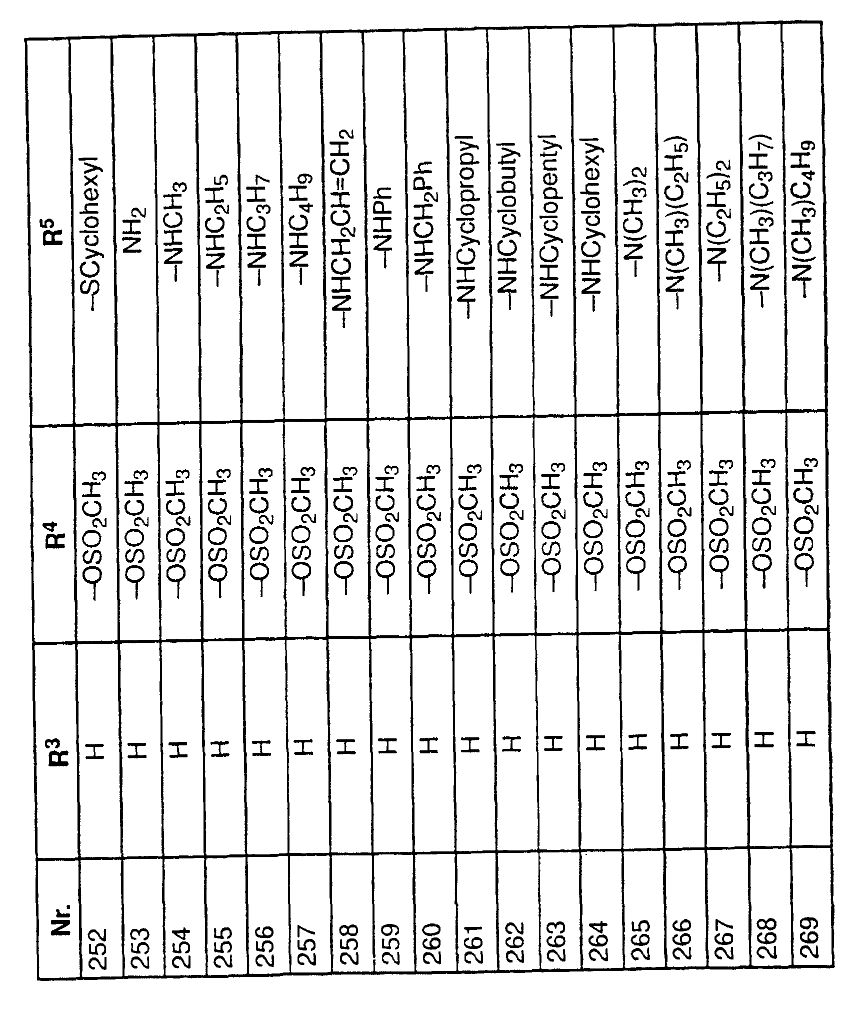

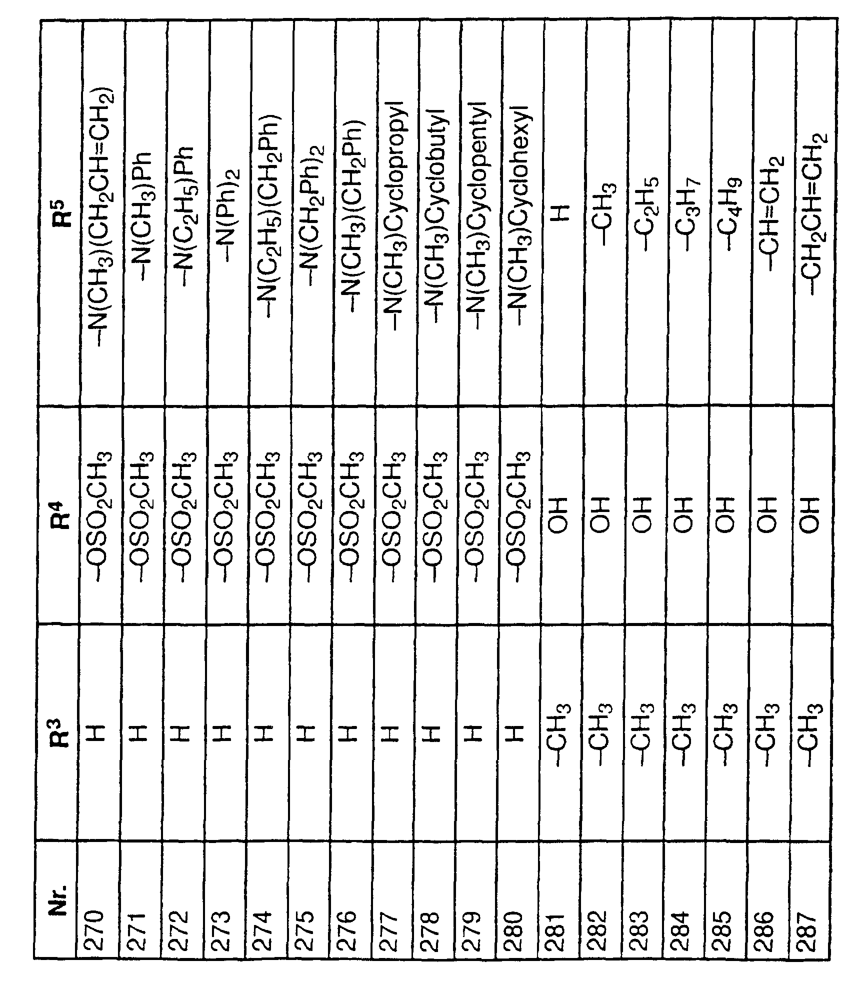

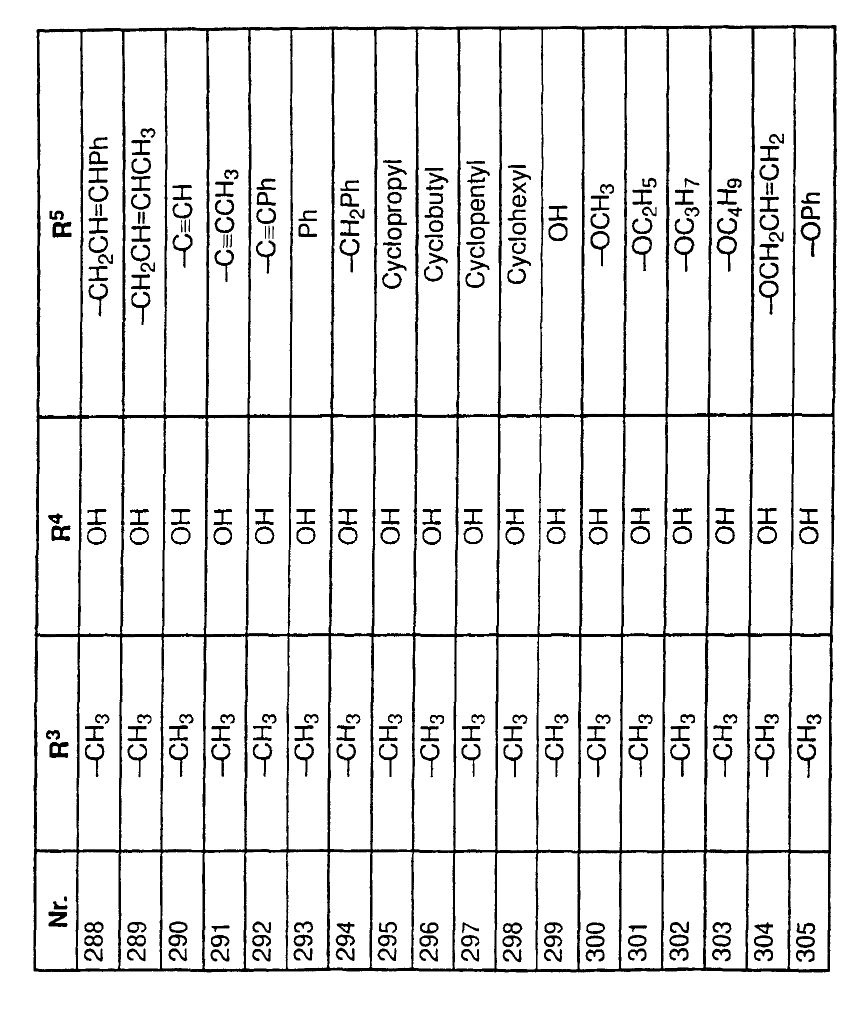

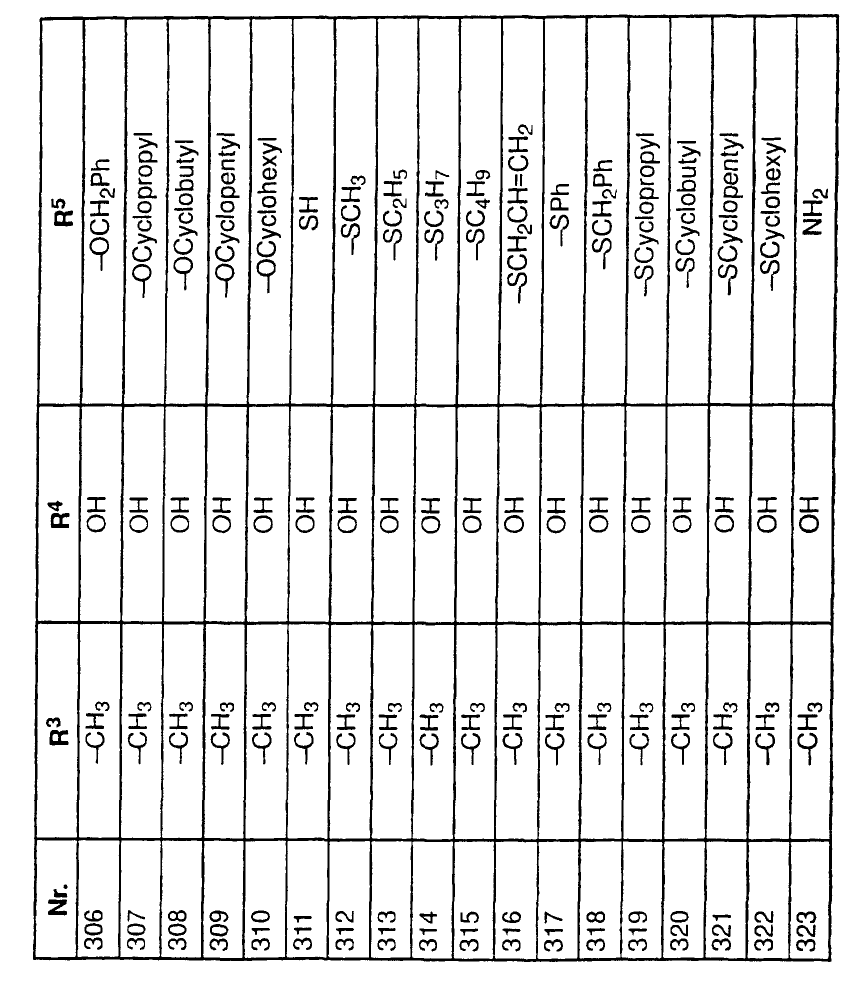

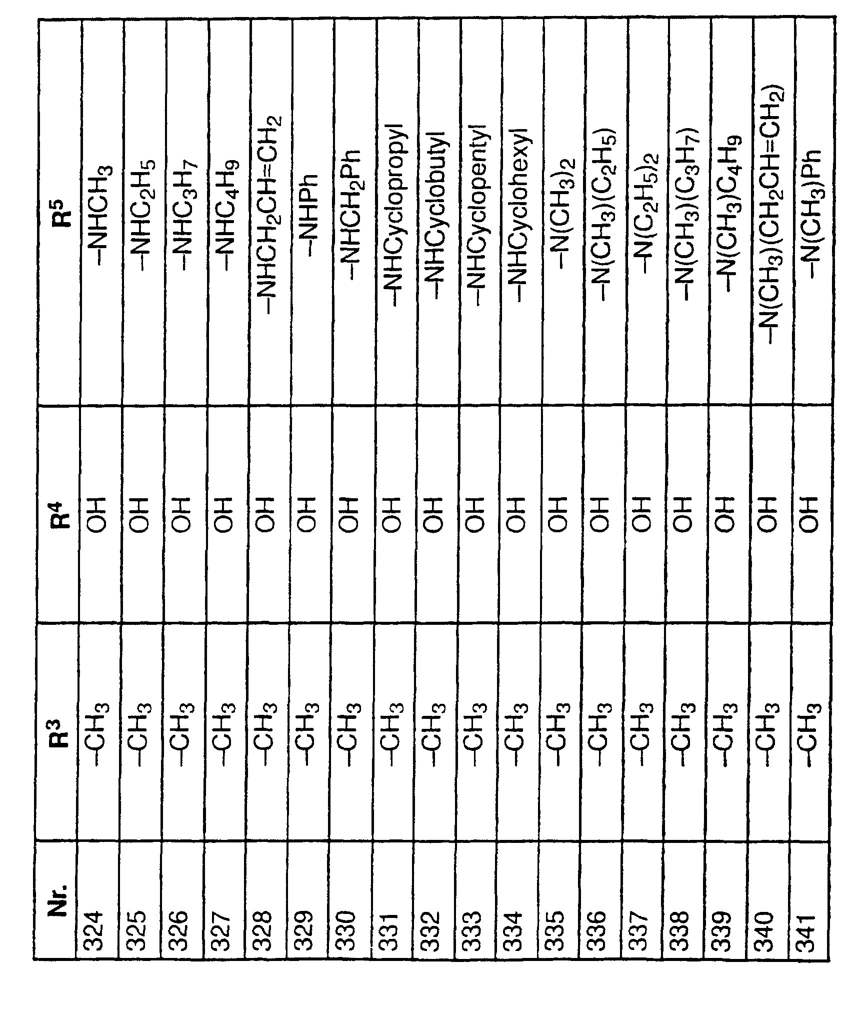

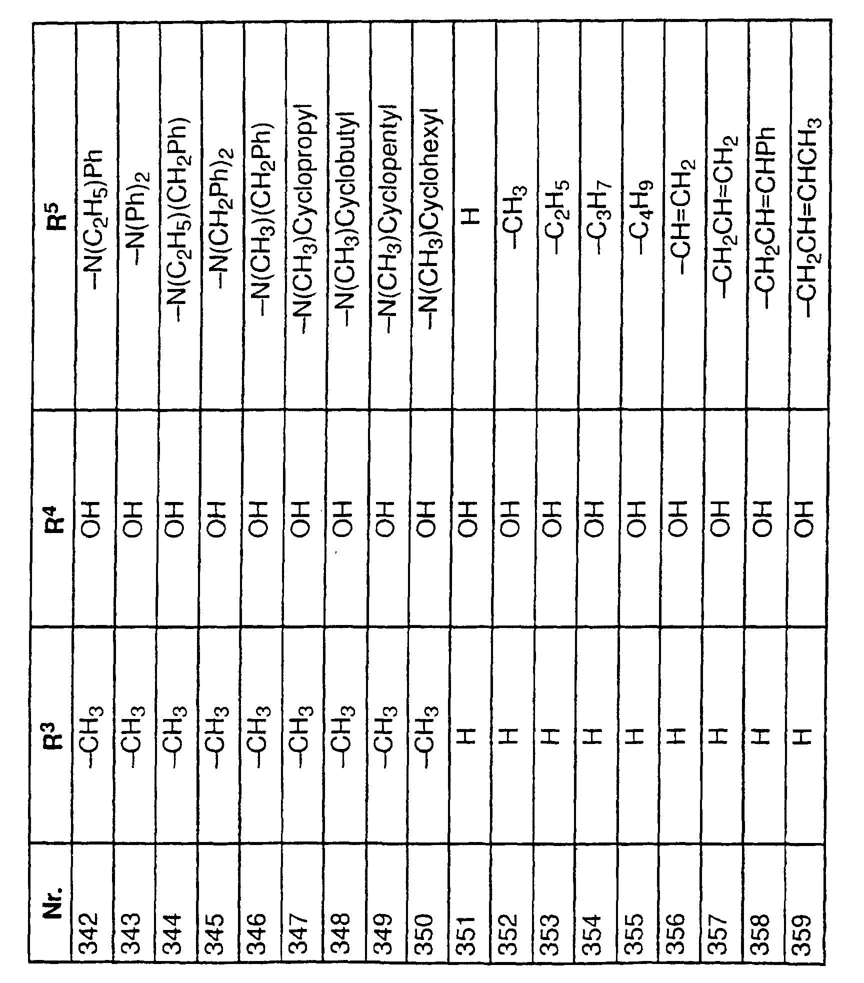

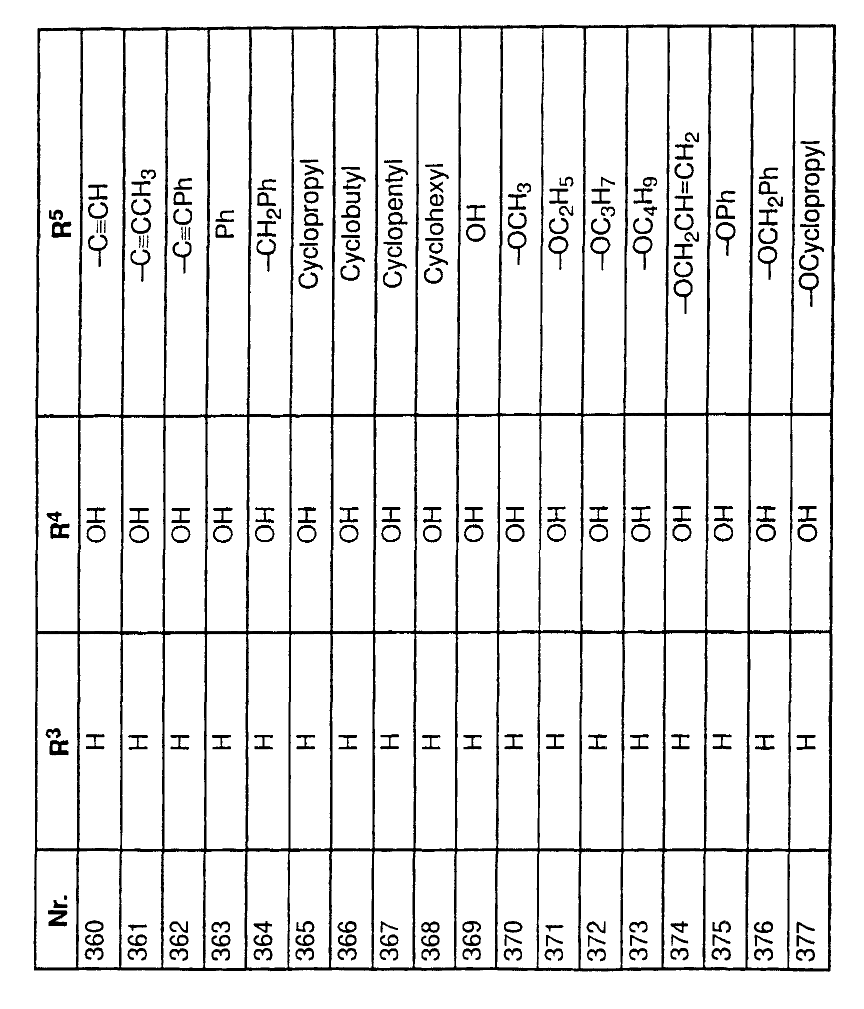

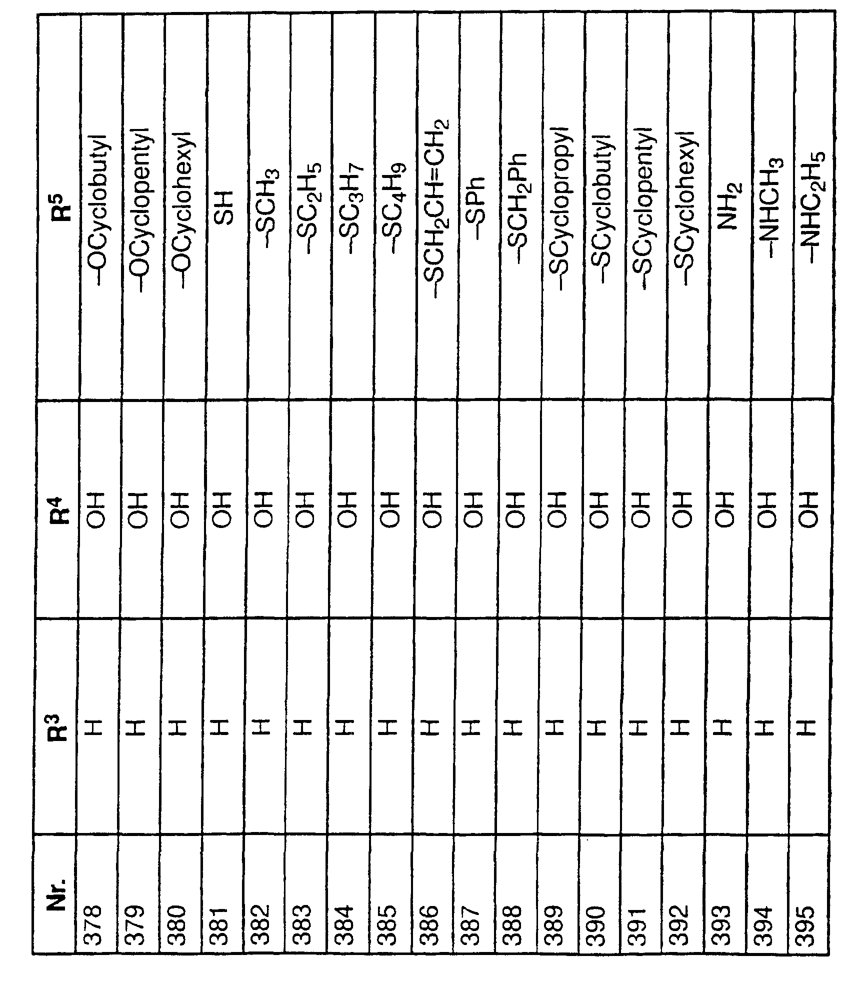

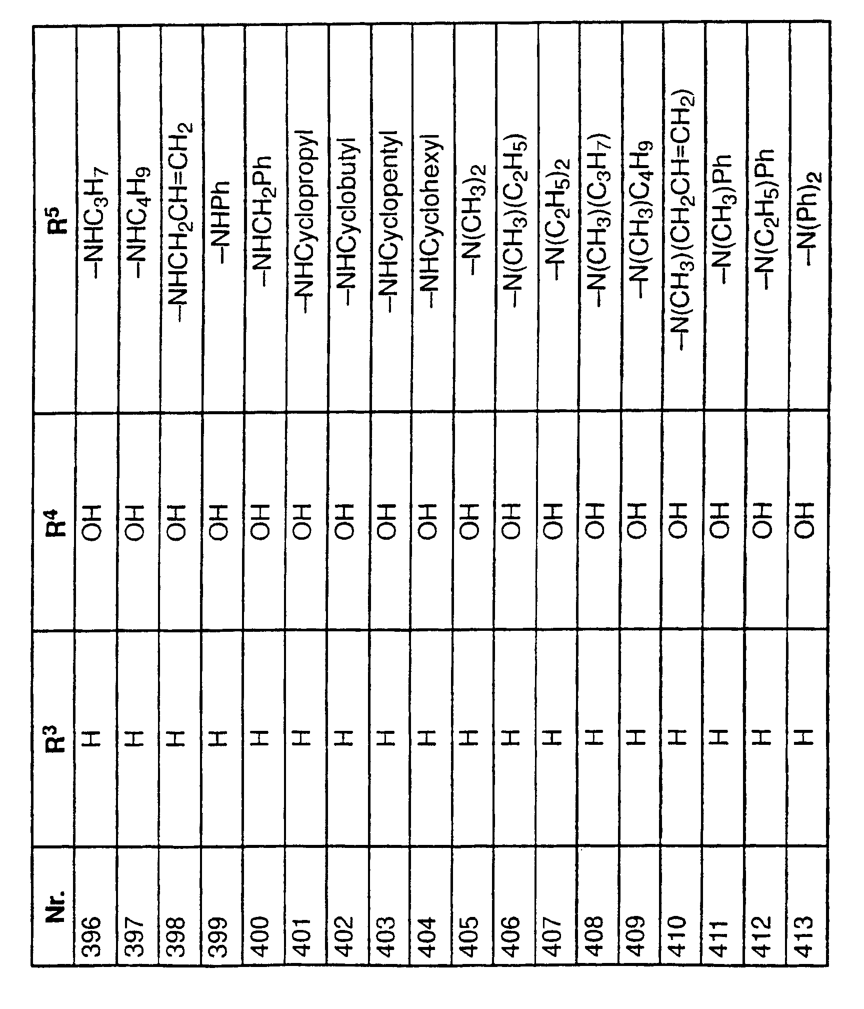

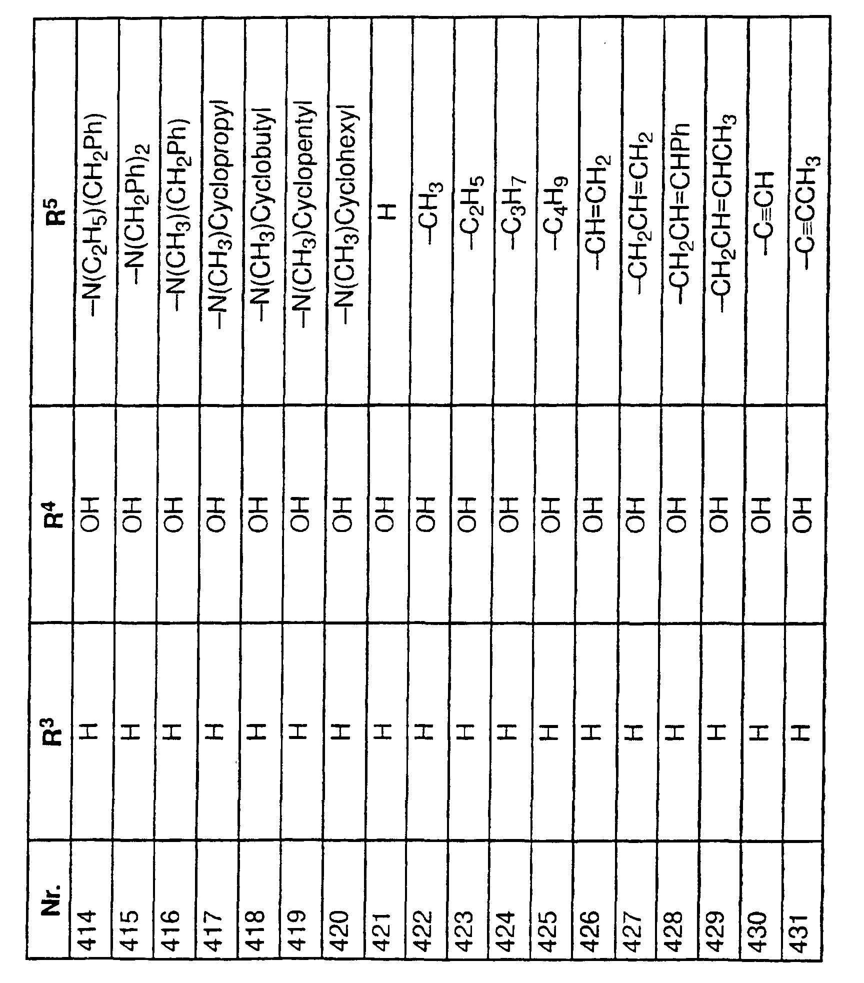

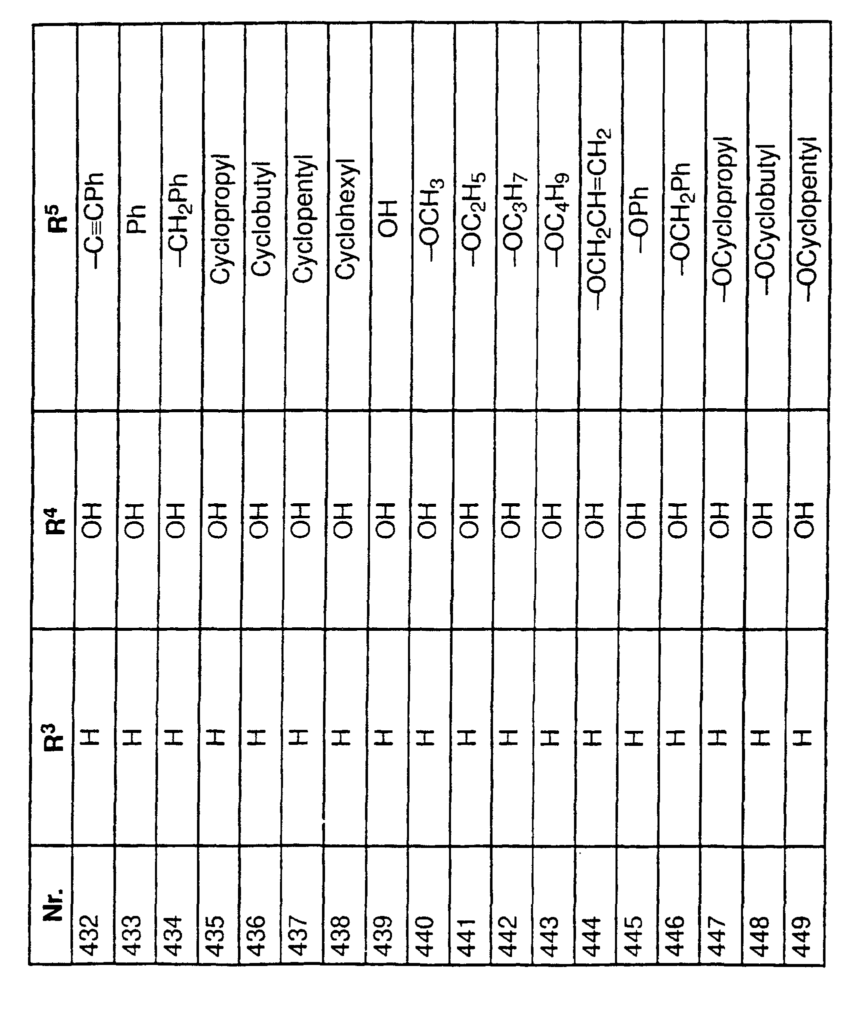

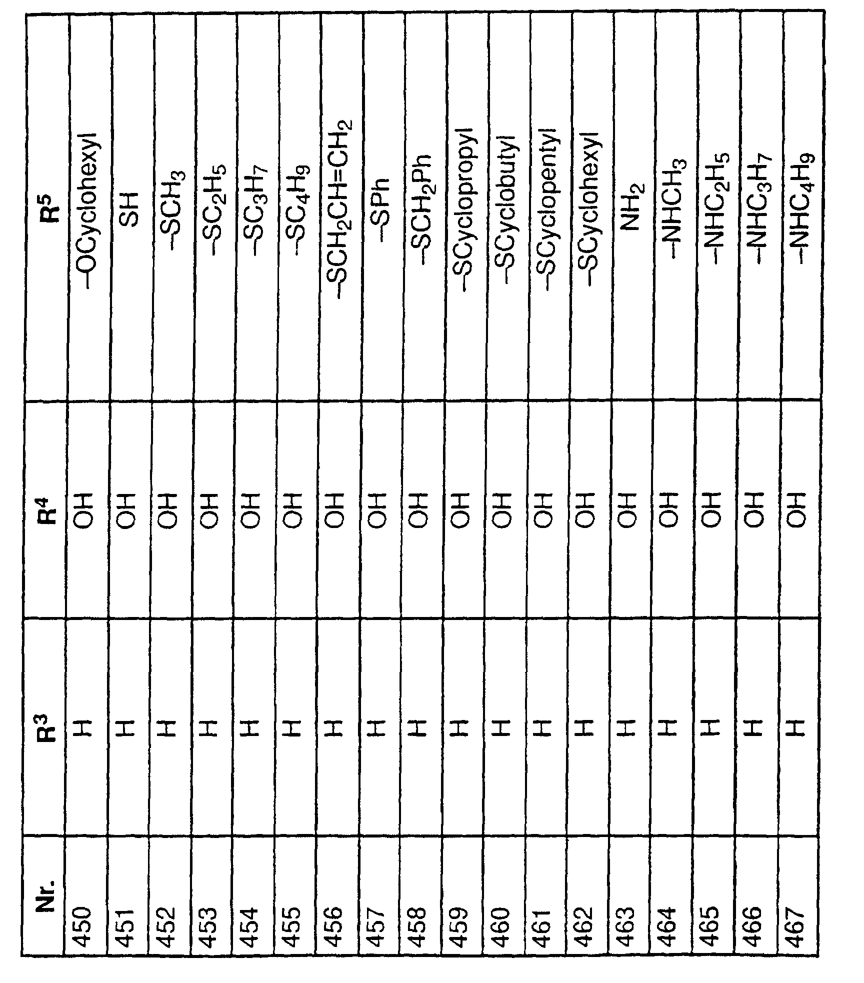

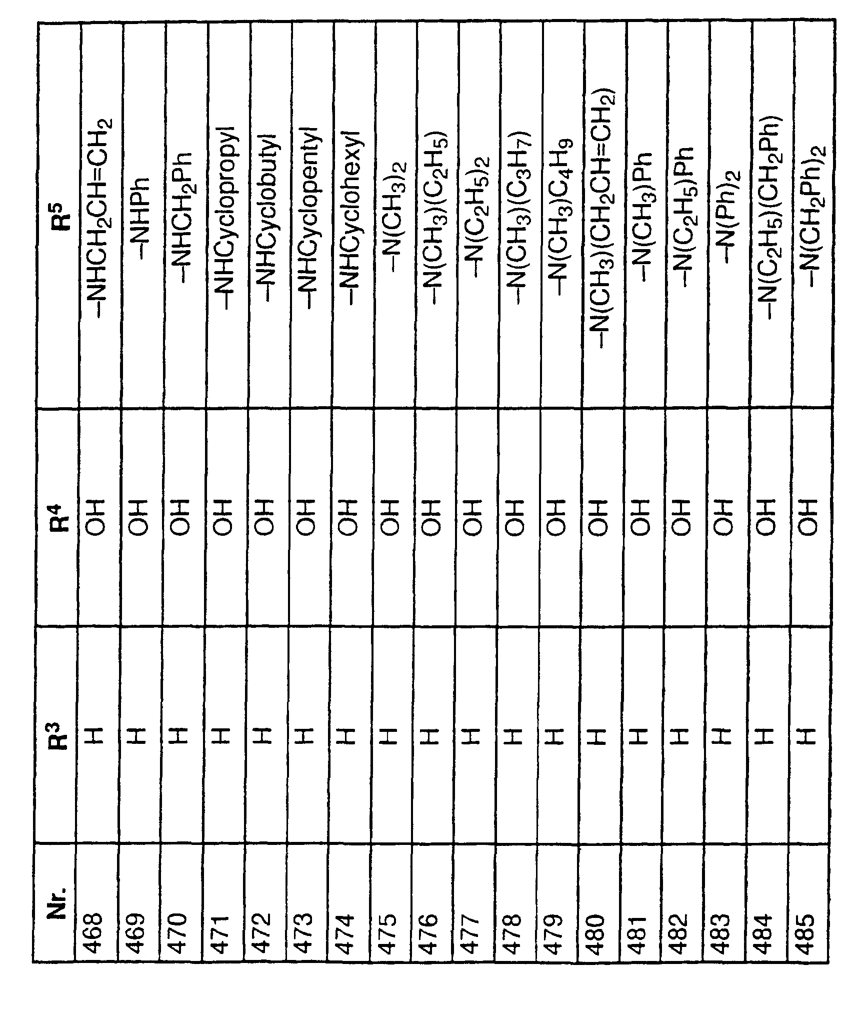

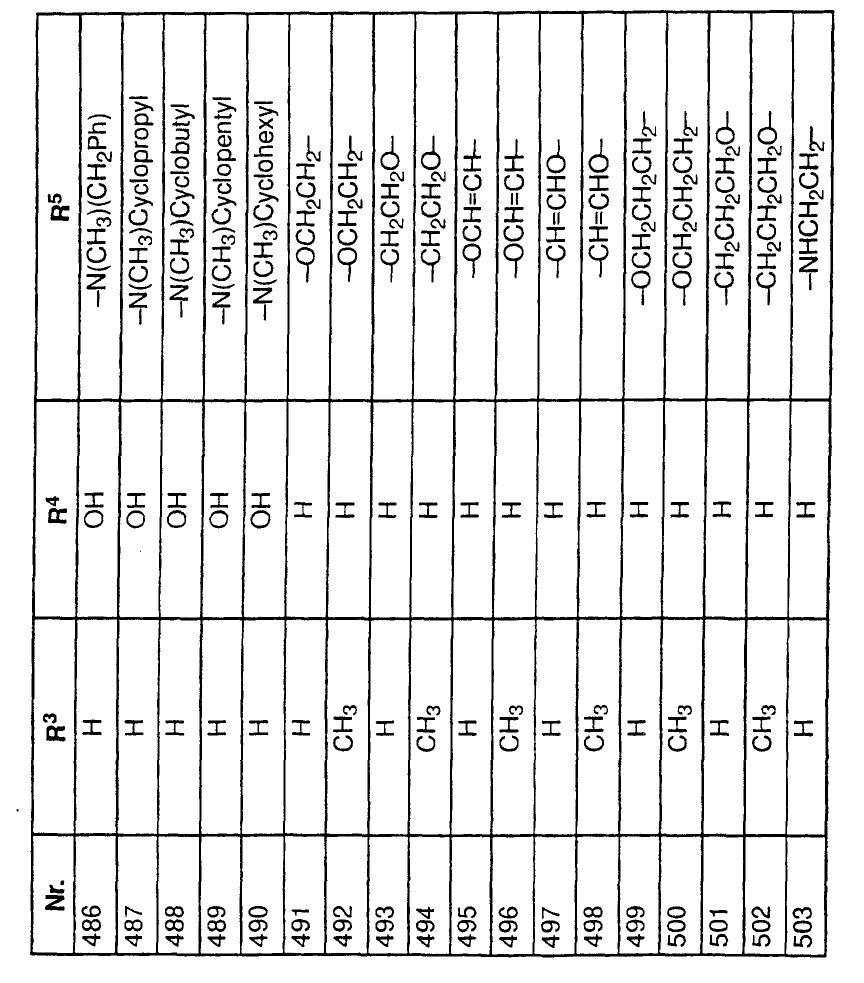

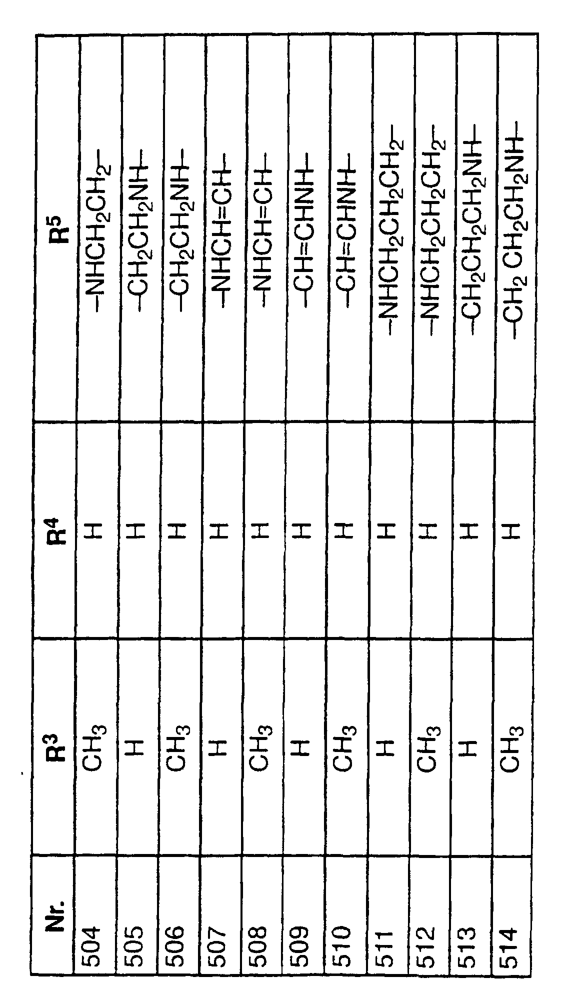

Die folgenden Tabellen 145-288 basieren auf den 4-Benzoyl-pyrazolen der Formel Ic:

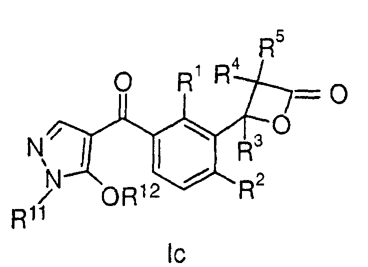

### Tabelle 145: Verbindungen 145.1-145.514

Verbindungen der allgemeinen Formel Ic, in der R¹ Cl, R² Methylsulfonyl, R¹¹ Methyl und R¹² Wasserstoff bedeutet und die Substituenten R³-R⁵ für jede einzelne Verbindung jeweils einer Zeile der Tabelle B entsprechen.

### Tabelle 146: Verbindungen 146.1-146.514

Verbindungen der allgemeinen Formel Ic, in der R¹ Cl, R² Methylsulfonyl, R¹¹ Ethyl, R¹² Wasserstoff bedeutet und die Substituenten R³-R⁵ für jede einzelne Verbindung jeweils einer Zeile der Tabelle B entsprechen.

### Tabelle 147: Verbindungen 147.1-147.514

Verbindungen der allgemeinen Formel Ic, in der R¹ Cl, R² Methylsulfonyl, R¹¹ n-Propyl, R¹² Wasserstoff bedeutet und die Substituenten R³-R⁵ für jede einzelne Verbindung jeweils einer Zeile der Tabelle B entsprechen.

### Tabelle 148: Verbindungen 148.1-148.514

Verbindungen der allgemeinen Formel Ic, in der R¹ Cl, R² Methylsulfonyl, R¹¹ Methyl, R¹² Methyl bedeutet und die Substituenten R³-R⁵ für jede einzelne Verbindung jeweils einer Zeile der Tabelle B entsprechen.

### Tabelle 149: Verbindungen 149.1-149.514

Verbindungen der allgemeinen Formel Ic, in der R¹ Cl, R² Methylsulfonyl, R¹¹ Ethyl, R¹² Methyl bedeutet und die Substituenten R³-R⁵ für jede einzelne Verbindung jeweils einer Zeile der Tabelle B entsprechen.

### Tabelle 150: Verbindungen 150.1-150.514

Verbindungen der allgemeinen Formel Ic, in der R¹ Cl, R² Methylsulfonyl, R¹¹ n-Propyl, R¹² Methyl bedeutet und die Substituenten R³-R⁵ für jede einzelne Verbindung jeweils einer Zeile der Tabelle B entsprechen.

### Tabelle 151: Verbindungen 151.1-151.514

Verbindungen der allgemeinen Formel Ic, in der R¹ Cl, R² Methylsulfonyl, R¹¹ Methyl, R¹² Ethyl bedeutet und die Substituenten R³-R⁵ für jede einzelne Verbindung jeweils einer Zeile der Tabelle B entsprechen.

### Tabelle 152: Verbindungen 152.1-152.514

Verbindungen der allgemeinen Formel Ic, in der R¹ Cl, R² Methylsulfonyl, R¹¹ Ethyl, R¹² Ethyl bedeutet und die Substituenten R³-R⁵ für jede einzelne Verbindung jeweils einer Zeile der Tabelle B entsprechen.

### Tabelle 153: Verbindungen 153.1-153.514

Verbindungen der allgemeinen Formel Ic, in der R¹ Cl, R² Methylsulfonyl, R¹¹ n-Propyl, R¹² Ethyl bedeutet und die Substituenten R³-R⁵ für jede einzelne Verbindung jeweils einer Zeile der Tabelle B entsprechen.

### Tabelle 154: Verbindungen 154.1-154.514

Verbindungen der allgemeinen Formel Ic, in der R¹ Cl, R² Methylsulfonyl, R¹¹ Methyl, R¹² Methylcarbonyl bedeutet und die Substituenten R³-R⁵ für jede einzelne Verbindung jeweils einer Zeile der Tabelle B entsprechen.

### Tabelle 155: Verbindungen 155.1-155.514

Verbindungen der allgemeinen Formel Ic, in der R¹ Cl, R² Methylsulfonyl, R¹¹ Ethyl, R¹² Methylcarbonyl bedeutet und die Substituenten R³-R⁵ für jede einzelne Verbindung jeweils einer Zeile der Tabelle B entsprechen.

### Tabelle 156: Verbindungen 156.1-156.514

Verbindungen der allgemeinen Formel Ic, in der R¹ Cl, R² Methylsulfonyl, R¹¹ n-Propyl, R¹² Methylcarbonyl bedeutet und die Substituenten R³-R⁵ für jede einzelne Verbindung jeweils einer Zeile der Tabelle B entsprechen.

### Tabelle 157: Verbindungen 157.1-157.514

Verbindungen der allgemeinen Formel Ic, in der R¹ Cl, R² Methylsulfonyl, R¹¹ Methyl, R¹² Ethylcarbonyl bedeutet und die Substituenten R³-R⁵ für jede einzelne Verbindung jeweils einer Zeile der Tabelle B entsprechen.

### Tabelle 158: Verbindungen 158.1-158.514

Verbindungen der allgemeinen Formel Ic, in der R¹ Cl, R² Methylsulfonyl, R¹¹ Ethyl, R¹² Ethylcarbonyl bedeutet und die Substituenten R³-R⁵ für jede einzelne Verbindung jeweils einer Zeile der Tabelle B entsprechen.

### Tabelle 159: Verbindungen 159.1-159.514

Verbindungen der allgemeinen Formel Ic, in der R¹ Cl, R² Methylsulfonyl, R¹¹ n-Propyl, R¹² Ethylcarbonyl bedeutet und die Substituenten R³-R⁵ für jede einzelne Verbindung jeweils einer Zeile der Tabelle B entsprechen.

### Tabelle 160: Verbindungen 160.1-160.514

Verbindungen der allgemeinen Formel Ic, in der R¹ Cl, R² Methylsulfonyl, R¹¹ Methyl, R¹² Methylsulfonyl bedeutet und die Substituenten R³-R⁵ für jede einzelne Verbindung jeweils einer Zeile der Tabelle B entsprechen.

### Tabelle 161: Verbindungen 161.1-161.514

Verbindungen der allgemeinen Formel Ic, in der R¹ Cl, R² Methylsulfonyl, R¹¹ Ethyl, R¹² Methylsulfonyl bedeutet und die Substituenten R³-R⁵ für jede einzelne Verbindung jeweils einer Zeile der Tabelle B entsprechen.

### Tabelle 162: Verbindungen 162.1-162.514

Verbindungen der allgemeinen Formel Ic, in der R¹ Cl, R² Methylsulfonyl, R¹¹ n-Propyl, R¹² Methylsulfonyl bedeutet und die Substituenten R³-R⁵ für jede einzelne Verbindung jeweils einer Zeile der Tabelle B entsprechen.

### Tabelle 163: Verbindungen 163.1-163.514

Verbindungen der allgemeinen Formel Ic, in der R¹ Cl, R² Methylsulfonyl, R¹¹ Methyl, R¹² Ethylsulfonyl bedeutet und die Substituenten R³-R⁵ für jede einzelne Verbindung jeweils einer Zeile der Tabelle B entsprechen.

### Tabelle 164: Verbindungen 164.1-164.514

Verbindungen der allgemeinen Formel Ic, in der R¹ Cl, R² Methylsulfonyl, R¹¹ Ethyl, R¹² Ethylsulfonyl bedeutet und die Substituenten R³-R⁵ für jede einzelne Verbindung jeweils einer Zeile der Tabelle B entsprechen.

### Tabelle 165: Verbindungen 165.1-165.514

Verbindungen der allgemeinen Formel Ic, in der R¹ Cl, R² Methylsulfonyl, R¹¹ n-Propyl, R¹² Ethylsulfonyl bedeutet und die Substituenten R³-R⁵ für jede einzelne Verbindung jeweils einer Zeile der Tabelle B entsprechen.

### Tabelle 166: Verbindungen 166.1-166.514

Verbindungen der allgemeinen Formel Ic, in der R¹ Cl, R² Methylsulfonyl, R¹¹ Methyl, R¹² 4-Methylphenylsulfonyl bedeutet und die Substituenten R³-R⁵ für jede einzelne Verbindung jeweils einer Zeile der Tabelle B entsprechen.

### Tabelle 167: Verbindungen 167.1-167.514

Verbindungen der allgemeinen Formel Ic, in der R¹ Cl, R² Methylsulfonyl, R¹¹ Ethyl, R¹² 4-Methylphenylsulfonyl bedeutet und die Substituenten R³-R⁵ für jede einzelne Verbindung jeweils einer Zeile der Tabelle B entsprechen.

### Tabelle 168: Verbindungen 168.1-168.514

Verbindungen der allgemeinen Formel Ic, in der R¹ Cl, R² Methylsulfonyl, R¹¹ n-Propyl, R¹² 4-Methylphenylsulfonyl bedeutet und die Substituenten R³-R⁵ für jede einzelne Verbindung jeweils einer Zeile der Tabelle B entsprechen.

### Tabelle 169: Verbindungen 169.1-160.514

Verbindungen der allgemeinen Formel Ic, in der R¹ Cl, R² Cl, R¹¹ Methyl und R¹² Wasserstoff bedeutet und die Substituenten R³-R⁵ für jede einzelne Verbindung jeweils einer Zeile der Tabelle B entsprechen.

### Tabelle 170: Verbindungen 170.1-170.514

Verbindungen der allgemeinen Formel Ic, in der R¹ Cl, R² Cl, R¹¹ Ethyl, R¹² Wasserstoff bedeutet und die Substituenten R³-R⁵ für jede einzelne Verbindung jeweils einer Zeile der Tabelle B entsprechen.

### Tabelle 171: Verbindungen 171.1-171.514

Verbindungen der allgemeinen Formel Ic, in der R¹ Cl, R² Cl, R¹¹ n-Propyl, R¹² Wasserstoff bedeutet und die Substituenten R³-R⁵ für jede einzelne Verbindung jeweils einer Zeile der Tabelle B entsprechen.

### Tabelle 172: Verbindungen 172.1-172.514

Verbindungen der allgemeinen Formel Ic, in der R¹ Cl, R² Cl, R¹¹ Methyl, R¹² Methyl bedeutet und die Substituenten R³-R⁵ für jede einzelne Verbindung jeweils einer Zeile der Tabelle B entsprechen.

### Tabelle 173: Verbindungen 173.1-173.514

Verbindungen der allgemeinen Formel Ic, in der R¹ Cl, R² Cl, R¹¹ Ethyl, R¹² Methyl bedeutet und die Substituenten R³-R⁵ für jede einzelne Verbindung jeweils einer Zeile der Tabelle B entsprechen.

### Tabelle 174: Verbindungen 174.1-174.514

Verbindungen der allgemeinen Formel Ic, in der R¹ Cl, R² Cl, R¹¹ n-Propyl, R¹² Methyl bedeutet und die Substituenten R³-R⁵ für jede einzelne Verbindung jeweils einer Zeile der Tabelle B entsprechen.

### Tabelle 175: Verbindungen 175.1-175.514

Verbindungen der allgemeinen Formel Ic, in der R¹ Cl, R² Cl, R¹¹ Methyl, R¹² Ethyl bedeutet und die Substituenten R³-R⁵ für jede einzelne Verbindung jeweils einer Zeile der Tabelle B entsprechen.

### Tabelle 176: Verbindungen 176.1-176.514

Verbindungen der allgemeinen Formel Ic, in der R¹ Cl, R² Cl, R¹¹ Ethyl, R¹² Ethyl bedeutet und die Substituenten R³-R⁵ für jede einzelne Verbindung jeweils einer Zeile der Tabelle B entsprechen.

### Tabelle 177: Verbindungen 177.1-177.514

Verbindungen der allgemeinen Formel Ic, in der R¹ Cl, R² Cl, R¹¹ n-Propyl, R¹² Ethyl bedeutet und die Substituenten R³-R⁵ für jede einzelne Verbindung jeweils einer Zeile der Tabelle B entsprechen.

### Tabelle 178: Verbindungen 178.1-178.514

Verbindungen der allgemeinen Formel Ic, in der R¹ Cl, R² Cl, R¹¹ Methyl, R¹² Methylcarbonyl bedeutet und die Substituenten R³-R⁵ für jede einzelne Verbindung jeweils einer Zeile der Tabelle B entsprechen.

### Tabelle 179: Verbindungen 179.1-179.514

Verbindungen der allgemeinen Formel Ic, in der R¹ Cl, R² Cl, R¹¹ Ethyl, R¹² Methylcarbonyl bedeutet und die Substituenten R³-R⁵ für jede einzelne Verbindung jeweils einer Zeile der Tabelle B entsprechen.

### Tabelle 180: Verbindungen 180.1-180.514

Verbindungen der allgemeinen Formel Ic, in der R¹ Cl, R² Cl, R¹¹ n-Propyl, R¹² Methylcarbonyl bedeutet und die Substituenten R³-R⁵ für jede einzelne Verbindung jeweils einer Zeile der Tabelle B entsprechen.

### Tabelle 181: Verbindungen 181.1-181.514

Verbindungen der allgemeinen Formel Ic, in der R¹ Cl, R² Cl, R¹¹ Methyl, R¹² Ethylcarbonyl bedeutet und die Substituenten R³-R⁵ für jede einzelne Verbindung jeweils einer Zeile der Tabelle B entsprechen.

### Tabelle 182: Verbindungen 182.1-182.514

Verbindungen der allgemeinen Formel Ic, in der R¹ Cl, R² Cl, R¹¹ Ethyl, R¹² Ethylcarbonyl bedeutet und die Substituenten R³-R⁵ für jede einzelne Verbindung jeweils einer Zeile der Tabelle B entsprechen.

### Tabelle 183: Verbindungen 183.1-183.514

Verbindungen der allgemeinen Formel Ic, in der R¹ Cl, R² Cl, R¹¹ n-Propyl, R¹² Ethylcarbonyl bedeutet und die Substituenten R³-R⁵ für jede einzelne Verbindung jeweils einer Zeile der Tabelle B entsprechen.

### Tabelle 184: Verbindungen 184.1-184.514

Verbindungen der allgemeinen Formel Ic, in der R¹ Cl, R² Cl, R¹¹ Methyl, R¹² Methylsulfonyl bedeutet und die Substituenten R³-R⁵ für jede einzelne Verbindung jeweils einer Zeile der Tabelle B entsprechen.

### Tabelle 185: Verbindungen 185.1-185.514

Verbindungen der allgemeinen Formel Ic, in der R¹ Cl, R² Cl, R¹¹ Ethyl, R¹² Methylsulfonyl bedeutet und die Substituenten R³-R⁵ für jede einzelne Verbindung jeweils einer Zeile der Tabelle B entsprechen.

### Tabelle 186: Verbindungen 186.1-186.514

Verbindungen der allgemeinen Formel Ic, in der R¹ Cl, R² Cl, R¹¹ n-Propyl, R¹² Methylsulfonyl bedeutet und die Substituenten R³-R⁵ für jede einzelne Verbindung jeweils einer Zeile der Tabelle B entsprechen.

### Tabelle 187: Verbindungen 187.1-187.514

Verbindungen der allgemeinen Formel Ic, in der R¹ Cl, R² Cl, R¹¹ Methyl, R¹² Ethylsulfonyl bedeutet und die Substituenten R³-R⁵ für jede einzelne Verbindung jeweils einer Zeile der Tabelle B entsprechen.

### Tabelle 188: Verbindungen 188.1-188.514

Verbindungen der allgemeinen Formel Ic, in der R¹ Cl, R² Cl, R¹¹ Ethyl, R¹² Ethylsulfonyl bedeutet und die Substituenten R³-R⁵ für jede einzelne Verbindung jeweils einer Zeile der Tabelle B entsprechen.

### Tabelle 189: Verbindungen 189.1-189.514

Verbindungen der allgemeinen Formel Ic, in der R¹ Cl, R² Cl, R¹¹ n-Propyl, R¹² Ethylsulfonyl bedeutet und die Substituenten R³-R⁵ für jede einzelne Verbindung jeweils einer Zeile der Tabelle B entsprechen.

### Tabelle 190: Verbindungen 190.1-190.514

Verbindungen der allgemeinen Formel Ic, in der R¹ Cl, R² Cl, R¹¹ Methyl, R¹² 4-Methylphenylsulfonyl bedeutet und die Substituenten R³-R⁵ für jede einzelne Verbindung jeweils einer Zeile der Tabelle B entsprechen.

### Tabelle 191: Verbindungen 191.1-191.514

Verbindungen der allgemeinen Formel Ic, in der R¹ Cl, R² Cl, R¹¹ Ethyl, R¹² 4-Methylphenylsulfonyl bedeutet und die Substituenten R³-R⁵ für jede einzelne Verbindung jeweils einer Zeile der Tabelle B entsprechen.

### Tabelle 192: Verbindungen 192.1-192.514

Verbindungen der allgemeinen Formel Ic, in der R¹ Cl, R² Cl, R¹¹ n-Propyl, R¹² 4-Methylphenylsulfonyl bedeutet und die Substituenten R³-R⁵ für jede einzelne Verbindung jeweils einer Zeile der Tabelle B entsprechen.

### Tabelle 193: Verbindungen 193.1-193.514

Verbindungen der allgemeinen Formel Ic, in der R¹ Cl, R² Trifluormethyl, R¹¹ Methyl und R¹² Wasserstoff bedeutet und die Substituenten R³-R⁵ für jede einzelne Verbindung jeweils einer Zeile der Tabelle B entsprechen.

### Tabelle 194: Verbindungen 194.1-194.514

Verbindungen der allgemeinen Formel Ic, in der R¹ Cl, R² Trifluormethyl, R¹¹ Ethyl, R¹² Wasserstoff bedeutet und die Substituenten R³-R⁵ für jede einzelne Verbindung jeweils einer Zeile der Tabelle B entsprechen.

### Tabelle 195: Verbindungen 195.1-195.514

Verbindungen der allgemeinen Formel Ic, in der R¹ Cl, R² Trifluormethyl, R¹¹ n-Propyl, R¹² Wasserstoff bedeutet und die Substituenten R³-R⁵ für jede einzelne Verbindung jeweils einer Zeile der Tabelle B entsprechen.

### Tabelle 196: Verbindungen 196.1-196.514

Verbindungen der allgemeinen Formel Ic, in der R¹ Cl, R² Trifluormethyl, R¹¹ Methyl, R¹² Methyl bedeutet und die Substituenten R³-R⁵ für jede einzelne Verbindung jeweils einer Zeile der Tabelle B entsprechen.

### Tabelle 197: Verbindungen 197.1-197.514

Verbindungen der allgemeinen Formel Ic, in der R¹ Cl, R² Trifluormethyl, R¹¹ Ethyl, R¹² Methyl bedeutet und die Substituenten R³-R⁵ für jede einzelne Verbindung jeweils einer Zeile der Tabelle B entsprechen.

### Tabelle 198: Verbindungen 198.1-198.514

Verbindungen der allgemeinen Formel Ic, in der R¹ Cl, R² Trifluormethyl, R¹¹ n-Propyl, R¹² Methyl bedeutet und die Substituenten R³-R⁵ für jede einzelne Verbindung jeweils einer Zeile der Tabelle B entsprechen.

### Tabelle 199: Verbindungen 199.1-199.514

Verbindungen der allgemeinen Formel Ic, in der R¹ Cl, R² Trifluormethyl, R¹¹ Methyl, R¹² Ethyl bedeutet und die Substituenten R³-R⁵ für jede einzelne Verbindung jeweils einer Zeile der Tabelle B entsprechen.

### Tabelle 200: Verbindungen 200.1-200.514

Verbindungen der allgemeinen Formel Ic, in der R¹ Cl, R² Trifluormethyl, R¹¹ Ethyl, R¹² Ethyl bedeutet und die Substituenten R³-R⁵ für jede einzelne Verbindung jeweils einer Zeile der Tabelle B entsprechen.

### Tabelle 201: Verbindungen 201.1-201.514

Verbindungen der allgemeinen Formel Ic, in der R¹ Cl, R² Trifluormethyl, R¹¹ n-Propyl, R¹² Ethyl bedeutet und die Substituenten R³-R⁵ für jede einzelne Verbindung jeweils einer Zeile der Tabelle B entsprechen.

### Tabelle 202: Verbindungen 202.1-202.514

Verbindungen der allgemeinen Formel Ic, in der R¹ Cl, R² Trifluormethyl, R¹¹ Methyl, R¹² Methylcarbonyl bedeutet und die Substituenten R³-R⁵ für jede einzelne Verbindung jeweils einer Zeile der Tabelle B entsprechen.

### Tabelle 203: Verbindungen 203.1-203.514

Verbindungen der allgemeinen Formel Ic, in der R¹ Cl, R² Trifluormethyl, R¹¹ Ethyl, R¹² Methylcarbonyl bedeutet und die Substituenten R³-R⁵ für jede einzelne Verbindung jeweils einer Zeile der Tabelle B entsprechen.

### Tabelle 204: Verbindungen 204.1-204.514

Verbindungen der allgemeinen Formel Ic, in der R¹ Cl, R² Trifluormethyl, R¹¹ n-Propyl, R¹² Methylcarbonyl bedeutet und die Substituenten R³-R⁵ für jede einzelne Verbindung jeweils einer Zeile der Tabelle B entsprechen.

### Tabelle 205: Verbindungen 205.1-205.514

Verbindungen der allgemeinen Formel Ic, in der R¹ Cl, R² Trifluormethyl, R¹¹ Methyl, R¹² Ethylcarbonyl bedeutet und die Substituenten R³-R⁵ für jede einzelne Verbindung jeweils einer Zeile der Tabelle B entsprechen.

### Tabelle 206: Verbindungen 206.1-206.514

Verbindungen der allgemeinen Formel Ic, in der R¹ Cl, R² Trifluormethyl, R¹¹ Ethyl, R¹² Ethylcarbonyl bedeutet und die Substituenten R³-R⁵ für jede einzelne Verbindung jeweils einer Zeile der Tabelle B entsprechen.

### Tabelle 207: Verbindungen 207.1-207.514

Verbindungen der allgemeinen Formel Ic, in der R¹ Cl, R² Trifluormethyl, R¹¹ n-Propyl, R¹² Ethylcarbonyl bedeutet und die Substituenten R³-R⁵ für jede einzelne Verbindung jeweils einer Zeile der Tabelle B entsprechen.

### Tabelle 208: Verbindungen 208.1-208.514

Verbindungen der allgemeinen Formel Ic, in der R¹ Cl, R² Trifluormethyl, R¹¹ Methyl, R¹² Methylsulfonyl bedeutet und die Substituenten R³-R⁵ für jede einzelne Verbindung jeweils einer Zeile der Tabelle B entsprechen.

### Tabelle 209: Verbindungen 209.1-209.514

Verbindungen der allgemeinen Formel Ic, in der R¹ Cl, R² Trifluormethyl, R¹¹ Ethyl, R¹² Methylsulfonyl bedeutet und die Substituenten R³-R⁵ für jede einzelne Verbindung jeweils einer Zeile der Tabelle B entsprechen.

### Tabelle 210: Verbindungen 210.1-210.514

Verbindungen der allgemeinen Formel Ic, in der R¹ Cl, R² Trifluormethyl, R¹¹ n-Propyl, R¹² Methylsulfonyl bedeutet und die Substituenten R³-R⁵ für jede einzelne Verbindung jeweils einer Zeile der Tabelle B entsprechen.

### Tabelle 211: Verbindungen 211.1-211.514

Verbindungen der allgemeinen Formel Ic, in der R¹ Cl, R² Trifluormethyl, R¹¹ Methyl, R¹² Ethylsulfonyl bedeutet und die Substituenten R³-R⁵ für jede einzelne Verbindung jeweils einer Zeile der Tabelle B entsprechen.

### Tabelle 212: Verbindungen 212.1-212.514

Verbindungen der allgemeinen Formel Ic, in der R¹ Cl, R² Trifluormethyl, R¹¹ Ethyl, R¹² Ethylsulfonyl bedeutet und die Substituenten R³-R⁵ für jede einzelne Verbindung jeweils einer Zeile der Tabelle B entsprechen.

### Tabelle 213: Verbindungen 213.1-213.514

Verbindungen der allgemeinen Formel Ic, in der R¹ Cl, R² Trifluormethyl, R¹¹ n-Propyl, R¹² Ethylsulfonyl bedeutet und die Substituenten R³-R⁵ für jede einzelne Verbindung jeweils einer Zeile der Tabelle B entsprechen.

### Tabelle 214: Verbindungen 214.1-214.514

Verbindungen der allgemeinen Formel Ic, in der R¹ Cl, R² Trifluormethyl, R¹¹ Methyl, R¹² 4-Methylphenylsulfonyl bedeutet und die Substituenten R³-R⁵ für jede einzelne Verbindung jeweils einer Zeile der Tabelle B entsprechen.

### Tabelle 215: Verbindungen 215.1-215.514

Verbindungen der allgemeinen Formel Ic, in der R¹ Cl, R² Trifluormethyl, R¹¹ Ethyl, R¹² 4-Methylphenylsulfonyl bedeutet und die Substituenten R¹-R⁵ für jede einzelne Verbindung jeweils einer Zeile der Tabelle B entsprechen.

### Tabelle 216: Verbindungen 216.1-216.514

Verbindungen der allgemeinen Formel Ic, in der R¹ Cl, R² Trifluormethyl, R¹¹ n-Propyl, R¹² 4-Methylphenylsulfonyl bedeutet und die Substituenten R³-R⁵ für jede einzelne Verbindung jeweils einer Zeile der Tabelle B entsprechen.

### Tabelle 217: Verbindungen 217.1-217.514

Verbindungen der allgemeinen Formel Ic, in der R¹ Methyl, R² Cl, R¹¹ Methyl und R¹² Wasserstoff bedeutet und die Substituenten R³-R⁵ für jede einzelne Verbindung jeweils einer Zeile der Tabelle B entsprechen.

### Tabelle 218: Verbindungen 218.1-218.514

Verbindungen der allgemeinen Formel Ic, in der R¹ Methyl, R² Cl, R¹¹ Ethyl, R¹² Wasserstoff bedeutet und die Substituenten R³-R⁵ für jede einzelne Verbindung jeweils einer Zeile der Tabelle B entsprechen.

### Tabelle 219: Verbindungen 219.1-219.514

Verbindungen der allgemeinen Formel Ic, in der R¹ Methyl, R² Cl, R¹¹ n-Propyl, R¹² Wasserstoff bedeutet und die Substituenten R³-R⁵ für jede einzelne Verbindung jeweils einer Zeile der Tabelle B entsprechen.

### Tabelle 220: Verbindungen 220.1-220.514

Verbindungen der allgemeinen Formel Ic, in der R¹ Methyl, R² Cl, R¹¹ Methyl, R¹² Methyl bedeutet und die Substituenten R³-R⁵ für jede einzelne Verbindung jeweils einer Zeile der Tabelle B entsprechen.

### Tabelle 221: Verbindungen 221.1-221.514

Verbindungen der allgemeinen Formel Ic, in der R¹ Methyl, R² Cl, R¹¹ Ethyl, R¹² Methyl bedeutet und die Substituenten R³-R⁵ für jede einzelne Verbindung jeweils einer Zeile der Tabelle B entsprechen.

### Tabelle 222: Verbindungen 222.1-222.514

Verbindungen der allgemeinen Formel Ic, in der R¹ Methyl, R² Cl, R¹¹ n-Propyl, R¹² Methyl bedeutet und die Substituenten R³-R⁵ für jede einzelne Verbindung jeweils einer Zeile der Tabelle B entsprechen.

### Tabelle 223: Verbindungen 223.1-223.514

Verbindungen der allgemeinen Formel Ic, in der R¹ Methyl, R² Cl, R¹¹ Methyl, R¹² Ethyl bedeutet und die Substituenten R³-R⁵ für jede einzelne Verbindung jeweils einer Zeile der Tabelle B entsprechen.

### Tabelle 224: Verbindungen 224.1-224.514

Verbindungen der allgemeinen Formel Ic, in der R¹ Methyl, R² Cl, R¹¹ Ethyl, R¹² Ethyl bedeutet und die Substituenten R³-R⁵ für jede einzelne Verbindung jeweils einer Zeile der Tabelle B entsprechen.

### Tabelle 225: Verbindungen 225.1-225.514

Verbindungen der allgemeinen Formel Ic, in der R¹ Methyl, R² Cl, R¹¹ n-Propyl, R¹² Ethyl bedeutet und die Substituenten R³-R⁵ für jede einzelne Verbindung jeweils einer Zeile der Tabelle B entsprechen.

### Tabelle 226: Verbindungen 226.1-226.514

Verbindungen der allgemeinen Formel Ic, in der R¹ Methyl, R² Cl, R¹¹ Methyl, R¹² Methylcarbonyl bedeutet und die Substituenten R³-R⁵ für jede einzelne Verbindung jeweils einer Zeile der Tabelle B entsprechen.

### Tabelle 227: Verbindungen 227.1-227.514

Verbindungen der allgemeinen Formel Ic, in der R¹ Methyl, R² Cl, R¹¹ Ethyl, R¹² Methylcarbonyl bedeutet und die Substituenten R³-R⁵ für jede einzelne Verbindung jeweils einer Zeile der Tabelle B entsprechen.

### Tabelle 228: Verbindungen 228.1-228.514

Verbindungen der allgemeinen Formel Ic, in der R¹ Methyl, R² Cl, R¹¹ n-Propyl, R¹² Methylcarbonyl bedeutet und die Substituenten R³-R⁵ für jede einzelne Verbindung jeweils einer Zeile der Tabelle B entsprechen.

### Tabelle 229: Verbindungen 229.1-229.514

Verbindungen der allgemeinen Formel Ic, in der R¹ Methyl, R² Cl, R¹¹ Methyl, R¹² Ethylcarbonyl bedeutet und die Substituenten R³-R⁵ für jede einzelne Verbindung jeweils einer Zeile der Tabelle B entsprechen.

### Tabelle 230: Verbindungen 230.1-230.514

Verbindungen der allgemeinen Formel Ic, in der R¹ Methyl, R² Cl, R¹¹ Ethyl, R¹² Ethylcarbonyl bedeutet und die Substituenten R³-R⁵ für jede einzelne Verbindung jeweils einer Zeile der Tabelle B entsprechen.

### Tabelle 231: Verbindungen 231.1-231.514

Verbindungen der allgemeinen Formel Ic, in der R¹ Methyl, R² Cl, R¹¹ n-Propyl, R¹² Ethylcarbonyl bedeutet und die Substituenten R³-R⁵ für jede einzelne Verbindung jeweils einer Zeile der Tabelle B entsprechen.

### Tabelle 232: Verbindungen 232.1-232.514

Verbindungen der allgemeinen Formel Ic, in der R¹ Methyl, R² Cl, R¹¹ Methyl, R¹² Methylsulfonyl bedeutet und die Substituenten R³-R⁵ für jede einzelne Verbindung jeweils einer Zeile der Tabelle B entsprechen.

### Tabelle 233: Verbindungen 233.1-233.514

Verbindungen der allgemeinen Formel Ic, in der R¹ Methyl, R² Cl, R¹¹ Ethyl, R¹² Methylsulfonyl bedeutet und die Substituenten R³-R⁵ für jede einzelne Verbindung jeweils einer Zeile der Tabelle B entsprechen.

### Tabelle 234: Verbindungen 234.1-234.514

Verbindungen der allgemeinen Formel Ic, in der R¹ Methyl, R² Cl, R¹¹ n-Propyl, R¹² Methylsulfonyl bedeutet und die Substituenten R³-R⁵ für jede einzelne Verbindung jeweils einer Zeile der Tabelle B entsprechen.

### Tabelle 235: Verbindungen 235.1-235.514

Verbindungen der allgemeinen Formel Ic, in der R¹ Methyl, R² Cl, R¹¹ Methyl, R¹² Ethylsulfonyl bedeutet und die Substituenten R³-R⁵ für jede einzelne Verbindung jeweils einer Zeile der Tabelle B entsprechen.

### Tabelle 236: Verbindungen 236.1-236.514

Verbindungen der allgemeinen Formel Ic, in der R¹ Methyl, R² Cl, R¹¹ Ethyl, R¹² Ethylsulfonyl bedeutet und die Substituenten R³-R⁵ für jede einzelne Verbindung jeweils einer Zeile der Tabelle B entsprechen.

### Tabelle 237: Verbindungen 237.1-237.514

Verbindungen der allgemeinen Formel Ic, in der R¹ Methyl, R² Cl, R¹¹ n-Propyl, R¹² Ethylsulfonyl bedeutet und die Substituenten R³-R⁵ für jede einzelne Verbindung jeweils einer Zeile der Tabelle B entsprechen.

### Tabelle 238: Verbindungen 238.1-238.514

Verbindungen der allgemeinen Formel Ic, in der R¹ Methyl, R² Cl, R¹¹ Methyl, R¹² 4-Methylphenylsulfonyl bedeutet und die Substituenten R³-R⁵ für jede einzelne Verbindung jeweils einer Zeile der Tabelle B entsprechen.

### Tabelle 239: Verbindungen 239.1-239.514

Verbindungen der allgemeinen Formel Ic, in der R¹ Methyl, R² Cl, R¹¹ Ethyl, R¹² 4-Methylphenylsulfonyl bedeutet und die Substituenten R³-R⁵ für jede einzelne Verbindung jeweils einer Zeile der Tabelle B entsprechen.

### Tabelle 240: Verbindungen 240.1-240.514

Verbindungen der allgemeinen Formel Ic, in der R¹ Methyl, R² Cl, R¹¹ n-Propyl, R¹² 4-Methylphenylsulfonyl bedeutet und die Substituenten R³-R⁵ für jede einzelne Verbindung jeweils einer Zeile der Tabelle B entsprechen.

### Tabelle 241: Verbindungen 241.1-241.514

Verbindungen der allgemeinen Formel Ic, in der R¹ Methyl, R² Methylsulfonyl, R¹¹ Methyl und R¹² Wasserstoff bedeutet und die Substituenten R³-R⁵ für jede einzelne Verbindung jeweils einer Zeile der Tabelle B entsprechen.

### Tabelle 242: Verbindungen 242.1-242.514

Verbindungen der allgemeinen Formel Ic, in der R¹ Methyl, R² Methylsulfonyl, R¹¹ Ethyl, R¹² Wasserstoff bedeutet und die Substituenten R³-R⁵ für jede einzelne Verbindung jeweils einer Zeile der Tabelle B entsprechen.

### Tabelle 243: Verbindungen 243.1-243.514

Verbindungen der allgemeinen Formel Ic, in der R¹ Methyl, R² Methylsulfonyl, R¹¹ n-Propyl, R¹² Wasserstoff bedeutet und die Substituenten R³-R⁵ für jede einzelne Verbindung jeweils einer Zeile der Tabelle B entsprechen.

### Tabelle 244: Verbindungen 244.1-244.514

Verbindungen der allgemeinen Formel Ic, in der R¹ Methyl, R² Methylsulfonyl, R¹¹ Methyl, R¹² Methyl bedeutet und die Substituenten R³-R⁵ für jede einzelne Verbindung jeweils einer Zeile der Tabelle B entsprechen.

### Tabelle 245: Verbindungen 245.1-245.514

Verbindungen der allgemeinen Formel Ic, in der R¹ Methyl, R² Methylsulfonyl, R¹¹ Ethyl, R¹² Methyl bedeutet und die Substituenten R³-R⁵ für jede einzelne Verbindung jeweils einer Zeile der Tabelle B entsprechen.

### Tabelle 246: Verbindungen 246.1-246.514

Verbindungen der allgemeinen Formel Ic, in der R¹ Methyl, R² Methylsulfonyl, R¹¹ n-Propyl, R¹² Methyl bedeutet und die Substituenten R³-R⁵ für jede einzelne Verbindung jeweils einer Zeile der Tabelle B entsprechen.

### Tabelle 247: Verbindungen 247.1-247.514

Verbindungen der allgemeinen Formel Ic, in der R¹ Methyl, R² Methylsulfonyl, R¹¹ Methyl, R¹² Ethyl bedeutet und die Substituenten R³-R⁵ für jede einzelne Verbindung jeweils einer Zeile der Tabelle B entsprechen.

### Tabelle 248: Verbindungen 248.1-248.514

Verbindungen der allgemeinen Formel Ic, in der R¹ Methyl, R² Methylsulfonyl, R¹¹ Ethyl, R¹² Ethyl bedeutet und die Substituenten R³-R⁵ für jede einzelne Verbindung jeweils einer Zeile der Tabelle B entsprechen.

### Tabelle 249: Verbindungen 249.1-249.514

Verbindungen der allgemeinen Formel Ic, in der R¹ Methyl, R² Methylsulfonyl, R¹¹ n-Propyl, R¹² Ethyl bedeutet und die Substituenten R³-R⁵ für jede einzelne Verbindung jeweils einer Zeile der Tabelle B entsprechen.

### Tabelle 250: Verbindungen 250.1-250.514

Verbindungen der allgemeinen Formel Ic, in der R¹ Methyl, R² Methylsulfonyl, R¹¹ Methyl, R¹² Methylcarbonyl bedeutet und die Substituenten R³-R⁵ für jede einzelne Verbindung jeweils einer Zeile der Tabelle B entsprechen.

### Tabelle 251: Verbindungen 251.1-251.514

Verbindungen der allgemeinen Formel Ic, in der R¹ Methyl, R² Methylsulfonyl, R¹¹ Ethyl, R¹² Methylcarbonyl bedeutet und die Substituenten R³-R⁵ für jede einzelne Verbindung jeweils einer Zeile der Tabelle B entsprechen.

### Tabelle 252: Verbindungen 252.1-252.514

Verbindungen der allgemeinen Formel Ic, in der R¹ Methyl, R² Methylsulfonyl, R¹¹ n-Propyl, R¹² Methylcarbonyl bedeutet und die Substituenten R³-R⁵ für jede einzelne Verbindung jeweils einer Zeile der Tabelle B entsprechen.

### Tabelle 253: Verbindungen 253.1-253.514

Verbindungen der allgemeinen Formel Ic, in der R¹ Methyl, R² Methylsulfonyl, R¹¹ Methyl, R¹² Ethylcarbonyl bedeutet und die Substituenten R³-R⁵ für jede einzelne Verbindung jeweils einer Zeile der Tabelle B entsprechen.

### Tabelle 254: Verbindungen 254.1-254.514

Verbindungen der allgemeinen Formel Ic, in der R¹ Methyl, R² Methylsulfonyl, R¹¹ Ethyl, R¹² Ethylcarbonyl bedeutet und die Substituenten R³-R⁵ für jede einzelne Verbindung jeweils einer Zeile der Tabelle B entsprechen.

### Tabelle 255: Verbindungen 255.1-255.514

Verbindungen der allgemeinen Formel Ic, in der R¹ Methyl, R² Methylsulfonyl, R¹¹ n-Propyl, R¹² Ethylcarbonyl bedeutet und die Substituenten R¹-R⁵ für jede einzelne Verbindung jeweils einer Zeile der Tabelle B entsprechen.

### Tabelle 256: Verbindungen 256.1-256.514

Verbindungen der allgemeinen Formel Ic, in der R¹ Methyl, R² Methylsulfonyl, R¹¹ Methyl, R¹² Methylsulfonyl bedeutet und die Substituenten R³-R⁵ für jede einzelne Verbindung jeweils einer Zeile der Tabelle B entsprechen.

### Tabelle 257: Verbindungen 257.1-257.514

Verbindungen der allgemeinen Formel Ic, in der R¹ Methyl, R² Methylsulfonyl, R¹¹ Ethyl, R¹² Methylsulfonyl bedeutet und die Substituenten R³-R⁵ für jede einzelne Verbindung jeweils einer Zeile der Tabelle B entsprechen.

### Tabelle 258: Verbindungen 258.1-258.514

Verbindungen der allgemeinen Formel Ic, in der R¹ Methyl, R² Methylsulfonyl, R¹¹ n-Propyl, R¹² Methylsulfonyl bedeutet und die Substituenten R¹-R⁵ für jede einzelne Verbindung jeweils einer Zeile der Tabelle B entsprechen.

### Tabelle 259: Verbindungen 259.1-259.514

Verbindungen der allgemeinen Formel Ic, in der R¹ Methyl, R² Methylsulfonyl, R¹¹ Methyl, R¹² Ethylsulfonyl bedeutet und die Substituenten R³-R⁵ für jede einzelne Verbindung jeweils einer Zeile der Tabelle B entsprechen.

### Tabelle 260: Verbindungen 260.1-260.514

Verbindungen der allgemeinen Formel Ic, in der R¹ Methyl, R² Methylsulfonyl, R¹¹ Ethyl, R¹² Ethylsulfonyl bedeutet und die Substituenten R³-R⁵ für jede einzelne Verbindung jeweils einer Zeile der Tabelle B entsprechen.

### Tabelle 261: Verbindungen 261.1-261.514

Verbindungen der allgemeinen Formel Ic, in der R¹ Methyl, R² Methylsulfonyl, R¹¹ n-Propyl, R¹² Ethylsulfonyl bedeutet und die Substituenten R³-R⁵ für jede einzelne Verbindung jeweils einer Zeile der Tabelle B entsprechen.

### Tabelle 262: Verbindungen 262.1-262.514

Verbindungen der allgemeinen Formel Ic, in der R¹ Methyl, R² Methylsulfonyl, R¹¹ Methyl, R¹² 4-Methylphenylsulfonyl bedeutet und die Substituenten R³-R⁵ für jede einzelne Verbindung jeweils einer Zeile der Tabelle B entsprechen.

### Tabelle 263: Verbindungen 263.1-263.514

Verbindungen der allgemeinen Formel Ic, in der R¹ Methyl, R² Methylsulfonyl, R¹¹ Ethyl, R¹² 4-Methylphenylsulfonyl bedeutet und die Substituenten R³-R⁵ für jede einzelne Verbindung jeweils einer Zeile der Tabelle B entsprechen.

### Tabelle 264: Verbindungen 264.1-264.514

Verbindungen der allgemeinen Formel Ic, in der R¹ Methyl, R² Methylsulfonyl, R¹¹ n-Propyl, R¹² 4-Methylphenylsulfonyl bedeutet und die Substituenten R³-R⁵ für jede einzelne Verbindung jeweils einer Zeile der Tabelle B entsprechen.

### Tabelle 265: Verbindungen 265.1-265.514

Verbindungen der allgemeinen Formel Ic, in der R¹ Methyl, R² Trifluormethyl, R¹¹ Methyl und R¹² Wasserstoff bedeutet und die Substituenten R³-R⁵ für jede einzelne Verbindung jeweils einer Zeile der Tabelle B entsprechen.

### Tabelle 266: Verbindungen 266.1-266.514

Verbindungen der allgemeinen Formel Ic, in der R¹ Methyl, R² Trifluormethyl, R¹¹ Ethyl, R¹² Wasserstoff bedeutet und die Substituenten R³-R⁵ für jede einzelne Verbindung jeweils einer Zeile der Tabelle B entsprechen.

### Tabelle 267: Verbindungen 267.1-267.514

Verbindungen der allgemeinen Formel Ic, in der R¹ Methyl, R² Trifluormethyl, R¹¹ n-Propyl, R¹² Wasserstoff bedeutet und die Substituenten R³-R⁵ für jede einzelne Verbindung jeweils einer Zeile der Tabelle B entsprechen.

### Tabelle 268: Verbindungen 268.1-268.514

Verbindungen der allgemeinen Formel Ic, in der R¹ Methyl, R² Trifluormethyl, R¹¹ Methyl, R¹² Methyl bedeutet und die Substituenten R³-R⁵ für jede einzelne Verbindung jeweils einer Zeile der Tabelle B entsprechen.

### Tabelle 269: Verbindungen 269.1-269.514

Verbindungen der allgemeinen Formel Ic, in der R¹ Methyl, R² Trifluormethyl, R¹¹ Ethyl, R¹² Methyl bedeutet und die Substituenten R³-R⁵ für jede einzelne Verbindung jeweils einer Zeile der Tabelle B entsprechen.

### Tabelle 270: Verbindungen 270.1-270.514

Verbindungen der allgemeinen Formel Ic, in der R¹ Methyl, R² Trifluormethyl, R¹¹ n-Propyl, R¹² Methyl bedeutet und die Substituenten R³-R⁵ für jede einzelne Verbindung jeweils einer Zeile der Tabelle B entsprechen.

### Tabelle 271: Verbindungen 271.1-271.514

Verbindungen der allgemeinen Formel Ic, in der R¹ Methyl, R² Trifluormethyl, R¹¹ Methyl, R¹² Ethyl bedeutet und die Substituenten R³-R⁵ für jede einzelne Verbindung jeweils einer Zeile der Tabelle B entsprechen.

### Tabelle 272: Verbindungen 272.1-272.514

Verbindungen der allgemeinen Formel Ic, in der R¹ Methyl, R² Trifluormethyl, R¹¹ Ethyl, R¹² Ethyl bedeutet und die Substituenten R³-R⁵ für jede einzelne Verbindung jeweils einer Zeile der Tabelle B entsprechen.

### Tabelle 273: Verbindungen 273.1-273.514

Verbindungen der allgemeinen Formel Ic, in der R¹ Methyl, R² Trifluormethyl, R¹¹ n-Propyl, R¹² Ethyl bedeutet und die Substituenten R³-R⁵ für jede einzelne Verbindung jeweils einer Zeile der Tabelle B entsprechen.

### Tabelle 274: Verbindungen 274.1-274.514

Verbindungen der allgemeinen Formel Ic, in der R¹ Methyl, R² Trifluormethyl, R¹¹ Methyl, R¹² Methylcarbonyl bedeutet und die Substituenten R³-R⁵ für jede einzelne Verbindung jeweils einer Zeile der Tabelle B entsprechen.

### Tabelle 275: Verbindungen 275.1-275.514

Verbindungen der allgemeinen Formel Ic, in der R¹ Methyl, R² Trifluormethyl, R¹¹ Ethyl, R¹² Methylcarbonyl bedeutet und die Substituenten R³-R⁵ für jede einzelne Verbindung jeweils einer Zeile der Tabelle B entsprechen.

### Tabelle 276: Verbindungen 276.1-276.514

Verbindungen der allgemeinen Formel Ic, in der R¹ Methyl, R² Trifluormethyl, R¹¹ n-Propyl, R¹² Methylcarbonyl bedeutet und die Substituenten R³-R⁵ für jede einzelne Verbindung jeweils einer Zeile der Tabelle B entsprechen.

### Tabelle 277: Verbindungen 277.1-277.514

Verbindungen der allgemeinen Formel Ic, in der R¹ Methyl, R² Trifluormethyl, R¹¹ Methyl, R¹² Ethylcarbonyl bedeutet und die Substituenten R³-R⁵ für jede einzelne Verbindung jeweils einer Zeile der Tabelle B entsprechen.

### Tabelle 278: Verbindungen 278.1-278.514

Verbindungen der allgemeinen Formel Ic, in der R¹ Methyl, R² Trifluormethyl, R¹¹ Ethyl, R¹² Ethylcarbonyl bedeutet und die Substituenten R³-R⁵ für jede einzelne Verbindung jeweils einer Zeile der Tabelle B entsprechen.

### Tabelle 279: Verbindungen 279.1-279.514

Verbindungen der allgemeinen Formel Ic, in der R¹ Methyl, R² Trifluormethyl, R¹¹ n-Propyl, R¹² Ethylcarbonyl bedeutet und die Substituenten R³-R⁵ für jede einzelne Verbindung jeweils einer Zeile der Tabelle B entsprechen.

### Tabelle 280: Verbindungen 280.1-280.514

Verbindungen der allgemeinen Formel Ic, in der R¹ Methyl, R² Trifluormethyl, R¹¹ Methyl, R¹² Methylsulfonyl bedeutet und die Substituenten R³-R⁵ für jede einzelne Verbindung jeweils einer Zeile der Tabelle B entsprechen.

### Tabelle 281: Verbindungen 281.1-281.514

Verbindungen der allgemeinen Formel Ic, in der R¹ Methyl, R² Trifluormethyl, R¹¹ Ethyl, R¹² Methylsulfonyl bedeutet und die Substituenten R³-R⁵ für jede einzelne Verbindung jeweils einer Zeile der Tabelle B entsprechen.

### Tabelle 282: Verbindungen 282.1-282.514

Verbindungen der allgemeinen Formel Ic, in der R¹ Methyl, R² Trifluormethyl, R¹¹ n-Propyl, R¹² Methylsulfonyl bedeutet und die Substituenten R³-R⁵ für jede einzelne Verbindung jeweils einer Zeile der Tabelle B entsprechen.

### Tabelle 283: Verbindungen 283.1-283.514

Verbindungen der allgemeinen Formel Ic, in der R¹ Methyl, R² Trifluormethyl, R¹¹ Methyl, R¹² Ethylsulfonyl bedeutet und die Substituenten R³-R⁵ für jede einzelne Verbindung jeweils einer Zeile der Tabelle B entsprechen.

### Tabelle 284: Verbindungen 284.1-284.514

Verbindungen der allgemeinen Formel Ic, in der R¹ Methyl, R² Trifluormethyl, R¹¹ Ethyl, R¹² Ethylsulfonyl bedeutet und die Substituenten R³-R⁵ für jede einzelne Verbindung jeweils einer Zeile der Tabelle B entsprechen.

### Tabelle 285: Verbindungen 285.1-285.514

Verbindungen der allgemeinen Formel Ic, in der R¹ Methyl, R² Trifluormethyl, R¹¹ n-Propyl, R¹² Ethylsulfonyl bedeutet und die Substituenten R³-R⁵ für jede einzelne Verbindung jeweils einer Zeile der Tabelle B entsprechen.

### Tabelle 286: Verbindungen 286.1-286.514

Verbindungen der allgemeinen Formel Ic, in der R¹ Methyl, R² Trifluormethyl, R¹¹ Methyl, R¹² 4-Methylphenylsulfonyl bedeutet und die Substituenten R³-R⁵ für jede einzelne Verbindung jeweils einer Zeile der Tabelle B entsprechen.

### Tabelle 287: Verbindungen 287.1-287.514

Verbindungen der allgemeinen Formel Ic, in der R¹ Methyl, R² Trifluonnethyl, R¹¹ Ethyl, R¹² 4-Methylphenylsulfonyl bedeutet und die Substituenten R³-R⁵ für jede einzelne Verbindung jeweils einer Zeile der Tabelle B entsprechen.

### Tabelle 288: Verbindungen 288.1-288.514

Verbindungen der allgemeinen Formel Ic, in der R¹ Methyl, R² Trifluormethyl, R¹¹ n-Propyl, R¹² 4-Methylphenylsulfonyl bedeutet und die Substituenten R³-R⁵ für jede einzelne Verbindung jeweils einer Zeile der Tabelle B entsprechen.

Beispielsweise seien dié Verbindungen Id der Tabellen 289 bis 432 genannt, wobei die Substituenten R³, R⁸ und R⁹ für jede einzelne Verbindung Id jeweils einer Zeile der Tabelle C entsprechen.

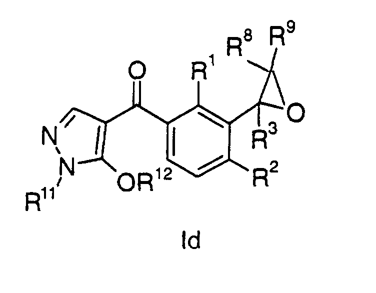

**Tabelle C**

| Nr. | R³ | R⁸ | R⁹ |
|---|---|---|---|
| 1 | H | H | H |
| 2 | H | CH₃ | H |
| 3 | H | C₂H₅ | H |
| 4 | H | C₃H₇ | H |
| 5 | H | C₄H₉ | H |
| 6 | H | CH(CH₃)₂ | H |
| 7 | H | cy-C3H₅ | H |
| 8 | H | cy-C₄H₇ | H |
| 9 | H | cy-C₅H₉ | H |
| 10 | H | cy-C₆-H₁₁ | H |
| 11 | H | C₆H₅ | H |
| 12 | H | CH₂-C₆H₅ | H |
| 13 | H | 2-Furyl | H |
| 14 | H | 3-Furyl | H |
| 15 | H | 2-Thienyl | H |
| 16 | H | 3-Thienyl | H |
| 17 | H | 2-Dioxanyl | H |
| 18 | H | CHO | H |
| 19 | H | COCH₃ | H |
| 20 | H | COOCH₃ | H |
| 21 | H | COOC₂H₅ | H |
| 22 | H | OCH₃ | H |
| 23 | H | CN | H |
| 24 | H | SCH₃ | H |
| 25 | H | COCF₃ | H |
| 26 | H | COC₆H₅ | H |
| 27 | H | CH = NOCH₃ | H |
| 28 | H | CH = NOC₂H₅ | H |
| 29 | H | C(CH₃) = NOCH₃ | H |
| 30 | H | CH₃ | CH₃ |
| 31 | H | C₂H₅ | CH₃ |
| 32 | H | C₃H₇ | CH₃ |
| 33 | H | C₄H₉ | CH₃ |
| 34 | H | CHO | CH₃ |
| 35 | H | COCH₃ | CH₃ |
| 36 | H | COOCH₃ | CH₃ |
| 37 | H | OCH₃ | CH₃ |
| 38 | H | C₆H₅ | CH₃ |
| 39 | H | CH₂-CHO | H |
| 40 | H | COOCH₂C₆H₅ | H |
| 41 | Cl | CH₃ | H |
| 42 | CH₃ | CH₃ | H |
| 43 | C₂H₅ | CH₃ | H |
| 44 | CF₃ | CH₃ | H |
| 45 | OCH₃ | CH₃ | H |
| 46 | OC₂H₅ | CH₃ | H |
| 47 | CH₂-C≡CH | CH₃ | H |
| 48 | CH₂-CH=CH₂ | CH₃ | H |
| 49 | Cl | CH₃ | H |
| 50 | CH₃ | CH₃ | H |
| 51 | CF₃ | CH₃ | H |
| 52 | OCH₃ | CH₃ | H |
| 53 | OC₂H₅ | CH₃ | H |
| 54 | CH₂-CH=CH₂ | CH₃ | H |
| 55 | CH₂-C≡CH | CH₃ | H |
| 56 | H | CH₃ | Ph |
| 57 | H | C₂H₅ | Ph |
| 58 | H | C₃H₇ | Ph |
| 59 | H | C₄H₉ | Ph |
| 60 | H | CHO | Ph |
| 61 | H | COCH₃ | Ph |
| 62 | H | COOCH₃ | Ph |
| 63 | H | OCH₃ | Ph |
| 64 | H | C₆H₅ | Ph |
| 65 | H | CH=NOCH₃ | Ph |
| 66 | H | C(CH₃)=NOCH₃ | Ph |
| 67 | CH₃ | 2-Cl-C₆H₄ | H |
| 68 | CH₃ | 3-Br-C₆H₄ | H |
| 69 | CH₃ | 4-F-C₆H₄ | H |
| 70 | CH₃ | 2,4-Cl₂-C₆H₃ | H |
| 71 | CH₃ | 2-NO₂-C₆H₄ | H |
| 72 | CH₃ | 3-CN-C₆H₄ | H |
| 73 | CH₃ | 4-Me-C₆H₄ | H |
| 74 | CH₃ | 2-OMe-C₆H₄ | H |
| 75 | CH₃ | 3-CF₃-C₆H₄ | H |
| 76 | CH₃ | 4-OCF₃-C₆H₄ | H |
| 77 | CH₃ | 2-Me-C₆H₄ | H |
| 78 | CH₃ | 3-Me-C₆H₄ | H |
| 79 | CH₃ | 2-SMe-C₆H₄ | H |
| 80 | CH₃ | 3-COOMe-C₆H₄ | H |
| 81 | CH₃ | 4-CF₃-C₆H₄ | H |
| 82 | CH₃ | 2-CF₃-C₆H₄ | H |
| 83 | CH₃ | 3-OMe-C₆H₄ | H |
| 84 | CH₃ | 4-OMe-C₆H₄ | H |
| 85 | H | 2-Furyl | CH₃ |
| 86 | H | 3-Furyl | CH₃ |
| 87 | H | 2-Thienyl | CH₃ |
| 88 | H | 3-Thienyl | CH₃ |
| 89 | H | 2-Pyridyl | CH₃ |
| 90 | H | 3-Pyridyl | CH₃ |
| 91 | H | 4-Pyridyl | CH₃ |
| 92 | H | 2-Thiazolyl | CH₃ |
| 93 | H | 4-Thiazolyl | CH₃ |
| 94 | H | 5-Thiazolyl | CH₃ |
| 95 | H | 2-Pyrrolyl | CH₃ |
| 96 | H | 3-Pyrrolyl | CH₃ |
| 97 | H | 4-Pyrrolyl | CH₃ |
| 98 | H | 3-Isoxazolyl | CH₃ |
| 99 | H | 4-Isoxazolyl | CH₃ |
| 100 | H | 5-Isoxazolyl | CH₃ |
| 101 | H | 2-Oxazolyl | CH₃ |
| 102 | H | 4-Oxazolyl | CH₃ |

Die folgenden Tabellen 289-432 basieren auf den 4-Benzoyl-pyrazolen der Formel Id:

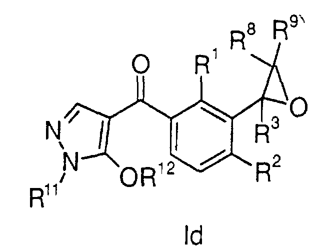

### Tabelle 289: Verbindungen 289.1-289.102

Verbindungen der allgemeinen Formel Id, in der R¹ Cl, R² Methylsulfonyl, R¹¹ Methyl und R¹² Wasserstoff bedeutet und die Substituenten R³, R⁸ und R⁹ für jede einzelne Verbindung jeweils einer Zeile der Tabelle C entsprechen.

### Tabelle 290: Verbindungen 290.1-290.102

Verbindungen der allgemeinen Formel Id, in der R¹ Cl, R² Methylsulfonyl, R¹¹ Ethyl, R¹² Wasserstoff bedeutet und die Substituenten R³, R⁸ und R⁹ für jede einzelne Verbindung jeweils einer Zeile der Tabelle C entsprechen.

### Tabelle 291: Verbindungen 291.1-291.102

Verbindungen der allgemeinen Formel Id, in der R¹ Cl, R² Methylsulfonyl, R¹¹ n-Propyl, R¹² Wasserstoff bedeutet und die Substituenten R³, R⁸ und R⁹ für jede einzelne Verbindung jeweils einer Zeile der Tabelle C entsprechen.

### Tabelle 292: Verbindungen 292.1-292.102

Verbindungen der allgemeinen Formel Id, in der R¹ Cl, R² Methylsulfonyl, R¹¹ Methyl, R¹² Methyl bedeutet und die Substituenten R³, R⁸ und R⁹ für jede einzelne Verbindung jeweils einer Zeile der Tabelle C entsprechen.

### Tabelle 293: Verbindungen 293.1-293.102

Verbindungen der allgemeinen Formel Id, in der R¹ Cl, R² Methylsulfonyl, R¹¹ Ethyl, R¹² Methyl bedeutet und die Substituenten R³, R⁸ und R⁹ für jede einzelne Verbindung jeweils einer Zeile der Tabelle C entsprechen.

### Tabelle 294: Verbindungen 294.1-294.102

Verbindungen der allgemeinen Formel Id, in der R¹ Cl, R² Methylsulfonyl, R¹¹ n-Propyl, R¹² Methyl bedeutet und die Substituenten R³, R⁸ und R⁹ für jede einzelne Verbindung jeweils einer Zeile der Tabelle C entsprechen.

### Tabelle 295: Verbindungen 295.1-295.102

Verbindungen der allgemeinen Formel Id, in der R¹ Cl, R² Methylsulfonyl, R¹¹ Methyl, R¹² Ethyl bedeutet und die Substituenten R³, R⁸ und R⁹ für jede einzelne Verbindung jeweils einer Zeile der Tabelle C entsprechen.

### Tabelle 296: Verbindungen 296.1-296.102

Verbindungen der allgemeinen Formel Id, in der R¹ Cl, R² Methylsulfonyl, R¹¹ Ethyl, R¹² Ethyl bedeutet und die Substituenten R³, R⁸ und R⁹ für jede einzelne Verbindung jeweils einer Zeile der Tabelle C entsprechen.

### Tabelle 297: Verbindungen 297.1-297.102

Verbindungen der allgemeinen Formel Id, in der R¹ Cl, R² Methylsulfonyl, R¹¹ n-Propyl, R¹² Ethyl bedeutet und die Substituenten R³, R⁸ und R⁹ für jede einzelne Verbindung jeweils einer Zeile der Tabelle C entsprechen.

### Tabelle 298: Verbindungen 298.1-298.102

Verbindungen der allgemeinen Formel Id, in der R¹ Cl, R² Methylsulfonyl, R¹¹ Methyl, R¹² Methylcarbonyl bedeutet und die Substituenten R³, R⁸ und R⁹ für jede einzelne Verbindung jeweils einer Zeile der Tabelle C entsprechen.

### Tabelle 299: Verbindungen 299.1-299.102

Verbindungen der allgemeinen Formel Id, in der R¹ Cl, R² Methylsulfonyl, R¹¹ Ethyl, R¹² Methylcarbonyl bedeutet und die Substituenten R³, R⁸ und R⁹ für jede einzelne Verbindung jeweils einer Zeile der Tabelle C entsprechen.

### Tabelle 300: Verbindungen 300.1-300.102

Verbindungen der allgemeinen Formel Id, in der R¹ Cl, R² Methylsulfonyl, R¹¹ n-Propyl, R¹² Methylcarbonyl bedeutet und die Substituenten R³, R⁸ und R⁹ für jede einzelne Verbindung jeweils einer Zeile der Tabelle C entsprechen.

### Tabelle 301: Verbindungen 301.1-301.102

Verbindungen der allgemeinen Formel Id, in der R¹ Cl, R² Methylsulfonyl, R¹¹ Methyl, R¹² Ethylcarbonyl bedeutet und die Substituenten R³, R⁸ und R⁹ für jede einzelne Verbindung jeweils einer Zeile der Tabelle C entsprechen.

### Tabelle 302: Verbindungen 302.1-302.102

Verbindungen der allgemeinen Formel Id, in der R¹ Cl, R² Methylsulfonyl, R¹¹ Ethyl, R¹² Ethylcarbonyl bedeutet und die Substituenten R³, R⁸ und R⁹ für jede einzelne Verbindung jeweils einer Zeile der Tabelle C entsprechen.

### Tabelle 303: Verbindungen 303.1-303.102

Verbindungen der allgemeinen Formel Id, in der R¹ Cl, R² Methylsulfonyl, R¹¹ n-Propyl, R¹² Ethylcarbonyl bedeutet und die Substituenten R³, R⁸ und R⁹ für jede einzelne Verbindung jeweils einer Zeile der Tabelle C entsprechen.

### Tabelle 304: Verbindungen 304.1-304.102

Verbindungen der allgemeinen Formel Id, in der R¹ Cl, R² Methylsulfonyl, R¹¹ Methyl, R¹² Methylsulfonyl bedeutet und die Substituenten R³, R⁸ und R⁹ für jede einzelne Verbindung jeweils einer Zeile der Tabelle C entsprechen.

### Tabelle 305: Verbindungen 305.1-305.102

Verbindungen der allgemeinen Formel Id, in der R¹ Cl, R² Methylsulfonyl, R¹¹ Ethyl, R¹² Methylsulfonyl bedeutet und die Substituenten R³, R⁸ und R⁹ für jede einzelne Verbindung jeweils einer Zeile der Tabelle C entsprechen.

### Tabelle 306: Verbindungen 306.1-306.102

Verbindungen der allgemeinen Formel Id, in der R¹ Cl, R² Methylsulfonyl, R¹¹ n-Propyl, R¹² Methylsulfonyl bedeutet und die Substituenten R³, R⁸ und R⁹ für jede einzelne Verbindung jeweils einer Zeile der Tabelle C entsprechen.

### Tabelle 307: Verbindungen 307.1-307.102

Verbindungen der allgemeinen Formel Id, in der R¹ Cl, R² Methylsulfonyl, R¹¹ Methyl, R¹² Ethylsulfonyl bedeutet und die Substituenten R³, R⁸ und R⁹ für jede einzelne Verbindung jeweils einer Zeile der Tabelle C entsprechen.

### Tabelle 308: Verbindungen 308.1-308.102

Verbindungen der allgemeinen Formel Id, in der R¹ Cl, R² Methylsulfonyl, R¹¹ Ethyl, R¹² Ethylsulfonyl bedeutet und die Substituenten R³, R⁸ und R⁹ für jede einzelne Verbindung jeweils einer Zeile der Tabelle C entsprechen.

### Tabelle 309: Verbindungen 309.1-309.102

Verbindungen der allgemeinen Formel Id, in der R¹ Cl, R² Methylsulfonyl, R¹¹ n-Propyl, R¹² Ethylsulfonyl bedeutet und die Substituenten R³, R⁸ und R⁹ für jede einzelne Verbindung jeweils einer Zeile der Tabelle C entsprechen.

### Tabelle 310: Verbindungen 310.1-310.102

Verbindungen der allgemeinen Formel Id, in der R¹ Cl, R² Methylsulfonyl, R¹¹ Methyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 311: Verbindungen 311.1-311.102

Verbindungen der allgemeinen Formel Id, in der R¹ Cl, R² Methylsulfonyl, R¹¹ Ethyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 312: Verbindungen 312.1-312.102

Verbindungen der allgemeinen Formel Id, in der R¹ Cl, R² Methylsulfonyl, R¹¹ n-Propyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 313: Verbindungen 313.1-313.102

Verbindungen der allgemeinen Formel Id, in der R¹ Cl, R² Cl, R¹¹ Methyl und R¹² Wasserstoff bedeutet und die Substituenten R³, R⁸ und R⁹ für jede einzelne Verbindung jeweils einer Zeile der Tabelle C entsprechen.

### Tabelle 314: Verbindungen 314.1-314.102

Verbindungen der allgemeinen Formel Id, in der R¹ Cl, R² Cl, R¹¹ Ethyl, R¹² Wasserstoff bedeutet und die Substituenten R³, R⁸ und R⁹ für jede einzelne Verbindung jeweils einer Zeile der Tabelle C entsprechen.

### Tabelle 315: Verbindungen 315.1-315.102

Verbindungen der allgemeinen Formel Id, in der R¹ Cl, R² Cl, R¹¹ n-Propyl, R¹² Wasserstoff bedeutet und die Substituenten R³, R⁸ und R⁹ für jede einzelne Verbindung jeweils einer Zeile der Tabelle C entsprechen.

### Tabelle 316: Verbindungen 316.1-316.102

Verbindungen der allgemeinen Formel Id, in der R¹ Cl, R² Cl, R¹¹ Methyl, R¹² Methyl bedeutet und die Substituenten R³, R⁸ und R⁹ für jede einzelne Verbindung jeweils einer Zeile der Tabelle C entsprechen.

### Tabelle 317: Verbindungen 317.1-317.102

Verbindungen der allgemeinen Formel Id, in der R¹ Cl, R² Cl, R¹¹ Ethyl, R¹² Methyl bedeutet und die Substituenten R³, R⁸ und R⁹ für jede einzelne Verbindung jeweils einer Zeile der Tabelle C entsprechen.

### Tabelle 318: Verbindungen 318.1-318.102

Verbindungen der allgemeinen Formel Id, in der R¹ Cl, R² Cl, R¹¹ n-Propyl, R¹² Methyl bedeutet und die Substituenten R³, R⁸ und R⁹ für jede einzelne Verbindung jeweils einer Zeile der Tabelle C entsprechen.

### Tabelle 319: Verbindungen 319.1-319.102

Verbindungen der allgemeinen Formel Id, in der R¹ Cl, R² Cl, R¹¹ Methyl, R¹² Ethyl bedeutet und die Substituenten R³, R⁸ und R⁹ für jede einzelne Verbindung jeweils einer Zeile der Tabelle C entsprechen.

### Tabelle 320: Verbindungen 320.1-320.102

Verbindungen der allgemeinen Formel Id, in der R¹ Cl, R² Cl, R¹¹ Ethyl, R¹² Ethyl bedeutet und die Substituenten R³, R⁸ und R⁹ für jede einzelne Verbindung jeweils einer Zeile der Tabelle C entsprechen.

### Tabelle 321: Verbindungen 321.1-321.102

Verbindungen der allgemeinen Formel Id, in der R¹ Cl, R² Cl, R¹¹ n-Propyl, R¹² Ethyl bedeutet und die Substituenten R³, R⁸ und R⁹ für jede einzelne Verbindung jeweils einer Zeile der Tabelle C entsprechen.

### Tabelle 322: Verbindungen 322.1-322.102

Verbindungen der allgemeinen Formel Id, in der R¹ Cl, R² Cl, R¹¹ Methyl, R¹² Methylcarbonyl bedeutet und die Substituenten R³, R⁸ und R⁹ für jede einzelne Verbindung jeweils einer Zeile der Tabelle C entsprechen.

### Tabelle 323: Verbindungen 323.1-323.102

Verbindungen der allgemeinen Formel Id, in der R¹ Cl, R² Cl, R¹¹ Ethyl, R¹² Methylcarbonyl bedeutet und die Substituenten R³, R⁸ und R⁹ für jede einzelne Verbindung jeweils einer Zeile der Tabelle C entsprechen.

### Tabelle 324: Verbindungen 324.1-324.102

Verbindungen der allgemeinen Formel Id, in der R¹ Cl, R² Cl, R¹¹ n-Propyl, R¹² Methylcarbonyl bedeutet und die Substituenten R³, R⁸ und R⁹ für jede einzelne Verbindung jeweils einer Zeile der Tabelle C entsprechen.

### Tabelle 325: Verbindungen 325.1-325.102

Verbindungen der allgemeinen Formel Id, in der R¹ Cl, R² Cl, R¹¹ Methyl, R¹² Ethylcarbonyl bedeutet und die Substituenten R³, R⁸ und R⁹ für jede einzelne Verbindung jeweils einer Zeile der Tabelle C entsprechen.

### Tabelle 326: Verbindungen 326.1-326.102

Verbindungen der allgemeinen Formel Id, in der R¹ Cl, R² Cl, R¹¹ Ethyl, R¹² Ethylcarbonyl bedeutet und die Substituenten R³, R⁸ und R⁹ für jede einzelne Verbindung jeweils einer Zeile der Tabelle C entsprechen.

### Tabelle 327: Verbindungen 327.1-327.102

Verbindungen der allgemeinen Formel Id, in der R¹ Cl, R² Cl, R¹¹ n-Propyl, R¹² Ethylcarbonyl bedeutet und die Substituenten R³, R⁸ und R⁹ für jede einzelne Verbindung jeweils einer Zeile der Tabelle C entsprechen.

### Tabelle 328: Verbindungen 328.1-328.102

Verbindungen der allgemeinen Formel Id, in der R¹ Cl, R² Cl, R¹¹ Methyl, R¹² Methylsulfonyl bedeutet und die Substituenten R³, R⁸ und R⁹ für jede einzelne Verbindung jeweils einer Zeile der Tabelle C entsprechen.

### Tabelle 329: Verbindungen 329.1-329.102

Verbindungen der allgemeinen Formel Id, in der R¹ Cl, R² Cl, R¹¹ Ethyl, R¹² Methylsulfonyl bedeutet und die Substituenten R³, R⁸ und R⁹ für jede einzelne Verbindung jeweils einer Zeile der Tabelle C entsprechen.

### Tabelle 330: Verbindungen 330.1-330.102

Verbindungen der allgemeinen Formel Id, in der R¹ Cl, R² Cl, R¹¹ n-Propyl, R¹² Methylsulfonyl bedeutet und die Substituenten R³, R⁸ und R⁹ für jede einzelne Verbindung jeweils einer Zeile der Tabelle C entsprechen

### Tabelle 331: Verbindungen 331.1-331.102

Verbindungen der allgemeinen Formel Id, in der R¹ Cl, R² Cl, R¹¹ Methyl, R¹² Ethylsulfonyl bedeutet und die Substituenten R³, R⁸ und R⁹ für jede einzelne Verbindung jeweils einer Zeile der Tabelle C entsprechen.

### Tabelle 332: Verbindungen 332.1-332.102

Verbindungen der allgemeinen Formel Id, in der R¹ Cl, R² Cl, R¹¹ Ethyl, R¹² Ethylsulfonyl bedeutet und die Substituenten R³, R⁸ und R⁹ für jede einzelne Verbindung jeweils einer Zeile der Tabelle C entsprechen.

### Tabelle 333: Verbindungen 333.1-333.102

Verbindungen der allgemeinen Formel Id, in der R¹ Cl, R² Cl, R¹¹ n-Propyl, R¹² Ethylsulfonyl bedeutet und die Substituenten R³, R⁸ und R⁹ für jede einzelne Verbindung jeweils einer Zeile der Tabelle C entsprechen.

### Tabelle 334: Verbindungen 334.1-334.102

Verbindungen der allgemeinen Formel Id, in der R¹ Cl, R² Cl, R¹¹ Methyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 335: Verbindungen 335.1-335.102

Verbindungen der allgemeinen Formel Id, in der R¹ Cl, R² Cl, R¹¹ Ethyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 336: Verbindungen 336.1-336.102

Verbindungen der allgemeinen Formel Id, in der R¹ Cl, R² Cl, R¹¹ n-Propyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 337: Verbindungen 337.1-337.102

Verbindungen der allgemeinen Formel Id, in der R¹ Cl, R² Trifluormethyl, R¹¹ Methyl und R¹² Wasserstoff bedeutet und die Substituenten R³, R⁸ und R⁹ für jede einzelne Verbindung jeweils einer Zeile der Tabelle C entsprechen.

### Tabelle 338: Verbindungen 338.1-338.102

Verbindungen der allgemeinen Formel Id, in der R¹ Cl, R² Trifluormethyl, R¹¹ Ethyl, R¹² Wasserstoff bedeutet und die Substituenten für jede einzelne Verbindung jeweils einer Zeile der Tabelle C entsprechen.

### Tabelle 339: Verbindungen 339.1-339.102

Verbindungen der allgemeinen Formel Id, in der R¹ Cl, R² Trifluormethyl, R¹¹ n-Propyl, R¹² Wasserstoff bedeutet und die Substituenten R³, R⁸ und R⁹ für jede einzelne Verbindung jeweils einer Zeile der Tabelle C entsprechen.

### Tabelle 340: Verbindungen 340.1-340.102

Verbindungen der allgemeinen Formel Id, in der R¹ Cl, R² Trifluormethyl, R¹¹ Methyl, R¹² Methyl bedeutet und die Substituenten R³, R⁸ und R⁹ für jede einzelne Verbindung jeweils einer Zeile der Tabelle C entsprechen.

### Tabelle 341: Verbindungen 341.1-341.102

Verbindungen der allgemeinen Formel Id, in der R¹ Cl, R² Trifluormethyl, R¹¹ Ethyl, R¹² Methyl bedeutet und die Substituenten R³, R⁸ und R⁹ für jede einzelne Verbindung jeweils einer Zeile der Tabelle C entsprechen.

### Tabelle 342: Verbindungen 342.1-342.102

Verbindungen der allgemeinen Formel Id, in der R¹ Cl, R² Trifluormethyl, R¹¹ n-Propyl, R¹² Methyl bedeutet und die Substituenten R³, R⁸ und R⁹ für jede einzelne Verbindung jeweils einer Zeile der Tabelle C entsprechen.

### Tabelle 343: Verbindungen 343.1-343.102

Verbindungen der allgemeinen Formel Id, in der R¹ Cl, R² Trifluormethyl, R¹¹ Methyl, R¹² Ethyl bedeutet und die Substituenten R³, R⁸ und R⁹ für jede einzelne Verbindung jeweils einer Zeile der Tabelle C entsprechen.

### Tabelle 344: Verbindungen 344.1-344.102

Verbindungen der allgemeinen Formel Id, in der R¹ Cl, R² Trifluormethyl, R¹¹ Ethyl, R¹² Ethyl bedeutet und die Substituenten R³, R⁸ und R⁹ für jede einzelne Verbindung jeweils einer Zeile der Tabelle C entsprechen.

### Tabelle 345: Verbindungen 345.1-345.102

Verbindungen der allgemeinen Formel Id, in der R¹ Cl, R² Trifluormethyl, R¹¹ n-Propyl, R¹² Ethyl bedeutet und die Substituenten R³, R⁸ und R⁹ für jede einzelne Verbindung jeweils einer Zeile der Tabelle C entsprechen.

### Tabelle 346: Verbindungen 346.1-346.102

Verbindungen der allgemeinen Formel Id, in der R¹ Cl, R² Trifluormethyl, R¹¹ Methyl, R¹² Methylcarbonyl bedeutet und die Substituenten R³, R⁸ und R⁹ für jede einzelne Verbindung jeweils einer Zeile der Tabelle C entsprechen.

### Tabelle 347: Verbindungen 347.1-347.102

Verbindungen der allgemeinen Formel Id, in der R¹ Cl, R² Trifluormethyl, R¹¹ Ethyl, R¹² Methylcarbonyl bedeutet und die Substituenten R³, R⁸ und R⁹ für jede einzelne Verbindung jeweils einer Zeile der Tabelle C entsprechen.

### Tabelle 348: Verbindungen 348.1-348.102

Verbindungen der allgemeinen Formel Id, in der R¹ Cl, R² Trifluormethyl, R¹¹ n-Propyl, R¹² Methylcarbonyl bedeutet und die Substituenten R³, R⁸ und R⁹ für jede einzelne Verbindung jeweils einer Zeile der Tabelle C entsprechen.

### Tabelle 349: Verbindungen 349.1-349.102

Verbindungen der allgemeinen Formel Id, in der R¹ Cl, R² Trifluormethyl, R¹¹ Methyl, R¹² Ethylcarbonyl bedeutet und die Substituenten R³, R⁸ und R⁹ für jede einzelne Verbindung jeweils einer Zeile der Tabelle C entsprechen.

### Tabelle 350: Verbindungen 350.1-350.102

Verbindungen der allgemeinen Formel Id, in der R¹ Cl, R² Trifluormethyl, R¹¹ Ethyl, R¹² Ethylcarbonyl bedeutet und die Substituenten R³, R⁸ und R⁹ für jede einzelne Verbindung jeweils einer Zeile der Tabelle C entsprechen.

### Tabelle 351: Verbindungen 351.1-351.102

Verbindungen der allgemeinen Formel Id, in der R¹ Cl, R² Trifluormethyl, R¹¹ n-Propyl, R¹² Ethylcarbonyl bedeutet und die Substituenten R³, R⁸ und R⁹ für jede einzelne Verbindung jeweils einer Zeile der Tabelle C entsprechen.

### Tabelle 352: Verbindungen 352.1-352.102

Verbindungen der allgemeinen Formel Id, in der R¹ Cl, R² Trifluormethyl, R¹¹ Methyl, R¹² Methylsulfonyl bedeutet und die Substituenten R³, R⁸ und R⁹ für jede einzelne Verbindung jeweils einer Zeile der Tabelle C entsprechen.

### Tabelle 353: Verbindungen 353.1-353.102

Verbindungen der allgemeinen Formel Id, in der R¹ Cl, R² Trifluormethyl, R¹¹ Ethyl, R¹² Methylsulfonyl bedeutet und die Substituenten R³, R⁸ und R⁹ für jede einzelne Verbindung jeweils einer Zeile der Tabelle C entsprechen.

### Tabelle 354: Verbindungen 354.1-354.102

Verbindungen der allgemeinen Formel Id, in der R¹ Cl, R² Trifluormethyl, R¹¹ n-Propyl, R¹² Methylsulfonyl bedeutet und die Substituenten R³, R⁸ und R⁹ für jede einzelne Verbindung jeweils einer Zeile der Tabelle C entsprechen.

### Tabelle 355: Verbindungen 355.1-355.102

Verbindungen der allgemeinen Formel Id, in der R¹ Cl, R² Trifluormethyl, R¹¹ Methyl, R¹² Ethylsulfonyl bedeutet und die Substituenten R³, R⁸ und R⁹ für jede einzelne Verbindung jeweils einer Zeile der Tabelle C entsprechen.

### Tabelle 356: Verbindungen 356.1-356.102

Verbindungen der allgemeinen Formel Id, in der R¹ Cl, R² Trifluormethyl, R¹¹ Ethyl, R¹² Ethylsulfonyl bedeutet und die Substituenten R³, R⁸ und R⁹ für jede einzelne Verbindung jeweils einer Zeile der Tabelle C entsprechen.

### Tabelle 357: Verbindungen 357.1-357.102

Verbindungen der allgemeinen Formel Id, in der R¹ Cl, R² Trifluormethyl, R¹¹ n-Propyl, R¹² Ethylsulfonyl bedeutet und die Substituenten R³, R⁸ und R⁹ für jede einzelne Verbindung jeweils einer Zeile der Tabelle C entsprechen.

### Tabelle 358: Verbindungen 358.1-358.102

Verbindungen der allgemeinen Formel Id, in der R¹ Cl, R² Trifluormethyl, R¹¹ Methyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 359: Verbindungen 359.1-359.102

Verbindungen der allgemeinen Formel Id, in der R¹ Cl, R² Trifluormethyl, R¹¹ Ethyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 360: Verbindungen 360.1-360.102

Verbindungen der allgemeinen Formel Id, in der R¹ Cl, R² Trifluormethyl, R¹¹ n-Propyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 361: Verbindungen 361.1-361.102

Verbindungen der allgemeinen Formel Id, in der R¹ Methyl, R² Cl, R¹¹ Methyl und R¹² Wasserstoff bedeutet und die Substituenten R³, R⁸ und R⁹ für jede einzelne Verbindung jeweils einer Zeile der Tabelle C entsprechen.

### Tabelle 362: Verbindungen 362.1-362.102

Verbindungen der allgemeinen Formel Id, in der R¹ Methyl, R² Cl, R¹¹ Ethyl, R¹² Wasserstoff bedeutet und die Substituenten R³, R⁸ und R⁹ für jede einzelne Verbindung jeweils einer Zeile der Tabelle C entsprechen.

### Tabelle 363: Verbindungen 363.1-363.102

Verbindungen der allgemeinen Formel Id, in der R¹ Methyl, R² Cl, R¹¹ n-Propyl, R¹² Wasserstoff bedeutet und die Substituenten R³, R⁸ und R⁹ für jede einzelne Verbindung jeweils einer Zeile der Tabelle C entsprechen.

### Tabelle 364: Verbindungen 364.1-364.102

Verbindungen der allgemeinen Formel Id, in der R¹ Methyl, R² Cl, R¹¹ Methyl, R¹² Methyl bedeutet und die Substituenten R³, R⁸ und R⁹ für jede einzelne Verbindung jeweils einer Zeile der Tabelle C entsprechen.

### Tabelle 365: Verbindungen 365.1-365.102

Verbindungen der allgemeinen Formel Id, in der R¹ Methyl, R² Cl, R¹¹ Ethyl, R¹² Methyl bedeutet und die Substituenten R³, R⁸ und R⁹ für jede einzelne Verbindung jeweils einer Zeile der Tabelle C entsprechen.

### Tabelle 366: Verbindungen 366.1-366.102

Verbindungen der allgemeinen Formel Id, in der R¹ Methyl, R² Cl, R¹¹ n-Propyl, R¹² Methyl bedeutet und die Substituenten R³, R⁸ und R⁹ für jede einzelne Verbindung jeweils einer Zeile der Tabelle C entsprechen.

### Tabelle 367: Verbindungen 367.1-367.102

Verbindungen der allgemeinen Formel Id, in der R¹ Methyl, R² Cl, R¹¹ Methyl, R¹² Ethyl bedeutet und die Substituenten R³, R⁸ und R⁹ für jede einzelne Verbindung jeweils einer Zeile der Tabelle C entsprechen.

### Tabelle 368: Verbindungen 368.1-368.102

Verbindungen der allgemeinen Formel Id, in der R¹ Methyl, R² Cl, R¹¹ Ethyl, R¹² Ethyl bedeutet und die Substituenten R³, R⁸ und R⁹ für jede einzelne Verbindung jeweils einer Zeile der Tabelle C entsprechen.

### Tabelle 369: Verbindungen 369.1-369.102

Verbindungen der allgemeinen Formel Id, in der R¹ Methyl, R² Cl, R¹¹ n-Propyl, R¹² Ethyl bedeutet und die Substituenten R³, R⁸ und R⁹ für jede einzelne Verbindung jeweils einer Zeile der Tabelle C entsprechen.

### Tabelle 370: Verbindungen 370.1-370.102

Verbindungen der allgemeinen Formel Id, in der R¹ Methyl, R² Cl, R¹¹ Methyl, R¹² Methylcarbonyl bedeutet und die Substituenten R³, R⁸ und R⁹ für jede einzelne Verbindung jeweils einer Zeile der Tabelle C entsprechen.

### Tabelle 371: Verbindungen 371.1-371.102

Verbindungen der allgemeinen Formel Id, in der R¹ Methyl, R² Cl, R¹¹ Ethyl, R¹² Methylcarbonyl bedeutet und die Substituenten R³, R⁸ und R⁹ für jede einzelne Verbindung jeweils einer Zeile der Tabelle C entsprechen.

### Tabelle 372: Verbindungen 372.1-372.102

Verbindungen der allgemeinen Formel Id, in der R¹ Methyl, R² Cl, R¹¹ n-Propyl, R¹² Methylcarbonyl bedeutet und die Substituenten R³, R⁸ und R⁹ für jede einzelne Verbindung jeweils einer Zeile der Tabelle C entsprechen.

### Tabelle 373: Verbindungen 373.1-373.102

Verbindungen der allgemeinen Formel Id, in der R¹ Methyl, R² Cl, R¹¹ Methyl, R¹² Ethylcarbonyl bedeutet und die Substituenten R³, R⁸ und R⁹ für jede einzelne Verbindung jeweils einer Zeile der Tabelle C entsprechen.

### Tabelle 374: Verbindungen 374.1-374.102

Verbindungen der allgemeinen Formel Id, in der R¹ Methyl, R² Cl, R¹¹ Ethyl, R¹² Ethylcarbonyl bedeutet und die Substituenten R³, R⁹ und R⁹ für jede einzelne Verbindung jeweils einer Zeile der Tabelle C entsprechen.

### Tabelle 375: Verbindungen 375.1-375.102

Verbindungen der allgemeinen Formel Id, in der R¹ Methyl, R² Cl, R¹¹ n-Propyl, R¹² Ethylcarbonyl bedeutet und die Substituenten R³, R⁸ und R⁹ für jede einzelne Verbindung jeweils einer Zeile der Tabelle C entsprechen.

### Tabelle 376: Verbindungen 376.1-376.102

Verbindungen der allgemeinen Formel Id, in der R¹ Methyl, R² Cl, R¹¹ Methyl, R¹² Methylsulfonyl bedeutet und die Substituenten R³, R⁸ und R⁹ für jede einzelne Verbindung jeweils einer Zeile der Tabelle C entsprechen.

### Tabelle 377: Verbindungen 377.1-377.102

Verbindungen der allgemeinen Formel Id, in der R¹ Methyl, R² Cl, R¹¹ Ethyl, R¹² Methylsulfonyl bedeutet und die Substituenten R³, R⁸ und R⁹ für jede einzelne Verbindung jeweils einer Zeile der Tabelle C entsprechen.

### Tabelle 378: Verbindungen 378.1-378.102

Verbindungen der allgemeinen Formel Id, in der R¹ Methyl, R² Cl, R¹¹ n-Propyl, R¹² Methylsulfonyl bedeutet und die Substituenten R³, R⁸ und R⁹ für jede einzelne Verbindung jeweils einer Zeile der Tabelle C entsprechen.

### Tabelle 379: Verbindungen 379.1-379.102

Verbindungen der allgemeinen Formel Id, in der R¹ Methyl, R² Cl, R¹¹ Methyl, R¹² Ethylsulfonyl bedeutet und die Substituenten R³, R⁸ und R⁹ für jede einzelne Verbindung jeweils einer Zeile der Tabelle C entsprechen.

### Tabelle 380: Verbindungen 380.1-380.102

Verbindungen der allgemeinen Formel Id, in der R¹ Methyl, R² Cl, R¹¹ Ethyl, R¹² Ethylsulfonyl bedeutet und die Substituenten R³, R⁸ und R⁹ für jede einzelne Verbindung jeweils einer Zeile der Tabelle C entsprechen.

### Tabelle 381: Verbindungen 381.1-381.102

Verbindungen der allgemeinen Formel Id, in der R¹ Methyl, R² Cl, R¹¹ n-Propyl, R¹² Ethylsulfonyl bedeutet und die Substituenten R³, R⁸ und R⁹ für jede einzelne Verbindung jeweils einer Zeile der Tabelle C entsprechen.

### Tabelle 382: Verbindungen 382.1-382.102

Verbindungen der allgemeinen Formel Id, in der R¹ Methyl, R² Cl, R¹¹ Methyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 383: Verbindungen 383.1-383.102

Verbindungen der allgemeinen Formel Id, in der R¹ Methyl, R² Cl, R¹¹ Ethyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 384: Verbindungen 384.1-384.102

Verbindungen der allgemeinen Formel Id, in der R¹ Methyl, R² Cl, R¹¹ n-Propyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 385: Verbindungen 385.1-385.102

Verbindungen der allgemeinen Formel Id, in der R¹ Methyl, R² Methylsulfonyl, R¹¹ Methyl und R¹² Wasserstoff bedeutet und die Substituenten R³, R⁸ und R⁹ für jede einzelne Verbindung jeweils einer Zeile der Tabelle C entsprechen.

### Tabelle 386: Verbindungen 386.1-386.102

Verbindungen der allgemeinen Formel Id, in der R¹ Methyl, R² Methylsulfonyl, R¹¹ Ethyl, R¹² Wasserstoff bedeutet und die Substituenten R³, R⁸ und R⁹ für jede einzelne Verbindung jeweils einer Zeile der Tabelle C entsprechen.

### Tabelle 387: Verbindungen 387.1-387.102

Verbindungen der allgemeinen Formel Id, in der R¹ Methyl, R² Methylsulfonyl, R¹¹ n-Propyl, R¹² Wasserstoff bedeutet und die Substituenten R³, R⁸ und R⁹ für jede einzelne Verbindung jeweils einer Zeile der Tabelle C entsprechen.

### Tabelle 388: Verbindungen 388.1-388.102

Verbindungen der allgemeinen Formel Id, in der R¹ Methyl, R² Methylsulfonyl, R¹¹ Methyl, R¹² Methyl bedeutet und die Substituenten R³, R⁸ und R⁹ für jede einzelne Verbindung jeweils einer Zeile der Tabelle C entsprechen.

### Tabelle 389: Verbindungen 389.1-389.102

Verbindungen der allgemeinen Formel Id, in der R¹ Methyl, R² Methylsulfonyl, R¹¹ Ethyl, R¹² Methyl bedeutet und die Substituenten R³, R⁸ und R⁹ für jede einzelne Verbindung jeweils einer Zeile der Tabelle C entsprechen.

### Tabelle 390: Verbindungen 390.1-390.102

Verbindungen der allgemeinen Formel Id, in der R¹ Methyl, R² Methylsulfonyl, R¹¹ n-Propyl, R¹² Methyl bedeutet und die Substituenten R³, R⁸ und R⁹ für jede einzelne Verbindung jeweils einer Zeile der Tabelle C entsprechen.

### Tabelle 391: Verbindungen 391.1-391.102

Verbindungen der allgemeinen Formel Id, in der R¹ Methyl, R² Methylsulfonyl, R¹¹ Methyl, R¹² Ethyl bedeutet und die Substituenten R³, R⁸ und R⁹ für jede einzelne Verbindung jeweils einer Zeile der Tabelle C entsprechen.

### Tabelle 392: Verbindungen 392.1-392.102

Verbindungen der allgemeinen Formel Id, in der R¹ Methyl, R² Methylsulfonyl, R¹¹ Ethyl, R¹² Ethyl bedeutet und die Substituenten R³, R⁸ und R⁹ für jede einzelne Verbindung jeweils einer Zeile der Tabelle C entsprechen.

### Tabelle 393: Verbindungen 393.1-393.102

Verbindungen der allgemeinen Formel Id, in der R¹ Methyl, R² Methylsulfonyl, R¹¹ n-Propyl, R¹² Ethyl bedeutet und die Substituenten R³, R⁸ und R⁹ für jede einzelne Verbindung jeweils einer Zeile der Tabelle C entsprechen.

### Tabelle 394: Verbindungen 394.1-394.102

Verbindungen der allgemeinen Formel Id, in der R¹ Methyl, R² Methylsulfonyl, R¹¹ Methyl, R¹² Methylcarbonyl bedeutet und die Substituenten R³, R⁸ und R⁹ für jede einzelne Verbindung jeweils einer Zeile der Tabelle C entsprechen.

### Tabelle 395: Verbindungen 395.1-395.102

Verbindungen der allgemeinen Formel Id, in der R¹ Methyl, R² Methylsulfonyl, R¹¹ Ethyl, R¹² Methylcarbonyl bedeutet und die Substituenten R³, R⁸ und R⁹ für jede einzelne Verbindung jeweils einer Zeile der Tabelle C entsprechen.

### Tabelle 396: Verbindungen 396.1-396.102

Verbindungen der allgemeinen Formel Id, in der R¹ Methyl, R² Methylsulfonyl, R¹¹ n-Propyl, R¹² Methylcarbonyl bedeutet und die Substituenten R³, R⁸ und R⁹ für jede einzelne Verbindung jeweils einer Zeile der Tabelle C entsprechen.

### Tabelle 397: Verbindungen 397.1-397.102

Verbindungen der allgemeinen Formel Id, in der R¹ Methyl, R² Methylsulfonyl, R¹¹ Methyl, R¹² Ethylcarbonyl bedeutet und die Substituenten R³, R⁸ und R⁹ für jede einzelne Verbindung jeweils einer Zeile der Tabelle C entsprechen.

### Tabelle 398: Verbindungen 398.1-398.102

Verbindungen der allgemeinen Formel Id, in der R¹ Methyl, R² Methylsulfonyl, R¹¹ Ethyl, R¹² Ethylcarbonyl bedeutet und die Substituenten R³, R⁸ und R⁹ für jede einzelne Verbindung jeweils einer Zeile der Tabelle C entsprechen.

### Tabelle 399: Verbindungen 399.1-399.102

Verbindungen der allgemeinen Formel Id, in der R¹ Methyl, R² Methylsulfonyl, R¹¹ n-Propyl, R¹² Ethylcarbonyl bedeutet und die Substituenten R³, R⁸ und R⁹ für jede einzelne Verbindung jeweils einer Zeile der Tabelle C entsprechen.

### Tabelle 400: Verbindungen 400.1-400.102

Verbindungen der allgemeinen Formel Id, in der R¹ Methyl, R² Methylsulfonyl, R¹¹ Methyl, R¹² Methylsulfonyl bedeutet und die Substituenten R³, R⁸ und R⁹ für jede einzelne Verbindung jeweils einer Zeile der Tabelle C entsprechen.

### Tabelle 401: Verbindungen 401.1-401.102

Verbindungen der allgemeinen Formel Id, in der R¹ Methyl, R² Methylsulfonyl, R¹¹ Ethyl, R¹² Methylsulfonyl bedeutet und die Substituenten R³, R⁸ und R⁹ für jede einzelne Verbindung jeweils einer Zeile der Tabelle C entsprechen.

### Tabelle 402: Verbindungen 402.1-402.102

Verbindungen der allgemeinen Formel Id, in der R¹ Methyl, R² Methylsulfonyl, R¹¹ n-Propyl, R¹² Methylsulfonyl bedeutet und die Substituenten R³, R⁸ und R⁹ für jede einzelne Verbindung jeweils einer Zeile der Tabelle C entsprechen.

### Tabelle 403: Verbindungen 403.1-403.102

Verbindungen der allgemeinen Formel Id, in der R¹ Methyl, R² Methylsulfonyl, R¹¹ Methyl, R¹² Ethylsulfonyl bedeutet und die Substituenten R³, R⁸ und R⁹ für jede einzelne Verbindung jeweils einer Zeile der Tabelle C entsprechen.

### Tabelle 404: Verbindungen 404.1-404.102

Verbindungen der allgemeinen Formel Id, in der R¹ Methyl, R² Methylsulfonyl, R¹¹ Ethyl, R¹² Ethylsulfonyl bedeutet und die Substituenten R³, R⁸ und R⁹ für jede einzelne Verbindung jeweils einer Zeile der Tabelle C entsprechen.

### Tabelle 405: Verbindungen 405.1-405.102

Verbindungen der allgemeinen Formel Id, in der R¹ Methyl, R² Methylsulfonyl, R¹¹ n-Propyl, R¹² Ethylsulfonyl bedeutet und die Substituenten R³, R⁸ und R⁹ für jede einzelne Verbindung jeweils einer Zeile der Tabelle C entsprechen.

### Tabelle 406: Verbindungen 406.1-406.102

Verbindungen der allgemeinen Formel Id, in der R¹ Methyl, R² Methylsulfonyl, R¹¹ Methyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 407: Verbindungen 407.1-407.102

Verbindungen der allgemeinen Formel Id, in der R¹ Methyl, R² Methylsulfonyl, R¹¹ Ethyl, R¹² 4-Methylphenylsulfonyl bedeutet und die Substituenten R³, R⁸ und R⁹ R¹-R⁸ für jede einzelne Verbindung jeweils einer Zeile der Tabelle C entsprechen.

### Tabelle 408: Verbindungen 408.1-408.102

Verbindungen der allgemeinen Formel Id, in der R¹ Methyl, R² Methylsulfonyl, R¹¹ n-Propyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 409: Verbindungen 409.1-409.102

Verbindungen der allgemeinen Formel Id, in der R¹ Methyl, R² Trifluormethyl, R¹¹ Methyl und R¹² Wasserstoff bedeutet und die Substituenten R³, R⁸ und R⁹ für jede einzelne Verbindung jeweils einer Zeile der Tabelle C entsprechen.

### Tabelle 410: Verbindungen 410.1-410.102

Verbindungen der allgemeinen Formel Id, in der R¹ Methyl, R² Trifluormethyl, R¹¹ Ethyl, R¹² Wasserstoff bedeutet und die Substituenten R³, R⁸ und R⁹ für jede einzelne Verbindung jeweils einer Zeile der Tabelle C entsprechen.

### Tabelle 411: Verbindungen 411.1-411.102

Verbindungen der allgemeinen Formel Id, in der R¹ Methyl, R² Trifluormethyl, R¹¹ n-Propyl, R¹² Wasserstoff bedeutet und die Substituenten R³, R⁸ und R⁹ für jede einzelne Verbindung jeweils einer Zeile der Tabelle C entsprechen.

### Tabelle 412: Verbindungen 412.1-412.102

Verbindungen der allgemeinen Formel Id, in der R¹ Methyl, R² Trifluormethyl, R¹¹ Methyl, R¹² Methyl bedeutet und die Substituenten R³, R⁸ und R⁹ für jede einzelne Verbindung jeweils einer Zeile der Tabelle C entsprechen.

### Tabelle 413: Verbindungen 413.1-413.102

Verbindungen der allgemeinen Formel Id, in der R¹ Methyl, R² Trifluormethyl, R¹¹ Ethyl, R¹² Methyl bedeutet und die Substituenten R³, R⁸ und R⁹ für jede einzelne Verbindung jeweils einer Zeile der Tabelle C entsprechen.

### Tabelle 414: Verbindungen 414.1-414.102

Verbindungen der allgemeinen Formel Id, in der R¹ Methyl, R² Trifluormethyl, R¹¹ n-Propyl, R¹² Methyl bedeutet und die Substituenten R³, R⁸ und R⁹ für jede einzelne Verbindung jeweils einer Zeile der Tabelle C entsprechen.

### Tabelle 415: Verbindungen 415.1-415.102

Verbindungen der allgemeinen Formel Id, in der R¹ Methyl, R² Trifluormethyl, R¹¹ Methyl, R¹² Ethyl bedeutet und die Substituenten R³, R⁸ und R⁹ für jede einzelne Verbindung jeweils einer Zeile der Tabelle C entsprechen.

### Tabelle 416: Verbindungen 416.1-416.102

Verbindungen der allgemeinen Formel Id, in der R¹ Methyl, R² Trifluormethyl, R¹¹ Ethyl, R¹² Ethyl bedeutet und die Substituenten R³, R⁸ und R⁹ für jede einzelne Verbindung jeweils einer Zeile der Tabelle C entsprechen.

### Tabelle 417: Verbindungen 417.1-417.102

Verbindungen der allgemeinen Formel Id, in der R¹ Methyl, R² Trifluormethyl, R¹¹ n-Propyl, R¹² Ethyl bedeutet und die Substituenten R³, R⁸ und R⁹ für jede einzelne Verbindung jeweils einer Zeile der Tabelle C entsprechen.

### Tabelle 418: Verbindungen 418.1-418.102

Verbindungen der allgemeinen Formel Id, in der R¹ Methyl, R² Trifluormethyl, R¹¹ Methyl, R¹² Methylcarbonyl bedeutet und die Substituenten R³, R⁸ und R⁹ für jede einzelne Verbindung jeweils einer Zeile der Tabelle C entsprechen.

### Tabelle 419: Verbindungen 419.1-419.102

Verbindungen der allgemeinen Formel Id, in der R¹ Methyl, R² Trifluormethyl, R¹¹ Ethyl, R¹² Methylcarbonyl bedeutet und die Substituenten R³, R⁸ und R⁹ für jede einzelne Verbindung jeweils einer Zeile der Tabelle C entsprechen.

### Tabelle 420: Verbindungen 420.1-420.102

Verbindungen der allgemeinen Formel Id, in der R¹ Methyl, R² Trifluormethyl, R¹¹ n-Propyl, R¹² Methylcarbonyl bedeutet und die Substituenten R³, R⁸ und R⁹ für jede einzelne Verbindung jeweils einer Zeile der Tabelle C entsprechen.

### Tabelle 421: Verbindungen 421.1-421.102

Verbindungen der allgemeinen Formel Id, in der R¹ Methyl, R² Trifluormethyl, R¹¹ Methyl, R¹² Ethylcarbonyl bedeutet und die Substituenten R³, R⁸ und R⁹ für jede einzelne Verbindung jeweils einer Zeile der Tabelle C entsprechen.

### Tabelle 422: Verbindungen 422.1-422.102

Verbindungen der allgemeinen Formel Id, in der R¹ Methyl, R² Trifluormethyl, R¹¹ Ethyl, R¹² Ethylcarbonyl bedeutet und die Substituenten R³, R⁸ und R⁹ für jede einzelne Verbindung jeweils einer Zeile der Tabelle C entsprechen.

### Tabelle 423: Verbindungen 423.1-423.102

Verbindungen der allgemeinen Formel Id, in der R¹ Methyl, R² Trifluormethyl, R¹¹ n-Propyl, R¹² Ethylcarbonyl bedeutet und die Substituenten R³, R⁸ und R⁹ für jede einzelne Verbindung jeweils einer Zeile der Tabelle C entsprechen.

### Tabelle 424: Verbindungen 424.1-424.102

Verbindungen der allgemeinen Formel Id, in der R¹ Methyl, R² Trifluormethyl, R¹¹ Methyl, R¹² Methylsulfonyl bedeutet und die Substituenten R³, R⁸ und R⁹ für jede einzelne Verbindung jeweils einer Zeile der Tabelle C entsprechen.

### Tabelle 425: Verbindungen 425.1-425.102

Verbindungen der allgemeinen Formel Id, in der R¹ Methyl, R² Trifluormethyl, R¹¹ Ethyl, R¹² Methylsulfonyl bedeutet und die Substituenten R³, R⁸ und R⁹ für jede einzelne Verbindung jeweils einer Zeile der Tabelle C entsprechen.

### Tabelle 426: Verbindungen 426.1-426.102

Verbindungen der allgemeinen Formel Id, in der R¹ Methyl, R² Trifluormethyl, R¹¹ n-Propyl, R¹² Methylsulfonyl bedeutet und die Substituenten R³, R⁸ und R⁹ für jede einzelne Verbindung jeweils einer Zeile der Tabelle C entsprechen.

### Tabelle 427: Verbindungen 427.1-427.102

Verbindungen der allgemeinen Formel Id, in der R¹ Methyl, R² Trifluormethyl, R¹¹ Methyl, R¹² Ethylsulfonyl bedeutet und die Substituenten R³, R⁸ und R⁹ für jede einzelne Verbindung jeweils einer Zeile der Tabelle C entsprechen.

### Tabelle 428: Verbindungen 428.1-428.102

Verbindungen der allgemeinen Formel Id, in der R¹ Methyl, R² Trifluormethyl, R¹¹ Ethyl, R¹² Ethylsulfonyl bedeutet und die Substituenten R³, R⁸ und R⁹ für jede einzelne Verbindung jeweils einer Zeile der Tabelle C entsprechen.

### Tabelle 429: Verbindungen 429.1-429.102

Verbindungen der allgemeinen Formel Id, in der R¹ Methyl, R² Trifluormethyl, R¹¹ n-Propyl, R¹² Ethylsulfonyl bedeutet und die Substituenten R³, R⁸ und R⁹ für jede einzelne Verbindung jeweils einer Zeile der Tabelle C entsprechen.

### Tabelle 430: Verbindungen 430.1-430.102

Verbindungen der allgemeinen Formel Id, in der R¹ Methyl, R² Trifluormethyl, R¹¹ Methyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 431: Verbindungen 431.1-431.102

Verbindungen der allgemeinen Formel Id, in der R¹ Methyl, R² Trifluormethyl, R¹¹ Ethyl, R¹² 4-Methylphenylsulfonyl bedeutet und die Substituenten R³, R⁸ und R⁹ für jede einzelne Verbindung jeweils einer Zeile der Tabelle C entsprechen.

### Tabelle 432: Verbindungen 432.1-432.102

Verbindungen der allgemeinen Formel Id, in der R¹ Methyl, R² Trifluormethyl, R¹¹ n-Propyl, R¹² 4-Methylphenylsulfonyl bedeutet und die Substituenten R³, R⁸ und R⁹ für jede einzelne Verbindung jeweils einer Zeile der Tabelle C entsprechen.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus orficinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser. Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die 4-Benzoyl-pyrazole als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und DIdutylnaphthalinsulfonsäure, sowie von Fettsäuren, Alkyl- und Alkylarylsulfonaten, Alkyl-, Laurylether-und Fettalkoholsulfaten, sowie Salze sulfatierter Hexa-, Hepta-und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, TrIdutylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Sektrum) eingesetzt.

Die erfindungsgemäßen Verbindungen I können beispielsweise wie folgt formuliert werden:
- I: 20 Gewichtsteile der Verbindung Nr. 434.01 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.
- II: 20 Gewichtsteile der Verbindung Nr. 434.01 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
- III: 20 Gewichtsteile des Wirkstoffs Nr. 434.01 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
- IV: 20 Gewichtsteile des Wirkstoffs Nr. 434.01 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
- V: 3 Gewichtsteile des Wirkstoffs Nr. 434.01 werden mit 97 Gewichtsteilen feinteiligem Kaolin vermischt. Man erhält auf diese Weise ein Stäubemittel, das 3 Gew.-% des Wirkstoffs enthält.

- VI: 20 Gewichtsteile des Wirkstoffs Nr. 434.01 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
- VII: 1 Gewichtsteil der Verbindung 434.01 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
- VIII1: Gewichtsteil der Verbindung 434.01 wird in einer Mischung gelöst, die aus 80 Gewichtsteilen Cyclohexanon und 20 Gewichtsteilen Wettol ® EM 31 (nicht ionischer Emulgator auf der Basis von ethoxyliertem Ricinusöl). Man erhält ein stabiles Emulsionskonzentrat.

Zur Verbreiterung des Wirkungsspektrums und zur Erzielung synergistischer Effekte können die 4-Benzoyl-pyrazole I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF3-phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden. Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.).

Nachfolgend sind die Synthesen einiger Ausgangsstoffe aufgeführt:

### 2,4-Dichlor-3-(3'-hydroxy-3'-methyl-2'-oxetanyl)-benzoesäure (Verbindung 5.03)

### Stufe a: 2,4-Dichlor-3-(3'-trimethylsilyloxy-3'-methyl-2'-oxetanyl)-benzoesäuremethylester (Verbindung 5.01)

Eine Lösung aus 10 g (0.043 M) 2,4-Dichlor-3-formyl-benzoesäuremethylester und 8.4 g (0.065 M) 2-Trimethylsilyloxy-propen in 1.0 1 n-Hexan wurde 24 h bei Raumtemperatur mit einem UV-Strahler (Heraeus, TQ 150W) bestrahlt. Anschließend wurde das Lösungsmittel im Vakuum abdestilliert und der Rückstand an 100 g Kieselgel (0.04 - 0.06 mm) mit Gemischen aus Cyclohexan und Essigsäureethylester 100:1 bis 5:1 (v/v) gereinigt. Man erhält 6.8 g 2,4-Dichlor-3-(3'-trimethylsilyloxy-3'-methyl-2'-oxetanyl)-benzoesäuremethylester.
1H-NMR (CDCl₃) δ [ppm): 1.3 (t, 3 H), 3.9 (dd, 3 H), 4.6 (m, 2 H), 6.4 (d, 1 H), 7.0 (s, 1 H), 7.3 (m, 2 H)

### Stufe b: 2,4-Dichlor-3-(3'-hydroxy-3'-methyl-2'-oxetanyl)-benzoesäuremethylester (Verbindung 5.02)

Eine Lösung aus 14 g (0.039 M) 2,4-Dichlor-3-(3'-trimethylsilyloxy-3'-methyl-2'-oxetanyl)-benzoesäuremethylester und 14 g Ionenaustauscher (Dowex 50 WX2, Serva) wurde in 100 ml Methanol 12 h bei Raumtemperatur gerührt. Anschließend wurde das Lösungsmittel im Vakuum abdestilliert und der Rückstand an 100 g Kieselgel (0.04 - 0.06 mm) mit Gemischen aus Cyclohexan und Essigsäureethylester 100:1 bis 2:1 (v/v) gereinigt. Man erhält 6.1 g 2,4-Dichlor-3- (3'-hydroxy-3'-methyl-2'-oxetanyl)-benzoesäuremethylester.
1H-NMR (CDCl₃) δ [ppm]: 1.7 (s, 3 H), 3.9 (s, 3 H), 4.6 (d, 1 H), 4.8 (d, 1 H), 6.3 (s, 1 H), 7.4 (d, 1 H), 7.6 (d, 1 H) alternativ:

### Stufe c: 2,4-Dichlor-3-(3'-hydroxy-3'-methyl-2'-oxetanyl)-benzoesäuremethylester (Verbindung 5.02)

Eine Lösung aus 1 g (0.003 M) 2,4-Dichlor-3-(3'-trimethylsilyloxy-3'-methyl-2'-oxetanyl)-benzoesäuremethylester und 5 ml 10 %iger methanolischer Lösung von Chlorwasserstoff wurde in 30 ml Methanol 12 h bei Raumtemperatur gerührt. Anschließend wurde das Lösungsmittel im Vakuum abdestilliert und der Rückstand in Diethylether aufgenommen. Diese etherische Lösung wurde mit Wasser neutral gewaschen, mit Natriumsulfat getrocknet, filtriert und das Lösungsmittel im Vakuum abdestilliert. Man erhält 0.7 g 2,4-Dichlor-3-(3'-hydroxy-3'-methyl-2'-oxetanyl)-benzoesäuremethylester.
1H-NMR (CDCl₃) δ [ppm]: 1.7 (s, 3 H), 3.9 (s, 3 H), 4.6 (d, 1 H), 4.8 (d, 1 H), 6.3 (s, 1 H), 7.4 (d, 1 H), 7.6 (d, 1 H)

### Stufe d: 2,4-Dichlor-3-(3'-hydroxy-3'-methyl-2'-oxetanyl)-benzoesäure (Verbindung 5.03)

Eine Lösung aus 4.9 g (0.013 M) 2,4-Dichlor-3-(3'-hydroxy-3'-methyl-2'-oxetanyl)-benzoesäuremethylester und 0.5 g (0.020 M) Lithiumhydroxid wurde in einem Gemisch aus 20 ml Tetrahydrofuran und 20 ml Wasser 12 h bei 0°C gerührt. Anschließend wurde die Lösung mit 10 %iger wäßriger Salzsäure auf pH 1-2 eingestellt und mit Diethylether extrahiert. Die vereinigten organischen Phasen wurden anschließend mit Natriumsulfat getrocknet, filtriert und das Lösungsmittel im Vakuum abdestilliert. Man erhält 3.5 g 2,4-Dichlor-3-(3'-hydroxy-3'-methyl-2'-oxetanyl)-benzoesäure.
1H-NMR (CDCl₃) δ [ppm]: 2.5 (s, 3 H), 4.7 (d, 1 H), 4.9 (d, 1 H), 6.4 (s, 1 H), 7.4 (d, 1 H), 7.7 (d, 1 H), 8.6 (breites s, 1 H)

In der nachfolgenden Tabelle 433 sind neben den voranstehenden Verbindungen weitere Benzoesäurederivate der Formel Vd aufgeführt, die in analoger Weise hergestellt wurden oder herstellbar sind.

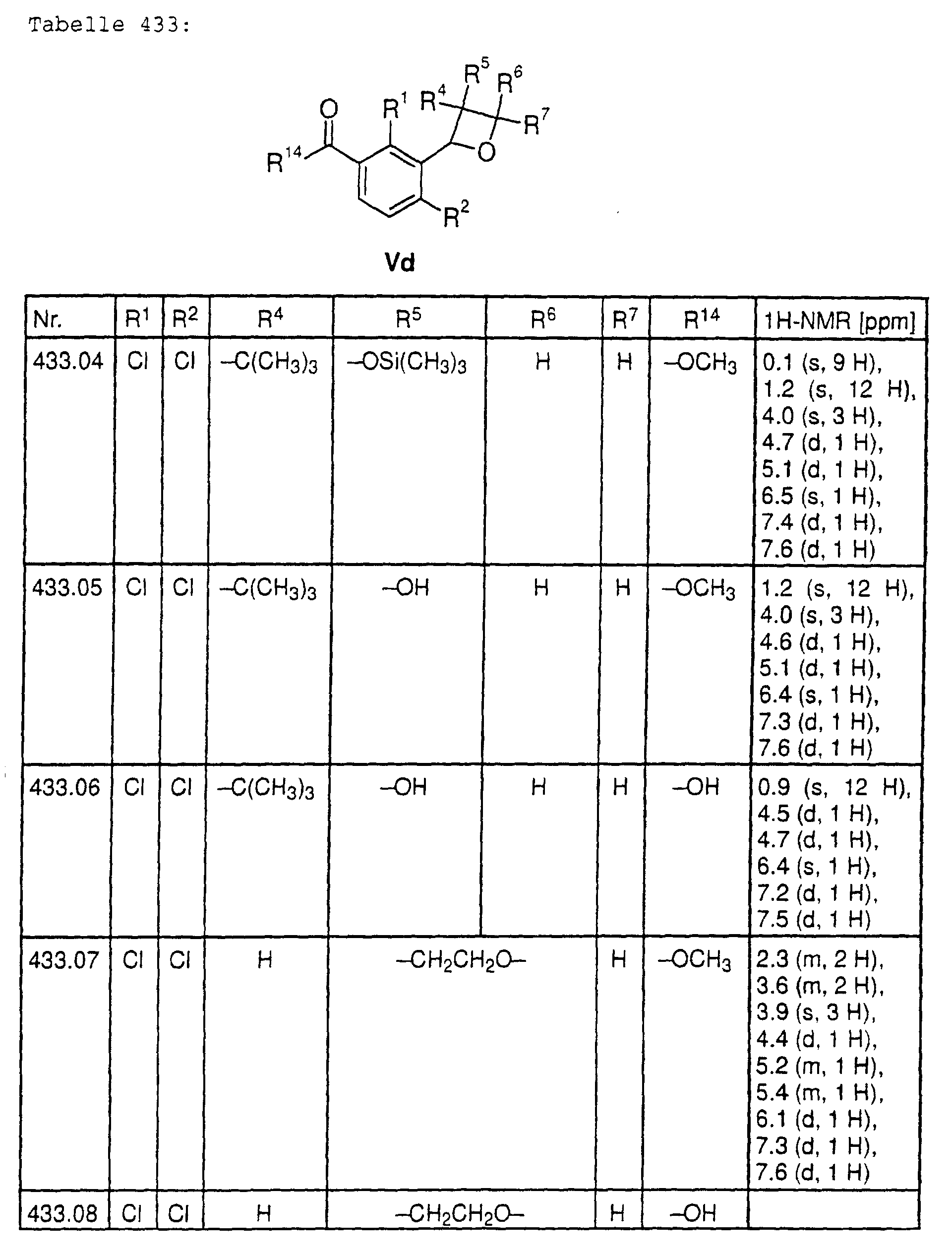

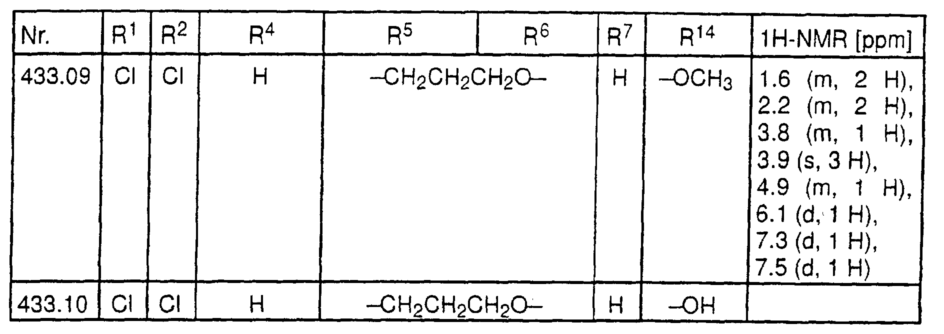

### Herstellung der Endprodukte

### 4-(2',4'-Dichlor-3'-(3''-hydroxy-3''-methyl-2''-oxetanyl-benzoyl)-2-ethyl-3-hydroxypyrazol (Verbindung 434.01)

Eine Lösung aus 1.40 g (0.005 M) 2,4-Dichlor-3-(3'-hydroxy-3'-methyl-2'-oxetanyl)-benzoesäure, 0.57 g (0.005 M) 2-Ethyl-3-hydroxypyrazol und 1.08 g (0.005 M) Dicyclohexylcarbodiimid in 50 ml trockenem Tetrahydrofuran wurde 12 h bei Raumtemperatur gerührt. Anschließend wurde der Niederschlag abgesaugt und das Filtrat in Wasser aufgenommen. Diese wäßrige Lösung wurde mit Essigsäureethylester extrahiert. Die vereinigten organischen Phasen wurden mit Natriumsulfat getrocknet, filtriert und das Lösungsmittel im Vakuum abdestilliert. Der ölige Rückstand wurde an 50 g Kieselgel (0.04 - 0.06 mm) mit einem Gemisch aus Cyclohexan und Essigsäureethylester 10 : 1 (v/v) gereinigt.

Das so erhaltene farblose Öl wurde in 10 ml Acetonitril aufgenommen, mit 0.3 g (0.003 M) Triethylamin und 0.1 g (0.001 M) Trimethylsilylcyanid versetzt und 4 h unter Rückfluß erhitzt. Nach Abkühlen wurde das Lösungsmittel im Vakuum entfernt und der Rückstand in Wasser aufgenommen. Die wäßrige Phase wurde mit 10 %iger wäßriger Salzsäurelösung auf pH 1-2 eingestellt und mit Essigsäureethylester extrahiert. Die vereinigten organischen Phasen wurden anschließend mit Wasser neutral gewaschen, mit Natriumsulfat getrocknet und das Lösungsmittel im Vakuum abdestilliert. Man erhält 0.3 g 4-(2',4'-Dichlor-3'-(3''-hydroxy-3''-methyl-2''-oxetanyl-benzoyl)-2-ethyl-3-hydroxypyrazol, das zur Reinigung aus Methyl-*tert*.butylether mit Petrolether ausgefällt wurde.

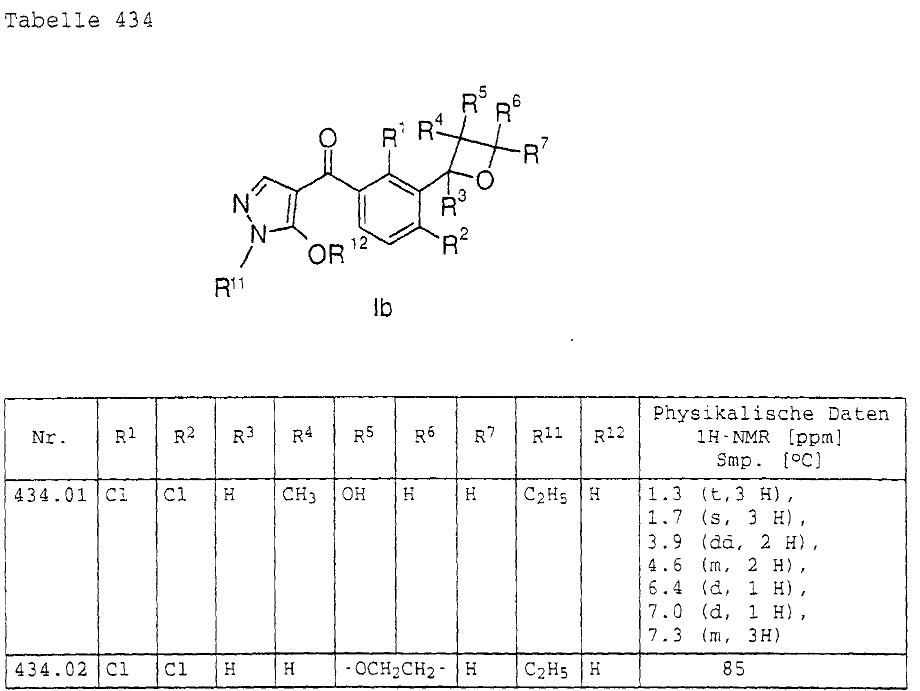

### Anwendungsbeispiele

Die herbizide Wirkung der 4-Benzoyl-pyrazole der Formel I ließ sich durch Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkte ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0.5 bzw. 0.25 kg/ha a. S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name | Abkürzungen |
|---|---|---|---|
| Echinochloa crus-galli | Hühnerhirse | barnyardgrass | ECHCG |
| Setaria faberii | Borstenhirse | giant foxtail | SETFA |
| Setaria viridis | Grüne Borstenhirse | green foxtail | SETVI |
| Chenopodium album | Weißer Gänsefuß | lambsquarters (goosefoot) | CHEAL |
| Polygonum persicaria | Flohknöterich | ladythumb | POLPE |
| Solanum nigrum | Schwarzer Nachtschatten | black nightshade | SOLNI |

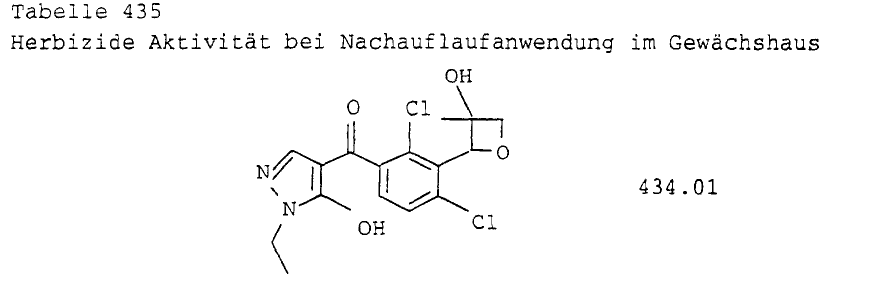

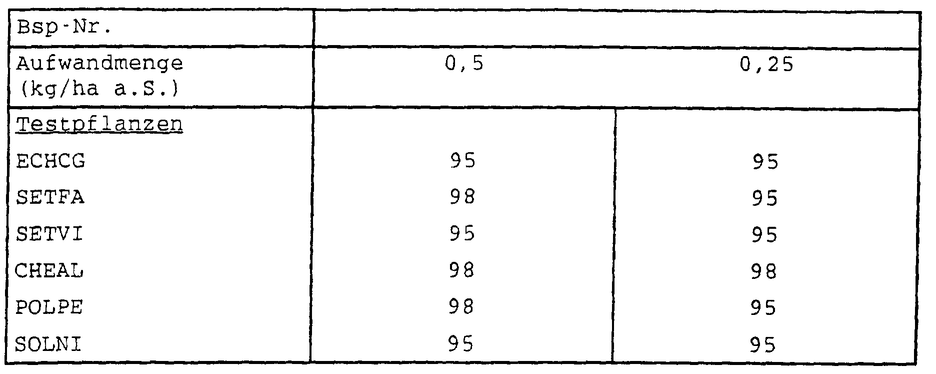

## Patentansprüche

1. 4-Benzoyl-pyrazole der Formel I 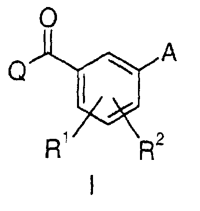 in der die Substituenten folgende Bedeutung haben:
R¹, R² Wasserstoff, Mercapto, Nitro, Halogen, Cyano, Rhodano, C₁-C₆-Alkyl, C₁-C₆-Halogenalkyl, C₁-C₆-Alkoxy, C₂-C₆-Alkenyl, C₂-C₆-Alkinyl, -OR¹⁰, -OCOR¹⁰, -OSO₂R¹⁰, -S(O)ₙR¹⁰, -SO₂OR¹⁰, -SO₂NR³R¹⁰, -NR¹⁰SO₂R¹⁰ oder -NR¹⁰COR;
Q ein in 4-Stellung verknüpftes Pyrazol der Formel II, 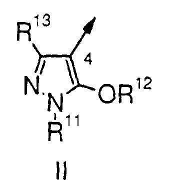
wobei
R¹¹ für C₁-C₆-Alkyl, C₁-C₆-Halogenalkyl, Phenyl oder Phenyl das partiell oder vollständig halogeniert ist und/oder einen bis drei der folgenden Reste trägt:
Nitro, Cyano, C₁-C₄-Alkyl, C₁-C₄-Halogen-alkyl, C₁-C₄-Alkoxy, C₁-C₄-Halogenalkoxy;
R¹² für Wasserstoff, C₁-C₆-Alkyl, C₁-C₆-Halogen-alkyl, C₁-C₆-Alkylcarbonyl, C₁-C₆-Halogen-alkylcarbonyl, C₁-C₆-Alkoxycarbonyl, C₁-C₆-Alkylsulfonyl, C₁-C₆-Halogenalkyl-sulfonyl, Phenylcarbonyl, Phenylcarbonylme-thyl, Phenoxycarbonyl oder Phenylsulfonyl, wobei die vier letztgenannten Substituenten unsubstituiert sind oder der Phenylring jeweils partiell oder vollständig halogeniert ist und/oder einen bis drei der folgenden Reste trägt:
Nitro, Cyano, C₁-C₄-Alkyl, C₁-C₄-Halogen-alkyl, C₁-C₄-Alkoxy, C₁-C₄-Halogenalkoxy;
R¹³ für Wasserstoff, C₁-C₆-Alkyl oder C₁-C₆-Halogenalkyl;
stehen;
A eine Gruppe der Formel IIIa, IIIb oder IV 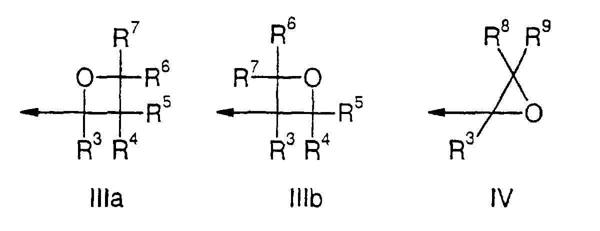 in der die Substituenten folgende Bedeutung haben:
R³ Wasserstoff, C₁-C₆-Alkyl oder Phenyl,
wobei der genannte Alkyl und Phenylrest partiell oder vollständig halogeniert sein kann und/oder eine bis drei der folgenden Gruppen tragen kann: Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxy-amino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
R⁴ - R⁷ können gleich oder verschieden sein und stehen unabhängig voneinander für:
Wasserstoff, Hydroxy, Mercapto, Amino, Halogen, C₁-C₆-Alkyl, C₃-C₆-Cycloalkyl, C₄-C₆-Cycloalkenyl, Phenyl, -OR¹⁰, -S(O)ₙR¹⁰, -OS(O)ₙR¹⁰, -PO(OR¹⁰)₂, -NR³R¹⁰, -Si(R¹⁰)₃ oder -OCOR¹⁰, wobei die genannten Alkyl und Cycloalkylreste sowie R³ und R¹⁰ der Reste -OR¹⁰, -S(O)ₙR¹⁰, -OS(O)ₙR¹⁰, -PO(OR¹⁰)₂, -NR³R¹⁰, -Si(R¹⁰)₃, -OCOR¹⁰ partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxy-amino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
R⁴, R⁵ können zusammen eine gegebenenfalls durch ein Stick-stoff- oder ein Sauerstoffatom ein- oder zweifach unterbrochene C₂-C₅-Alkylen- oder C₂-C₅-Alkenylenkette oder eine Gruppe =X bilden, wobei X für ein Sauer-stoffatom oder für eine Gruppe CR³R¹⁰, NR¹⁰, NNR³R¹⁰, oder NOR¹⁰ stehen kann;
R⁶, R⁷ können zusammen eine gegebenenfalls durch ein Stick-stoff- oder ein Sauerstoffatom einfach oder zweifach unterbrochene C₂-C₅-Alkylen- oder C₂-C₅-Alkenylenkette oder eine Gruppe =X bilden, wobei X für ein Sauerstoffatom oder für eine Gruppe CR³R¹⁰, NR¹⁰, NNR³R¹⁰ oder NOR¹⁰ stehen kann;
n null, eins, zwei;
R⁵, R⁶ können darüber hinaus, wenn sie an benachbarte Kohlenstoffatome gebunden sind, zusammen eine gegebenenfalls durch ein Stickstoff- oder ein Sauerstoffatom unterbrochene C₃-C₄-Alkylen- oder C₃-C₄-Alkeny-lenkette bilden, wenn R⁴ und R⁷ für Wasserstoff stehen;
R⁸, R⁹ können gleich oder verschieden sein und stehen unabhängig voneinander für:
Wasserstoff, Nitro, Halogen, Cyano, C₁-C₆-Alkyl, C₃-C₆-Cycloalkyl, C₄-C₆-Cycloalkenyl, C₅-C₆-Hetero-cyclyl, -OR¹⁰, -SR¹⁰, -COR¹⁰, -COOR¹⁰, -CONR³R¹⁰, Phenyl, Phenyl-C₁-C₆-alkyl und fünf- oder sechsgliedriges Hetaryl,
wobei die genannten Alkyl und Cycloalkylreste sowie R³ und R¹⁰ der Reste -OR¹⁰, -SR¹⁰, -COR¹⁰, -COOR¹⁰, -CONR³R¹⁰ partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxy-amino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
R⁸, R⁹ können darüber hinaus zusammen eine gegebenenfalls durch ein Stickstoff- oder ein Sauerstoffatom ein-oder zweifach unterbrochene C₂-C₅-Alkylen- oder C₂-C₅-Alkenylenkette bilden;
R¹⁰ Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl; wobei die genannten Alkylreste partiell oder vollständig halogeniert sein können und/-oder eine bis drei der folgenden Gruppen tragen können:
Hydroxy, Mercapto, Amino, Cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-Alkyliminooxy, C₁-C₄-Alkoxy-amino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
sowie deren landwirtschaftlich brauchbaren Salze.

2. 4-Benzoyl-pyrazole der Formel I nach Anspruch 1, in der
R¹ Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵ oder -S(O)ₙR⁷ bedeutet;
R² für Wasserstoff oder einen wie voranstehend unter R¹ genannten Rest steht.

3. 4-Benzoyl-pyrazole der Formel Ia nach Anspruch 1 oder 2, 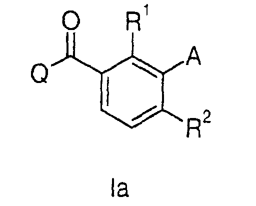 in der die Substituenten R¹, R², Q und A die unter Anspruch 1 genannte Bedeutung haben.

4. 4-Benzoyl-pyrazole der Formel Ia nach Anspruch 3, in der A für eine Gruppe der Formel IIIa oder IIIb steht.

5. 4-Benzoyl-pyrazole der Formel Ia nach Anspruch 3, in der A für eine Gruppe der Formel IV steht.

6. Verfahren zur Herstellung von 4-Benzoyl-pyrazolen der Formel I gemäß den Ansprüchen 1 bis 5, **dadurch gekennzeichnet, daß** man ein Pyrazol der Formel IIa, in der die Substituenten R¹¹ und R¹³ die unter Anspruch 1 genannte Bedeutung haben, 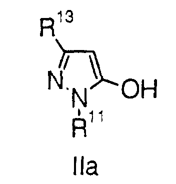 mit einer aktivierten Carbonsäure Va oder mit einer Carbonsäure Vb, 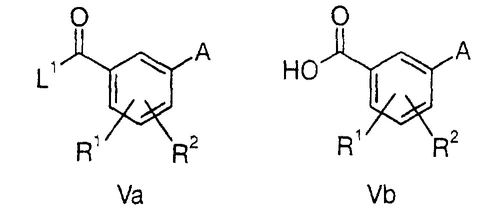 wobei die Substituenten R¹, R² und A die in Anspruch 1 genannte Bedeutung haben und L¹ für eine nucleophil austauschbare Abgangsgruppe steht, acyliert und das Acylierungsprodukt gegebenenfalls in Gegenwart eines Katalysators zu den Verbindungen I umlagert und falls gewünscht zur Herstellung von 4-Benzoyl-pyrazolen der allgemeinen Formel I mit R¹² ≠ H mit einer Verbindung der Formel VI, 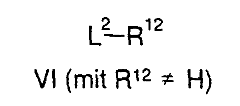 in der R¹² die unter Anspruch 1 genannte Bedeutung hat mit Ausnahme von Wasserstoff und L² für eine nucleophil austauschbare Abgangsgruppe steht, umsetzt.

7. Aktivierte Carbonsäuren der Formel Va und Carbonsäuren der Formel Vb gemäß Anspruch 6, wobei die Substituenten R¹, R² und A die in Anspruch 1 genannte Bedeutung haben und L¹ für eine nucleophil austauschbare Abgangsgruppe, ausgewählt aus der Gruppe Halogen, Hetaryl und Carboxylat, steht.

8. Verfahren zur Herstellung der Benzoesäureester Vc, 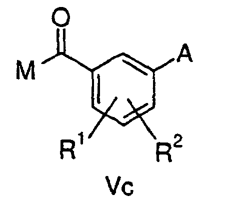 wobei die Substituenten R¹, R² und A die in Anspruch 1 genannte Bedeutung haben und M C₁-C₆-Alkoxy bedeutet, **dadurch gekennzeichnet, daß** man einen Aldehyd der Formel IX oder Ketone der Formel X 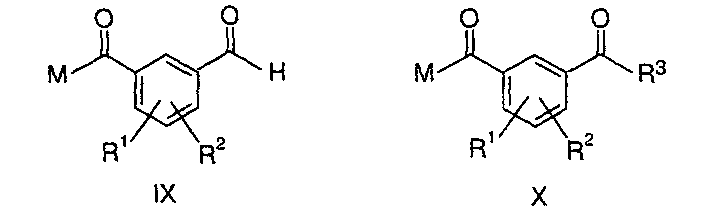 in Gegenwart von Olefinen der Formel XI 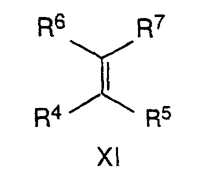 im UV-Bereich belichtet.

9. Mittel, enthaltend eine herbizid wirksame Menge mindestens eines 4-Benzoyl-pyrazols der Formel I oder eines landwirtschaftlich brauchbaren Salzes von I gemäß den Ansprüchen 1 bis 5 und für die Formulierung von Pflanzenschutzmitteln übliche Hilfsmittel.

10. Verfahren zur Herstellung von herbizid wirksamen Mitteln gemäß Anspruch 8, **dadurch gekennzeichnet, daß** man eine herbizid wirksame Menge mindestens eines 4-Benzoyl-pyrazols der Formel I oder eines landwirtschaftlich brauchbaren Salzes von I gemäß den Ansprüchen 1 bis 5 und für die Formulierung von Pflanzenschutzmitteln übliche Hilfsmittel mischt.

11. Verfahren zur Bekämpfung von unerwünschtem Pflanzenwuchs, **dadurch gekennzeichnet, daß** man eine herbizid wirksame Menge mindestens eines 4-Benzoyl-pyrazols der Formel I oder eines landwirtschaftlich brauchbaren Salzes von I gemäß den Ansprüchen 1 bis 5 auf Pflanzen, deren Lebensraum und/-oder auf Samen einwirken läßt.

12. Verwendung der 4-Benzoyl-pyrazole der Formel I und deren landwirtschaftlich brauchbaren Salze gemäß den Ansprüchen 1 bis 5 als Herbizide.

## Claims

1. 4-Benzoylpyrazoles of the formula I 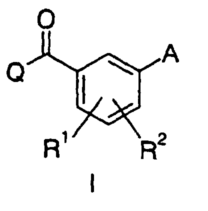 where:
R¹ and R² are each hydrogen, mercapto, nitro, halogen, cyano, thiocyanato, C₁-C₆-alkyl, C₁-C₆-haloalkyl, C₁-C₆-alkoxy, C₂-C₆-alkenyl, C₂-C₆-alkynyl, -OR¹⁰, -OCOR¹⁰, -OSO₂R¹⁰, -S(O)ₙR¹⁰, -SO₂OR¹⁰, -SO₂NR³R¹⁰, -NR¹⁰SO₂R¹⁰ or -NR¹⁰COR;
Q is a pyrazole of the formula II 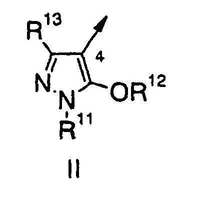
which is attached in position 4 and where
R¹¹ is C₁-C₆-alkyl, C₁-C₆-haloalkyl, phenyl or phenyl which is partially or fully halogenated and/or carries one to three of the following radicals: nitro, cyano, C₁-C₄-alkyl, C₁-C₄-haloalkyl, C₁-C₄-alkoxy, C₁-C₄-haloalkoxy;
R¹² is hydrogen, C₁-C₆-alkyl, C₁-C₆-haloalkyl, C₁-C₆-alkylcarbonyl, C₁-C₆-haloalkylcarbonyl, C₁-C₆-alkoxycarbonyl, C₁-C₆-alkylsulfonyl, C₁-C₆-haloalkylsulfonyl, phenylcarbonyl, phenylcarbonylmethyl, phenoxycarbonyl or phenylsulfonyl,
where the last four substituents are unsubstituted or the phenyl ring in question is partially or fully halogenated and/or carries one to three of the following radicals: nitro, cyano, C₁-C₄-alkyl, C₁-C₄-haloalkyl, C₁-C₄-alkoxy, C₁-C₄-haloalkoxy;
R¹³ is hydrogen, C₁-C₆-alkyl or C₁-C₆-haloalkyl;
A is a group of the formula IIIa, IIIb or IV 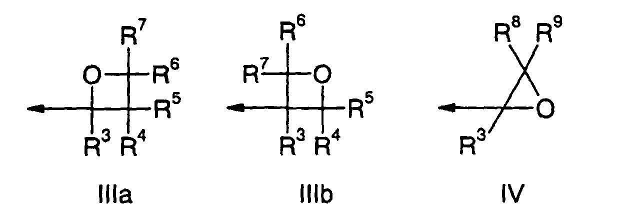 where:
R³ is hydrogen, C₁-C₆-alkyl or phenyl,
where the alkyl and phenyl radicals mentioned may be partially or fully halogenated and/or may carry one to three of the following groups:
hydroxyl, mercapto, amino, cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁₋C₄-alkyliminooxy, C₁-C₄-alkoxyamino, C₁-C₄-alkylcarbonyl, C₁-C₄-alkoxy-C₂-C₆-alkoxycarbonyl, C₁-C₄-alkylsulfonyl, heterocyclyl, heterocyclyloxy, phenyl, benzyl, hetaryl, phenoxy, benzyloxy and hetaryloxy, where the last eight radicals may in turn be substituted;
R⁴ - R⁷ may be identical or different and, independently of the others, each is:
hydrogen, hydroxyl, mercapto, amino, halogen, C₁-C₆-alkyl, C₃-C₆-cycloalkyl, C₄-C₆-cycloalkenyl, phenyl, -OR¹⁰, -S(O)ₙR¹⁰, -OS(O)ₙR¹⁰, -PO(OR¹⁰)₂, -NR³R¹⁰, -Si(R¹⁰)₃ or -OCOR¹⁰,
where the alkyl and cycloalkyl radicals mentioned and R³ and R¹⁰ of the radicals -OR¹⁰, -S(O)ₙR¹⁰, -OS(O)ₙR¹⁰, -PO(OR¹⁰)₂, -NR³R¹⁰, -Si(R¹⁰)₃, -OCOR¹⁰ may be partially or fully halogenated and/or may carry one to three of the following groups:
hydroxyl, mercapto, amino, cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-alkyliminooxy, C₁-C₄-alkoxyamino, C₁-C₄-alkylcarbonyl, C₁-C₄-alkoxy-C₂-C₆-alkoxycarbonyl, C₁-C₄-alkylsulfonyl, heterocyclyl, heterocyclyloxy, phenyl, benzyl, hetaryl, phenoxy, benzyloxy and hetaryloxy, where the last eight radicals may in turn be substituted;
R⁴ and R⁵ together may form a C₂-C₅-alkylene or C₂₋C₅-alkenylene chain which may be interrupted once or twice by a nitrogen or an oxygen atom, or may form a group =X, where X is an oxygen atom or a group CR³R¹⁰, NR¹⁰, NNR³R¹⁰, or NOR¹⁰;
R⁶ and R⁷ together may form a C₂-C₅-alkylene or C₂-C₅-alkenylene chain which may be interrupted once or twice by a nitrogen or an oxygen atom, or may form a group =X, where X is an oxygen atom or a group CR³R¹⁰, NR¹⁰, NNR³R¹⁰, or NOR¹⁰;
n is zero, one, two;
R⁵ and R⁶ together may furthermore, if they are attached to adjacent carbon atoms and if R⁴ and R⁷ are each hydrogen, form a C₃-C₄-alkylene or C₃-C₄-alkenylene chain which may be interrupted by a nitrogen or an oxygen atom;
R⁸ and R⁹ may be identical or different and, independently of the other, each is:
hydrogen, nitro, halogen, cyano, C₁-C₆-alkyl, C₃-C₆-cycloalkyl, C₄-C₆-cycloalkenyl, C₅-C₆-heterocyclyl, -OR¹⁰, -SR¹⁰, -COR¹⁰, -COOR¹⁰, -CONR³R¹⁰, phenyl, phenyl-C₁-C₆-alkyl and five-or six-membered hetaryl,
where the alkyl and cycloalkyl radicals mentioned and R³ and R¹⁰ of the radicals -OR¹⁰, -SR¹⁰, -COR¹⁰, -COOR¹⁰, -CONR³R¹⁰ may be partially or fully halogenated and/or may carry one to three of the following groups: hydroxyl, mercapto, amino, cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-alkyliminooxy, C₁-C₄-alkoxyamino, C₁-C₄-alkylcarbonyl, C₁-C₄-alkoxy-C₂-C₆-alkoxycarbonyl, C₁-C₄-alkylsulfonyl, heterocyclyl, heterocyclyloxy, phenyl, benzyl, hetaryl, phenoxy, benzyloxy and hetaryloxy, where the last eight radicals may in turn be substituted;
R⁸ and R⁹ together may furthermore form a C₂-C₅-alkylene or C₂-C₅-alkenylene chain which may interrupted once or twice by a nitrogen or an oxygen atom;
R¹⁰ is hydrogen, C₁-C₆-alkyl, C₁-C₆-haloalkyl, C₂-C₆-alkenyl, C₂-C₆-alkynyl, phenyl or phenyl-C₁-C₆-alkyl, where the alkyl radicals mentioned may be partially or fully halogenated and/or may carry one to three of the following groups:
hydroxyl, mercapto, amino, cyano, R¹⁰, -OR¹⁰, -SR¹⁰, -NR³R¹⁰, =NOR¹⁰, -OCOR¹⁰, -SCOR¹⁰, -NR³COR¹⁰, -CO₂R¹⁰, -COSR¹⁰, -CONR³R¹⁰, C₁-C₄-alkyliminooxy, C₁-C₄-alkoxyamino, C₁-C₄-alkylcarbonyl, C₁-C₄-alkoxy-C₂-C₆-alkoxycarbonyl, C₁-C₄-alkylsulfonyl, heterocyclyl, heterocyclyloxy, phenyl, benzyl, hetaryl, phenoxy, benzyloxy and hetaryloxy, where the last eight radicals may in turn be substituted;
and agriculturally useful salts thereof.

2. 4-Benzoylpyrazoles of the formula I as claimed in claim 1 in which
R¹ is nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR⁵ or -S(O)ₙR⁷;
R² is hydrogen or one of the radicals mentioned above under R¹.

3. 4-Benzoylpyrazoles of the formula Ia as claimed in claim 1 or 2. 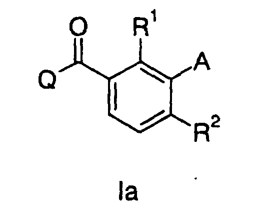 where the substituents R¹, R², Q and A are each as defined in claim 1.

4. 4-Benzoylpyrazoles of the formula Ia as claimed in claim 3 in which A is a group of the formula IIIa or IIIb.

5. 4-Benzoylpyrazoles of the formula Ia as claimed in claim 3 in which A is a group of the formula IV.

6. A process for preparing 4-benzoylpyrazoles of the formula I as claimed in any of claims 1 to 5, which comprises acylating a pyrazole of the formula IIa, where the substituents R¹¹ and R¹³ are each as defined in claim 1, 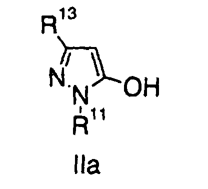 with an activated carboxylic acid Va or a carboxylic acid Vb, 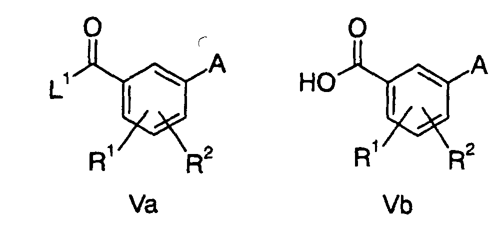 where the substituents R¹, R² and A are each as defined in claim 1 and L¹ is a nucleophilically replaceable leaving group, and rearranging the acylation product, if appropriate in the presence of a catalyst, to the compounds I and, if desired, reacting them with a compound of the formula VI 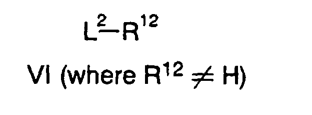 in which R¹² is as defined in claim 1 except for hydrogen and L² is a nucleophilically replaceable leaving group, for preparing 4-benzoylpyrazoles of the general formula I where R¹² ≠ H.

7. Activated carboxylic acids of the formula Va and carboxylic acids of the formula Vb as claimed in claim 6 where the substituents R¹, R² and A are each as defined in claim 1 and L¹ is a nucleophilically replaceable leaving group selected from the group consisting of halogen, hetaryl and carboxylate.

8. A process for preparing the benzoic esters Vc, 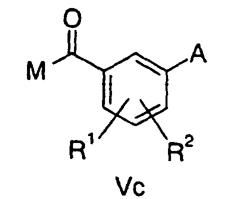 where the substituents R¹, R² and A are each as defined in claim 1 and M is C₁-C₆-alkoxy, which comprises irradiating an aldehyde of the formula IX or ketones of the formula X 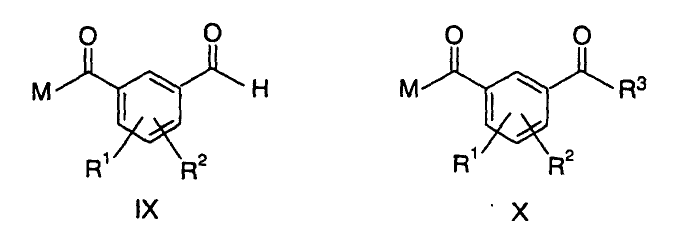 in the presence of olefins of the formula XI 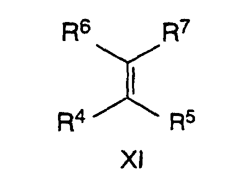 in the UV range.

9. A composition, which comprises a herbicidally active amount of at least one 4-benzoylpyrazole of the formula I or an agriculturally useful salt of I as claimed in any of claims 1 to 5 and auxiliaries which are customarily used for formulating crop protection agents.

10. A process for preparing herbicides as claimed in claim 8, which comprises mixing a herbicidally effective amount of at least one 4-benzoylpyrazole of the formula I or an agriculturally useful salt of I as claimed in any of claims 1 to 5 and auxiliaries which are customarily used for formulating crop protection agents.

11. A method for controlling undesirable vegetation, which comprises allowing a herbicidally effective amount of at least one 4-benzoylpyrazole of the formula I or an agriculturally useful salt of I as claimed in any of claims 1 to 5 to act on plants, their habitat and/or on seeds.

12. The use of the 4-benzoylpyrazoles of the formula I and agriculturally useful salts thereof as claimed in any of claims 1 to 5 as herbicides.

## Revendications

1. 4-benzoyl-pyrazole répondant à la formule I 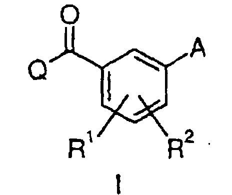 dans laquelle les substituants ont la signification suivante :
R¹, R² représentent un atome d'hydrogène, un groupe mercapto, nitro, halogéno, cyano, rhodano, alkyle en C₁-C₆, halogénoalkyle en C₁-C₆, alcoxy en C₁-C₆, alcényle en C₂-C₆, alcynyle en C₂-C₆, -OR¹⁰, -OCOR¹⁰, -OSO₂R¹⁰, - S(O)ₙR¹⁰, -SO₂OR¹⁰, -SO₂NR³R¹⁰, -NR¹⁰SO₂R¹⁰ ou -NR¹⁰COR ;
Q est un pyrazole lié en position 4 répondant à la formule II, 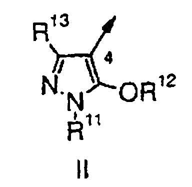
dans laquelle
R¹¹ représente un groupe alkyle en C₁-C₆, halogénoalkyle en C₁-C₆, phényle ou phényle partiellement ou totalement halogéné et/ou portant entre un et trois des radicaux suivants :
nitro, cyano, alkyle en C₁-C₄, halogénoalkyle en C₁-C₄, alcoxy en C₁-C₄, halogénoalcoxy en C₁-C₄ ;
R¹² représente un atome d'hydrogène, un groupe alkyle en C₁-C₆, halogénoalkyle en C₁-C₆, (alkyle en C₁-C₆)carbonyle, (halogénoalkyle en C₁-C₆)carbonyle, (alcoxy en C₁-C₆)carbonyle, alkylsulfonyle en C₁-C₆, halogénoalkylsulfonyle en C₁-C₆, phénylcarbonyle, phénylcarbonylméthyle, phénoxycarbonyle ou phénylsulfonyle, les quatre derniers substituants cités étant non substitués ou le cycle phénylique étant pour chacun partiellement ou totalement halogéné et/ou portant entre un et trois des radicaux suivants :
nitro, cyano, alkyle en C₁-C₄, halogénoalkyle en C₁-C₄, alcoxy en C₁-C₄, halogénoalcoxy en C₁-C₄ ;
R¹³ représente un atome d'hydrogène, un groupe alkyle en C₁-C₆ ou halogénoalkyle en C₁-C₆ ;
A représente un groupe répondant à la formule IIIa, IIIb ou IV 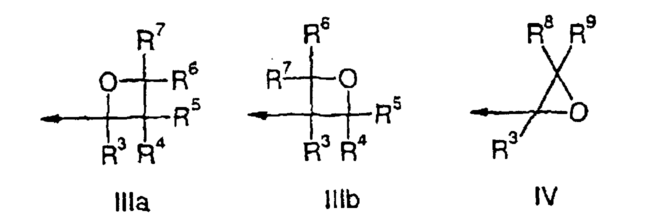 dans laquelle les substituants ont la signification suivante :
R³ représente un atome d'hydrogène, un groupe alkyle en C₁-C₆ ou phényle, l'alkyle cité et le radical phényle pouvant être partiellement ou totalement halogénés et/ou pouvant porter entre un et trois des groupes suivants :
hydroxy, mercapto, amino, cyano, R¹⁰, -OR¹⁰, -SR¹⁰, NR³R¹⁰, =NOR¹⁰, - OCOR¹⁰, -SCOR¹⁰, NR³COR¹⁰, -CO₂R¹⁰, -COSR¹⁰, -CONR³R¹⁰, alkyliminooxy en C₁-C₄, alcoxyamino en C₁-C₄, (alkyle en C₁-C₄)carbonyle, (alcoxy en C₁-C₄)-(alcoxy en C₂-C₆)carbonyle, alkylsulfonyle en C₁-C₄, hétérocyclyle, hétérocyclyloxy, phényle, benzyle, hétéroaryle, phénoxy, benzyloxy et hétéroaryloxy, les huit derniers radicaux cités pouvant eux-mêmes être substitués ;
R⁴ - R⁷ peuvent être identiques ou différents et représentent, indépendamment l'un de l'autre :
un atome d'hydrogène, un groupe hydroxy, mercapto, amino, halogéno, alkyle en C₁-C₆, cycloalkyle en C₃-C₆, cycloalcényle en C₄-C₆, phényle, -OR¹⁰, - S(O)ₙR¹⁰, -OS(O)ₙR¹⁰, -PO(OR¹⁰)₂, -NR³R¹⁰, -Si(R¹⁰)₃ ou -OCOR¹⁰,
les radicaux alkyle et cycloalkyle cités ainsi que R³ et R¹⁰ des radicaux - OR¹⁰, -S(O)ₙR¹⁰, -OS(O)ₙR¹⁰, -PO(OR¹⁰)₂, -NR³R¹⁰, -Si(R¹⁰)₃, -OCOR¹⁰ pouvant être partiellement ou totalement halogénés et/ou pouvant porter entre un et trois des groupes suivants :
hydroxy, mercapto, amino, cyano, R¹⁰, -OR¹⁰, -SR¹⁰, -NR³R¹⁰, =NOR¹⁰, - OCOR¹⁰, -SCOR¹⁰, -NR³COR¹⁰, -CO₂R¹⁰, -COSR¹⁰, -CONR³R¹⁰, alkyliminooxy en C₁-C₄, alcoxyamino en C₁-C₄, (alkyle en C₁-C₄)carbonyle, (alcoxy en C₁-C₄)-(alcoxy en C₂-C₆)carbonyle, alkylsulfonyle en C₁-C₄, hétérocyclyle, hétérocyclyloxy, phényle, benzyle, hétéroaryle, phénoxy, benzyloxy et hétéroaryloxy, les huit derniers radicaux cités pouvant eux-mêmes être substitués ;
R⁴, R⁵ peuvent former ensemble une chaîne alkylène en C₂-C₅ ou une chaîne alcénylène en C₂-C₅ pouvant éventuellement être interrompues une ou deux fois par un atome d'azote ou d'oxygène ou bien peuvent former un groupe =X, X pouvant représenter un atome d'oxygène ou un groupe CR³R¹⁰, NR¹⁰, NNR³R¹⁰, ou NOR¹⁰ ;
R⁶, R⁷ peuvent former ensemble une chaîne alkylène en C₂-C₅ ou une chaîne alcénylène en C₂-C₅ pouvant éventuellement être interrompues une ou deux fois par un atome d'azote ou d'oxygène ou bien peuvent former un groupe =X, dans lequel X peut représenter un atome d'oxygène ou un groupe CR³R¹⁰, NR¹⁰, NNR³R¹⁰, ou NOR¹⁰ ;
n vaut zéro, un, deux ;
R⁵ - R⁶ peuvent en outre, lorsqu'ils sont liés à un atome de carbone adjacent, former ensemble une chaîne alkylène en C₃-C₄ ou une chaîne alcénylène en C₃-C₄ qui peuvent éventuellement être interrompues par un atome d'azote ou d'oxygène, lorsque R⁴ et R⁷ représentent un atome d'hydrogène ;
R⁸ - R⁹ peuvent être identiques ou différents et représentent, indépendamment l'un de l'autre :
un atome d'hydrogène, un groupe nitro, halogéno, cyano, alkyle en C₁-C₆, cycloalkyle en C₃-C₆, cycloalcényle en C₄-C₆, hétérocyclyle en C₅-C₆, -OR¹⁰, -SR¹⁰, -COR¹⁰, -COOR¹⁰, -CONR³R¹⁰, phényle, phényl-(alkyle en C₁-C₆), et un groupe hétéroaryle ayant cinq ou six chaînons,
les radicaux alkyle et cycloalkyle cités ainsi que R³ et R¹⁰ des radicaux - OR¹⁰, -SR¹⁰, -COR¹⁰, -COOR¹⁰, -CONR³R¹⁰ pouvant être partiellement ou totalement halogénés et/ou pouvant porter entre un et trois des groupes suivants :
hydroxy, mercapto, amino, cyano, R¹⁰, -OR¹⁰, -SR¹⁰, -NR³R¹⁰, =NOR¹⁰, - OCOR¹⁰, -SCOR¹⁰, -NR³COR¹⁰, -CO₂R¹⁰, -COSR¹⁰, -CONR³R¹⁰, alkyliminooxy en C₁-C₄, alcoxyamino en C₁-C₄, (alkyle en C₁-C₄)carbonyle, (alcoxy en C₁-C₄)-(alcoxy en C₂-C₆)carbonyle, alkylsulfonyle en C₁-C₄, hétérocyclyle, hétérocyclyloxy, phényle, benzyle, hétéroaryle, phénoxy, benzyloxy et hétéroaryloxy, les huit derniers radicaux cités pouvant eux-mêmes être substitués ;
R⁸ - R⁹ peuvent en outre former ensemble une chaîne alkylène en C₂-C₅ ou une chaîne alcénylène en C₂-C₅ qui peuvent éventuellement être interrompues une ou deux fois par un atome d'azote ou d'oxygène ;
R¹⁰ représente un atome d'hydrogène, un groupe alkyle en C₁-C₆, halogénoalkyle en C₁-C₆, alcényle en C₂-C₆, alcynyle en C₂-C₆, phényle, ou phényl-(alkyle en C₁-C₆) ; les radicaux alkyle cités pouvant être partiellement ou totalement halogénés et/ou pouvant porter entre un et trois des groupes suivants :
hydroxy, mercapto, amino, cyano, R¹⁰, -OR¹⁰, -SR¹⁰, -NR³R¹⁰, =NOR¹⁰, - OCOR¹⁰, -SCOR¹⁰, -NR³COR¹⁰, -CO₂R¹⁰, -COSR¹⁰, -CONR³R¹⁰, alkyliminooxy en C₁-C₄, alcoxyamino en C₁-C₄, (alkyle en C₁-C₄)carbonyle, (alcoxy en C₁-C₄)-alcoxy en C₂-C₆)carbonyle, alkylsulfonyle en C₁-C₄, hétérocyclyle, hétérocyclyloxy, phényle, benzyle, hétéroaryle, phénoxy, benzyloxy et hétéroaryloxy, les huit derniers radicaux cités pouvant eux-mêmes être substitués ;
ainsi que les sels d'un tel composé destinés à un usage agricole.

2. 4-benzoyl-pyrazole répondant à la formule I selon la revendication 1, dans laquelle
R¹ représente un groupe nitro, halogéno, cyano, rhodano, alkyle en C₁-C₆, halogénoalkyle en C₁-C₆, alcoxy-(en C₁-C₆)-alkyle-(en C₁-C₆), alcényle en C₂-C₆, alcynyle en C₂-C₆, -OR⁵ ou -S(O)ₙR⁷;
R² représente un atome d'hydrogène ou un radical tel que cité précédemment pour R¹.

3. 4-benzoyl-pyrazole répondant à la formule la selon la revendication 1 ou 2, 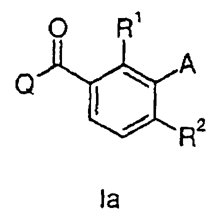 les substituants R¹, R², Q et A ayant la signification indiquée dans la revendication 1.

4. 4-benzoyl-pyrazole répondant à la formule la selon la revendication 3, A représentant un groupe répondant à la formule IIIa ou IIIb.

5. 4-benzoyl-pyrazole répondant à la formule la selon la revendication 3, A représentant un groupe répondant à la formule IV.

6. Procédé de fabrication de composés de 4-benzoyl-pyrazole répondant à la formule I selon les revendications 1 à 5, **caractérisé en ce qu'**on acyle un pyrazole répondant à la formule ils, dans laquelle les substituants R¹¹ et R¹³ ont la signification indiquée dans la revendication 1, 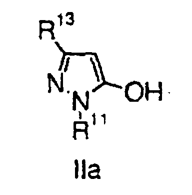 avec un acide carboxylique activé Va ou avec un acide carboxylique Vb, 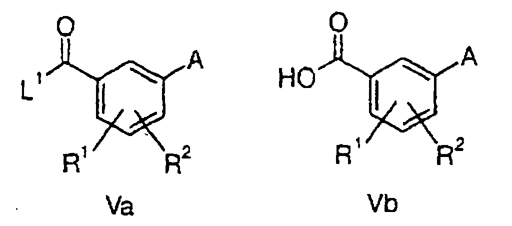 les substituants R¹, R² et A ayant la signification indiquée dans la revendication 1 et L¹ représentant un groupe sortant pouvant être substitué par réaction nucléophile et **en ce qu'**on transforme le produit d'acylation éventuellement en présence d'un catalyseur pour obtenir les composés I et, si cela est souhaité, dans le but de fabriquer des composés de 4-benzoyle-pyrazole répondant à la formule générale I avec R¹² ≠ H, qu'on fait réagir ce produit d'acylation avec un composé répondant à la formule VI, 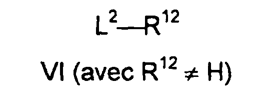 dans laquelle R¹² a la signification indiquée dans la revendication 1, à l'exception de l'atome d'hydrogène, et L² représente un groupe sortant pouvant être substitué par réaction nucléophile.

7. Acide carboxylique activé répondant à la formule Va et acide carboxylique répondant à la formule Vb selon la revendication 6, les substituants R¹, R² et A ayant la signification indiquée dans la revendication 1 et L¹ représentant un groupe sortant pouvant être substitué par réaction nucléophile choisi parmi le groupe halogéno, hétéroaryle et carboxylate.

8. Procédé de fabrication des esters d'acide benzoïque Vc, 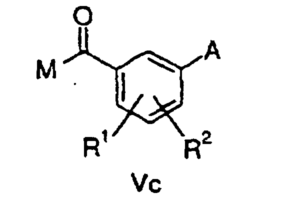 les substituants R¹, R² et A ayant la signification indiquée dans la revendication 1 et M désignant un groupe alcoxy en C₁-C₆, **caractérisé en ce qu'**on expose à une lumière UV un aldéhyde répondant à la formule IX ou une cétone répondant à la formule X 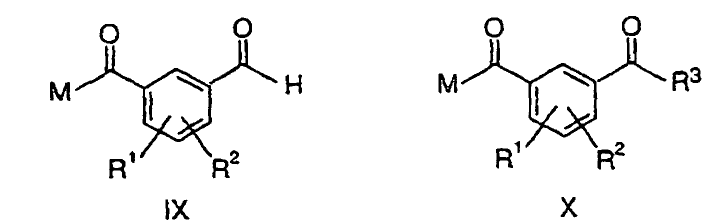 en présence d'oléfines répondant à la formula XI

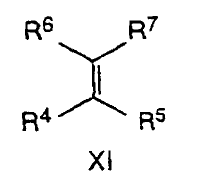

9. Produit contenant une quantité efficace à effet herbicide d'au moins un 4-benzoyl-pyrazole répondant à la formule I ou d'un sel de I approprié à un usage agricole selon les revendications 1 à 5 et des adjuvants usuels pour la formulation de produits phytosanitaires.

10. Procédé de fabrication de produits efficaces à effet herbicide selon la revendication 8, **caractérisé en ce qu'**on mélange une quantité efficace à effet herbicide d'au moins un 4-benzoyl-pyrazole répondant à la formule I ou d'un sel de I approprié à un usage agricole selon les revendications 1 à 5 et des adjuvants usuels pour la formulation de produits phytosanitaires.

11. Procédé de lutte contre la croissance de plantes non souhaitables, **caractérisé en ce qu'**on fait agir sur des plantes, sur leur milieu et/ou sur des semences une quantité efficace à effet herbicide d'au moins un 4-benzoyl-pyrazole répondant à la formule I ou d'un sel de I approprié à un usage agricole, selon les revendications 1 à 5.

12. Utilisation du 4-benzoyl-pyrazole répondant à la formule I et de leurs sels, destinés à un usage agricole comme herbicide, selon les revendications 1 à 5.
